# EUROPEAN PATENT APPLICATION

(11) **EP 3 811 978 A1**
(43) Date of publication of application: **28.04.2021**
(21) Application number: 19818561.3
(22) Date of filing: 14.06.2019
(51) Int. Cl.: A61K 47/64, A61K 39/395, A61P 35/00, C07K 2/00, C07K 4/00, C07K 14/00, C07K 16/00

(54) **SUBSTANCE HAVING AFFINITY FOR ANTIBODY, AND COMPOUND OR SALT THEREOF HAVING BIOORTHOGONAL FUNCTIONAL GROUP**

(30) Priority: 14.06.2018 JP 2018113962
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); HATADA, Noriko, Kawasaki-shi, Kanagawa 210-8681 (JP); YAMADA, Kei, Kawasaki-shi, Kanagawa 210-8681 (JP); SHIKIDA, Natsuki, Kawasaki-shi, Kanagawa 210-8681 (JP); SHIMBO, Kazutaka, Kawasaki-shi, Kanagawa 210-8681 (JP); FUJII, Tomohiro, Kawasaki-shi, Kanagawa 210-8681 (JP); HIRASAWA, Shigeo, Kawasaki-shi, Kanagawa 210-8681 (JP)
(74) Representative: Mewburn Ellis LLP
(86) International application number: PCT/JP2019/023779
(87) International publication number: WO 2019/240288

(57) **Abstract**

The present invention provides a technique enabling modification of an antibody, particularly regioselective modification of an antibody. More specifically, the present invention provides a compound having an affinity substance to an antibody and a bioorthogonal functional group, represented by the following Formula (I):

A-L-E-B (I)

wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group, or a salt thereof, and the like.

## Description

### TECHNICAL FIELD

The present invention relates to a compound having an affinity substance to an antibody and a bioorthogonal functional group, or a salt thereof, and the like.

### BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) (Non-Patent Literatures 1 to 3).

ADCs including T-DM1 have had the problem of their nonuniformity from the beginning of their development. That is, a small compound drug is randomly reacted with about 70 to 80 Lys residues in an antibody, and thus a drug/antibody ratio (DAR) and a conjugation position are not constant. It is known that such a random conjugation method normally provides a DAR within a range of 0 to 8, producing a plurality of medicines having different numbers of bonds of a drug. In recent years, it has been reported that when the number of bonds and the bond positions of a drug of an ADC are changed, pharmacokinetics, and a releasing rate and effects of the drug change. Given these circumstances, next-generation ADCs are required to control the number and positions of a drug to be conjugated. It is believed that when the number and positions are fixed, the problems of expected efficacy, variations in conjugation medicines, and lot difference, or what is called regulation, will be solved (Non-Patent Literature 4).

Although methods for regioselectively modifying antibodies are being investigated worldwide, most of them are methods of modification using genetic engineering techniques or enzymes. For the genetic engineering methods of modification, problems have been pointed out such as reductions in the expression efficiency of antibodies themselves (reductions in total yield when ADCs are prepared), although regioselectivity and number selectivity can be controlled. In addition, there is a problem in that it takes long years to construct an antibody expression system and the like (Non-Patent Literatures 5 to 7).

In recent years, methods that chemically modify proteins under complicated environments such as intracellular ones using a small molecule probe have been reported. The methods are used for imaging or identification of receptors in repositioning small compound drugs. In the field of chemical biology, organic chemical methods of protein modification using a synthesized small molecule probe are attracting attention (Non-Patent Literatures 8 to 11).

A chemical conjugation by affinity peptide (CCAP) method has recently been developed. This method has succeeded in regioselective modification of antibodies by a method that reacts a peptide reagent in which an NHS-activated ester and a drug are coupled with an affinity peptide with an antibody (that is, a method for producing an ADC through a linker comprising a peptide portion). This method has succeeded in regioselectively modifying an antibody Fc region with a drug by a chemical synthetic technique first in the world, and besides, practically favorable results [reaction time: 30 minutes, yield: 70% (for DAR 1), and regioselectivity: 100%] have been determined. It has been demonstrated that control with a DAR of 2 can be achieved by adding about five equivalents of the peptide reagent, which is epoch-making in that a modified position can also be controlled (Patent Literature 1) .

### PRIOR ART REFERENCES

### Patent Literature

Patent Literature 1: WO 2016/186206

### Non-Patent Literature

Non-Patent Literature 1: Reichert JM et al., Nat Biotechnol 2005; 23: 1073-8
Non-Patent Literature 2: Kubota T et al., Cancer Sci 2009; 100: 1566-72
Non-Patent Literature 3: Wu AM et al., Nat Biotechnol 2005; 23: 1137-46
Non-Patent Literature 4: Junutula JR et al., Nat Biotechnol 2008; 26: 925-32
Non-Patent Literature 5: Shen BQ et al., Nat Biotechnol 2012; 30: 184-9
Non-Patent Literature 6: Hofer T et al., Biochemistry 2009; 48: 12047-57
Non-Patent Literature 7: Liu W et al., Nat Methods 2007; 4: 239-44
Non-Patent Literature 8: S. T. Laughlin et al., Science 2008; 320,664
Non-Patent Literature 9: A. E. Speers et al., ChemBioChem 2004; 5, 41
Non-Patent Literature 10: Y. Takaoka et al., Angew. Chem. Int. Ed. 2013; 52, 4088
Non-Patent Literature 11: S. Fujishima et al., J. Am. Chem. Soc, 2012; 134: 3961-64

### DISCLOSURE OF INVENTION

### PROBLEM TO BE SOLVED BY THE INVENTION

The object of the present invention is to develop a technique enabling modification of an antibody, particularly regioselective modification of an antibody.

### MEANS FOR SOLVING PROBLEM

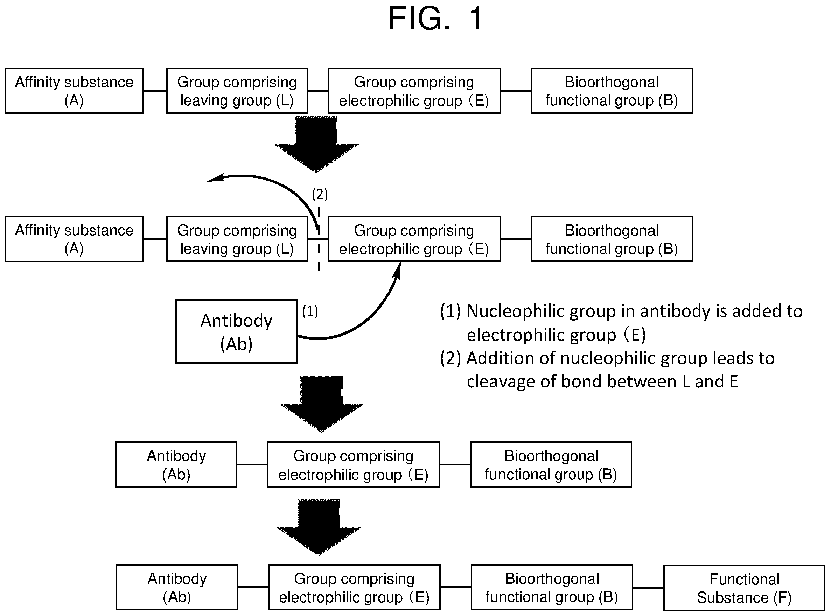

The inventors of the present invention made intensive studies, and as a result, have found out that a compound having a structural unit of A-L-E (where A is an affinity substance to an antibody, L is a divalent group comprising a certain leaving group, and E is a divalent group comprising an electrophilic group (i) coupled with the leaving group and (ii) having ability to react with a nucleophilic group in the antibody) or a salt thereof is useful for regiospecific modification of an antibody. For example, it has been found out that a certain compound having an affinity substance to an antibody and a bioorthogonal functional group, represented by Formula (I), is useful for regiospecific modification of an antibody (e.g., FIG. 1 and various Examples). In addition, it has been found out that a compound having an affinity substance to an antibody and a functional substance, represented by formula (IV), or a salt thereof is useful for regiospecific modification of an antibody (e.g., Examples 13 and 14). The inventors of the present invention have also found out that use of such a compound can prepare an antibody comprising no peptide portion as a linker and regioselectively having a functional substance or functional substances (e.g., a drug) (antibody drug conjugate (ADC)), for example. Avoidance of use of a linker comprising a peptide portion, which has potential immunogenicity and is easily hydrolyzed in the blood, is desirable in the clinical application of ADC. That is, the method developed by the inventors of the present invention has succeeded in regioselectively modifying an antibody Fc region with a drug by a chemical synthetic technique, and besides, without using any linker comprising a peptide portion.

Specifically, the present invention is as follows.
[1] A compound having an affinity substance to an antibody and a bioorthogonal functional group, represented by the following Formula (I):

   A-L-E-B (I)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   B is a bioorthogonal functional group, and
   the leaving group has ability to be cleaved and eliminated from E by a reaction between the nucleophilic group and the electrophilic group, or
   a salt thereof.
[2] The compound or salt thereof according to [1], wherein the affinity substance is a peptide.
[3] The compound or salt thereof according to [2], wherein the peptide is a peptide having ability to bind to a constant region of a monoclonal antibody.
[4] The compound or salt thereof according to [2] or [3], wherein the peptide is a peptide having ability to bind to an Fc region of a monoclonal antibody.
[5] The compound or salt thereof according to [4], wherein the peptide is a peptide having ability to bind to an Fc region of IgG.
[6] The compound or salt thereof according to any of [2] to [5], wherein the peptide has 10 to 40 amino acid residues.
[7] The compound or salt thereof according to any of [2] to [6], wherein the peptide comprises
   (a) (a-1-1) the amino acid sequence of FNMQQQRRFYEALHDPNLNEEQRNARIRSIRDD (SEQ ID NO: 11) or
      (a-1-2) the amino acid sequence of FNMQCQRRFYEALHDPNLNEEQRNARIRSIRDDC (SEQ ID NO: 12),
      wherein any one to three amino acid residues in the sequence, which may be the same or different, are each substituted with one amino acid residue selected from the group consisting of a lysine residue, an aspartic acid residue, and a glutamic acid residue, or
      (a-2-1) the amino acid sequence of β-Ala-NMQQQRRFYEALHDPNLNEEQRNARIRSIRDD (SEQ ID NO: 13) or
      (a-2-2) the amino acid sequence of β-Ala-NMQCQRRFYEALHDPNLNEEQRNARIRSIRDDC (SEQ ID NO: 14),
      wherein any one to three amino acid residues in the sequence, which may be the same or different, are each substituted with one amino acid residue selected from the group consisting of a lysine residue, an aspartic acid residue, and a glutamic acid residue, and
   (b) an amino acid sequence having 85% or more identity to each of the amino acid sequences of SEQ ID NOs: 11 to 14.
[8] The compound or salt thereof according to any of [2] to [6], wherein
   the peptide comprises any of amino acid sequences of
   Formula 1-1: (X₀₋₃)ₐ-C-Xaa1-Xaa2-Xaa3-Xaa4-Xaa5-Xaa6-I-I-W-C-(X₀₋₃)_{b} (SEQ ID NO: 15),
   Formula 1-2: (X₀₋₃)ₐ-C-Xaa1-Xaa2-Xaa3-Xaa4-Xaa5-Xaa6-I-V-W- C-(X₀₋₃)_{b} (SEQ ID NO: 16),
   Formula 1-3: (X₀₋₃)ₐ-C-Xaa1-Xaa2-Xaa3-Xaa4-Xaa5-Xaa6-V-V-W-C-(X₀₋₃)_{b} (SEQ ID NO: 17),
   Formula 1-4: (X₀₋₃)ₐ-C-Xaa1-Xaa2-Xaa3-Xaa4-Xaa5-Xaa6-A-V-W-C-(X₀₋₃)_{b} (SEQ ID NO: 18),
   Formula 1-5: (X₀₋₃)ₐ-C-Xaa1-Xaa2-Xaa3-Xaa4-Xaa5-Xaa6-L-L-W-C-(X₀₋₃)_{b} (SEQ ID NO: 19),
   Formula 1-6: (X₀₋₃)ₐ-C-Xaa1-Xaa2-Xaa3-Xaa4-Xaa5-Xaa6-L-I-W-C-(X₀₋₃)_{b} (SEQ ID NO: 20),
   Formula 1-7: (X₀₋₃)ₐ-C-Xaa1-Xaa2-Xaa3-Xaa4-Xaa5-Xaa6-L-V-F-C-(X₀₋₃)_{b} (SEQ ID NO: 21),
   Formula 1-8 (X₀₋₃)ₐ-C-Xaa1-Xaa2-Xaa3-Xaa4-Xaa5-Xaa6-Q-V-W-C-(X₀₋₃)_{b} (SEQ ID NO: 22),
   Formula 1-9: (X₀₋₃)ₐ-C-Xaa1-Xaa2-Xaa3-Xaa4-Xaa5-Xaa6-E-V-W-C-(X₀₋₃)_{b} (SEQ ID NO: 23) wherein
   (X₀₋₃)ₐ is absence, or an arginine residue-glycine residue-asparagine residue, an aspartic acid residue, a glycine residue-asparagine residue, or an asparagine residue,
   (X₀₋₃)_{b} is absence, or a threonine residue-tyrosine residue-histidine residue, or a threonine residue,
   Xaa1 is an alanine residue,
   Xaa2 is a tyrosine residue, a tryptophan residue, or a histidine residue,
   Xaa3 is a histidine residue, a phenylalanine residue, a tyrosine residue, a tryptophan residue, an arginine residue, or a glycine residue,
   Xaa4 is a lysine residue, an aspartic acid residue, or a glutamic acid residue,
   Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue,
   Xaa6 is a glutamine residue, a glutamic acid residue, an asparagine residue, or an aspartic acid residue, and Formula 2-1: (X₀₋₃')ₐ-C-(Xaa1')-(Xaa2')-(Xaa3')-(Xaa4')-(Xaa5')-(Xaa6')-L-V-W-C-(X₀₋₃')_{b} (SEQ ID NO: 24)
      wherein
   (X₀₋₃')ₐ and (X₀₋₃')_{b} are the same as the above (X₀₋₃)ₐ and (X₀₋₃)_{b}, respectively, and
   Xaa1', Xaa2', Xaa3', Xaa4', Xaa5', and Xaa6' are the same as the above Xaa1, Xaa2, Xaa3, Xaa4, Xaa5, and Xaa6, respectively.
[9] The compound or salt thereof according to any of [1] to [8], wherein the leaving group is (1) a group selected from the group consisting of -O-, -S-, -Se-, -SO₂-O-,- SO₂-N(R)-, -SO₂-, -C≡C-CH₂-O-, -N(OR)-, -N(R)-, and -O-N(R)- where R is a hydrogen atom or C₁₋₆ alkyl, or (2) heteroarylene.
[10] The compound or salt thereof according to any one of [1] to [9], wherein the nucleophilic group is selected from the group consisting of NH₂ in a side chain of a lysine residue, OH in a side chain of a tyrosine residue, OH in a side chain of a serine residue, OH in a side chain of a threonine residue, and SH in a side chain of a cysteine residue.
[11] The compound or salt thereof according to any one of [1] to [10], wherein the electrophilic group is selected from the group consisting of -C(=O)-, -SO₂-, and -CH₂-.
[12] The compound or salt thereof according to any of [1] to [11], wherein the bioorthogonal functional group is selected from the group consisting of an azide residue, an aldehyde residue, a thiol residue, an alkyne residue, an alkene residue, a halogen residue, a tetrazine residue, a nitron residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue.
[13] The compound or salt thereof according to any one of [1] to [12], wherein the bioorthogonal functional group is selected from the group consisting of an azide residue, a thiol residue, an alkyne residue, a maleimide residue, and a disulfide residue.
[14] The compound or salt thereof according to any one of [1] to [13], wherein the compound represented by the above Formula (I) is a compound represented by Formula (I-1) below:

   A-L₁-L₂-E₁-E₂-E₃-B (I-1)

   wherein
   A and B are the same as those in the above Formula (I),
   L₁ is a bond or a divalent group,
   L₂ is a leaving group,
   E₁ is an electrophilic group (i) coupled with the leaving group and (ii) having ability to react with a nucleophilic group in the antibody,
   E₂ is a group represented by (a) -X-Y-
      wherein
   X which binds to E₁ is C(R₁) (R₂) where R₁ and R₂ are each independently a hydrogen atom or C₁₋₆ alkyl, N(R₃) where R₃ is a hydrogen atom or C₁₋₆ alkyl, O, S, or Se, and
   Y which binds to E₃ is C(R₄)(R₅) where R₄ and R₅ are each independently a hydrogen atom or C₁₋₆ alkyl, or
   E₂ is a group represented by (b) the following formula (i) : 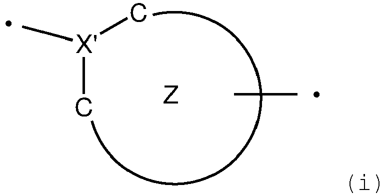
      where ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and · is a bond,
      when E₂ is -X-Y-, E₃ is a divalent group, and when E₂ is a group represented by Formula (i), E₃ is a bond or a divalent group, and
      the leaving group has ability to be cleaved and eliminated from E₁ by a reaction between the nucleophilic group and the electrophilic group.
[15] The compound or salt thereof according to [14], wherein the L₂ is
   (a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- where R is a hydrogen atom or C₁₋₆ alkyl,
   (b) heteroarylene, or
   (c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- where R is a hydrogen atom or C₁₋₆ alkyl.
[16] The compound or salt thereof according to [14] or [15], wherein the L₂ is a group selected from the group consisting of the following structural formulae: 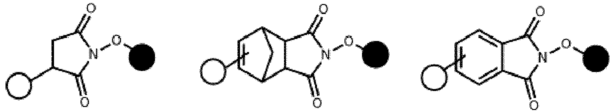 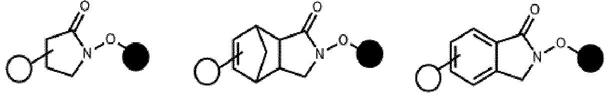 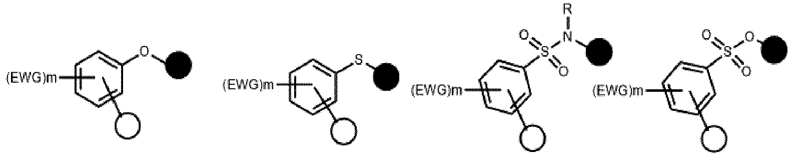 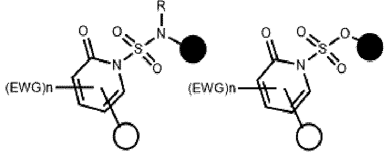 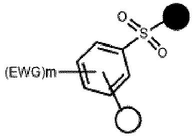 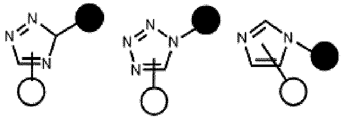 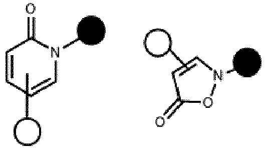 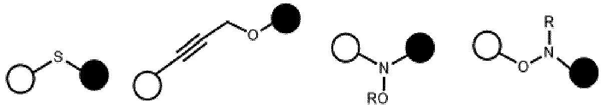
   where EWG is an electron-withdrawing group,
   m is an integer of 0 to 4,
   n is an integer of 0 to 3,
   R is a hydrogen atom or C₁₋₆ alkyl,
   a symbol of "white circle" is a bond to L₁, and a symbol of "black circle" is a bond to E₁.
[17] The compound or salt thereof according to any of [1] to [16], wherein a main chain of L or L₁-L₂ coupling A with E consists of 20 or less atoms.
[18] The compound or salt thereof according to any of [14] to [17], wherein the compound represented by the above Formula (I-1) is a compound represented by the following Formula (I-2):

   A-L₁-L₂-E₁-X-Y-E₃-B (1-2)

   wherein
   A, L₁, X, Y, and B are the same as those in the above Formula (I-1),
   L₂ is
      (a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- where R is a hydrogen atom or C₁₋₆ alkyl,
      (b) heteroarylene, or
      (c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- where R is a hydrogen atom or C₁₋₆ alkyl,
   E₁ is selected from the group consisting of -C(=O)-, - SO₂-, and -CH₂-, and
   E₃ is a divalent group.
[19] The compound or salt thereof according to any of [14] to [17], wherein the compound represented by the above Formula (I-1) is a compound represented by the following Formula (I-3): 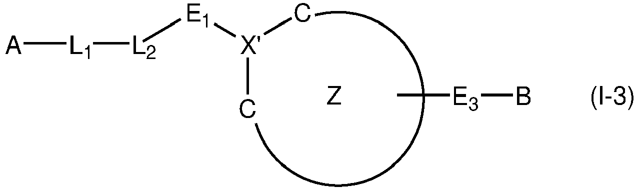 wherein
   A, L₁, ring Z, and B are the same as those in the above Formula (I-1),
   L₂ is
      (a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- where R is a hydrogen atom or C₁₋₆ alkyl,
      (b) heteroarylene, or
      (c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- (where R is a hydrogen atom or C₁₋₆ alkyl,
      E₁ is selected from the group consisting of -C(=O)-, - SO₂-, and -CH₂-, and
      E₃ is a bond or a divalent group.
[20] A reagent of regioselectively modifying an antibody by a bioorthogonal functional group, the reagent comprising
   a compound having an affinity substance to an antibody and a bioorthogonal functional group, represented by the following Formula (I):

   A-L-E-B (I)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   B is a bioorthogonal functional group, and
   the leaving group has ability to be cleaved and eliminated from E by a reaction between the nucleophilic group and the electrophilic group, or
   a salt thereof.
[21] A method for producing an antibody having a bioorthogonal functional group or bioorthogonal functional groups or a salt thereof, the method comprising:
   reacting a compound having an affinity substance to an antibody and a bioorthogonal functional group, represented by the following Formula (I):

      A-L-E-B (I)

      wherein
      A is an affinity substance to an antibody,
      L is a divalent group comprising a leaving group,
      E is a divalent group comprising an electrophilic group (i) coupled with the leaving group and (ii) having ability to react with a nucleophilic group in the antibody,
      B is a bioorthogonal functional group, and
      the leaving group has ability to be cleaved and eliminated from E by a reaction between the nucleophilic group and the electrophilic group, or a salt thereof with an antibody
   to form an antibody having a bioorthogonal functional group or bioorthogonal functional groups, represented by the following Formula (II):

      Ab-E-B (II)

      wherein
      E and B are the same as those in the above Formula (I), and
      Ab is an antibody, or
      a salt thereof.
[22] A method for producing an antibody having a functional substance or functional substances or a salt thereof, the method comprising:
   (1) reacting a compound having an affinity substance to an antibody and a bioorthogonal functional group, represented by the following Formula (I):

      A-L-E-B (I)

      wherein
      A is an affinity substance to an antibody,
      L is a divalent group comprising a leaving group,
      E is a divalent group comprising an electrophilic group (i) coupled with the leaving group and (ii) having ability to react with a nucleophilic group in the antibody,
      B is a bioorthogonal functional group, and
      the leaving group has ability to be cleaved and eliminated from E by a reaction between the nucleophilic group and the electrophilic group, or a salt thereof with an antibody
      to form an antibody having a bioorthogonal functional group or bioorthogonal functional groups, represented by the following Formula (II):

      Ab-E-B (II)

      wherein
      E and B are the same as those in the above Formula (I), and
      Ab is an antibody, or
      a salt thereof; and
   (2) reacting the antibody having a bioorthogonal functional group or bioorthogonal functional groups, represented by the above Formula (II), or a salt thereof with a functional substance via the bioorthogonal functional group
      to form an antibody having a functional substance or functional substances, represented by the following Formula (III) :

      Ab-E-B'-F (III)

      wherein Ab is the same as that in Formula (II),
      E is the same as that in Formula (I),
      B' is a divalent group comprising a portion formed by a reaction between the functional substance and the bioorthogonal functional group, and
      F is a functional substance, or a salt thereof.
[23] An antibody regioselectively having a bioorthogonal functional group or bioorthogonal functional groups, represented by the following Formula (II-1):

   Ab-E₁-E₂-E₃-B (II-1)

   wherein
   Ab is an antibody,
   E₁ is an electrophilic group coupled with a nucleophilic group in the antibody,
   E₂ is a group represented by (a) -X-Y-
   wherein X which binds to E₁ is C(R₁)(R₂) where R₁ and R₂ are each independently a hydrogen atom or C₁₋₆ alkyl, N(R₃) where R₃ is a hydrogen atom or C₁₋₆ alkyl, O, S, or Se, and
   Y which binds to E₃ is C(R₄)(R₅) where R₄ and R₅ are each independently a hydrogen atom or C₁₋₆ alkyl, or
   E₂ is a group represented by (b) the following formula (i) : 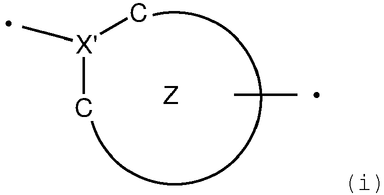
      wherein ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and · is a bond,
      when E₂ is -X-Y-, E₃ is a divalent group, and when E₂ is a group represented by Formula (i), E₃ is a bond or a divalent group, and
      B is a bioorthogonal functional group, or
      a salt thereof.
[24] The antibody or salt thereof according to [23], wherein the antibody is an antibody having a bioorthogonal functional group or bioorthogonal functional groups only in a constant region of a monoclonal antibody.
[25] The antibody or salt thereof according to [23] or [24], wherein the antibody is an antibody having a bioorthogonal functional group or bioorthogonal functional groups only in an Fc region of a monoclonal antibody.
[26] The antibody or salt thereof according to any of [23] to [25], wherein the antibody is a human IgG regioselectively having a bioorthogonal functional group or bioorthogonal functional groups in a region consisting of amino acid residues at positions 246 to 248 or 288 to 290 in a human IgG Fc region.
[27] The antibody or salt thereof according to any of [23] to [26], wherein the antibody represented by the above Formula (II-1) is an antibody regioselectively having a bioorthogonal functional group or bioorthogonal functional groups, represented by the following formula (II-2):

   Ab-E₁-X-Y-E₃-B (II-2)

   wherein
   Ab, X, Y, and B are the same as those in the above Formula (II-1),
   E₁ is selected from the group consisting of -C(=O)-, - SO₂-, and -CH₂-, and
   E₃ is a divalent group.
[28] The antibody or salt thereof according to any of [23] to [26], wherein the antibody represented by the above Formula (II-1) is an antibody regioselectively having a bioorthogonal functional group or bioorthogonal functional groups, represented by the following formula (II-3): 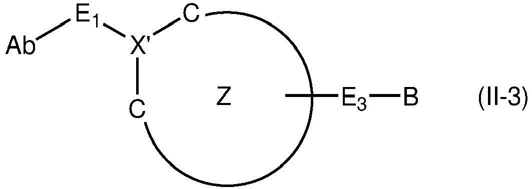 wherein
   Ab, ring-constituting atom X', ring Z, and B are the same as those in the above Formula (II-1),
   E₁ is selected from the group consisting of -C(=O)-, - SO₂-, and -CH₂-, and
   E₃ is a bond or a divalent group.
[29] An antibody regioselectively having a functional substance or functional substances, represented by the following Formula (III-1):

   Ab-E₁-E₂-E₃-B'-F (III-1)

   wherein
   Ab is an antibody,
   E₁ is an electrophilic group coupled with a nucleophilic group in the antibody,
   E₂ is a group represented by (a) -X-Y-
   wherein X which binds to E₁ is C(R₁)(R₂) where R₁ and R₂ are each independently a hydrogen atom or C₁₋₆ alkyl, N(R₃) where R₃ is a hydrogen atom or C₁₋₆ alkyl, O, S, or Se, and Y which binds to E₃ is C(R₄)(R₅) where R₄ and R₅ are each independently a hydrogen atom or C₁₋₆ alkyl, or E₂ is a group represented by (b) the following formula (i): 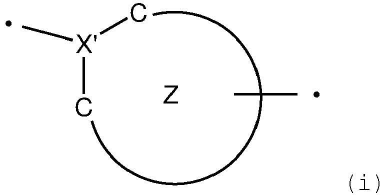
      wherein ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and · is a bond,
      E₃ is a divalent group when E₂ is -X-Y-, and is a bond or a divalent group when E₂ is a group represented by Formula (i),
      B' is a divalent group comprising a portion formed by a reaction between the functional substance and the bioorthogonal functional group, and
      F is a functional substance, or
      a salt thereof.
[30] The antibody or salt thereof according to [29], wherein the antibody is an antibody having a bioorthogonal functional group or bioorthogonal functional groups only in a constant region of a monoclonal antibody.
[31] The antibody or salt thereof according to [29] or [30], wherein the antibody is an antibody having a bioorthogonal functional group or bioorthogonal functional groups only in an Fc region of a monoclonal antibody.
[32] The antibody or salt thereof according to any of [29] to [31], wherein the antibody is a human IgG regioselectively having a functional substance or functional substances in a region consisting of amino acid residues at positions 246 to 248 or 288 to 290 in a human IgG Fc region.
[33] The antibody or salt thereof according to any of [29] to [32], wherein the antibody represented by the above Formula (III-1) is an antibody regioselectively having a functional substance or functional substances, represented by the following formula (III-2):

   Ab-E₁-X-Y-E₃-B'-F (III-2)

   wherein
   Ab, X, Y, B', and F are the same as those in the above Formula (III-1),
   E₁ is selected from the group consisting of -C(=O)-, - SO₂-, and -CH₂-, and
   E₃ is a divalent group.
[34] The antibody or salt thereof according to any of [29] to [32], wherein the antibody represented by the above Formula (III-1) is an antibody regioselectively having a functional substance or functional substances, represented by the following formula (III-3): 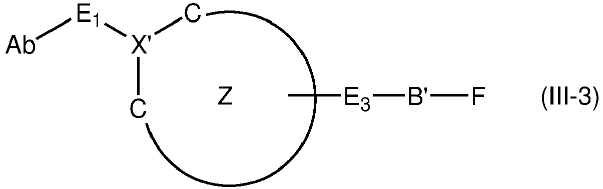 wherein
   Ab, ring-constituting atom X', ring Z, B', and F are the same as those in the above Formula (III-1),
   E₁ is selected from the group consisting of -C(=O)-, - SO₂-, and -CH₂-, and
   E₃ is a bond or a divalent group.
[35] A compound having an affinity substance to an antibody and a functional substance, represented by the following Formula (IV):

   A-L-E-F (IV)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   F is a functional substance, and
   the leaving group has ability to be cleaved and eliminated from E by a reaction between the nucleophilic group and the electrophilic group, or
   a salt thereof.
[36] A reagent of regioselectively modifying an antibody by a functional substance, represented by the following Formula (IV):

   A-L-E-F (IV)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   F is a functional substance, and
   the leaving group has ability to be cleaved and eliminated from E by a reaction between the nucleophilic group and the electrophilic group, or
   a salt thereof.
[37] A method for producing an antibody having a functional substance or functional substances or a salt thereof, the method comprising
   reacting a compound having an affinity substance to an antibody and a functional substance, represented by the following Formula (IV):

   A-L-E-F (IV)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   F is a functional substance, and
   the leaving group has ability to be cleaved and eliminated from E by a reaction between the nucleophilic group and the electrophilic group, or a salt thereof with an antibody
   to form the antibody having a functional substance or functional substances, represented by the following Formula (III) :

   Ab-E-F (III)

   wherein
   Ab is an antibody, and
   E and F are the same as those in the above Formula (IV), or a salt thereof.

### Advantageous Effects of Invention

The compound of the present invention having an affinity substance to an antibody and a bioorthogonal functional group or a functional substance, or a salt thereof is useful for regioselective modification of an antibody, for example.

The antibody of the present invention regioselectively having a bioorthogonal functional group or bioorthogonal functional groups or a salt thereof is useful as an intermediate in preparation of an antibody regioselectively having a functional substance or functional substances or a salt thereof, for example.

The antibody of the present invention regioselectively having a functional substance or functional substances or a salt thereof is useful as pharmaceuticals or reagents (e.g., diagnostic reagents and reagents for research), for example.

### BRIEF DESCRIPTION OF DRAWINGS

FIG. 1 is a schematic diagram illustrating an outline of the present invention.

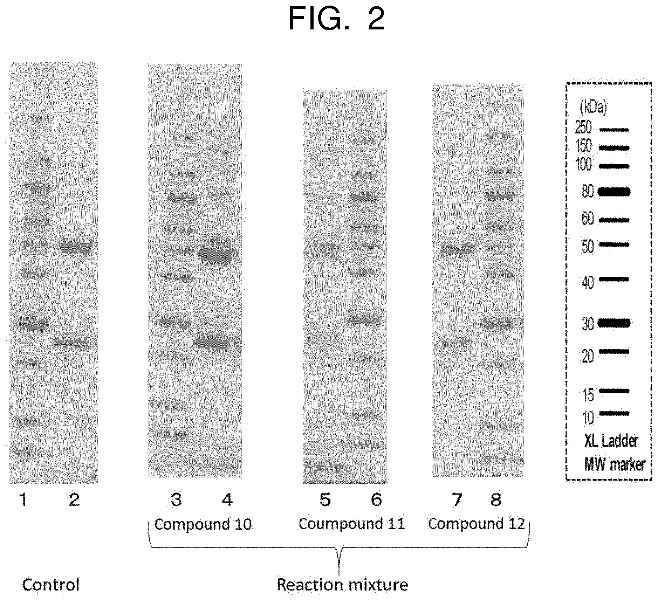

FIG. 2 is a diagram illustrating analysis results by SDS-PAGE in synthesis of an IgG antibody trastuzumab-peptide conjugate. Lanes 1, 3, 6, and 8: molecular weight markers; Lane 2: unreacted IgG antibody trastuzumab (control, A band near a molecular weight of 50,000 indicates a heavy chain, and a band near a molecular weight of 25,000 indicates a light chain); Lane 4: a conjugate of IgG antibody trastuzumab and compound 10 (An upper band near a molecular weight of 50,000 indicates that compound 10 has been conjugated with a heavy chain of trastuzumab. A lower band near the molecular weight of 50,000 indicates an unreacted heavy chain, and a band near a molecular weight of 25,000 indicates an unreacted light chain); Lane 5: a conjugate of IgG antibody trastuzumab and compound 11 (An upper band near a molecular weight of 50,000 indicates that compound 11 has been conjugated with a heavy chain of trastuzumab. A lower band near the molecular weight of 50,000 indicates an unreacted heavy chain, and a band near a molecular weight of 25,000 indicates an unreacted light chain); Lane 7: a reaction mixture after conjugation of IgG antibody trastuzumab with compound 12 (A band near a molecular weight of 50,000 indicates an unreacted heavy chain, and a band near a molecular weight of 25,000 indicates an unreacted light chain. A band indicating conjugation of compound 12 is not observed).

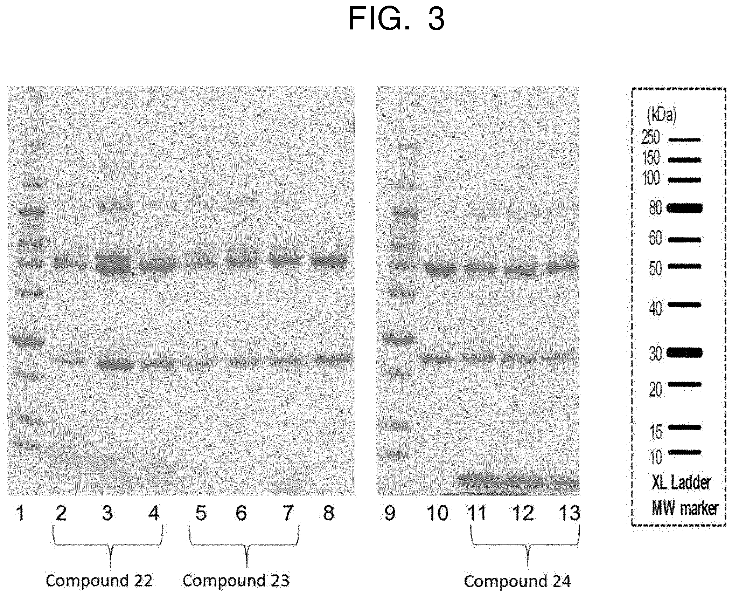

FIG. 3 is a diagram illustrating analysis results by SDS-PAGE of a trastuzumab-peptide conjugate synthesized by regiospecifically introducing maleimide to IgG antibody trastuzumab and then conjugating the resulting product with a peptide reagent having a thiol. Lanes 1 and 9: molecular weight markers. Lane 2: a conjugate obtained by treating IgG antibody trastuzumab with compound 22 (10 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lane 3: a conjugate obtained by treating IgG antibody trastuzumab with compound 22 (20 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lane 4: a conjugate obtained by treating IgG antibody trastuzumab with compound 22 (40 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lane 5: a conjugate obtained by treating IgG antibody trastuzumab with compound 23 (10 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lane 6: a conjugate obtained by treating IgG antibody trastuzumab with compound 23 (20 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lane 7: a conjugate obtained by treating IgG antibody trastuzumab with compound 23 (40 molar equivalents to the antibody) to introduce maleimide thereto regioselectively, and then conjugating the resulting product with compound 25. An upper band near a molecular weight of 50,000 indicates that maleimide has been introduced to a heavy chain of trastuzumab and the trastuzumab has been conjugated with compound 25. A lower band near the molecular weight of 50,000 indicates an unreacted heavy chain, and a band near a molecular weight of 25,000 indicates an unreacted light chain. Lanes 8 and 10: unreacted IgG antibody trastuzumab (control, A band near a molecular weight of 50,000 indicates a heavy chain, and a band near a molecular weight of 25,000 indicates a light chain) Lane 11: a reaction mixture obtained by treating IgG antibody trastuzumab with compound 24 (10 molar equivalents to the antibody) and then adding compound 25 thereto. A band near a molecular weight of 50,000 indicates an unreacted heavy chain, and a band near a molecular weight of 25,000 indicates an unreacted light chain. Lane 12: a reaction mixture obtained by treating IgG antibody trastuzumab with compound 24 (20 molar equivalents to the antibody) and then adding compound 25 thereto. A band near a molecular weight of 50,000 indicates an unreacted heavy chain, and a band near a molecular weight of 25,000 indicates an unreacted light chain. Lane 13: a reaction mixture obtained by treating IgG antibody trastuzumab with compound 24 (40 molar equivalents to the antibody) and then adding compound 25 thereto. A band near a molecular weight of 50,000 indicates an unreacted heavy chain, and a band near a molecular weight of 25,000 indicates an unreacted light chain.
FIG. 4 is a diagram illustrating ESI-TOFMS of maleimide-modified trastuzumab synthesized in (4-5-1) under post-reduction conditions. An upper part indicates measurement results of unreacted trastuzumab, and a lower part indicates modified trastuzumab.
FIG. 5 is a diagram illustrating ESI-TOFMS of azide-modified trastuzumab (azide-modified antibody 1) synthesized in (6-1-1) under post-reduction conditions. A lower part indicates measurement results of unreacted trastuzumab, and an upper part indicates modified trastuzumab.
FIG. 6 is a diagram illustrating ESI-TOFMS of azide-modified trastuzumab (azide-modified antibody 3) synthesized in (6-1-2) under post-reduction conditions. A lower part indicates measurement results of unreacted trastuzumab, and an upper part indicates modified trastuzumab.
FIG. 7 is a diagram illustrating ESI-TOFMS of azide-modified trastuzumab (azide-modified antibody 6) synthesized in (6-1-3) under post-reduction conditions. An upper part indicates measurement results of unreacted trastuzumab, and a lower part indicates modified trastuzumab.
FIG. 8 is a diagram illustrating ESI-TOFMS of azide-modified trastuzumab (azide-modified antibody 8) synthesized in (6-1-3) under post-reduction conditions. An upper part indicates measurement results of unreacted trastuzumab, and a lower part indicates modified trastuzumab.
FIG. 9 is a diagram illustrating ESI-TOFMS of azide-modified trastuzumab (azide-modified antibody 10) synthesized in (6-1-3) under post-reduction conditions. An upper part indicates measurement results of unreacted trastuzumab, and a lower part indicates modified trastuzumab.
FIG. 10 is a diagram illustrating ESI-TOFMS of azide-modified adalimumab (azide-modified antibody 28) synthesized in (6-1-4) under post-reduction conditions. An upper part indicates measurement results of unreacted adalimumab, and a lower part indicates modified adalimumab.
FIG. 11 is a diagram illustrating ESI-TOFMS of denosumab-azide-modified antibody (azide-modified antibody 29) synthesized in (6-1-4) under post-reduction conditions. An upper part indicates measurement results of unreacted denosumab, and a lower part indicates modified denosumab.
FIG. 12 is a diagram illustrating ESI-TOFMS of a dupilumab-azide-modified antibody (azide-modified antibody 30) synthesized in (6-1-4) under post-reduction conditions. An upper part indicates measurement results of unreacted dupilumab, and a lower part indicates modified dupilumab.
FIG. 13 is a diagram illustrating ESI-TOFMS of a rituximab-azide-modified antibody (azide-modified antibody 31) synthesized in (6-1-4) under post-reduction conditions. A lower part indicates measurement results of unreacted rituximab, and an upper part indicates modified rituximab.
FIG. 14 is a diagram illustrating ESI-TOFMS of protected thiol-modified trastuzumab synthesized in (8-1-1) under post-reduction conditions. An upper part indicates measurement results of unreacted trastuzumab, and a lower part indicates modified trastuzumab.
FIG. 15 is a diagram illustrating ESI-TOFMS of thiol-modified trastuzumab deprotected in (8-3-1) under post-reduction conditions. An upper part indicates measurement results of unreacted trastuzumab, and a lower part indicates modified trastuzumab.
FIG. 16 is a diagram illustrating ESI-TOFMS of azide-modified trastuzumab synthesized in (9-1-1) under post-reduction conditions. An upper part indicates measurement results of unreacted trastuzumab, and a lower part indicates modified trastuzumab.
FIG. 17 is a diagram illustrating ESI-TOFMS of azide-modified trastuzumab synthesized in (9-1-5) under post-reduction conditions. An upper part indicates measurement results of unreacted trastuzumab, and a lower part indicates modified trastuzumab.
FIG. 18 is a diagram illustrating ESI-TOFMS of azide-modified trastuzumab synthesized in (9-2-2) under post-reduction conditions. An upper part indicates measurement results of unreacted trastuzumab, and a lower part indicates modified trastuzumab.
FIG. 19 is a diagram illustrating ESI-TOFMS of a trastuzumab-Cy3 conjugate synthesized in (10-1-1). A lower part indicates measurement results of unreacted trastuzumab, a middle part indicates measurement results of azide-modified trastuzumab synthesized in (6-1-1), and an upper part indicates the trastuzumab-Cy3 conjugate.
FIG. 20 is a diagram illustrating ESI-TOFMS of a trastuzumab-Cy3 conjugate treated in (10-1-2) under reduction conditions. A lower part indicates measurement results of unreacted trastuzumab, a middle part indicates measurement results of azide-modified trastuzumab synthesized in (6-1-1), and an upper part indicates the trastuzumab-Cy3 conjugate.
FIG. 21 is a diagram illustrating ESI-TOFMS of a trastuzumab-peptide conjugate synthesized in (10-2-2). A lower part indicates measurement results of unreacted trastuzumab, a middle part indicates measurement results of azide-modified trastuzumab synthesized in (6-1-1), and an upper part indicates the trastuzumab-Cy3 conjugate.
FIG. 22 is a diagram illustrating ESI-TOFMS of a trastuzumab-Cy3 conjugate treated in (10-2-3) under reduction conditions. A lower part indicates measurement results of unreacted trastuzumab, a middle part indicates measurement results of azide-modified trastuzumab synthesized in (6-1-1), and an upper part indicates the trastuzumab-peptide conjugate.
FIG. 23 is a diagram illustrating ESI-TOFMS of a trastuzumab-maleimide compound conjugate treated in (10-3-1) under reduction conditions. An upper part indicates measurement results of thiol-introduced trastuzumab, and a lower part indicates a trastuzumab-maleimide compound conjugate.
FIG. 24 is a diagram illustrating ESI-TOFMS of a reaction product treated in (10-3-2) under reduction conditions. An upper part indicates measurement results of thiol-introduced trastuzumab, and a lower part indicates a trastuzumab-maleimide compound conjugate.
FIG. 25 is a diagram illustrating (1) an amino acid sequence of a heavy chain of trastuzumab (SEQ ID NO: 2) and (2) an amino acid sequence of a light chain of trastuzumab (SEQ ID NO: 4).
FIG. 26 is a diagram illustrating (1) an amino acid sequence of a heavy chain of denosumab (SEQ ID NO: 104) and (2) an amino acid sequence of a light chain of denosumab (SEQ ID NO: 105).
FIG. 27 is a diagram illustrating (1) an amino acid sequence of a heavy chain of dupilumab (SEQ ID NO: 106) and (2) an amino acid sequence of a light chain of dupilumab (SEQ ID NO: 107).

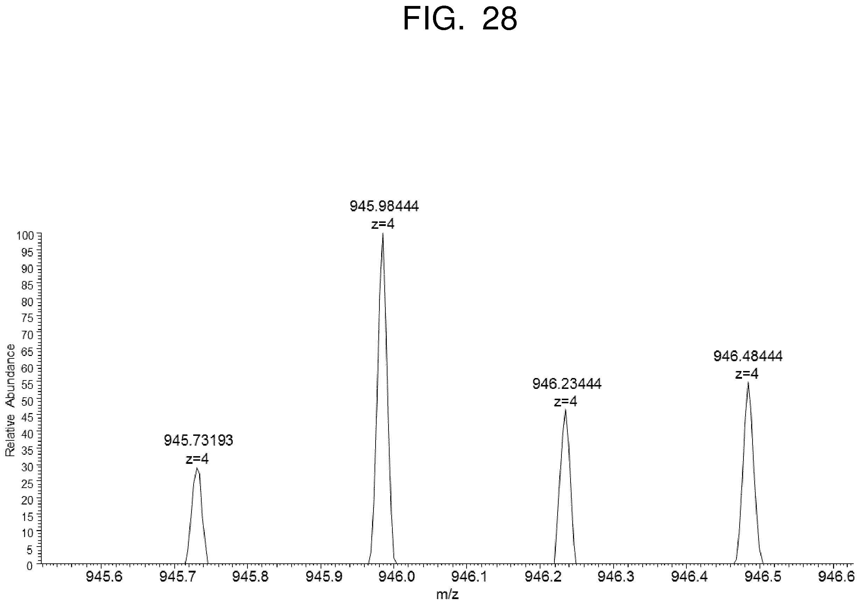

FIG. 28 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 29 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 30 is a diagram illustrating an MS spectrum of a peptide fragment of FNWYVDGVEVHNAKTKPR (SEQ ID NO: 12) comprising a modified site to a lysine residue by trypsin digestion of azidobenzoic acid-modified trastuzumab.
FIG. 31 is a diagram illustrating a CID spectrum of a peptide fragment of FNWYVDGVEVHNAKTKPR (SEQ ID NO: 12) comprising a modified site (aminebenzoic acid-introduced compound (+119.037 Da)) to a lysine residue by trypsin digestion of azidobenzoic acid-modified trastuzumab.
FIG. 32 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of mercaptopropionic acid-added maleimide-modified trastuzumab.
FIG. 33 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of maleimide MPA-modified trastuzumab.
FIG. 34 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of alkyl azide-modified trastuzumab.
FIG. 35 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of alkyl azide-modified trastuzumab.
FIG. 36 is a diagram illustrating an MS spectrum of a peptide fragment of VVSVLTVLHQDWLNGKEYK (SEQ ID NO: 101) comprising a modified site to a lysine residue by trypsin digestion of azidobenzoic acid-modified trastuzumab.
FIG. 37 is a diagram illustrating a CID spectrum of a peptide fragment of VVSVLTVLHQDWLNGKEYK (SEQ ID NO: 101) comprising a modified site to a lysine residue by trypsin digestion of azidobenzoic acid-modified trastuzumab.
FIG. 38 is a diagram illustrating analysis results by BioPharma Finder that a lysine residue at position 317 of azidobenzoic acid-modified trastuzumab is highly selectively modified.
FIG. 39 is a diagram illustrating an MS spectrum of a peptide fragment of FNWYVDGVEVHNAKTKPR (SEQ ID NO: 12) comprising a modified site to a lysine residue by trypsin digestion of azidobenzoic acid-modified trastuzumab.
FIG. 40 is a diagram illustrating a CID spectrum of a peptide fragment of FNWYVDGVEVHNAKTKPR (SEQ ID NO: 12) comprising a modified site to a lysine residue by trypsin digestion of azidobenzoic acid-modified trastuzumab.
FIG. 41 is a diagram illustrating analysis results by BioPharma Finder that a lysine residue at position 288 or 290 of azidobenzoic acid-modified trastuzumab is highly selectively modified.
FIG. 42 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 43 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 44 is a diagram illustrating analysis results by BioPharma Finder that a lysine residue at position 246 or 248 of azidobenzoic acid-modified trastuzumab is highly selectively modified.
FIG. 45 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of acetylthiol-modified trastuzumab.
FIG. 46 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of acetylthiol-modified trastuzumab.
FIG. 47 is a diagram illustrating analysis results by BioPharma Finder that a lysine residue at position 246 or 248 of acetylthiol-modified trastuzumab is highly selectively modified.
FIG. 48 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 49 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of azidobenzoic acid-modified trastuzumab.
FIG. 50 is a diagram illustrating an MS spectrum of a peptide fragment of FNWYVDGVEVHNAKTKPR (SEQ ID NO: 12) comprising a modified site to a lysine residue by trypsin digestion of azidobenzoic acid-modified trastuzumab.
FIG. 51 is a diagram illustrating a CID spectrum of a peptide fragment of FNWYVDGVEVHNAKTKPR (SEQ ID NO: 12) comprising a modified site to a lysine residue by trypsin digestion of azidobenzoic acid-modified trastuzumab.
FIG. 52 is a diagram illustrating an MS spectrum of a peptide fragment of THTCPPCPAPELLGGPSVFLFPPKPKDTLMISR (SEQ ID NO: 11) comprising a modified site to a lysine residue by trypsin digestion of acetylthiol and azidobenzoic acid-modified trastuzumab.
FIG. 53 is a diagram illustrating a CID spectrum of a peptide fragment of THTCPPCPAPELLGGPSVFLFPPKPKDTLMISR (SEQ ID NO: 11) comprising a modified site to a lysine residue by trypsin digestion of acetylthiol and azidobenzoic acid-modified trastuzumab.
FIG. 54 is a diagram illustrating an MS spectrum of a peptide fragment of FNWYVDGVEVHNAKTKPR (SEQ ID NO: 12) comprising a modified site to a lysine residue by trypsin digestion of acetylthiol and azidobenzoic acid-modified trastuzumab.
FIG. 55 is a diagram illustrating a CID spectrum of a peptide fragment of FNWYVDGVEVHNAKTKPR (SEQ ID NO: 12) comprising a modified site to a lysine residue by trypsin digestion of acetylthiol and azidobenzoic acid-modified trastuzumab.
FIG. 56 is a diagram illustrating analysis results by BioPharma Finder that lysine residues at position 246 or 248 and 288 or 290 of acetylthiol and azidobenzoic acid-modified trastuzumab are highly selectively modified.
FIG. 57 is a diagram illustrating an MS spectrum of a peptide fragment of CCVECPPCPAPPVAGPSVFLFPPKPKDTLMISR (SEQ ID NO: 102) comprising a modified site to a lysine residue by trypsin digestion of benzoic acid-modified denosumab.
FIG. 58 is a diagram illustrating a CID spectrum of a peptide fragment of CCVECPPCPAPPVAGPSVFLFPPKPKDTLMISR (SEQ ID NO: 102) comprising a modified site to a lysine residue by trypsin digestion of benzoic acid-modified denosumab.
FIG. 59 is a diagram illustrating analysis results by BioPharma Finder that a lysine residue at position 247 or 249 of benzoic acid-modified denosumab is highly selectively modified.
FIG. 60 is a diagram illustrating an MS spectrum of a peptide fragment of YGPPCPPCPAPEFLGGPSVFLFPPKPKDTLMISR (SEQ ID NO: 103) comprising a modified site to a lysine residue by trypsin digestion of benzoic acid-modified dupilumab.
FIG. 61 is a diagram illustrating a CID spectrum of a peptide fragment of YGPPCPPCPAPEFLGGPSVFLFPPKPKDTLMISR (SEQ ID NO: 103) comprising a modified site to a lysine residue by trypsin digestion of benzoic acid-modified dupilumab.
FIG. 62 is a diagram illustrating analysis results by BioPharma Finder that a lysine residue at position 251 or 253 of benzoic acid-modified dupilumab is highly selectively modified.
FIG. 63 is a diagram illustrating analysis results of a trastuzumab-DM1 conjugate synthesized in (12-1-1) by ESI-TOFMS (under non-reduction conditions).
FIG. 64 is a diagram illustrating analysis results of a trastuzumab-DM1 conjugate synthesized in (12-1-1) by ESI-TOFMS (under reduction conditions).
FIG. 65 is a diagram illustrating analysis results of a trastuzumab-MMAE conjugate synthesized in (12-2-1) by ESI-TOFMS (under non-reduction conditions).
FIG. 66 is a diagram illustrating analysis results of a trastuzumab-MMAE conjugate synthesized in (12-2-1) by ESI-TOFMS (under reduction conditions).
FIG. 67 is a diagram illustrating analysis results of a rituximab-DM1 conjugate synthesized in (12-3-1) by ESI-TOFMS (under non-reduction conditions).
FIG. 68 is a diagram illustrating analysis results of a rituximab-DM1 conjugate synthesized in (12-3-1) by ESI-TOFMS (under reduction conditions).
FIG. 69 is a diagram illustrating analysis results of a rituximab-DM1 conjugate synthesized in (12-4-1) by ESI-TOFMS (under non-reduction conditions).
FIG. 70 is a diagram illustrating analysis results of a rituximab-DM1 conjugate synthesized in (12-4-1) by ESI-TOFMS (under reduction conditions).
FIG. 71 is a diagram illustrating (1) a consensus amino acid sequence between an Fc region in a heavy chain of trastuzumab and an IgG1 Fc region (SEQ ID NO: 1), and (2) an amino acid sequence of an IgG1 Fc region (SEQ ID NO: 3) .

### EMBODIMENTS FOR CARRYING OUT THE INVENTION

### 1. Compound Having Affinity Substance to Antibody and Bioorthogonal Functional Group, or Salt Thereof

### 1-1. Outline

The present invention provides a compound having an affinity substance to an antibody and a bioorthogonal functional group, represented by Formula (I), or a salt thereof.

A-L-E-B (I)

wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group.

In expressions of Formula (I) and other formulae presented in relation to the present invention, - (a hyphen) indicates that two units present on both sides thereof covalently bind to each other. Consequently, in Formula (I), A covalently binds to L, L covalently binds to A and E, E covalently binds to L and B, and B covalently binds to E. The compound having an affinity substance to an antibody and a bioorthogonal functional group, represented by Formula (I), or a salt thereof indicates that the affinity substance to an antibody (A) contains a structural unit having L-E-B via a covalent bond between A and L. Consequently, in Formula (I), the affinity substance to an antibody (A) may contain one structural unit having L-E-B, or a plurality of (e.g., two to five, preferably two to four, more preferably two or three) structural units each having L-E-B (same or different).

Also in other formulae, the affinity substance to an antibody (A) or an antibody (Ab) contains a specific structural unit (structural unit excluding A or Ab) in each of the formulae via a covalent bond. Consequently, also in other formulae, the affinity substance to an antibody (A) or the antibody (Ab) may contain one specific structural unit or a plurality of (e.g., 2 to 5, preferably 2 to 4, more preferably 2 or 3) specific structural units (same or different).

### 1-2. Affinity Substance to Antibody (A)

In Formula (I), A is an affinity substance to an antibody. The affinity substance to an antibody is a substance having binding ability through a noncovalent bond to an antibody.

The affinity substance used in the present invention targets an antibody. The antibody may be an antibody modified with a biomolecule (e.g., sugar) or an antibody unmodified with a biomolecule. As the antibody, any antibody to any component such as a bio-derived component, a virus-derived component, or a component found in an environment can be used, but an antibody to a bio-derived component or a virus-derived component is preferable. Examples of the bio-derived component include components derived from animals such as mammals and birds (e.g., chickens), insects, microorganisms, plants, fungi, and fishes (e.g., protein). The bio-derived component is preferably a component derived from mammals. Examples of the mammals include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). The bio-derived component is more preferably a component derived from primates or rodents (e.g., protein), and even more preferably a human-derived component (e.g., protein) in view of the clinical application of the present invention. Examples of the virus-derived component include components derived from influenza viruses (e.g., avian influenza viruses and swine influenza viruses), AIDS virus, Ebola virus, and phage viruses (e.g., protein).

The antibody is a polyclonal antibody or a monoclonal antibody, and is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, scFv antibodies, Fab antibodies, F(ab')₂ antibodies, VHH antibodies, Fc region proteins, and Fc-fusion proteins. The antibody may be a divalent antibody (e.g., IgG, IgD, or IgE) or a tetravalent or higher antibody (e.g., IgA antibody or IgM antibody).

The antibody as a target of the affinity substance may comprise any amino acid residues and preferably comprises 20 natural L-α-amino acid residues normally contained in proteins. Examples of such amino acid residues include L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H), L-lysine (K), and glycine (G) (hereinafter, the expression of L is omitted). The antibody may comprise e.g., 100 or more, preferably 120 or more, more preferably 150 or more, even more preferably 180 or more, and particularly preferably 200 or more amino acid residues. The antibody may comprise e.g., 1,000 or less, preferably 900 or less, more preferably 800 or less, even more preferably 700 or less, and particularly preferably 600 or less amino acid residues. More specifically, the antibody may comprise e.g., 100 to 1,000, preferably 120 to 900, more preferably 150 to 800, even more preferably 180 to 700, and particularly preferably 200 to 600 amino acid residues. When the antibody is an antibody (e.g., the monoclonal antibody described above), the above number of amino acid residues may correspond to amino acid residues of a heavy chain of the antibody.

The antibody as the target of the affinity substance is further a protein comprising specific amino acid residues having a side chain or a terminal (an N-terminal and/or a C-terminal), preferably a side chain, with which a bioorthogonal functional group described below is capable of reacting at one position or a plurality of positions (preferably a plurality of positions). Examples of such specific amino acid residues include amino acid residues described below; preferred are amino acid residues selected from the group consisting of a lysine residue, a tyrosine residue, a serine residue, a threonine residue, and a cysteine residue. Considering that the compound of the present invention can regioselectively modify an antibody, preferred is an antibody comprising such specific amino acid residues at a plurality of positions. The positions are not limited to particular positions so long as they are two or more positions and may be e.g., three or more positions, preferably five or more positions, more preferably ten or more positions, even more preferably 20 or more positions, and particularly preferably 30 or more positions. The positions may be e.g., 200 or less positions, preferably 180 or less positions, more preferably 150 or less positions, even more preferably 120 or less positions, and particularly preferably 100 or less positions. More specifically, the positions may be e.g., 3 to 200 positions, preferably 5 to 180 positions, more preferably 10 to 150 positions, even more preferably 20 to 120 positions, and particularly preferably 30 to 100 positions. Even for such an antibody comprising the specific amino acid residues at a plurality of positions, the compound of the present invention can regioselectively modify one or two specific amino acid residues present in a specific region. It is said that the number of lysine residues of human IgG1 is generally about 70 to 90, for example, although it depends on an amino acid composition in a variable region. The present invention has succeeded in regioselectively modifying such one or two lysine residues present in a specific region of human IgG1.

More specifically, in the present invention, in view of, while maintaining the function of an antibody (that is, while maintaining native folding without denaturing the antibody), modifying amino acid residues present at specific target sites in the antibody, preferred is regioselective modification of amino acid residues exposed to the surface of the antibody. In human IgG such as human IgG1, for example, exposed lysine residues and exposed tyrosine residues are present at the following positions (refer to http://www.imgt.org/IMGTScientificChart/Numbering/Hu_IGHGnb er.html by EU numbering).

### (1) Exposed lysine residues

CH2 domain (position 246, position 248, position 274, position 288, position 290, position 317, position 320, position 322, and position 338)
CH3 domain (position 360, position 414, and position 439)

### (2) Exposed tyrosine residues

CH2 domain (position 278, position 296, and position 300)
CH3 domain (position 436)

### (3) Exposed serine residues

CH2 domain (position 254, position 267, position 298, and position 324)
CH3 domain (position 375, position 400, position 415, position 440, and position 442)

### (4) Exposed threonine residue

CH2 domain (position 256, position 289, and position 307)
CH3 domain (position 335, position 359, position 393, and position 437)

Consequently, when human IgG such as human IgG1 is modified with a lysine residue or a tyrosine residue, modification at the above positions is preferred.

When human IgG such as human IgG1 is modified with a lysine residue, a tyrosine residue, a serine residue, or a threonine residue, among the positions of (1) to (4), lysine residues, tyrosine residues, serine residues, or tyrosine residues present at the following positions, which are high in the degree of exposure to the surface, may be preferably modified.

### (1') Exposed lysine residues

CH2 domain (position 246, position 248, position 274, position 288, position 290, position 317, position 320, and position 322)
CH3 domain (position 360, position 414, and position 439)

### (2') Exposed tyrosine residues

CH2 domain (position 278, position 296, and position 300)
CH3 domain (position 436)

### (3') Exposed serine residues

CH2 domain (position 254, position 267, and position 298)
CH3 domain (position 400, position 415, and position 440)

### (4') Exposed threonine residues

CH2 domain (position 256 and position 289)
CH3 domain (position 335 and position 359)

Consequently, when human IgG such as human IgG1 is modified with a lysine residue, a tyrosine residue, a serine residue, or a threonine residue, modification at the above positions is more preferred.

When human IgG such as human IgG1 is modified with a lysine residue, among the positions of (1), lysine residues present at certain positions (e.g., position 246, position 248, position 288, position 290, and position 317) in the CH2 domain, which can be efficiently modified in the present invention, may be more preferably modified.

In a specific embodiment, the antibody as the target of the affinity substance, when comprising the specific amino acid residues at a plurality of positions as described above, may comprise one or more specific amino acid residues in a target region consisting of 1 to 50 continuous amino acid residues and comprise five or more of the specific amino acid residues in a non-target region other than the target region. The target region may consist of preferably 1 to 30, more preferably 1 to 20, and even more preferably one to ten, one to five, or one to three (that is, one, two, or three) amino acid residues. The target region may be particularly preferably a region consisting of a specific amino acid residue present at a specific position. Such a specific position, which varies depending on the types of the target protein and the affinity substance and the like, may be e.g., a specific position in a specific region of a constant region of an antibody (e.g., CH1, CH2, and CH3) and preferably a position in CH2 of an antibody. The target region may be more specifically the following residues following Eu numbering in human IgG Fc:
(1) a Lys248 residue (hereinafter, also referred to simply as "Lys248" in the present specification and corresponding to the 18th residue in a human IgG CH2 region (SEQ ID NO: 1)) or a Lys246 residue (hereinafter, also referred to simply as "Lys246" in the present specification and corresponding to the 16th residue in the human IgG CH2 region (SEQ ID NO: 1));
(2) a Lys288 residue (hereinafter, also referred to simply as "Lys288" in the present specification and corresponding to the 58th residue in the human IgG CH2 region (SEQ ID NO: 1)) or a Lys290 residue (hereinafter, also referred to simply as "Lys290" in the present specification and corresponding to the 60th residue in the human IgG CH2 region (SEQ ID NO: 1)); and
(3) a Lys317 residue (hereinafter, also referred to simply as "Lys317" in the present specification and corresponding to the 87th residue in the human IgG CH2 region (SEQ ID NO: 1)).

The present invention can modify the specific amino acid residue in the target region highly regioselectively. Such regioselectivity may be e.g., 30% or more, preferably 40% or more, more preferably 50% or more, even more preferably 60% or more, and particularly preferably 70% or more, 80% or more, 90% or more, 95% or more, 96% or more, 97% or more, 98% or more, 99% or more, or 100%.

The target region does not necessarily comprise the same kind of amino acid residue as the specific amino acid residue other than the specific amino acid residue present at the specific position in a region up to a remote position of "a" (where "a" is any integer of 1 to 10) amino acid residues to an N-terminal side and a C-terminal side each with respect to the specific amino acid present at the specific position. The symbol "a" is preferably an integer of 1 to 5, more preferably an integer of 1 to 3, even more preferably 1 or 2, and particularly preferably 1.

In a preferred embodiment, the antibody is a monoclonal antibody. Examples of the isotype of the monoclonal antibody and the like include IgG (e.g., IgG1, IgG2, IgG3, and IgG4), IgM, IgA, IgD, IgE, and IgY. The monoclonal antibody is a full-length antibody or an antibody fragment (e.g., F(ab')₂, Fab', Fab, Fv, and a single-chain antibody); the full-length antibody is preferred. The antibody is particularly preferably a human antibody, a humanized antibody, or a chimeric antibody having human IgG (e.g., IgG1, IgG2, IgG3, and IgG4) in a constant region.

The antibody is an antibody to any antigen. Such an antigen may be a component found in organisms and viruses described above, for example. Examples of such an antigen include a protein [comprising an oligopeptide and a polypeptide, which may be a protein modified with a biomolecule such as sugar (e.g., glycoprotein)], a sugar chain, a nucleic acid, and a small compound.

The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, Siglec-15

### (8) Blood Diseases

vWF, Factor IXa, Factor X, IFNγ, C5, BMP-6, Ferroportin, TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

In a more preferred embodiment, the affinity substance to an antibody is an affinity substance to a monoclonal antibody. The isotype of the monoclonal antibody is similar to those described above for the antibody; IgG (e.g., IgG1, IgG2, IgG3, and IgG4) is preferred. The monoclonal antibody is preferably a full-length monoclonal antibody.

In an even more preferred embodiment, the affinity substance to an antibody is an affinity substance to a chimeric antibody, a humanized antibody, or a human antibody (e.g., IgG including IgG1, IgG2, IgG3, and IgG4) as a full-length monoclonal antibody.

In a particularly preferred embodiment, the affinity substance to an antibody is an affinity substance to an antibody, comprising any one Fc region protein selected from the group consisting of the following (A) to (C) and having antigen-binding ability:
(A) an Fc region protein comprising the amino acid sequence of SEQ ID NO: 1;
(B) an Fc region protein comprising an amino acid sequence with one or several amino acid residues inserted, added, deleted, or substituted in the amino acid sequence of SEQ ID NO: 1; and
(C) an Fc region protein comprising an amino acid sequence having 90% or more identity to the amino acid sequence of SEQ ID NO: 1.

The amino acid sequence of SEQ ID NO: 1 is an Fc region protein. It is known that such an Fc region protein has secretion ability. Consequently, the Fc region proteins of (A) to (C) can have secretion ability. An antibody comprising such an Fc region protein can have antigen-binding ability. The amino acid residue at position 18 in SEQ ID NO: 1 is any amino acid residue, preferably a neutral amino acid residue, more preferably an amino acid residue having a nonpolar side chain described below, and even more preferably leucine, isoleucine, or alanine, and particularly preferably leucine or alanine. The amino acid residue at position 19 in SEQ ID NO: 1 is any amino acid residue, preferably a neutral amino acid residue or an acidic amino acid residue, more preferably an amino acid residue having a nonpolar side chain or an acidic amino acid residue, and even more preferably leucine or glutamic acid. The amino acid residue at position 21 in SEQ ID NO: 1 is any amino acid residue, preferably a neutral amino acid residue, more preferably an amino acid residue having a nonpolar side chain, and even more preferably glycine or alanine. The amino acid residue at position 140 in SEQ ID NO: 1 is any amino acid residue, preferably an acidic amino acid residue, and more preferably glutamic acid or aspartic acid. The amino acid residue at position 142 in SEQ ID NO: 1 is any amino acid residue, preferably a neutral amino acid residue, more preferably an amino acid residue having a nonpolar side chain, even more preferably methionine, leucine, or isoleucine, and particularly preferably methionine or leucine. The amino acid residue at position 177 in SEQ ID NO: 1 is any amino acid residue, preferably a neutral amino acid residue, more preferably an amino acid residue having an uncharged polar side chain or an amino acid residue having a nonpolar side chain described below, even more preferably threonine, alanine, or glycine, and particularly preferably threonine or alanine.

In a preferred embodiment, the amino acid sequence of SEQ ID NO: 1 may be an amino acid sequence consisting of the amino acid residues at positions 220 to 449 in the amino acid sequence of SEQ ID NO: 2.

In another preferred embodiment, the amino acid sequence of SEQ ID NO: 1 may be an amino acid sequence consisting of the amino acid residues at positions 7 to 236 in the amino acid sequence of SEQ ID NO: 3.

In a specific embodiment, the antibody comprising the Fc region protein comprising the amino acid sequence described above may be an antibody comprising the Fc region protein comprising the amino acid sequence described above and a constant region of an antibody. Such a constant region of an antibody may be a constant region of a chimeric antibody, a humanized antibody, or a human antibody (e.g., IgG including IgG1, IgG2, IgG3, and IgG4).

In (B) the Fc region protein, one or several amino acid residues can be modified by one, two, three, or four variations selected from the group consisting of deletion, substitution, addition, and insertion of amino acid residues. The variations of amino acid residues may be introduced to one region in the amino acid sequence or intruded to a plurality of different regions. The term "one or several" indicates a number that does not significantly impair protein activity. The number indicated by the term "one or several" is e.g., 1 to 100, preferably 1 to 80, more preferably 1 to 50, 1 to 30, 1 to 20, 1 to 10, or 1 to 5 (e.g., one, two, three, four, or five) .

In (C) the Fc region protein, the percent identity to the amino acid sequence of SEQ ID NO: 1 may be 70% or more, 75% or more, 80% or more, 85% or more, 90% or more, 91% or more, 92% or more, 93% or more, 94% or more, 95% or more, 96% or more, 97% or more, 98% or more, or 99% or more. In the present invention, calculation of the percent identity of peptides and polypeptides (proteins) can be performed by Algorithm blastp. More specifically, calculation of the percent identity of polypeptides can be performed using Scoring Parameters (Matrix: BLOSUM62; Gap Costs: Existence = 11 Extension = 1; Compositional Adjustments: Conditional compositional score matrix adjustment) as default settings in Algorithm blastp provided in National Center for Biotechnology Information (NCBI). Calculation of the percent identity of polynucleotides (genes) can be performed by Algorithm blastn. More specifically, calculation of the percent identity of polynucleotides can be performed using Scoring Parameters (Match/Mismatch Scores = 1, -2; Gap Costs = Linear) as default settings in Algorithm blastn provided in NCBI.

Secretion in secretion ability has the same meaning as the secretion (what is called solubility) of the secretory protein. Consequently, "having secretion ability" means functioning as an antibody in a manner similar to normal antibodies.

In the antibody comprising the Fc region protein, a variation may be introduced to a specific site so long as target characteristics (e.g., secretion ability and antigen-binding ability) are maintained. The position of an amino acid residue to which a variation may be introduced that can maintain the target characteristics is obvious to a person skilled in the art. Specifically, a person skilled in the art can 1) compare amino acid sequences of a plurality of proteins having homogeneous characteristics with each other, 2) clarify a relatively preserved region and a relatively non-preserved region, and then 3) predict a region capable of playing an important role for a function and a region incapable of playing an important role for a function from the relatively preserved region and the relatively non-preserved region each and can thus recognize structure-function correlation. Consequently, a person skilled in the art can identify the position of an amino acid residue to which a variation may be introduced in the amino acid sequence of the antibody comprising the Fc region protein.

When an amino acid residue is varied by substitution, the substitution of the amino acid residue may be preservative substitution. The term "preservative substitution" when used in the present specification refers to substituting a certain amino acid residue with an amino acid residue having a similar side chain. Families of amino acid residues having a similar side chain are known in the field concerned. Examples of such families include amino acids having a basic side chain (e.g., lysine, arginine, and histidine), amino acids having an acidic side chain (e.g., aspartic acid, and glutamic acid), amino acids having an uncharged polar side chain (e.g., asparagine, glutamine, serine, threonine, tyrosine, and cysteine), amino acids having a nonpolar side chain (e.g., glycine, alanine, valine, leucine, isoleucine, proline, phenylalanine, methionine, and tryptophan), amino acids having a β-position-branched side chain (e.g., threonine, valine, and isoleucine), amino acids having an aromatic side chain (e.g., tyrosine, phenylalanine, tryptophan, and histidine), amino acids having a hydroxy group (e.g., alcoholic and phenolic)-containing side chain (e.g., serine, threonine, tyrosine), and amino acids having a sulfur-containing side chain (e.g., cysteine and methionine). The preservative substitution of the amino acid may be preferably substitution between aspartic acid and glutamic acid, substation among arginine, lysine, and histidine, substitution between tryptophan and phenylalanine, substitution between phenylalanine and valine, substitution among leucine, isoleucine, and alanine, and substitution between glycine and alanine.

Examples of the antibody used in the present invention or the antibody comprising any one Fc region selected from the group consisting of (A) to (C) include chimeric antibodies (e.g., rituximab, basiliximab, infliximab, cetuximab, siltuximab, dinutuximab, and altertoxaximab), humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and human antibodies (e.g., adalimumab, panitumumab, golimumab, ustekinumab, canakinumab, ofatumumab, denosumab (IgG2), ipilimumab, belimumab, raxibacumab, ramucirumab, nivolumab (IgG4), secukinumab, evolocumab (IgG2), alirocumab, necitumumab, brodalumab (IgG2), olaratumab, and dupilumab (IgG4)) (cases not referring to the IgG subtype indicate that they are IgG1).

Examples of the affinity substance to an antibody described above include peptides (comprising oligopeptides, polypeptides, and proteins), small compounds, nucleic acids, nucleic acid-peptide conjugates, peptide-small compound conjugates, and nucleic acid-small compound conjugates.

In a specific embodiment, the affinity substance to an antibody described above may be a peptide (comprising an oligopeptide, a polypeptide, and a protein, which may be a glycoprotein). As such a peptide, for example, the following peptides have been reported:
(1) an IgG binding peptide having an affinity to a specific region (CH2 region) of human IgG in general (that is, human IgG1, IgG2, IgG3, and IgG4. Hereinafter the same) (e.g., refer to WO 2016/186206, WO 2013/027796, and WO 2008/054030);
(2) a protein A Mimetic (PAM) peptide having an affinity to a specific region (CH2 region) of human IgG in general (e.g., refer to Fassina G et al., JOURNAL OF MOLECULAR RECOGNITION, 1996, VOL.6, 564-569);
(3) EPIHRSTLTALL (SEQ ID NO: 25) having an affinity to a specific region (CH2 region) of human IgG in general (e.g., refer to Ehrlich G. K et al., J. Biochem. Biophys. Methods, 2001, VOL. 49, 443-454);
(4) (NH2-Cys1-X1-X2-X3-X4)2-Lys-Gly-OH having an affinity to a specific region (Fc region) of human IgG in general (e.g., refer to Ruvo M et al., ChemBioChem, 2005, VOL. 6, 1242-1253);
(5) FARLVSSIRY (SEQ ID NO: 26), FGRLVSSIRY (SEQ ID NO: 27), and TWKTSRISIF (SEQ ID NO: 28) having an affinity to a specific region (Fc region) of human IgG in general (e.g., refer to Krook M et al., Journal of Immunological Methods, 1998, VOL. 221, 151-157);
(6) QSYP (SEQ ID NO: 29) having an affinity to a specific region of human IgG in general (e.g., refer to Jacobs J. M. et al., Bio. Techniques, 2003, VOL. 34, 132-141) ;
(7) HWRGWV (SEQ ID NO: 30), HYFKFD (SEQ ID NO: 31), and HFRRHL (SEQ ID NO: 32) having an affinity to a specific region (Fc region) of human IgG in general (e.g., refer to Carbonell R. G. et al., Journal of Chromatography A, 2009, VOL. 1216, 910-918);
(8) DAAG (SEQ ID NO: 33) having an affinity to a specific region (Fc region) of human IgG in general (e.g., refer to Lund L. N. et al., Journal of Chromatography A, 2012, VOL. 1225, 158-167);
(9) Fc-I, Fc-II, and Fc-III having an affinity to a specific region (Fc region) of human IgG in general (e.g., refer to Warren L. Delano et al., Science, 2000, VOL. 287, 1279-1283; WO 2001/045746); and
(10) NARKFYKG (SEQ ID NO: 34) and NKFRGKYK (SEQ ID NO: 35) having an affinity to a specific region (Fc region) of human IgG in general (e.g., refer to Biochemical Engineering Journal, 2013, VOL. 79, 33-40).

In another specific embodiment, the affinity substance to an antibody described above may be a substance other than the peptide. Reported examples of such a substance include an aptamer having affinity to a specific region (the CH2 region, especially a side chain of Lys340) of human IgG (e.g., human IgG1 to 4) [e.g., GGUG(C/A)(U/T) motif-containing aptamers such as GGUGCU and GGUGAU] (e.g., refer to WO 2007/004748; Nomura Y et al., Nucleic Acids Res., 2010 Nov; 38(21): 7822-9; and Miyakawa S et al., RNA., 2008 Jun; 14(6): 1154-63).

The affinity substance to an antibody described above can be obtained by any known method in the field concerned. The affinity substance to an antibody can be obtained by producing an antibody (e.g., the hybridoma method) using the entire antibody or a partial peptide in the antibody (e.g., when an antibody surface exposed region is known, a partial peptide present in the region) or screening the affinity substance (e.g., the phage display method, the systematic evolution of ligands with exponential enrichment (SELEX) method, the mRNA display method, the ribosome display method, the cDNA display method, and the yeast display method) from a library from which the affinity substance is available (e.g., peptide libraries, antibody libraries, antibody-forming cell libraries, aptamer libraries, phage libraries, mRNA libraries, and cDNA libraries), for example. When the affinity substance to an antibody is an affinity substance to an Fc region (a soluble region) of an antibody, a partial peptide present in a specific region (e.g., CH1, CH2, and CH3) of the Fc region of various kinds of antibodies (e.g., IgG, IgA, IgM, IgD, and IgE) is used, whereby an affinity substance (e.g., an antibody and an aptamer) capable of selectively binding to any portion in the Fc region of the antibody can be efficiently obtained. The thus obtained affinity substances comprise a mixture of substances relatively strong and weak in affinitive binding ability. However, even an affinity substance weak in affinitive biding ability can strengthen its affinitive binding ability by using it in an excessive amount.

In a preferred embodiment, the affinity substance to an antibody is a peptide. Such a peptide is preferably a peptide having ability to bind to a constant region of a monoclonal antibody, more preferably a peptide having ability to bind to an Fc region of the monoclonal antibody, and even more preferably a peptide having ability to bind to an Fc region of IgG. The length of the peptide is not particularly limited, but is, for example, a peptide consisting of 10 to 40 (e.g., 10 to 20, 20 to 30, and 30 to 40) amino acid residues. Examples of the amino acid residue constituting the peptide include 20 L-α-amino acid residues constituting natural proteins, stereoisomers thereof (e.g., D-amino acid), and isomers thereof (e.g., β-amino acid).

In a specific embodiment, the affinity substance to an antibody described above may be a peptide comprising any of the following amino acid sequences:
A peptide comprising
(a-1-1) an amino acid sequence of FNMQQQRRFYEALHDPNLNEEQRNARIRSIRDD (SEQ ID NO: 11) (e.g., refer to compounds 22 to 24 and 29) or
(a-1-2) an amino acid sequence of FNMQCQRRFYEALHDPNLNEEQRNARIRSIRDDC (SEQ ID NO: 12) (an amino acid sequence in which two Ks in the known sequence Z34C are substituted with Rs), wherein any one to three amino acid residues in the sequence, which may be the same or different, are each substituted with one amino acid residue selected from the group consisting of a lysine residue, an aspartic acid residue, and a glutamic acid residue, or
(a-2-1) an amino acid sequence of β-Ala-NMQQQRRFYEALHDPNLNEEQRNARIRSIRDD (SEQ ID NO: 13) (e.g., refer to compounds 26 to 28) or
(a-2-2) an amino acid sequence of β-Ala-NMQCQRRFYEALHDPNLNEEQRNARIRSIRDDC (SEQ ID NO: 14) (an amino acid sequence in which two Ks in the known sequence Z34C are substituted with Rs, and an N-terminal F is substituted with β-Ala), wherein any one to three amino acid residues in the sequence, which may be the same or different, are each substituted with one amino acid residue selected from the group consisting of a lysine residue, an aspartic acid residue, and a glutamic acid residue, and
(b) an amino acid sequence having 85% or more identity to each of the amino acid sequences of SEQ ID NOs: 11 to 14.

Such a peptide has ability to bind to an Fc region of a monoclonal antibody.

The peptide consisting of the amino acid sequence of SEQ ID NO: 12 is obtained by changing two Ks (lysine) of the 26th and 28th counted from the N-terminal to Rs (arginine) in the affinity peptide known as Z34C for peptide reagent synthetic reasons. The compound of the present invention comprising a peptide containing any of the above amino acid sequences is useful for regioselective modification of a specific amino acid residue (e.g., Lys248 residue, Lys246 residue, Lys288, Lys290, or Lys317 according to Eu numbering, or amino acid residues other than these residues) in human IgG Fc. The amino acid sequence of Z34C is FNMQCQRRFYEALHDPNLNEEQRNAKIKSIRDDC (SEQ ID NO: 36) (e.g., refer to Starovasnik, M. A. et al., Structural mimicry of a native protein by a minimized binding domain., Proc. Natl. Acad. Sci. USA., 94, 10080-10085 (1997)).

The affinity peptide can have an affinity to human IgG (e.g., human IgG described above. Preferably human IgG1). When the above affinity peptide comprises two cysteine residues (e.g., positions 5 and 34), the affinity peptide may form a cyclic peptide through a disulfide bond by the two cysteine residues.

For a position to which a lysine residue, an aspartic acid residue, or a glutamic acid residue (an amino acid residue easily modified with a cross-linking agent) is introduced, any position can be used so long as it has affinity to human IgG such as human IgG1. A person skilled in the art can easily identify such a position. The position to which a lysine residue, an aspartic acid residue, or a glutamic acid residue is introduced may be an amino acid residue other than a cysteine residue. The position to which the amino acid residue capable of being easily modified with a cross-linking agent is introduced is more preferably the amino acid residues at position 1, position 3, position 6, position 7, position 13, position 20, position 24, position 31, and position 32, for example.

The amino acid sequence having the characteristics of (a) and (b) also preferably has one specific amino acid residue selected from the group consisting of a lysine residue, an aspartic acid residue, and a glutamic acid residue (the amino acid residue capable of being easily modified with a cross-linking agent) (preferably a lysine residue) at a certain position and has variations of the normal 20 amino acid residues forming natural proteins (preferably 17 amino acid residues other than a lysine residue, an aspartic acid residue, and a glutamic acid residue, and more preferably 19 amino acid residues other than a lysine residue) at positions other than the certain position. Such a certain position is not limited to a particular position; examples thereof include position 1, position 3, position 6, position 7, position 13, position 20, position 24, position 31, and position 32. The amino acid sequence having the characteristics of (a) and (b) maintains the two cysteine residues, and these two cysteine residues may bind to each other through a disulfide bond. The amino acid sequence having 85% or more identity to each of the amino acid sequences of SEQ ID NOs: 11 to 14 may comprise one to three (preferably one or two, and more preferably one) modified amino acid residues by one, two, three, or four variations selected from the group consisting of deletion, substitution, addition, and insertion (preferably substitution) of amino acid residues. The variations of amino acid residues may be introduced to one region in the amino acid sequence or intruded to a plurality of different regions.

The amino acid sequence having the characteristics of (a) and (b) may be more preferably the following (c) or (d).
(c) An amino acid sequence selected from the group consisting of the following amino acid sequences of (1) to (16) :
   (1) FNMQQQRRFYEALHDPNLNEEQRNARIRSIKDD(SEQ ID NO: 5);
   (2)FNMQQQRRFYEALHDPNLNEEQRNARIKSIRDD(SEQ ID NO: 6);
   (3) β-Ala-NMQQQRRFYEALHDPNLNEEQRNARIRSIRDD(SEQ ID NO: 7) ;
   (4) FNMQQQRRFYEALHDPNLNEEQRNAKIKSIKDD(SEQ ID NO: 8);
   (5)KNMQCQRRFYEALHDPNLNEEQRNARIRSIRDDC(SEQ ID NO: 37);
   (6)FNMQCQKRFYEALHDPNLNEEQRNARIRSIRDDC(SEQ ID NO: 38);
   (7)FNMQCQRRFYEAKHDPNLNEEQRNARIRSIRDDC(SEQ ID NO: 39);
   (8)FNMQCQRRFYEALHDPNLNEEQRKARIRSIRDDC(SEQ ID NO: 40);
   (9)FNMQCQRRFYEALHDPNLNKEQRNARIRSIRDDC(SEQ ID NO: 41);
   (10) FNMQCQRRFYEALHDPNLNEEQRNARIRSIKDDC(SEQ ID NO: 42);
   (11) FNKQCQRRFYEALHDPNLNEEQRNARIRSIRDDC(SEQ ID NO: 43);
   (12) FNMQCKRRFYEALHDPNLNEEQRNARIRSIRDDC(SEQ ID NO: 44);
   (13) FNMQCQRRFYEALHDPNLNEEQRNARIRSIRKDC(SEQ ID NO: 45);
   (14) β-Ala-NMQQQRRFYEALHDPNLEEQRNARIRSI(SEQ ID NO: 97);
   (15)FNMQQQRRFYEALHDPNLNKEQRNARIRSIRDD(SEQ ID NO: 98); and
   (16) β-Ala-NMQQQRRFYEALHDPNLEEQRNARIRSIKDD(SEQ ID NO: 100); or
(d) an amino acid sequence having 90% or more identity to any of the amino acid sequences of (1) to (16) (may be modification of the number of amino acid residues described above) having variations of 19 amino acid residues other than a lysin residue at positions other than the one lysine residue and the two cysteine residues (e.g., position 1, position 3, position 6, position 7, position 13, position 20, position 24, position 31, and position 32) in any of the amino acid sequences of (1) to (16).

It has been determined that a peptide comprising such an amino acid sequence has ability to bind to an Fc region of IgG. The affinity peptide comprising the amino acid sequence of (d) is preferably a peptide having ability to bind to a constant region of a monoclonal antibody, more preferably a peptide having ability to bind to an Fc region of the monoclonal antibody, and even more preferably a peptide having ability to bind to an Fc region of IgG.

The affinity peptide may have additional variations of amino acid residues other than the introduction of one amino acid residue easily modified with a cross-linking agent so long as it has 85% or more identity to each of the amino acid sequences of SEQ ID NOs: 11 to 14 or the amino acid sequences of (1) to (16). A person skilled in the art can easily identify a position to which the additional variations of amino acids can be introduced. The phenylalanine residue at position 1, the arginine residue at position 6, the leucine residue at position 13, the glutamic acid residue at position 20, the asparagine residue at position 24, or the arginine residue at position 31 (except the position to which the amino acid residue capable of being easily modified with a cross-linking agent has already been introduced) can also be used, for example. Examples of amino acids that can be introduced by the additional variations of amino acids include alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). These 19 amino acids other than lysine may be preferably used. The amino acids may each be an L-body or a D-body; an L-body is preferred (in Examples, the amino acid residues forming the peptides are all L-bodies).

The degree of percent identity to each of the amino acid sequences of SEQ ID NOs: 11 to 14 or the amino acid sequences of (1) to (16) can be determined as described above. The degree of percent identity is preferably 90% or more, 92% or more, more preferably 94% or more, even more preferably 95% or more, and particularly preferably 97% or more (that is, variation of only one amino acid residue is included).

In another specific embodiment, the affinity substance to an antibody described above is a peptide comprising any of the following amino acid sequences:

Formula 1-1: (X₀₋₃)ₐ-C-Xaa1-Xaa2-Xaa3-Xaa4-Xaa5-Xaa6-I-I-W-C-(X₀₋₃)_{b} (SEQ ID NO: 15)

Formula 1-2: (X₀₋₃)ₐ-C-Xaa1-Xaa2-Xaa3-Xaa4-Xaa5-Xaa6-I-V-W-C-(X₀₋₃)_{b}(SEQ ID NO: 16)

Formula 1-3: (X₀₋₃)ₐ-C-Xaa1-Xaa2-Xaa3-Xaa4-Xaa5-Xaa6-V-V-W-C-(X₀₋₃)_{b} (SEQ ID NO: 17)

Formula 1-4: (X₀₋₃)ₐ-C-Xaa1-Xaa2-Xaa3-Xaa4-Xaa5-Xaa6-A-V-W-C-(X₀₋₃)_{b} (SEQ ID NO: 18)

Formula 1-5: (X₀₋₃)ₐ-C-Xaa1-Xaa2-Xaa3-Xaa4-Xaa5-Xaa6-L-L-W-C-(X₀₋₃)_{b}(SEQ ID NO: 19)

Formula 1-6: (X₀₋₃)ₐ-C-Xaa1-Xaa2-Xaa3-Xaa4-Xaa5-Xaa6-L-I-W-C-(X₀₋₃)_{b}(SEQ ID NO: 20)

Formula 1-7: (X₀₋₃)ₐ-C-Xaa1-Xaa2-Xaa3-Xaa4-Xaa5-Xaa6-L-V-F-C-(X₀₋₃)_{b} (SEQ ID NO: 21)

Formula 1-8: (X₀₋₃)ₐ-C-Xaa1-Xaa2-Xaa3-Xaa4-Xaa5-Xaa6-Q-V-W-C-(X₀₋₃)_{b} (SEQ ID NO: 22)

Formula 1-9: (X₀₋₃)ₐ-C-Xaa1-Xaa2-Xaa3-Xaa4-Xaa5-Xaa6-E-V-W-C-(X₀₋₃)_{b}(SEQ ID NO: 23)

wherein
(X₀₋₃)ₐ is absence, or an arginine residue-glycine residue-asparagine residue, a glycine residue-asparagine residue, an aspartic acid residue, or an asparagine residue,
(X₀₋₃)_{b} is absence, or a threonine residue-tyrosine residue-histidine residue, or a threonine residue,
Xaa1 is an alanine residue,
Xaa2 is a tyrosine residue, a tryptophan residue, or a histidine residue,
Xaa3 is a histidine residue, a phenylalanine residue, a tyrosine residue, a tryptophan residue, an arginine residue, or a glycine residue,
Xaa4 is a lysine residue, an aspartic acid residue, or a glutamic acid residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, or an aspartic acid residue, or

   Formula 2-1: (X₀₋₃')ₐ-C-(Xaa1')-(Xaa2')-(Xaa3')-(Xaa4')-(Xaa5')-(Xaa6')-L-V-W-C- (X₀₋₃')_{b} (SEQ ID NO: 24)

   wherein
   (X₀₋₃')ₐ and (X₀₋₃')_{b} are the same as the above (X₀₋₃)ₐ and (X₀₋₃)_{b}, respectively,
   Xaa1', Xaa2', Xaa3', Xaa4', Xaa5', and Xaa6' are the same as the above Xaa1, Xaa2, Xaa3, Xaa4, Xaa5, and Xaa6, respectively

Such a peptide has ability to bind to an Fc region of a monoclonal antibody.

In the amino acid sequences represented by the above formulae 1-1 to 1-9 and formula 2-1, (X₀₋₃)ₐ is absence, or an arginine residue-glycine residue-asparagine residue, a glycine residue-asparagine residue, an aspartic acid residue, or an asparagine residue, and preferably absence, or an arginine residue-glycine residue-asparagine residue, an aspartic acid residue, or an asparagine residue.

In the amino acid sequences represented by the above formulae 1-1 to 1-9 and formula 2-1, Xaa2 is a tyrosine residue, a tryptophan residue, or a histidine residue, and preferably a tyrosine residue or a tryptophan residue.

In the amino acid sequences represented by the above formulae 1-1 to 1-9 and formula 2-1, Xaa3 is a histidine residue, a phenylalanine residue, a tyrosine residue, a tryptophan residue, an arginine residue, or a glycine residue, and preferably a histidine residue.

In the amino acid sequences represented by the above formulae 1-1 to 1-9 and formula 2-1, Xaa4 is a lysine residue, an aspartic acid residue, or a glutamic acid residue, and preferably a lysine residue.

In the amino acid sequences represented by the above formulae 1-1 to 1-9 and formula 2-1, Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and preferably a glycine residue, a threonine residue, or a leucine residue.

In the amino acid sequences represented by the above formulae 1-1 to 1-9 and formula 2-1, Xaa6 is a glutamine residue, a glutamic acid residue, an asparagine residue, or an aspartic acid residue, and more preferably a glutamine residue.

The peptide comprising any of the amino acid sequences represented by the above formulae 1-1 to 1-9 and formula 2-1 is preferably a peptide comprising an amino acid sequence selected from the group consisting of the following:
(1') RGNCAYHKGQIIWCTYH (SEQ ID NO: 46);
(2') RGNCAYHKGQIVWCTYH (SEQ ID NO: 47);
(3') RGNCAYHKGQVVWCTYH (SEQ ID NO: 48);
(4') RGNCAYHKGQAVWCTYH (SEQ ID NO: 49);
(5') RGNCAYHKGQLLWCTYH (SEQ ID NO: 50);
(6') RGNCAYHKGQLIWCTYH (SEQ ID NO: 51);
(7') DCAYHKGQIVWCT (SEQ ID NO: 52);
(8') DCAYHKGQVVWCT (SEQ ID NO: 53);
(9') DCAYHKGQAVWCT (SEQ ID NO: 54);
(10') RGNCAYHKSQIIWCTYH (SEQ ID NO: 55);
(11') RGNCAYHKNQIIWCTYH (SEQ ID NO: 56);
(12') RGNCAYHKDQIIWCTYH (SEQ ID NO: 57);
(13') RGNCAYHKQQIIWCTYH (SEQ ID NO: 58);
(14') RGNCAYHKEQIIWCTYH (SEQ ID NO: 59);
(15') RGNCAYHKFQIIWCTYH (SEQ ID NO: 60);
(16') RGNCAYHKYQIIWCTYH (SEQ ID NO: 61);
(17') RGNCAYHKWQIIWCTYH (SEQ ID NO: 62);
(18') RGNCAYHKHQIIWCTYH (SEQ ID NO: 63);
(19') RGNCAYHKTQIIWCTYH (SEQ ID NO: 64);
(20') RGNCAYHKLQIIWCTYH (SEQ ID NO: 65);
(21') CAYHKLQIVWC (SEQ ID NO: 66);
(22') CAYHKLQLIWC (SEQ ID NO: 67);
(23') CAYHKSQIVWC (SEQ ID NO: 68);
(24') RGNCAYHKGQLVFCTYH (SEQ ID NO: 69);
(25') RGNCAYHKGQQVWCTYH (SEQ ID NO: 70);
(26') RGNCAYHKGQEVWCTYH (SEQ ID NO: 71);
(27') CAYHKGQLVWC (SEQ ID NO: 72);
(28') RGNCAYHKAQLVWCTYH (SEQ ID NO: 73);
(29') RGNCAYHKVQLVWCTYH (SEQ ID NO: 74);
(30') RGNCAYHKLQLVWCTYH (SEQ ID NO: 75);
(31') RGNCAYHKIQLVWCTYH (SEQ ID NO: 76);
(32') RGNCAYHKSQLVWCTYH (SEQ ID NO: 77);
(33') RGNCAYHKTQLVWCTYH (SEQ ID NO: 78);
(34') RGNCAYHKNQLVWCTYH (SEQ ID NO: 79);
(35') RGNCAYHKDQLVWCTYH (SEQ ID NO: 80);
(36') RGNCAYHKQQLVWCTYH (SEQ ID NO: 81);
(37') RGNCAYHKEQLVWCTYH (SEQ ID NO: 82);
(38') RGNCAYHKFQLVWCTYH (SEQ ID NO: 83);
(39') RGNCAYHKRQLVWCTYH (SEQ ID NO: 84);
(40') RGNCAYHKHQLVWCTYH (SEQ ID NO: 85);
(41') RGNCAYHKWQLVWCTYH (SEQ ID NO: 86);
(42') RGNCAYHKYQLVWCTYH (SEQ ID NO: 87);
(43') RGNCAYFKGQLVWCTYH (SEQ ID NO: 88);
(44') RGNCAYYKGQLVWCTYH (SEQ ID NO: 89);
(45') RGNCAYWKGQLVWCTYH (SEQ ID NO: 90);
(46') RGNCAYRKGQLVWCTYH (SEQ ID NO: 91);
(47') RGNCAYGKGQLVWCTYH (SEQ ID NO: 92);
(48') DCAYHKGQLVWC (SEQ ID NO: 93);
(49') NCAYHKGQLVWC (SEQ ID NO: 94);
(50') CAYHKGQLVWCT (SEQ ID NO: 95);
(51') CAYHKSQLVWC (SEQ ID NO: 96);
(52') GNCAYHKGQIIWCTYH (SEQ ID NO: 99); and
(53') RGNCAYHEGQIIWCTYH (SEQ ID NO: 108).

It has been determined that a peptide comprising such an amino acid sequence has ability to bind to an Fc region of IgG.

At least two cysteine residues separated from each other in each amino acid sequence of the peptide can form a cyclic peptide through a disulfide bond. Alternatively, in the peptide, the sulfide groups in the two cysteine residues may be coupled with each other through a carbonyl group-containing linker represented by the following.

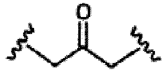

The broken line portions of the carbonyl group-containing linker represented by the above mean bond portions with the sulfide groups. The linker is more stable than a normal disulfide bond against a reduction reaction and the like. Such a peptide can be prepared by a method described in WO 2016/186206, for example.

The compound of the present invention comprising a peptide containing any of the above amino acid sequences is useful for regioselective modification of a specific amino acid residue (e.g., Lys248 residue, Lys246 residue, Lys288, Lys290, or Lys317 according to Eu numbering, or amino acid residues other than these residues) in human IgG Fc. An amino acid forming the peptide may each be an L-body or a D-body; an L-body is preferred (in Examples, the amino acid residues forming the peptides are all L-bodies).

The peptide may have a specific amino acid residue modified with a cross-linking agent. Examples of such a specific amino acid residue include a lysine residue, an aspartic acid residue, and a glutamic acid residue; preferred is a lysine residue. Examples of the cross-linking agent include cross-linking agents comprising preferably two or more succinimidyl groups such as disuccinimidyl glutarate (DSG) and disuccinimidyl suberate (DSS); cross-linking agents comprising preferably two or more imide acid portions such as dimethyl adipimidate·2HCl (DMA), dimethyl pimelimidate·2HCl (DMP), and dimethyl suberimidate·2HCl (DMS); and cross-linking agents having an SS bond such as dimethyl 3,3'-dithiobispropionimidate·2HCl (DTBP) and dithiobis(succinimidyl propionate) (DSP) (e.g., WO 2016/186206).

When the affinity substance to an antibody is a peptide, a terminal amino group and a terminal carboxy group of the peptide may be protected. Examples of a protecting group for the N-terminal amino group include an alkylcarbonyl group (an acyl group) (e.g., an acetyl group, a propoxy group, and a butoxycarbonyl group such as a tertbutoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group). The protecting group for the N-terminal amino group is preferably an acetyl group. Examples of a protecting group for the C-terminal carboxy group include a group capable of forming an ester or an amide. Examples of the group capable of forming an ester or an amide include an alkyloxy group (e.g., methyloxy, ethyloxy, propyloxy, butyloxy, pentyloxy, and hexyloxy), an aryloxy group (e.g., phenyloxy and naphthyloxy), an aralkyloxy group (e.g., benzyloxy), and an amino group. The protecting group for the C-terminal carboxy group is preferably an amino group.

### 1-3. Divalent Group Comprising Leaving Group (L)

In Formula (I), L is a divalent group comprising a leaving group.

The leaving group has ability to be cleaved and eliminated from E by a reaction between a nucleophilic group in an antibody and an electrophilic group contained in the electrophilic group-comprising divalent group (E). Such a leaving group is common technical knowledge in the field concerned (e.g., Fujishima, S. et al J. Am. Chem. Soc, 2012, 134, 3961-3964 (described above); Chem. Sci. 2015 3217-3224; Nature Chemistry volume 8, pages 542-548 (2016)). The leaving group is not particularly limited so long as it has ability to be cleaved from E by the above reaction, but examples thereof include (1) a group selected from the group consisting of -O-, -S-, -Se-, -SO₂-O-,- SO₂-N(R)-, - SO₂-, -C≡C-CH₂-O-, -N(OR)-, -N(R)-, and -O-N(R)- (where R is a hydrogen atom or C₁₋₆ alkyl), and (2) heteroarylene.

Examples of C₁₋₆ alkyl include methyl, ethyl, n-propyl, i-propyl, n-butyl, s-butyl, isobutyl, t-butyl, pentyl, and hexyl. C₁₋₆ alkyl is preferably C₁₋₄ alkyl.

The group selected from the group consisting of - O-, -S-, -Se-, -SO₂-O-,- SO₂-N(R) -, -SO₂-, -C≡C-CH₂-O-, - N(OR)-, -N(R)-, and -O-N(R)-, which are examples of the leaving group, is the following (1) or (2):
(1) a group consisting of -O-, -S-, -Se-, -SO₂-O-,-SO₂-N(R)-, -SO₂-, -C≡C-CH₂-O-, -N(OR)-, -N(R)-, or -O-N(R)-; or
(2) a group comprising -O-, -S-, -Se-, -SO₂-O-,- SO₂-N(R) -, -SO₂-, -C≡C-CH₂-O-, -N(OR)-, -N(R)-, or -O-N(R)- and a group that enhances ability thereof to be eliminated.

The group that enhances ability of -O-, -S-, -Se-, -SO₂-O-,- SO₂-N(R)-, -SO₂-, -C≡C-CH₂-O-, -N(OR)-, -N(R)-, or -O-N(R)- to be eliminated is a group that enhances ability of -O-, -S-, -Se-, -SO₂-O-,- SO₂-N(R)-, -SO₂-, -C≡C-CH₂-O-, -N(OR)-, -N(R)-, or -O-N(R)- to be eliminated from an electrophilic group when the group is present adjacent to - O-, -S-, -Se-, -SO₂-O-,- SO₂-N(R)-, -SO₂-, -C≡C-CH₂-O-, - N(OR)-, -N(R)-, or -O-N(R)- as compared to when the group is not present adjacent to -O-, -S-, -Se-, -SO₂-O-,- SO₂-N(R)-, -SO₂-, -C≡C-CH₂-O-, -N(OR)-, -N(R)-, or -O-N(R)-. In other words, the group that enhances ability of -O-, -S-, - Se-, -SO₂-O-, - SO₂-N(R)-, -SO₂-, -C≡C-CH₂-O-, -N(OR)-, - N(R)-, or -O-N(R)- to be eliminated is a group having ability to withdraw an electron of an oxygen atom, a sulfur atom, a selenium atom, or a nitrogen atom.

Preferred example of the group that enhances ability of -O-, -S-, -Se-, -SO₂-O-,- SO₂-N(R)-, -SO₂-, -C≡C-CH₂-O-, -N(OR)-, -N(R)-, or -O-N(R)- to be eliminated include an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone. The number of electron-withdrawing groups that may be comprised in each of the arylene and the heteroarylene is 1 or more (e.g., 1 to 3, preferably 1 or 2). Examples of the electron-withdrawing group include a halogen atom, a halogen atom-substituted alkyl (e.g., trifluoromethyl), a boronic acid residue, mesyl, tosyl, triflate, nitro, cyano, a phenyl group, and a keto group (e.g., acyl).

The "arylene" in the "arylene optionally substituted with an electron-withdrawing group" is preferably C₆₋₂₄ arylene, more preferably C₆₋₁₈ arylene, even more preferably C₆₋₁₄ arylene, and most preferably C₆₋₁₀ arylene. The arylene optionally substituted with an electron-withdrawing group may be further substituted or is not necessarily substituted with a substituent other than the electron-withdrawing group. The number of carbon atoms does not comprise the number of carbon atoms of the electron-withdrawing group and the other substituents. Examples of the arylene include phenylene, naphthylene, and anthracenylene.

The "heteroarylene" in the "heteroarylene optionally substituted with an electron-withdrawing group" is preferably C₁₋₂₁ heteroarylene, more preferably C₁₋₁₅ heteroarylene, even more preferably C₁₋₉ heteroarylene, and most preferably C₁₋₆ heteroarylene. The heteroarylene optionally substituted with an electron-withdrawing group may be further substituted or is not necessarily substituted with a substituent other than the electron-withdrawing group. The number of carbon atoms does not comprise the number of carbon atoms of the electron-withdrawing group and the other substituents. The heteroarylene comprises one or more (for example, 1 to 5, preferably 1 to 4, more preferably 1 to 3) heteroatoms selected from the group consisting of a nitrogen atom, an oxygen atom, and a sulfur atom as ring-constituting atoms. Examples of the heteroarylene include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The optionally condensed 2,5-diketopyrrolidine, the optionally condensed 2,6-diketopiperidine, the optionally condensed 2-ketopyrrolidine, the optionally condensed 2-ketopiperidine, and 2-pyridone may be substituted or are not necessarily substituted with a substituent such as an electron-withdrawing group.

The heteroarylene, which is an example of the leaving group, is a heteroarylene having a low π electron density (that is, less than 1). The heteroarylene, which is a leaving group, is preferably a heteroarylene containing a nitrogen atom as a ring-constituting atom. The heteroarylene containing a nitrogen atom as a ring-constituting atom is preferably C₁₋₂₁ heteroarylene containing a nitrogen atom as a ring-constituting atom, more preferably C₁₋₁₅ heteroarylene containing a nitrogen atom as a ring-constituting atom, and even more preferably C₁₋₉ heteroarylene containing a nitrogen atom as a ring-constituting atom. The heteroarylene, which is a leaving group, may be substituted or is not necessarily substituted with a substituent such as an electron-withdrawing group. The number of carbon atoms does not comprise the number of carbon atoms of the substituent. Examples of the heteroarylene which is a leaving group and contains a nitrogen atom as a ring-constituting atom include imidazolediyl, triazolediyl, tetrazolediyl, and 2-pyridonediyl (that is, 2-hydroxypyridinediyl).

A group such as the "arylene optionally substituted with an electron-withdrawing group", which is an example of the group that enhances ability of -O-, -S-, -Se-, -SO₂-O-,- SO₂-N(R)-, -SO₂-, -C≡C-CH₂-O-, -N(OR)-, - N(R)-, or -O-N(R)- to be eliminated, and the "heteroarylene", which is an example of the leaving group, may each have, for example, one to five, preferably one to three, more preferably one or two substituents, or do not each necessarily have a substituent. It is preferable not to have such a substituent in view of synthesizing a compound having a simple chemical structure. On the other hand, when such a group as described above has a substituent, examples of the substituent include the following:
(i) a halogen atom;
(ii) a monovalent hydrocarbon group;
(iii) aralkyl;
(iv) a monovalent heterocyclic group;
(v) Rₐ-O-, Rₐ-C(=O)-, Rₐ-O-C(=O)-, and Rₐ-C(=O)-O-wherein Rₐ indicates a hydrogen atom or a monovalent hydrocarbon group;
(vi) NR_{b}R_{c}-, NR_{b}R_{c}-C(=O)-, NR_{b}R_{c}-C(=O)-O-, and R_{b}-C (=O) -NR_{c}- wherein R_{b} and R_{c} are the same as or different from each other, and each indicate a hydrogen atom or a monovalent hydrocarbon group; and
(vii) a nitro group, a sulfuric acid group, a sulfonic acid group, a cyano group, and a carboxy group.

Examples of the halogen atom include a fluorine atom, a chlorine atom, a bromine atom, and an iodine atom.

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. Alkyl, alkenyl, and alkynyl may be linear or branched.

The alkyl is preferably C₁₋₁₂ alkyl, more preferably C₁₋₆ alkyl, and even more preferably C₁₋₄ alkyl. The number of carbon atoms does not comprise the number of carbon atoms of the substituent. Examples of C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C₂₋₁₂ alkenyl, more preferably C₂₋₆ alkenyl, and even more preferably C₂₋₄ alkenyl. The number of carbon atoms does not comprise the number of carbon atoms of the substituent. Examples of C₂₋₁₂ alkenyl include vinyl, propenyl, and n-butenyl.

The alkynyl is preferably C₂₋₁₂ alkynyl, more preferably C₂₋₆ alkynyl, and even more preferably C₂₋₄ alkynyl. The number of carbon atoms does not comprise the number of carbon atoms of the substituent. Examples of C₂₋₁₂ alkynyl include ethynyl, propynyl, and n-butynyl.

The monovalent chain hydrocarbon group is preferably alkyl.

The monovalent alicyclic hydrocarbon group means a hydrocarbon group comprising only alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

Cycloalkyl is preferably C₃₋₁₂ cycloalkyl, more preferably C₃₋₆ cycloalkyl, and even more preferably C₅₋₆ cycloalkyl. The number of carbon atoms does not comprise the number of carbon atoms of the substituent. Examples of C₃₋₁₂ cycloalkyl include cyclopropyl, cyclobutyl, cyclopentyl, and cyclohexyl.

The cycloalkenyl is preferably C₃₋₁₂ cycloalkenyl, more preferably C₃₋₆ cycloalkenyl, and even more preferably C₅₋₆ cycloalkenyl. The number of carbon atoms does not comprise the number of carbon atoms of the substituent. Examples of C₃₋₁₂ cycloalkenyl include cyclopropenyl, cyclobutenyl, cyclopentenyl, and cyclohexenyl.

The cycloalkynyl is preferably C₃₋₁₂ cycloalkynyl, more preferably C₃₋₆ cycloalkynyl, and even more preferably C₅₋₆ cycloalkynyl. The number of carbon atoms does not comprise the number of carbon atoms of the substituent. Examples of C₃₋₁₂ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C₆₋₁₂ aryl, more preferably C₆₋₁₀ aryl, and even more preferably C₆ aryl. The number of carbon atoms does not comprise the number of carbon atoms of the substituent. Examples of C₆₋₁₂ aryl include phenyl and naphthyl.

The monovalent aromatic hydrocarbon group is preferably phenyl.

Among these, the monovalent hydrocarbon group is preferably alkyl, cycloalkyl, and aryl and more preferably alkyl.

Aralkyl refers to arylalkyl. The definitions, examples, and preferred examples of aryl and alkyl in arylalkyl are as described above. The aralkyl is preferably C₃₋₁₅ aralkyl. Examples of such an aralkyl include benzoyl, phenethyl, naphthylmethyl, and naphthylethyl.

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom contained in the heterocyclic group.

The monovalent aromatic heterocyclic group is preferably a C₁₋₁₅ aromatic heterocyclic group, more preferably a C₁₋₉ aromatic heterocyclic group, and even more preferably a C₁₋₆ aromatic heterocyclic group. The number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and even more preferably a C₂₋₆ nonaromatic heterocyclic group. The number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these, the monovalent heterocyclic group is preferably a five-membered or six-membered heterocyclic group.

The substituent may be preferably the following:
(i') a halogen atom;
(ii') C₁₋₁₂ alkyl, phenyl, or naphthyl;
(iii') C₃₋₁₅ aralkyl;
(iv') a five-membered or six-membered heterocyclic group;
(v') Rₐ-O-, Rₐ-C(=O)-, Rₐ-O-C(=O)-, or Rₐ-C(=O)-O-wherein Rₐ indicates a hydrogen atom or C₁₋₁₂ alkyl;
(vi') NR_{b}R_{c}-, NR_{b}R_{c}-C (=O) -, NR_{b}R_{c}-C (=O) -O-, or R_{b}-C (=O) - NR_{c}- wherein R_{b} and R_{c} are the same as or different from each other, and each indicate a hydrogen atom or C₁₋₁₂ alkyl; or
(vii') the same groups as those enumerated in (vii).

The substituent may be more preferably the following:
(i") a halogen atom;
(ii") C₁₋₁₂ alkyl;
(iii'') Rₐ-O-, Rₐ-C(=O)-, Rₐ-O-C(=O)-, or Rₐ-C(=O)-O-(wherein Rₐ indicates a hydrogen atom or C₁₋₁₂ alkyl);
(iv") NR_{b}R_{c}-, NR_{b}R_{c}-C (=O) -, NR_{b}R_{c}-C (=O) -O-, or R_{b}-C(=O)-NR_{c}- wherein R_{b} and R_{c} are the same as or different from each other, and each indicate a hydrogen atom or C₁₋₁₂ alkyl; or
(v") the same groups as those enumerated in (vii).

The substituent may be even more preferably the following:
(i"') a halogen atom;
(ii"') C₁₋₆ alkyl;
(iii''') Rₐ-O-, Rₐ-C(=O)-, Rₐ-O-C(=O)-, or Rₐ-C(=O) -O-(wherein Rₐ indicates a hydrogen atom or C₁₋₆ alkyl);
(iv''') NR_{b}R_{c}-, NR_{b}R_{c}-C (=O) -, NR_{b}R_{c}-C (=O) -O-, or R_{b}-C(=O)-NR_{c}- wherein R_{b} and R_{c} are the same as or different from each other, and each indicate a hydrogen atom or C₁₋₆ alkyl; or
(v''') the same groups as those enumerated in (vii).

The substituent may be particularly preferably the following:
(i'''') a halogen atom;
(ii"") C₁₋₄ alkyl;
(iii'''') Rₐ-O-, Rₐ-C(=O) -, Rₐ-O-C (=O) -, or Rₐ-C(=O) -O-(wherein Rₐ indicates a hydrogen atom or C₁₋₄ alkyl);
(iv'''') NR_{b}R_{c}-, NR_{b}R_{c}-C(=O)-, NR_{b}R_{c}-C(=O) -O-, or R_{b}-C(=O)-NR_{c}- wherein R_{b} and R_{c} are the same as or different from each other, and each indicate a hydrogen atom or C₁₋₄ alkyl; or
(v'''') the same groups as those enumerated in (vii).

More specifically, the leaving group may be any of the following (a) to (c).
(a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
(b) heteroarylene, or
(c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)] .

As the leaving group, (a) or (b) is preferable among (a), (b), and (c), and (a) is particularly preferable. Ring P in (a) is any of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, or 2-pyridone. Among these, an arylene substituted with an electron-withdrawing group, a heteroarylene substituted with an electron-withdrawing group, 2,5-diketopyrrolidine, and 2,6-diketopiperidine are more preferable, and an arylene substituted with an electron-withdrawing group, 2,5-diketopyrrolidine, and 2,6-diketopiperidine are even more preferable.

Ring P in (a) is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone. The details of these groups are the same as those described as preferred examples of the group that enhances ability of - N(R)-, -N(OR)-, -O-, -S-, or -Se- to be eliminated.

Q in (a) is selected from the group consisting of -O-, -S-, -Se-, -SO₂-O-, -SO₂-N(R)-, -SO₂-, -C≡C-CH₂-O-, - N(OR)-, -N(R)-, and -O-N(R)- (where R is a hydrogen atom or C₁₋₆ alkyl) . Among these, Q is preferably -O-, -S-, -SO₂-O-, or -SO₂-N(R)-, more preferably -O- or -S-, and even more preferably -O-.

(b) is a heteroarylene, and is preferably imidazolediyl, triazolediyl, tetrazolediyl, or 2-pyridonediyl (= 2-hydroxypyridinediyl), and more preferably imidazolediyl or 2-pyridonediyl.

Q in (c) is selected from the group consisting of -O-, -S-, -Se-, -SO₂-O-, -SO₂-N(R)-, -SO₂-, -C≡C-CH₂-O-, - N(OR)-, -N(R)-, and -O-N(R)- (where R is a hydrogen atom or C₁₋₆ alkyl) . Q is preferably -S-, -C≡C-CH₂-O-, -N(OR)-, - N(R)-, or -O-N(R)-, and more preferably S, -N(OR), or -ON(R)-.

More specifically, the preferable leaving group may be selected from the group consisting of the following structural formulae. 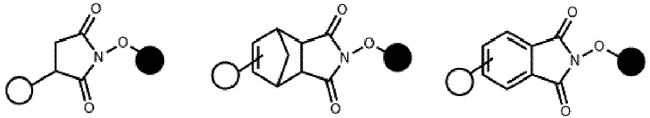 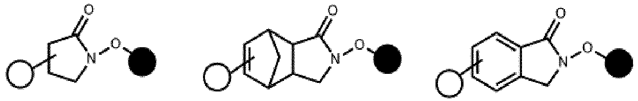 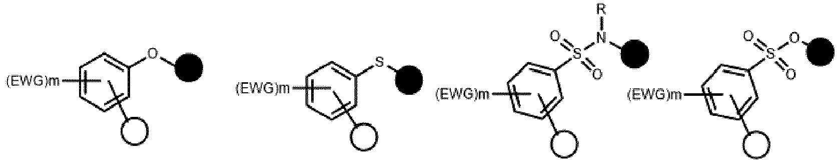 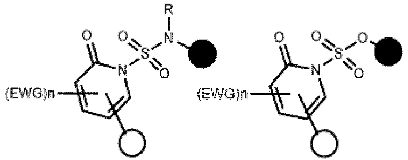 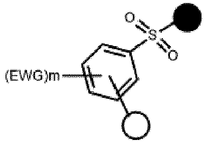 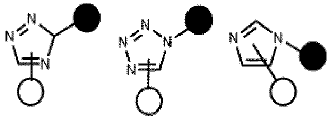 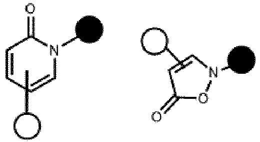 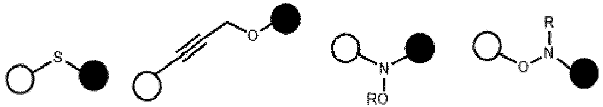
(where EWG is an electron-withdrawing group,
m is an integer of 0 to 4,
n is an integer of 0 to 3,
R is a hydrogen atom or C₁₋₆ alkyl,
a symbol of "white circle" is a bond to L₁, and a symbol of "black circle" is a bond to E₁).

The details of the electron-withdrawing group and C₁₋₆ alkyl are as described above. m is preferably an integer of 1 to 4, and more preferably 1, 2, or 3. n is preferably an integer of 1 to 3, and more preferably 1 or 2.

The divalent group comprising a leaving group, represented by L is a divalent group consisting of the above leaving group, or a divalent group comprising another divalent group in addition to the above leaving group. Examples of such another divalent group include a divalent hydrocarbon group, a divalent heterocyclic group, -C(=O)-, -NR_{L}- (R_{L} indicates a hydrogen atom or the above-described substituent), -O-, -S-, -C(=S)-, and a group consisting of a combination of two or more (e.g., two to eight, preferably two to six, and more preferably two to four) of these. The divalent hydrocarbon group and the divalent heterocyclic group may each have, for example, one to five, preferably one to three, more preferably one or two substituents, or do not each necessarily have a substituent. It is preferable not to have such a substituent in view of synthesizing a compound having a simple chemical structure. On the other hand, when the above group has a substituent, examples and preferred examples of the substituent are the same as those described above.

The divalent hydrocarbon group is a linear, branched, or cyclic divalent hydrocarbon group and preferably a linear or branched divalent hydrocarbon group. Examples of the divalent hydrocarbon group include alkylene, alkenylene, alkynylene, and arylene.

The alkylene is preferably C₁₋₁₂ alkylene, more preferably C₁₋₆ alkylene, and particularly preferably C₁₋₄ alkylene. The number of carbon atoms does not comprise the number of carbon atoms of the substituent. Alkylene may be any of linear, branched, or cyclic one and is preferably linear alkylene. Examples of such an alkylene include methylene, ethylene, propylene, butylene, pentylene, and hexylene.

The alkenylene is preferably C₂₋₁₂ alkenylene, more preferably C₂₋₆ alkenylene, and particularly preferably C₂₋₄ alkenylene. The number of carbon atoms does not comprise the number of carbon atoms of the substituent. Alkenylene may be any of linear, branched, or cyclic one and is preferably linear alkenylene. Examples of such an alkenylene include ethylenylene, propynylene, butenylene, pentenylene, and hexenylene.

The alkynylene is preferably C₂₋₁₂ alkynylene, more preferably C₂₋₆ alkynylene, and particularly preferably C₂₋₄ alkynylene. The number of carbon atoms does not comprise the number of carbon atoms of the substituent. Alkynylene may be any of linear, branched, or cyclic one and is preferably linear alkynylene. Examples of such an alkynylene include ethynylene, propynylene, butynylene, pentynylene, and hexynylene.

The arylene is preferably C₆₋₂₄ arylene, more preferably C₆₋₁₈ arylene, even more preferably C₆₋₁₄ arylene, and still even more preferably C₆₋₁₀ arylene. The number of carbon atoms does not comprise the number of carbon atoms of the substituent. Examples of the arylene include phenylene, naphthylene, and anthracenylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₁₋₂₁ divalent aromatic heterocyclic group, more preferably a C₁₋₁₅ divalent aromatic heterocyclic group, even more preferably a C₁₋₉ divalent aromatic heterocyclic group, and still even more preferably a C₁₋₆ divalent aromatic heterocyclic group. The number of carbon atoms does not comprise the number of carbon atoms of the substituent. More specific examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₂₋₂₁ nonaromatic heterocyclic group, more preferably a C₂₋₁₅ nonaromatic heterocyclic group, even more preferably a C₂₋₉ nonaromatic heterocyclic group, and still even more preferably a C₂₋₆ nonaromatic heterocyclic group. The number of carbon atoms does not comprise the number of carbon atoms of the substituent. More specifically, examples of the divalent nonaromatic heterocyclic group include pyrroldionediyl, pyrrolinedionediyl, pyrrolinediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

In a specific embodiment, L can be expressed by L₁-L₂.

L₁ is a bond or a divalent group. The definitions, examples, and preferred examples of the divalent group represented by L₁ are the same as those of another divalent group when the divalent group comprising a leaving group, represented by L is a divalent group comprising another divalent group in addition to the leaving group.

L₂ is a leaving group. The definitions, examples, and preferred examples of the leaving group represented by L₂ are the same as those of the leaving group in L.

The leaving group represented by L₂ is preferably any of the above (a) to (c). Examples and preferred examples of the leaving group represented by L₂ are also the same as those described in the above (a) to (c).

The length of a main chain of L (divalent group comprising a leaving group) or L₁ (bond or a divalent group)-L₂ (leaving group) that couples A (affinity substance) with E (divalent group comprising an electrophilic group) can be designed as appropriate according to various factors such as the types of an antibody and an affinity substance, and a relationship between a target site of the affinity substance in the antibody and a specific amino acid residue in the antibody to be regioselectively modified. The main chain of L or L₁-L₂ refers to a chain structure coupling A with E and consisting of a plurality of atoms covalently binding to each other, and excludes a hydrogen atom, a branched structure portion, and a substituent. When a compound represented by Formula (I) is brought into contact with an antibody, A first associates with the antibody. Next, a nucleophilic group in a side chain of a specific amino acid residue to be modified in the antibody (for example, an amino group in a side chain of a lysine residue) present near an antibody association site reacts with an electrophilic group in E. As a result, the leaving group contained in L or L₁ can be eliminated from E while the nucleophilic group binds to the electrophile. At this time, when there is no other amino acid residue of the same type as a specific amino acid residue to be modified near a space between the antibody association site and the specific amino acid residue, the electrophilic group in E can regioselectively bind to a nucleophilic group in the side chain of the specific amino acid residue to be modified in the antibody without strictly controlling the length of the main chain. It is understood that even when another specific amino acid residue of the same type as the specific amino acid residue is present in such regions, the electrophilic group in E can regioselectively bind to the specific amino acid residue by controlling the length of the main chain.

The length of the main chain of L or L₁-L₂ coupling A with E is not particularly limited so long as it can regioselectively modify a specific amino acid residue in an antibody. For example, when a specific amino acid residue in human IgG Fc (e.g., Lys248 residue, Lys246 residue, Lys288, Lys290, or Lys317 according to Eu numbering, or amino acid residues other than these residues) is regioselectively modified, the length preferably consists of 20 or fewer atoms. The length of the main chain of L or L₁-L₂ may be preferably 1 or more, more preferably 2 or more, even more preferably 3 or more, and particularly preferably 4 or more or 5 or more. The length of the main chain of L or L₁-L₂ may be preferably 50 or less, more preferably 30 or less, even more preferably 20 or less, and particularly preferably 15 or less or 10 or less. More specifically, the length of the main chain of L or L₁-L₂ may be preferably 1 to 50, more preferably 1 to 30, even more preferably 1 to 20, and particularly preferably 1 to 15 or 1 to 10. Alternatively, the length of the main chain of L or L₁-L₂ may be preferably 2 to 30, even more preferably 3 to 20, and particularly preferably 4 to 15 or 5 to 10.

When the main chain has a structure comprising no cyclic structure, the number of atoms of the main chain can be determined by counting the number of atoms in a chain structure (excluding the number of hydrogen atoms, and the number of atoms of branched structure portions and substituents).

On the other hand, when the main chain has a structure containing a cyclic structure, in view of defining the length of the main chain, the number of atoms of the main chain can be counted for convenience's sake. Specifically, the number of atoms of the main chain in such a case can be determined by counting the number of atoms of the shortest route connecting two bonds in the cyclic structure in addition to the number of atoms in a chain structure comprising no divalent cyclic structure in the main chain (excluding the number of hydrogen atoms, and the number of atoms of branched structure portions and substituents) (e.g., refer to the (a) to (d) thick routes below). 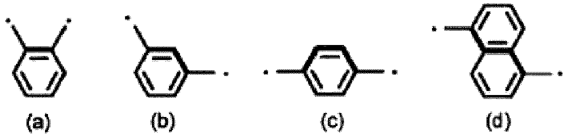 . is a bond.

In the case of (a), the shortest route is the thick route, and thus the number of atoms in the divalent cyclic structure counted as the number of atoms of the main chain is two.

In the case of (b), the shortest route is the thick route, and thus the number of atoms in the divalent cyclic structure counted as the number of atoms of the main chain is three.

In the case of (c), any route is the shortest route (the same distance), and thus the number of atoms in the divalent cyclic structure counted as the number of atoms of the main chain is four.

In the case of (d), the route of the condensed site is the shortest route, and thus the number of atoms in the divalent cyclic structure counted as the number of atoms of the main chain is four.

The main chain of L or L₁-L₂ may be preferably a chain structure in which the "other divalent group" in L or the "divalent group" in L₁ does not comprise a divalent cyclic structure. Consequently, the "other divalent group" in L or the "divalent group" in L₁ may be a divalent linear or branched hydrocarbon group, -C(=O)-, -NR_{L}- (R_{L} indicates a hydrogen atom or the above-described substituent), -O-, - S-, -C(=S)-, or a group consisting of a combination of two or more (e.g., two to eight, preferably two to six, and more preferably two to four) of these. The divalent linear or branched hydrocarbon group may have, for example, one to five, preferably one to three, more preferably one or two substituents, or does not necessarily have a substituent. It is preferable not to have such a substituent in view of synthesizing a compound having a simple chemical structure. On the other hand, when the group as described above has a substituent, examples and preferred examples of such a substituent are the same as those of the substituent which may be comprised in the heteroarylene, which is an example of the leaving group.

### 1-4. Divalent Group Comprising Electrophilic Group (E)

In Formula (I), L is a divalent group comprising an electrophilic group (i) coupled with the leaving group and (ii) having ability to react with a nucleophilic group in the antibody.

Examples of the nucleophilic group in an antibody include NH₂ in a side chain of a lysine residue, OH in a side chain of a tyrosine residue, OH in a side chain of a serine residue, OH in a side chain of a threonine residue, and SH in a side chain of a cysteine residue. The nucleophilic group in an antibody is preferably NH₂ in a side chain of a lysine residue or OH in a side chain of a tyrosine residue, and more preferably NH₂ in a side chain of a lysine residue.

The electrophilic group comprised in E can be any electrophilic group capable of being coupled with a leaving group and having ability to react with the nucleophilic group in an antibody as described above. A group selected from the group consisting of -C(=O)-, -SO₂-, and -CH₂- is preferable. As the electrophilic group, -CH₂- can also be used depending on an electronic balance with an adjacent group (e.g., leaving group, L₂, or E₂). For example, when a tosyl group is used as the leaving group, -CH₂- can be suitably used as the electrophilic group (Tsukiji et al., Nature Chemical Biology, Vol. 5, No. 5, May 2009). The electrophilic group is more preferably -C(=O)- or -SO₂-, and even more preferably -C(=O)-.

The divalent group comprising an electrophilic group, represented by E is a divalent group consisting of the above electrophilic group, or a divalent group comprising another divalent group in addition to the above electrophilic group. Examples of such another divalent group include a divalent hydrocarbon group, a divalent heterocyclic group, -C(=O)-, -NR_{E}- (R_{E} indicates a hydrogen atom or the above-described substituent), -O-, -S-, -C(=S)-, and a group consisting of a combination of two or more (e.g., two to eight, preferably two to six, and more preferably two to four) of these. The divalent hydrocarbon group and the divalent heterocyclic group may each have, for example, one to five, preferably one to three, more preferably one or two substituents, or do not each necessarily have a substituent. It is preferable not to have such a substituent in view of synthesizing a compound having a simple chemical structure. On the other hand, when the group as described above has a substituent, examples and preferred examples of such a substituent are the same as those of the substituent which may be comprised in the heteroarylene, which is an example of the leaving group. E (and E₁-E₂-E₃ described below) can be designed so as to be free of a peptide portion having a problem of potential immunogenicity and being easily hydrolyzed in the blood.

The divalent hydrocarbon group is a linear, branched, or cyclic divalent hydrocarbon group and preferably a linear or branched divalent hydrocarbon group. Examples of the divalent hydrocarbon group include alkylene, alkenylene, alkynylene, and arylene.

The alkylene is preferably C1-12 alkylene, more preferably C₁₋₆ alkylene, and particularly preferably C₁₋₄ alkylene. The number of carbon atoms does not comprise the number of carbon atoms of the substituent. Alkylene may be any of linear, branched, or cyclic one and is preferably linear alkylene. Examples of such an alkylene include methylene, ethylene, propylene, butylene, pentylene, and hexylene.

The alkenylene is preferably C2-12 alkenylene, more preferably C2-6 alkenylene, and particularly preferably C2-4 alkenylene. The number of carbon atoms does not comprise the number of carbon atoms of the substituent. Alkenylene may be any of linear, branched, or cyclic one and is preferably linear alkenylene. Examples of such an alkenylene include ethylenylene, propynylene, butenylene, pentenylene, and hexenylene.

The alkynylene is preferably C2-12 alkynylene, more preferably C2-6 alkynylene, and particularly preferably C2-4 alkynylene. The number of carbon atoms does not comprise the number of carbon atoms of the substituent. Alkynylene may be any of linear, branched, or cyclic one and is preferably linear alkynylene. Examples of such an alkynylene include ethynylene, propynylene, butynylene, pentynylene, and hexynylene.

The arylene is preferably C6-24 arylene, more preferably C6-18 arylene, even more preferably C6-14 arylene, and still even more preferably C6-10 arylene. The number of carbon atoms does not comprise the number of carbon atoms of the substituent. Examples of the arylene include phenylene, naphthylene, and anthracenylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C1-21 divalent aromatic heterocyclic group, more preferably a C1-15 divalent aromatic heterocyclic group, even more preferably a C1-9 divalent aromatic heterocyclic group, and still even more preferably a C1-6 divalent aromatic heterocyclic group. The number of carbon atoms does not comprise the number of carbon atoms of the substituent. More specific examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C2-21 nonaromatic heterocyclic group, more preferably a C2-15 nonaromatic heterocyclic group, even more preferably a C2-9 nonaromatic heterocyclic group, and still even more preferably a C2-6 nonaromatic heterocyclic group. The number of carbon atoms does not comprise the number of carbon atoms of the substituent. More specifically, examples of the divalent nonaromatic heterocyclic group include pyrroldionediyl, pyrrolinedionediyl, pyrrolinediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

In a specific embodiment, E can be expressed by E₁-E₂-E₃.

In Formula (I), E₁ is an electrophilic group (i) coupled with the leaving group and (ii) having ability to react with a nucleophilic group in the antibody. The definitions, examples, and preferred examples of the electrophilic group of E₁ are the same as those of the electrophilic group in E.

E₂ is (a) or (b) below:
E₂ is a group represented by
(a) -X-Y-
   wherein X which binds to E₁ is C(R₁) (R₂) where R₁ and R₂ are each independently a hydrogen atom or C₁₋₆ alkyl, N(R₃) where R₃ is a hydrogen atom or C₁₋₆ alkyl, O, S, or Se, and
   Y which binds to E₃ is C(R₄) (R₅) where R₄ and R₅ are each independently a hydrogen atom or C₁₋₆ alkyl; or
(b) the following formula (i): 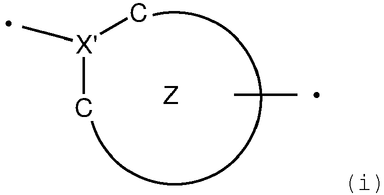 wherein the ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and · is a bond).

When E₂ is the above (a), C₁₋₆ alkyl in R₁ to R₅ is the same as that described above.

When E₂ is the above (a), X which binds to E₁ is preferably C(R₁) (R₂), N(R₃), O, S, or Se, more preferably C(R₁) (R₂), N(R₃), O, or S, even more preferably C(R₁) (R₂) , N(R₃), or O, still even more preferably C(R₁) (R₂) or N(R₃), and most preferably C(R₁) (R₂) in view of improving the reactivity and stability of the compound of the present invention.

X in E₂ can be defined in relation to a leaving group (e.g., refer to L and L₂). When X is an atom or a group such as N(R₃), O, S, or Se, not only the leaving group but X can also be eliminated from the electrophilic group. However, when a predetermined amount of a compound represented by Formula (I) is used for a reaction with an antibody, the reaction can be controlled such that X in at least part of the compound represented by Formula (I) is not eliminated from the electrophilic group. Therefore, in the present invention, an atom or a group as described above can be used as X. X can be preferably an atom or a group that is less likely to be eliminated than the leaving group (that is, an atom or a group having a pKa value larger than a pKa value of the leaving group) in view of improving the efficiency of a target reaction between the compound represented by Formula (I) and an antibody, and further improving the yield of an antibody having a bioorthogonal functional group or bioorthogonal functional groups. Consequently, X in E₂ preferably has ability to be eliminated equal to or less than the leaving group in view of more selectively eliminating the leaving group and suppressing the elimination of X in E₂ to improve the efficiency of a target reaction between the compound represented by Formula (I) and an antibody and to further improve the yield of an antibody having a bioorthogonal functional group or bioorthogonal functional groups. Such X may vary depending on the type of leaving group (e.g., - N(R)-, -N(OR)-, -O-, -S-, -Se-, or heteroarylene), presence or absence of a group comprising a group that enhances ability of a leaving group present adjacent to the leaving group to be eliminated, and the type thereof, but the outline of X is as follows.
(1) When the leaving group is -N(R)- or -N(OR)-, X is preferably C(R₁) (R₂) or N(R₃), and more preferably C(R₁) (R₂) .
(2) When the leaving group is -O-, X is preferably C(R₁) (R₂), N(R₃), or O, more preferably C(R₁) (R₂) or N(R₃), and even more preferably C(R₁) (R₂) .
(3) When the leaving group is -S-, X is preferably C(R₁) (R₂), N(R₃), O, or S, more preferably C(R₁) (R₂), N(R₃), or O, even more preferably C(R₁) (R₂) or N(R₃), and still even more preferably C(R₁) (R₂) .
(4) When the leaving group is -Se-, X is preferably C(R₁) (R₂), N(R₃), O, S, or Se, more preferably C(R₁) (R₂) , N(R₃), O, or S, even more preferably C(R₁) (R₂), N(R₃), or O, still even more preferably C(R₁) (R₂) or N(R₃), and most preferably C(R₁) (R₂) .
(5) When the leaving group is heteroarylene, X is preferably C(R₁) (R₂), N(R₃), O, S, or Se, more preferably C(R₁) (R₂), N(R₃), O, or S, even more preferably C(R₁) (R₂) , N(R₃), or O, still even more preferably C(R₁) (R₂) or N(R₃), and most preferably C(R₁) (R₂) .

When E₂ is the above (b), the ring-constituting atom X' which binds to E₁ comprised in the ring Z is a carbon atom or a nitrogen atom.

When the ring-constituting atom X' which binds to E₁ is a carbon atom, the ring Z is a divalent cyclic group in which all of the ring-constituting atom X' which binds to E₁ and ring-constituting atoms on both sides thereof are carbon atoms. The divalent cyclic group may have e.g., one to five, preferably one to three, and more preferably one or two substituents, or does not necessarily have a substituent. It is preferable not to have such a substituent in view of synthesizing a compound having a simple chemical structure. On the other hand, when the divalent cyclic group has a substituent, examples and preferred examples of such a substituent are the same as those of the substituent which may be comprised in the heteroarylene, which is an example of the leaving group. Examples of such a divalent cyclic group include a cyclic divalent hydrocarbon group (e.g., arylene, cyclic alkylene, cyclic alkenylene, or cyclic alkynylene), and a divalent heterocyclic group (e.g., a divalent aromatic heterocyclic group or a divalent nonaromatic heterocyclic group).

The divalent hydrocarbon group is a cyclic divalent hydrocarbon group, and examples of the divalent hydrocarbon group include cyclic alkylene, cyclic alkenylene, cyclic alkynylene, and arylene.

The cyclic alkylene is preferably C₃₋₁₂ alkylene, more preferably C₃₋₁₀ alkylene, and particularly preferably C₅₋₈ alkylene. The number of carbon atoms does not comprise the number of carbon atoms of the substituent. Examples of such an alkylene include cyclopropylene, cyclobutylene, cyclopentylene, cyclohexylene, cycloheptylene, cyclooctylene, cyclononylene, and cyclodekylene.

The cyclic alkenylene is preferably C₃₋₁₂ alkenylene, more preferably C₃₋₁₀ alkenylene, and particularly preferably C₅₋₈ alkenylene. The number of carbon atoms does not comprise the number of carbon atoms of the substituent. Examples of such an alkenylene include cyclopropenylene, cyclobutenylene, cyclopentenylene, cyclohexenylene, cycloheptenylene, cyclooctenylene, cyclononenylene, and cyclodekenylene.

The cyclic alkynylene is preferably C₆₋₁₂ alkynylene, more preferably C₇₋₁₂ alkynylene, and particularly preferably C₈₋₁₂ alkynylene. The number of carbon atoms does not comprise the number of carbon atoms of the substituent. Alkynylene may be any of linear, branched, or cyclic one and is preferably linear alkynylene. Examples of such an alkynylene include cyclohexynylene, cycloheptynylene, cyclooctynylene, cyclononynylene, cyclodekynylene, cycloundekynylene, and cyclododekynylene.

The arylene is preferably C6-24 arylene, more preferably C6-18 arylene, even more preferably C6-14 arylene, and still even more preferably C6-10 arylene. The number of carbon atoms does not comprise the number of carbon atoms of the substituent. Examples of the arylene include phenylene, naphthylene, and anthracenylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₃₋₂₁ divalent aromatic heterocyclic group, more preferably a C₃₋₁₅ divalent aromatic heterocyclic group, even more preferably a C₃₋₉ divalent aromatic heterocyclic group, and still even more preferably a C₃₋₆ divalent aromatic heterocyclic group. The number of carbon atoms does not comprise the number of carbon atoms of the substituent. More specifically, examples of the divalent aromatic heterocyclic group include pyrrolediyl, furandiyl, thiophenediyl, pyridinediyl, pyridazinediyl, pyrimidinediyl, pyrazolediyl, isothiazolediyl, isoxazolediyl, indolediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₂₁ nonaromatic heterocyclic group, more preferably a C₃₋₁₅ nonaromatic heterocyclic group, even more preferably a C₃₋₉ nonaromatic heterocyclic group, and still even more preferably a C₃₋₆ nonaromatic heterocyclic group. The number of carbon atoms does not comprise the number of carbon atoms of the substituent. More specifically, examples of the divalent nonaromatic heterocyclic group include pyrrolinedionediyl, pyrrolinediyl, azetidinediyl, oxetanediyl, thietanediyl, pyrrolidinediyl, dihydrofurandiyl, tetrahydrofurandiyl, tetrahydrothiophenediyl, pyrrolinediyl, piperidinediyl, dihydropyrandiyl, tetrahydropyrandiyl, tetrahydrothiopyrandiyl, piperazinediyl, dihydrooxazinediyl, tetrahydrooxazinediyl, dihydropyrimidinediyl, and tetrahydropyrimidinediyl.

When the ring-constituting atom X' which binds to E₁ is a nitrogen atom, the ring Z is a divalent heterocyclic group in which the ring-constituting atom X' which binds to E₁ is a nitrogen atom, and ring-constituting atoms on both sides of the nitrogen atom are carbon atoms. Such a divalent heterocyclic group is a divalent heterocyclic group comprising a nitrogen atom as a ring-constituting atom. The divalent aromatic heterocyclic group comprising a nitrogen atom as a ring-constituting atom is preferably a C₃₋₂₁ divalent heterocyclic group, more preferably a C₃₋₁₅ divalent heterocyclic group, even more preferably a C₃₋₉ divalent heterocyclic group, and still even more preferably a C₃₋₆ divalent heterocyclic group. The divalent heterocyclic group may have e.g., one to five, preferably one to three, and more preferably one or two substituents, or does not necessarily have a substituent. It is preferable not to have such a substituent in view of synthesizing a compound having a simple chemical structure. On the other hand, when the divalent heterocyclic group has a substituent, examples and preferred examples of such a substituent are the same as those of the substituent which may be comprised in the heteroarylene, which is an example of the leaving group. The number of carbon atoms does not comprise the number of carbon atoms of the substituent. Examples of the divalent heterocyclic group comprising a nitrogen atom as a ring-constituting atom include a divalent aromatic heterocyclic group comprising a nitrogen atom as a ring-constituting atom and a divalent nonaromatic heterocyclic group comprising a nitrogen atom as a ring-constituting atom. Examples of the divalent aromatic heterocyclic group comprising a nitrogen atom as a ring-constituting atom include pyrrolediyl, imidazolediyl, indolediyl, purinediyl, and carbazolediyl. Examples of the divalent nonaromatic heterocyclic group comprising a nitrogen atom as a ring-constituting atom include pyrroledionediyl, pyrrolinedionediyl, pyrrolinediyl, aziridinediyl, azetidinediyl, pyrrolidinediyl, pyrrolinediyl, imidazolidinediyl, piperidinediyl, morpholinediyl, thiomorpholinediyl, piperazinediyl, dihydropyrimidinediyl, and tetrahydropyrimidinediyl.

(b) The group represented by the above formula (i) is preferably (b') a group represented by the following formula (i'): 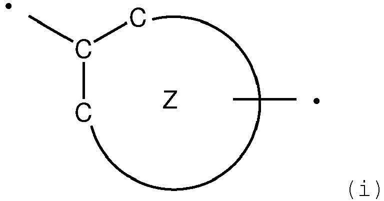 (where the ring Z is a divalent cyclic group in which all of a ring-constituting atom which binds to E₁ and ring-constituting atoms on both sides thereof are carbon atoms. · is a bond). The definitions, examples, and preferred examples of the divalent cyclic group for the ring Z in the group represented by the above Formula (i') are the same as those of the divalent cyclic group for the ring Z in the group represented by the above Formula (i).

E₃ is a divalent group when E₂ is -X-Y-, and is a bond or a divalent group when E₂ is a group represented by Formula (i). The divalent group represented by E₃ is the same as another divalent group when the divalent group comprising an electrophilic group, represented by E is a divalent group comprising another divalent group in addition to the electrophilic group.

The length of a main chain of E (divalent group comprising an electrophilic group) or E₁ (electrophilic group) - E₂ (the above (a) or (b)) - E₃ (bond or divalent group), coupling L (divalent group comprising a leaving group) with B (bioorthogonal functional group) cannot be involved in regioselective modification of the position of a specific amino acid residue in an antibody in a reaction between the compound represented by Formula (I) and the antibody, but can be involved in a distance between an antibody and a bioorthogonal functional group in the antibody having the bioorthogonal functional group, formed after the reaction. The main chain of E or E₁ - E₂ - E₃ refers to a chain structure coupling L with B and consisting of a plurality of atoms covalently binding to each other, and excludes a hydrogen atom, a branched structure portion, and a substituent. Consequently, the length of the main chain of E or E₁ - E₂ - E₃ can be designed as appropriate in view of adjusting the distance.

The length of the main chain of E or E₁ - E₂ - E₃ coupling L with B is not particularly limited, but may be a length consisting of three or more atoms.

When the main chain has a structure comprising no cyclic structure, the number of atoms of the main chain can be determined by counting the number of atoms in a chain structure (excluding the number of hydrogen atoms, and the number of atoms of branched structure portions and substituents).

On the other hand, when the main chain has a structure containing a cyclic structure, in view of defining the length of the main chain, the number of atoms of the main chain can be counted for convenience's sake. Specifically, the number of atoms of the main chain in such a case can be determined, as described above, by counting the number of atoms of the shortest route connecting two bonds in the cyclic structure in addition to the number of atoms in a chain structure comprising no divalent cyclic structure in the main chain (excluding the number of hydrogen atoms, and the number of atoms of branched structure portions and substituents).

The main chain of E or E₁ - E₂ - E₃ may be preferably a chain structure in which the "other divalent group" in E or the "divalent group" in E₃ does not comprise a divalent cyclic structure. Consequently, the "other divalent group" in E or the "divalent group" in E₃ may be a divalent linear or branched hydrocarbon group, -C(=O)-, - NR_{E}- (R_{E} indicates a hydrogen atom or the above-described substituent), -O-, -S-, -C(=S)-, or a group consisting of a combination of two or more (e.g., two to eight, preferably two to six, and more preferably two to four) of these. The divalent linear or branched hydrocarbon group may have, for example, one to five, preferably one to three, more preferably one or two substituents, or does not necessarily have a substituent. It is preferable not to have such a substituent in view of synthesizing a compound having a simple chemical structure. On the other hand, when the group as described above has a substituent, examples and preferred examples of such a substituent are the same as those of the substituent which may be comprised in the heteroarylene, which is an example of the leaving group.

### 1-5. Bioorthogonal Functional Group (B)

In Formula (I), B is a bioorthogonal functional group.

The bioorthogonal functional group refers to a group that does not react with biological components (e.g., amino acids, nucleic acids, lipids, sugars, and phosphoric acids) or has a low reaction rate to biological components but selectively reacts with components other than biological components. The bioorthogonal functional group is well known in the technical field concerned (e.g., refer to Sharpless K. B. et al., Angew. Chem. Int. Ed. 40, 2004 (2015); Bertozzi C. R. et al., Science 291, 2357 (2001); Bertozzi C. R. et al., Nature Chemical Biology 1, 13 (2005)).

When the target of the affinity substance is an antibody (protein), the bioorthogonal functional group is a bioorthogonal functional group to a protein. The bioorthogonal functional group to proteins is a group that does not react with side chains of 20 natural amino acid residues forming proteins and reacts with certain functional groups. The 20 natural amino acid residues forming proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Among these 20 natural amino acid residues, glycine, which has no side chain (that is, has a hydrogen atom), and alanine, isoleucine, leucine, phenylalanine, and valine, which have a hydrocarbon group as a side chain (that is, comprise no hetero atom selected from the group consisting of a sulfur atom, a nitrogen atom, and an oxygen atom in their side chains) are inactive to normal reactions. Consequently, the bioorthogonal functional group to proteins is a functional group incapable of reacting with, in addition to the side chains of these amino acids having side chains inactive to normal reactions, side chains of asparagine, glutamine, methionine, proline, serine, threonine, tryptophan, tyrosine, aspartic acid, glutamic acid, arginine, histidine, and lysin.

Examples of such a bioorthogonal functional group that cannot react with proteins include an azide residue, an aldehyde residue, a thiol residue, an alkene residue (in other words, it is only required to have a vinylene (ethenylene) portion, which is a minimum unit having a carbon-carbon double bond. Hereinafter the same), an alkyne residue (in other words, it is only required to have an ethynylene portion, which is a minimum unit having a carbon-carbon triple bond. Hereinafter the same), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (e.g., a carbonyl residue having a fluorine atom, a chlorine atom, a bromine atom, or an iodine atom at an α-position. Hereinafter the same), an isonitrile residue, a sydnone residue, and a selenium residue. The antibody can be a protein that cannot comprise a free thiol. In the protein that cannot comprise a free thiol, a thiol functions as a bioorthogonal functional group. Consequently, when a target of the affinity substance is an antibody, the bioorthogonal functional group comprises a thiol. The thiol residue may be an unprotected thiol residue (that is, -SH) or a protected thiol residue. Examples of a protecting group for the thiol residue in the protected thiol residue include a hydrocarbon group (e.g., an alkyl group, an alkenyl group, an alkynyl group, a cycloalkyl group, an aryl group (e.g., a phenyl group or a naphthyl group), or an aryl alkyl (aralkyl) group], an acyl group (e.g., an acetyl group, a propoxy group, a butoxycarbonyl group such as a tert-butoxycarbonyl group, or a benzoyl group), an arylalkyloxycarbonyl group (e.g., a fluorenylmethoxycarbonyl group), an aryloxycarbonyl group, an arylalkyl (aralkyl) oxycarbonyl group (e.g., a benzyloxycarbonyl group), an alkylthiol group (t-butylthio group), and an arylthiol group (e.g., a pyridyldithio group). Alternatively, the protected thiol residue may be a disulfide residue. In the arylalkyl in the arylalkyl (aralkyl) group and the arylalkyl (aralkyl) oxycarbonyl group, one or more (for example, 2, 3, 4, or 5) aryls bind to an alkyl. The number of carbon atoms of the protecting group for the thiol residue is, for example, 1 to 30, preferably 1 to 20, more preferably 1 to 15, even more preferably 1 to 10, and particularly preferably 1 to 6. In the present invention, the compound represented by Formula (I) may comprise one or more types (e.g., two, three, or four types) of bioorthogonal functional groups; the compound may preferably comprise one type of bioorthogonal functional group.

More specifically, the bioorthogonal functional group may correspond to any one chemical structure selected from the group consisting of the following: 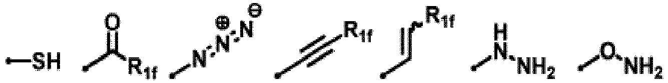 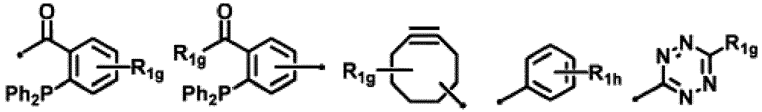 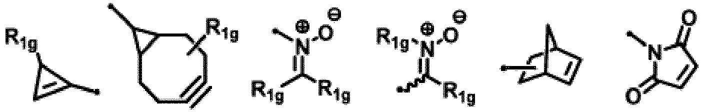 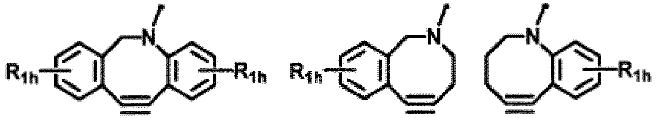 wherein
R_{1f}, one or a plurality of R_{1g}S, and one or a plurality of R₁ₕS are the same as or different from each other, and are each an atom or a group selected from the group consisting of (a) to (g) or an electron-withdrawing group, and
• is a bond.

Examples of the atom or group selected from the group consisting of (a) to (g) include:
a group selected from the group consisting of
   (a) a hydrogen atom or a halogen atom;
   (b) a monovalent hydrocarbon group;
   (c) an aralkyl;
   (d) a monovalent heterocyclic group;
   (e) Rₐ-O-, Rₐ-C(=O)-, Rₐ-O-C(=O)-, or Rₐ-C(=O)-O-wherein Rₐ indicates a hydrogen atom or a monovalent hydrocarbon group; (f) NR_{b}R_{c}-, NR_{b}R_{c}-C(=O)-, NR_{b}R_{c}-C(=O)-O-, or R_{b}-C(=O) -NR_{c}- wherein R_{b} and R_{c} are the same as or different from each other, and each indicate a hydrogen atom or a monovalent hydrocarbon group; and
   (g) a nitro group, a sulfuric acid group, a sulfonic acid group, a cyano group, and a carboxy group.

The definitions, examples, and preferred examples of the halogen atom, the monovalent hydrocarbon group, the aralkyl, the monovalent heterocyclic group, and the monovalent hydrocarbon group in Rₐ to R_{c} in (a) to (g) are the same as those in the above (i) to s(vii). The atom or group selected from the group consisting of (a) to (g) is particularly preferably the atom or group of (a) or (b).

The bioorthogonal functional group may be preferably a group selected from the group consisting of an azide residue, a thiol residue, an alkyne residue, a maleimide residue, and a disulfide residue among the bioorthogonal functional groups described above in view of improving reaction efficiency or the like.

Examples of the electron-withdrawing group include those described above, in which preferred are a halogen atom, a boronic acid residue, mesyl, tosyl, and triflate.

### 1-6. Preferred Structure of Compound Represented by Formula (I)

In a preferred embodiment, the compound represented by Formula (I) may be a compound represented by the following Formula (I-1):

A-L₁-L₂-E₁-E₂-E₃-B (I-1)

wherein
A and B are the same those as in Formula (I),
L₁ is a bond or a divalent group,
L₂ is a leaving group,
E₁ is an electrophilic group (i) coupled with a leaving group and (ii) having ability to react with a nucleophilic group in an antibody,
E₂ is a group represented by (a) -X-Y- [where X which binds to E₁ is C(R₁) (R₂) (where R₁ and R₂ are each independently a hydrogen atom or C₁₋₆ alkyl), N(R₃) (where R₃ is a hydrogen atom or C₁₋₆ alkyl), O, S, or Se, and Y which binds to E₃ is C(R₄) (R₅) (where R₄ and R₅ are each independently a hydrogen atom or C₁₋₆ alkyl), or (b) the following formula (i): 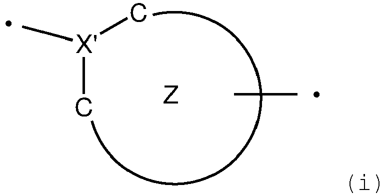
   (where the ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms. · is a bond),
   E₃ is a divalent group when E₂ is -X-Y-, and is a bond or a divalent group when E₂ is a group represented by Formula (i), and
   the leaving group has ability to be cleaved and eliminated from E₁ by a reaction between the nucleophilic group and the electrophilic group].

In Formula (I-1), the leaving group represented by L₂ is preferably any of the above (a) to (c). Examples and preferred examples of the leaving group represented by L₂ are also the same as those described in the above (a) to (c) .

In a preferred specific embodiment, the compound represented by Formula (I-1) may be a compound represented by the following Formula (1-2):

A-L₁-L₂-E₁-X-Y-E₃-B (1-2)

wherein
A, L₁, X, Y, and B are the same as those in Formula (I-1),
L₂ is
   (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R) -, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-, - SO₂-, and -CH₂-, and
E₃ is a divalent group.

In another preferred specific embodiment, the compound represented by Formula (I-1) may be a compound represented by the following Formula (1-3): 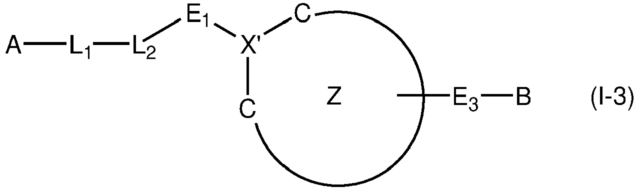 wherein
A, L₁, ring Z, ring-constituting atom X', and B are the same as those in the above Formula (I-1),
L₂ is
   (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-, - SO₂-, and -CH₂-, and
E₃ is a bond or a divalent group.

The compound represented by formula (1-3) may be preferably a compound represented by the following Formula (1-4) : 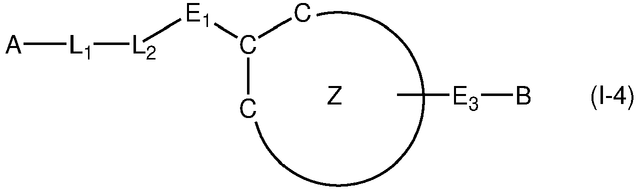 wherein
A, L₁, and B are the same as those in the above Formula (I-1),
L₂ is (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-, - SO₂-, and -CH₂-,
ring Z is a divalent cyclic group in which all of a ring-constituting atom which binds to E₁ and ring-constituting atoms on both sides thereof are carbon atoms, and
E₃ is a bond or a divalent group.

In the compounds represented by the above Formulae (I-1) to (1-4), the definitions, examples, and preferred examples of A, L₁, L₂, E₁, E₂, E₃, B, -X-Y-, C₁₋₆ alkyl in R₁ to R₅, and the group represented by Formula (i) (e.g., a divalent cyclic group or a divalent heterocyclic group) are the same as those described above. The definitions, examples, and preferred examples of (a) to (c) each of which is L₂, ring Z (e.g., a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms), ring P, and a group such as Q (e.g., C₁₋₆ alkyl in R) are also the same as those described above.

### 1-7. Method of Production

The compound having an affinity substance to an antibody and a bioorthogonal functional group, or a salt thereof can be prepared as appropriate. The compound having an affinity substance to an antibody and a bioorthogonal functional group, or a salt thereof is represented by Formula (I), preferably Formula (I-1), more preferably Formula (1-2), (1-3), or (1-4).

For the affinity substance to an antibody (A), one having any functional group can be selected as appropriate. Consequently, using a reactive group capable of reacting with the functional group, the affinity substance is reacted with a structural unit represented by L-E-B, whereby a structural unit represented by A-L-E-B can be prepared. Such a reaction can be conducted in an appropriate reaction system such as an organic solvent system or an aqueous solution system at an appropriate temperature (e.g., about 15°C to 200°C), for example. The reaction system may comprise an appropriate catalyst. The reaction time is e.g., 1 minute to 20 hours, preferably 10 minutes to 15 hours, more preferably 20 minutes to 10 hours, and even more preferably 30 minutes to 8 hours.

In the reaction system, a molar ratio (Y/X) of the structural unit (Y) represented by L-E-B to the affinity substance (X) is not limited to a particular ratio because it varies in accordance with the types of the structural unit and the affinity substance, the number of sites in the affinity substance to be modified with the structural unit, and the like. However, the molar ratio (Y/X) is e.g., 0.1 to 50, preferably 0.5 to 40, more preferably 1 to 35, even more preferably 2 to 25, and particularly preferably 3 to 15.

Determination of the formation of the compound having an affinity substance to an antibody and a bioorthogonal functional group, or a salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. The compound having an affinity substance to an antibody and a bioorthogonal functional group, or a salt thereof can be purified as appropriate by any method such as chromatography (e.g., the pieces of chromatography described above and affinity chromatography).

### 1-8. Others

In the inventions described below [e.g., inventions represented by Formulae (II) and (III), formulae having subordinate concepts thereof, and partial structural formulae], any symbols (e.g., A, L, E, and B) and the details of terms expressed in relation to the symbols (e.g., definitions, examples, and preferred examples) are common to those of the invention of the compound represented by the above Formula (I) or a formula having a subordinate concept thereof, or a salt thereof. Specific groups (e.g., a bioorthogonal functional group, a divalent group, and a substituent) that can define the inventions described below and any technical elements such as specific values (e.g., definitions, examples, and preferred examples) can also be common to those described above. Consequently, these matters can be quoted as appropriate in the inventions described below without any special reference. Similarly, the technical elements of a specific invention described in the inventions described below can be quoted as appropriate as the technical elements of the present invention and other inventions.

### 2. Method for producing antibody having bioorthogonal functional group

The present invention provides a method for producing an antibody having a bioorthogonal functional group or bioorthogonal functional groups or a salt thereof, the method comprising the following.
(1) Reacting a compound having an affinity substance to an antibody and a bioorthogonal functional group, represented by the following Formula (I):

   A-L-E-B (I)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   B is a bioorthogonal functional group, and
   the leaving group has ability to be cleaved and eliminated from E by a reaction between the nucleophilic group and the electrophilic group, or a salt thereof with an antibody
   to form an antibody having a bioorthogonal functional group or bioorthogonal functional groups, represented by the following Formula (II):

   Ab-E-B (II)

   wherein
   E and B are the same as those in Formula (I), and
   Ab is an antibody, or a salt thereof.

The antibody used in the method for producing an antibody having a bioorthogonal functional group or bioorthogonal functional groups is the same as the above-described antibody. The antibody is preferably a monoclonal antibody. Examples of the isotype of the monoclonal antibody include IgG (e.g., IgG1, IgG2, IgG3, and IgG4), IgM, IgA, IgD, IgE, and IgY. The monoclonal antibody is a full-length antibody or an antibody fragment (e.g., F(ab')₂, Fab', Fab, Fv, and a single-chain antibody); the full-length antibody is preferred. The antibody is particularly preferably a human antibody, a humanized antibody, or a chimeric antibody having human IgG (e.g., IgG1, IgG2, IgG3, and IgG4) in a constant region.

Ab in Formula (II) is the same as the above-described antibody and covalently binds to the electrophilic group in E. Examples of the nucleophilic group in the antibody to be subjected to covalent bond with the electrophilic group in E include NH₂ in a side chain of a lysine residue, OH in a side chain of a tyrosine residue, OH in a side chain of a serine residue, OH in a side chain of a threonine residue, and SH in a side chain of a cysteine residue.

E in Formula (II) is the same as E in the above Formula (I). E can be represented by E₁-E₂-E₃. E₁, E₂, and E₃ are the same as those described above. As described above, E and E₁-E₂-E₃ can be designed so as to be free of a peptide portion having a problem of potential immunogenicity and being easily hydrolyzed in the blood. In this case, the antibody having a bioorthogonal functional group or bioorthogonal functional groups, represented by Formula (II) can be used for preparing an antibody having a functional substance or functional substances not having such a problem.

The electrophilic group in E and the electrophilic group in E₁ covalently bind to the nucleophilic group in the antibody. Examples of the electrophilic group covalently binding to the nucleophilic group in the antibody include: NH-C(=O)-, NH-SO₂-, and NH-CH₂- (when the nucleophilic group in the antibody to be subjected to covalent bond with E is NH₂ in a side chain of a lysine residue); O-C(=O)-, O-SO₂-, and O-CH₂- (when the nucleophilic group in the antibody to be subjected to covalent bond with E is OH in a side chain of a tyrosine residue, a serine residue, or a threonine residue); and S-C(=O)- and S-CH₂- (when the nucleophilic group in the antibody to be subjected to covalent bond with E is SH in a side chain of a cysteine residue). The electrophilic group covalently binding to the nucleophilic group in the antibody is preferably NH-C(=O)- or NH-SO₂- (when the nucleophilic group in the antibody to be subjected to covalent bond with E is NH₂ in a side chain of a lysine residue), O-C(=O)- or O-SO₂- (when the nucleophilic group in the antibody to be subjected to covalent bond with E is OH in a side chain of a tyrosine residue), more preferably NH-C(=O)- or NH-SO₂- (when the nucleophilic group in the antibody to be subjected to covalent bond with E is NH₂ in a side chain of a lysine residue), and even more preferably NH-C(=O)- (when the nucleophilic group in the antibody to be subjected to covalent bond with E is NH₂ in a side chain of a lysine residue).

B in Formula (II) is the same as B in the above Formula (I).

The antibody having a bioorthogonal functional group or bioorthogonal functional groups produced by the production method of the present invention is preferably an antibody regioselectively having a bioorthogonal functional group or bioorthogonal functional groups. When the compound having an affinity substance to an antibody and a bioorthogonal functional group, or a salt thereof is represented by Formula (I), it is possible to produce an antibody regioselectively having a bioorthogonal functional group or bioorthogonal functional groups, represented by Formula (II). The antibody regioselectively having a bioorthogonal functional group or bioorthogonal functional groups is preferably an antibody having a bioorthogonal functional group or bioorthogonal functional groups only in a constant region, and more preferably an antibody having a bioorthogonal functional group or bioorthogonal functional groups only in an Fc region.

In the present specification, "regioselective" or "regioselectivity" refers to a state in which even though a specific amino acid residue is not present locally at a specific region in the antibody, a certain structural unit capable of binding to the specific amino acid residue in the antibody is present locally at a specific region in the antibody. Consequently, expressions related to regioselectivity such as "regioselectively having," "regioselective binding," and "binding with regioselectivity" mean that the binding rate or the possession rate of a certain structural unit in the target region comprising one or more specific amino acid residues is higher at a significant level than the possession rate or the binding rate of the structural unit in the non-target region comprising a plurality of amino acid residues homogeneous with respect to the specific amino acid residues in the target region. Such regioselective binding or possession can be achieved by the present invention, which enables the certain structural unit to preferentially react with the specific amino acid residues in the target region in the antibody, not by randomly reacting the certain structural unit with the specific amino acid residues in the antibody but by using a compound comprising an affinity substance to an antibody.

More specifically, when the antibody (Ab) comprises one or more specific amino acid residues (e.g., a lysine residue, a tyrosine residue, a threonine residue, a serine residue, or a cysteine residue) in a target region consisting of 1 to 50 continuous amino acid residues [e.g., a region consisting of amino acid residues at positions 246 to 248 in the human IgG Fc region, (b) a region consisting of amino acid residues at positions 288 to 290 in the human IgG Fc region, or (c) a region consisting of an amino acid residue at position 317 in the human IgG Fc region] and comprises five or more of the specific amino acid residues in a non-target region other than the target region, a partial structure other than the antibody can bind to one or more specific amino acid residues comprised in the target region with 30% or more regioselectivity. The regioselectivity may be e.g., preferably 40% or more, more preferably 50% or more, even more preferably 60% or more, and particularly preferably 70% or more, 80% or more, 90% or more, 95% or more, 96% or more, 97% or more, 98% or more, 99% or more, or 100%.

The antibody having a bioorthogonal functional group or bioorthogonal functional groups produced by the production method of the present invention can have the bioorthogonal functional group (that is, a structural unit represented by E-B) depending on the number of heavy chains. The antibody regioselectively having a bioorthogonal functional group or bioorthogonal functional groups can be produced by first associating the compound (A-L-E-B) represented by Formula (I) with a constant region of an antibody heavy chain via the affinity substance to an antibody (A), and then reacting an electrophilic group in E with a nucleophilic group in a side chain of a specific amino acid residue near the association site (constant region of the same heavy chain as the antibody heavy chain). Consequently, by using an antibody having a plurality of (e.g., 1 to 8, preferably 1 to 4, more preferably 2) antibody heavy chains in the production method of the present invention, it is possible to produce an antibody regioselectively having a plurality of structural units represented by E-B (or a plurality of structural units having a subordinate concept thereof) in the same target region of the plurality of antibody heavy chains. For example, by using an antibody having two antibody heavy chains (e.g., IgG, IgD, IgE, F(ab')₂ antibody, Fc region protein, or Fc fusion protein) in the production method of the present invention, it is possible to produce an antibody regioselectively having two structural units represented by E-B in the same target region of the two antibody heavy chains. That is, in the antibody having a bioorthogonal functional group or bioorthogonal functional groups, the modification mode by the bioorthogonal functional group can be the same between a plurality of (e.g., two) heavy chains.

The antibody having a bioorthogonal functional group or bioorthogonal functional groups can have the same or different types of bioorthogonal functional groups (e.g., a structural unit represented by E-B) in a plurality of (e.g., 2 to 5, preferably 2 to 4, more preferably 2 or 3) target regions of one antibody heavy chain. In this case, in the antibody having a bioorthogonal functional group or bioorthogonal functional groups, the modification mode by the bioorthogonal functional group can be the same between a plurality of (e.g., two) heavy chains.

The production method of the present invention may comprise subjecting a formed antibody to a specific treatment to form an antibody having a bioorthogonal functional group or bioorthogonal functional groups and further modified. Examples of such a specific treatment include an antibody fragmentation treatment (e.g., a treatment with a specific protease such as papain or pepsin).

When the compound represented by Formula (I) or a salt thereof is used in the production method of the present invention, an antibody having a bioorthogonal functional group or bioorthogonal functional groups represented by the above Formula (II) can be produced.

In a preferred embodiment, when a compound represented by Formula (I-1) is used as the compound represented by Formula (I) in the production method of the present invention, an antibody having a bioorthogonal functional group or bioorthogonal functional groups, represented by the following Formula (II-1) can be produced.

Ab-E₁-E₂-E₃-B (II-1)

wherein
Ab is an antibody,
E₁ is an electrophilic group coupled with a nucleophilic group in the antibody,
E₂ is a group represented by (a)-X-Y- [where X which binds to E₁ is C(R₁) (R₂) (where R₁ and R₂ are each independently a hydrogen atom or C₁₋₆ alkyl), N(R₃) (where R₃ is a hydrogen atom or C₁₋₆ alkyl), O, S, or Se, and Y which binds to E₃ is C(R₄) (R₅) (where R₄ and R₅ are each independently a hydrogen atom or C₁₋₆ alkyl), or (b) the following formula (i): 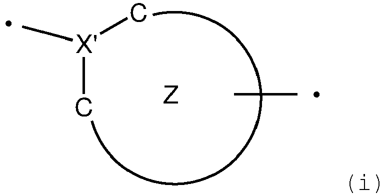
   (where the ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms. · is a bond),
   E₃ is a divalent group when E₂ is -X-Y-, and is a bond or a divalent group when E₂ is a group represented by Formula (i), and
   B is a bioorthogonal functional group].

In a preferred specific embodiment, when a compound represented by Formula (1-2) is used as the compound represented by Formula (I-1) in the production method of the present invention, an antibody having a bioorthogonal functional group or bioorthogonal functional groups, represented by the following Formula (II-2) can be produced.

Ab-E₁-X-Y-E₃-B (II-2)

wherein
Ab, X, Y, and B are the same as those in Formula (II-1),
E₁ is a group selected from the group consisting of - C(=O)-, -SO₂-, and -CH₂-, and
E₃ is a divalent group.

In another preferred specific embodiment, when a compound represented by Formula (1-3) is used as the compound represented by Formula (I-1) in the production method of the present invention, an antibody having a bioorthogonal functional group or bioorthogonal functional groups, represented by the following Formula (11-3) can be produced. 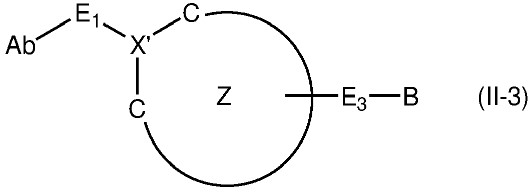 wherein
Ab, ring-constituting atom X', ring Z, and B are the same as those in Formula (II-1),
E₁ is a group selected from the group consisting of - C(=O)-, -SO₂-, and -CH₂-, and
E₃ is a bond or a divalent group.

When a compound represented by Formula (1-4) is used as the compound represented by Formula (1-3) in the production method of the present invention, an antibody having a bioorthogonal functional group or bioorthogonal functional groups, represented by the following Formula (II-4) can be preferably produced. 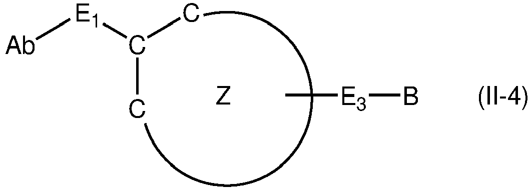 wherein
Ab, ring Z, and B are the same as those in Formula (II-1),
E₁ is a group selected from the group consisting of - C(=O)-, -SO₂-, and -CH₂-, and
E₃ is a bond or a divalent group.

Any symbols (e.g., E, E₁, E₂, E₃, and B) in Formula (II), (II-1), (II-2), (II-3) or (11-4) and the details of terms (e.g., antibody, electrophilic group, and bioorthogonal functional group) expressed in relation to the symbols (e.g., definitions, examples, and preferred examples) are common to those of the above Formula (I) or a formula having a subordinate concept thereof.

The compound having an affinity substance to an antibody and a bioorthogonal functional group, or a salt thereof has the electrophilic group in E or the electrophilic group in E₁, and therefore can react with the antibody. Such a reaction can be conducted as appropriate under a condition incapable of causing denaturation or decomposition (e.g., cleavage of an amide bond) of proteins (a mild condition). Such a reaction can be conducted in an appropriate reaction system such as a buffer at room temperature (e.g., about 15°C to 30°C), for example. The pH of the buffer is e.g., 5 to 9, preferably 5.5 to 8.5, and more preferably 6.0 to 8.0. The buffer may comprise an appropriate catalyst. The reaction time is e.g., 1 minute to 20 hours, preferably 10 minutes to 15 hours, more preferably 20 minutes to 10 hours, and even more preferably 30 minutes to 8 hours. For the details of such a reaction, refer to G. J. L. Bernardes et al., Chem. Rev., 115, 2174 (2015); G. J. L. Bernardes et al., Chem. Asian. J., 4,630 (2009); B. G. Davies et al., Nat. Commun., 5, 4740 (2014); A. Wagner et al., Bioconjugate. Chem., 25,825 (2014), for example.

In the reaction system, a molar ratio (Y/X) of the compound having an affinity substance to an antibody and a bioorthogonal functional group, or a salt thereof (Y) to the antibody (X) is not limited to a particular ratio because it varies in accordance with the types of the compound having an affinity substance to an antibody and a bioorthogonal functional group, or a salt thereof, and the antibody, the number of sites in the antibody to be modified by the compound having an affinity substance to an antibody and a bioorthogonal functional group, or a salt thereof (e.g., DAR), and the like. However, the molar ratio (Y/X) is e.g., 0.1 to 100, preferably 0.5 to 80, more preferably 1 to 70, even more preferably 2 to 50, and particularly preferably 3 to 30.

Determination of the formation of the antibody having a bioorthogonal functional group or bioorthogonal functional groups, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of bioorthogonal functional groups that the antibody having a bioorthogonal functional group or bioorthogonal functional groups has can be performed by electrophoresis, chromatography, or mass spectrometry, for example, and preferably mass spectrometry. The antibody having a bioorthogonal functional group or bioorthogonal functional groups can be purified as appropriate by any method such as chromatography (e.g., the pieces of chromatography described above and affinity chromatography).

3. Method for producing antibody having functional substance using compound having affinity substance to antibody and bioorthogonal functional group, or salt thereof

The present invention provides a method for producing an antibody having a functional substance or functional substances or a salt thereof, the method comprising the following.
(1) Reacting a compound having an affinity substance to an antibody and a bioorthogonal functional group, represented by the following Formula (I):

   A-L-E-B (I)

   wherein
   A is an affinity substance to an antibody,
   L is a divalent group comprising a leaving group,
   E is a divalent group comprising an electrophilic group (i) coupled with the leaving group and (ii) having ability to react with a nucleophilic group in the antibody,
   B is a bioorthogonal functional group, and
   the leaving group has ability to be cleaved and eliminated from E by a reaction between the nucleophilic group and the electrophilic group, or a salt thereof with an antibody
   to form an antibody having a bioorthogonal functional group or bioorthogonal functional groups, represented by the following Formula (II):

   Ab-E-B (II)

   wherein
   E and B are the same as those in the above Formula (I), and
   Ab is an antibody, or a salt thereof, and
(2) reacting the antibody having a bioorthogonal functional group or bioorthogonal functional groups, represented by the above Formula (II), or a salt thereof with a functional substance via the bioorthogonal functional group
   to form an antibody having a functional substance or functional substances, represented by the following Formula (III):

   Ab-E-B'-F (III)

   wherein Ab is the same as that in Formula (II),
   E is the same as that in Formula (I),
   B' is a divalent group comprising a portion formed by a reaction between the functional substance and the bioorthogonal functional group, and
   F is a functional substance, or a salt thereof.

Step (1) can be performed in a similar manner to the method for producing an antibody having a bioorthogonal functional group or bioorthogonal functional groups.

The functional substance (F) used in step (2) is not limited to a particular substance so long as it is a substance imparting any function to the antibody; examples thereof include drugs, labelling substances, and stabilizers; preferred are drugs and labelling substances. The functional substance may be a single functional substance or a substance in which two or more functional substances are coupled with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a drug that treats or prevents a certain disease, or a drug that alleviates side effects associated with use of the antibody to a target protein of the certain disease.

More specifically, the drug is an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{s}), radioisotopes of yttrium (e.g., ⁹⁰Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi).

Examples of labelling substances include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl)ruthenium, and luminol), and radioisotopes (e.g., those described above) or substances comprising them.

The functional substance is a large compound, a middle compound, or a small compound and is preferably a small compound. The small compound refers to compounds with a molecular weight of 1,500 or lower. The small compound is a natural compound or a synthesized compound. The molecular weight of the small compound may be 1,200 or lower, 1,000 or lower, 900 or lower, 800 or lower, 700 or lower, 600 or lower, 500 or lower, 400 or lower, or 300 or lower. The molecular weight of the small compound may be 30 or higher, 40 or higher, or 50 or higher. The small compound may be any of the drugs or labelling substances described above. Examples of the small compound include amino acids, oligopeptides, vitamins, nucleosides, nucleotides, oligonucleotides, monosaccharides, oligosaccharides, lipids, fatty acids, and salts thereof.

The functional substance has various functional groups corresponding to its structure. When the functional substance has a functional group easily reacting with the bioorthogonal functional group, the functional group of the functional substance and the bioorthogonal functional group can be reacted with each other as appropriate. The function group easily reacting with the bioorthogonal functional group can vary depending on a specific type of the bioorthogonal functional group. A person skilled in the art can select an appropriate functional group as the functional group easily reacting with the bioorthogonal functional group as appropriate (e.g., Boutureira et al., Chem. Rev., 2015, 115, 2174-2195). Examples of the functional group easily reacting with the bioorthogonal functional group include, but are not limited to, an azide residue when the bioorthogonal functional group is an alkyne residue, a hydrazine residue when the bioorthogonal functional group is an aldehyde residue or a ketone residue, and a maleimide residue and a disulfide residue when the bioorthogonal functional group is a thiol residue. For example, when the bioorthogonal functional group is an alkyne residue and the functional group easily reacting with the bioorthogonal functional group is an azide residue (or vice versa), the divalent group comprising a portion formed by a reaction between the functional substance and the bioorthogonal functional group may be a divalent group comprising a triazole residue (which may be condensed with another ring or is not necessarily condensed therewith); when the bioorthogonal functional group is an aldehyde residue or a ketone residue and the functional group easily reacting with the bioorthogonal functional group is a hydrazine residue (or vice versa), the divalent group comprising a portion formed by a reaction between the functional substance and the bioorthogonal functional group may be a divalent group comprising a hydrazone residue; when the bioorthogonal functional group is a thiol residue and the functional group easily reacting with the bioorthogonal functional group is a maleimide residue or a disulfide residue (or vice versa), the divalent group comprising a portion formed by a reaction between the functional substance and the bioorthogonal functional group may be a divalent group comprising a thiosuccinimide residue or a divalent group comprising a disulfide residue (e.g., Boutureira et al., Chem. Rev., 2015, 115, 2174-2195). The divalent group comprising a triazole residue (which may be condensed with another ring or is not necessarily condensed therewith), the divalent group comprising a hydrazone residue, the divalent group comprising a thiosuccinimide residue, or the divalent group comprising a disulfide residue is a preferred example of the divalent group comprising a portion formed by a reaction between the functional substance and the bioorthogonal functional group.

On the other hand, when the functional substance has no functional group easily reacting with the bioorthogonal functional group, a substance derivatized so as to have a desired functional group can be used as the functional substance. When the functional substance is a soluble protein, for example, a substance derivatized so as to have a functional group that the soluble protein does not naturally have can be used.

Derivatization is a common technical knowledge in the field concerned (e.g., WO 2004/010957, United States Patent Application Publication No. 2006/0074008, and United States Patent Application Publication No. 2005/0238649). Derivatization may be performed using the cross-linking agent described above, for example. Alternatively, derivatization may be performed using a specific linker having a desired functional group. Such a linker may be able to separate the functional substance and the antibody from each other through the cleavage of the linker under an appropriate condition (e.g., intracellular or extracellular), for example. Examples of such a linker include peptidyl linkers decomposed by specific proteases [e.g., intracellular proteases (e.g., proteases present in lysosome or endosome) and extracellular proteases (e.g., secretory proteases)] (e.g., United States Patent No. 6,214,345; Dubowchik et al., Pharm. Therapeutics 83: 67-123 (1999)) and linkers capable of being cleaved at local acidic sites present in living bodies (e.g., Unites States Patent Nos. 5,622,929, 5,122,368, and 5,824,805). The linker may be self-immolative (e.g., WO 02/083180, WO 04/043493, and WO 05/112919). In the present invention, the derivatized functional substance can also be referred to simply as the "functional substance."

Ab in Formula (III) is the same as the above-described antibody and covalently binds to the electrophilic group in E. Examples of the nucleophilic group in the antibody to be subjected to covalent bond with the electrophilic group in E include NH₂ in a side chain of a lysine residue, OH in a side chain of a tyrosine residue, OH in a side chain of a serine residue, OH in a side chain of a threonine residue, and SH in a side chain of a cysteine residue.

E in Formula (III) is the same as E in the above Formula (I). E can be represented by E₁-E₂-E₃. E₁, E₂, and E₃ are the same as those described above. As described above, E and E₁-E₂-E₃ can be designed so as to be free of a peptide portion having a problem of potential immunogenicity and being easily hydrolyzed in the blood. In this case, the antibody having a functional substance or functional substances, represented by Formula (III) can be suitably used as pharmaceuticals.

The electrophilic group in E and the electrophilic group in E₁ covalently bind to the nucleophilic group in the antibody. Examples of the electrophilic group covalently binding to the nucleophilic group in the antibody include: NH-C(=O)-, NH-SO₂-, and NH-CH₂- (when the nucleophilic group in the antibody to be subjected to covalent bond with E is NH₂ in a side chain of a lysine residue); O-C(=O)-, O-SO₂-, and O-CH₂- (when the nucleophilic group in the antibody to be subjected to covalent bond with E is OH in a side chain of a tyrosine residue, a serine residue, or a threonine residue); and S-C(=O)- and S-CH₂- (when the nucleophilic group in the antibody to be subjected to covalent bond with E is SH in a side chain of a cysteine residue). The electrophilic group covalently binding to the nucleophilic group in the antibody is preferably NH-C(=O)- or NH-SO₂- (when the nucleophilic group in the antibody to be subjected to covalent bond with E is NH₂ in a side chain of a lysine residue), O-C(=O)- or O-SO₂- (when the nucleophilic group in the antibody to be subjected to covalent bond with E is OH in a side chain of a tyrosine residue), more preferably NH-C(=O)- or NH-SO₂- (when the nucleophilic group in the antibody to be subjected to covalent bond with E is NH₂ in a side chain of a lysine residue), and even more preferably NH-C(=O)- (when the nucleophilic group in the antibody to be subjected to covalent bond with E is NH₂ in a side chain of a lysine residue).

In a specific embodiment, when the functional substance has a functional group easily reacting with the bioorthogonal functional group, or when the functional substance is derivatized so as to have a functional group easily reacting with the bioorthogonal functional group, the functional group easily reacting with the bioorthogonal functional group may be selected from the group consisting of an azide residue, an aldehyde residue, a thiol residue, an alkyne residue, an alkene residue, a halogen residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue.

In a more specific embodiment, when the functional substance has a functional group easily reacting with the bioorthogonal functional group, or when the functional substance is derivatized so as to have a functional group easily reacting with the bioorthogonal functional group, the functional group easily reacting with the bioorthogonal functional group may be selected from the group consisting of the groups illustrated below. 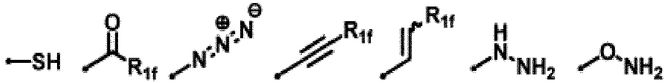 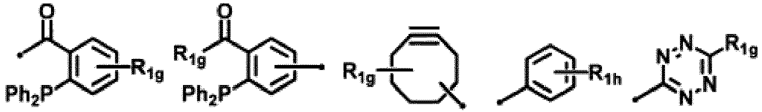 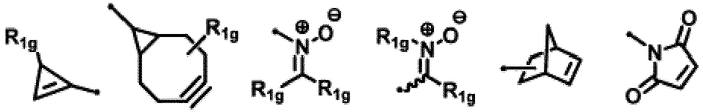 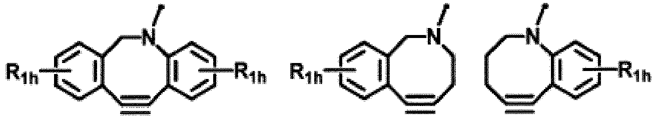 wherein
R_{1f}, one or a plurality of R_{1g}s, and one or a plurality of R₁ₕs are the same as or different from each other, and are each an atom or a group selected from the group consisting of the above (i) to (vii) or an electron-withdrawing group, and · is a bond to the functional substance.

The divalent group comprising a portion formed by a reaction between a functional substance and a bioorthogonal functional group, represented by B' in Formula (III) (1) may be a divalent group comprising a triazole residue, a hydrazone residue, or a thiosuccinimide residue, referred to in the above-described preferred examples, or (2) which is not limited to a particular group, may be a divalent group comprising a residue selected from the group consisting of a disulfide residue (this residue has been referred to in the above-described preferred examples), an acetal residue, a ketal residue, an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue, for example.

The divalent group comprising a portion formed by a reaction between a functional substance and a bioorthogonal functional group, which is not limited to a particular group, may be a divalent group comprising a residue corresponding to any one chemical structure selected from the group consisting of the following: 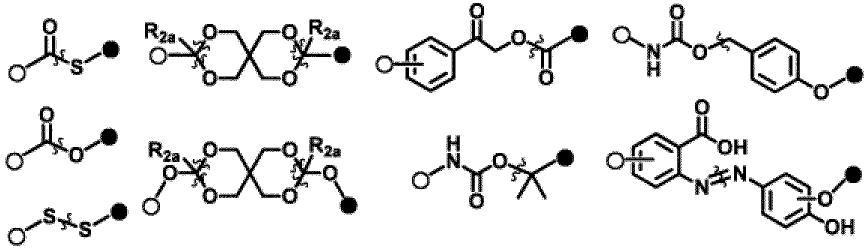 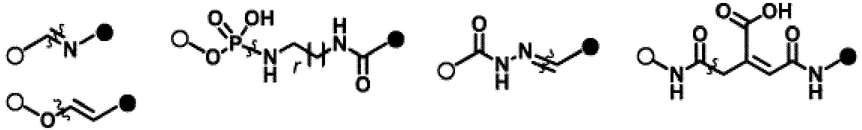 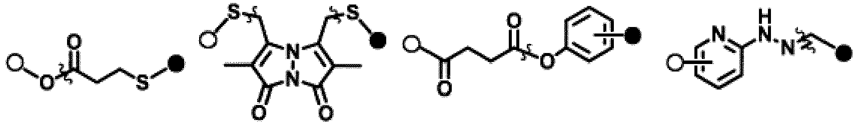 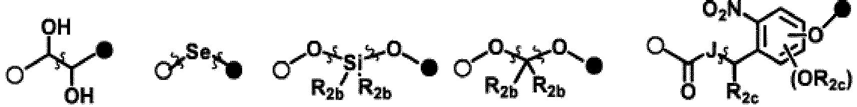 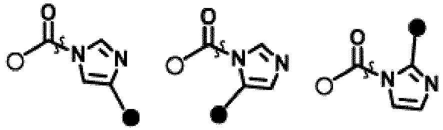 [where a wavy line orthogonal to a bond indicates a bond formed by a reaction,
a plurality of R₂ₐs are the same as or different from each other, a plurality of R_{2b}s are the same as or different from each other, and a plurality of R_{2c}s are the same as or different from each other, and are hydrogen atoms or the above-described substituents,
J is -CH₂-, -O-, or -S-,
r is any integer of 1 to 4,
a symbol of "white circle" indicates a bond to an F side portion, and a symbol of "black circle" indicates a bond to a B' side portion.

When the chemical structure is asymmetrical about a cleavable portion, • may indicate a bond to a B' side portion and o may indicate a bond to an F-side portion].

The antibody having a functional substance or functional substances produced in step (2) in the production method of the present invention is preferably an antibody regioselectively having a functional substance or functional substances. When the antibody regioselectively having a bioorthogonal functional group or bioorthogonal functional groups is represented by Formula (II), it is possible to produce an antibody regioselectively having a functional substance or functional substances, represented by Formula (III). The antibody regioselectively having a functional substance or functional substances is preferably an antibody having a functional substance or functional substances only in a constant region, and more preferably an antibody having a functional substance or functional substances only in an Fc region.

When the antibody (Ab) comprises one or more specific amino acid residues (e.g., a lysine residue, a tyrosine residue, a threonine residue, a serine residue, or a cysteine residue) in a target region consisting of 1 to 50 continuous amino acid residues [e.g., a region consisting of amino acid residues at positions 246 to 248 in the human IgG Fc region, (b) a region consisting of amino acid residues at positions 288 to 290 in the human IgG Fc region, or (c) a region consisting of an amino acid residue at position 317 in the human IgG Fc region] and comprises five or more of the specific amino acid residues in a non-target region other than the target region, a partial structure other than the antibody can bind to one or more specific amino acid residues comprised in the target region with 30% or more regioselectivity. The regioselectivity may be e.g., preferably 40% or more, more preferably 50% or more, even more preferably 60% or more, and particularly preferably 70% or more, 80% or more, 90% or more, 95% or more, 96% or more, 97% or more, 98% or more, 99% or more, or 100%.

The antibody having a functional substance or functional substances produced by the production method of the present invention can have a functional substance (that is, a structural unit represented by E-B'-F) depending on the number of heavy chains. This is because the antibody having a functional substance or functional substances can be produced from an antibody having a bioorthogonal functional group or bioorthogonal functional groups that can have a structural unit represented by E-B depending on the number of heavy chains. Consequently, by using an antibody having a plurality of (e.g., 1 to 8, preferably 1 to 4, more preferably 2) antibody heavy chains in the production method of the present invention, it is possible to produce an antibody regioselectively having a plurality of structural units represented by E-B'-F (or a plurality of structural units having a subordinate concept thereof) in the same target region of the plurality of antibody heavy chains. For example, by using an antibody having two antibody heavy chains (e.g., IgG, IgD, IgE, F(ab')₂ antibody, Fc region protein, or Fc fusion protein) in the production method of the present invention, it is possible to produce an antibody regioselectively having two structural units represented by E-B'-F in the same target region of the two antibody heavy chains. That is, in the antibody having a functional substance or functional substances, the modification mode by the functional substance can be the same between a plurality of (e.g., two) heavy chains.

The antibody having a functional substance or functional substances can have the same or different types of functional substances (e.g., a structural unit represented by E-B'-F) in a plurality of (e.g., 2 to 5, preferably 2 to 4, more preferably 2 or 3) target regions of one antibody heavy chain. In this case, in the antibody having a functional substance or functional substances, the modification mode by the functional substance can be the same between a plurality of (e.g., two) heavy chains.

The production method of the present invention may comprise subjecting a formed antibody to a specific treatment to form an antibody having a functional substance or functional substances and further modified. Examples of such a specific treatment include an antibody fragmentation treatment (e.g., a treatment with a specific protease such as papain or pepsin).

When the compound represented by Formula (I) or a salt thereof is used in the production method of the present invention, in step (1), an antibody having a bioorthogonal functional group or bioorthogonal functional groups represented by the above Formula (II) can be produced, and then in step (2), an antibody having a functional substance or functional substances represented by the above Formula (III) can be produced.

In a preferred embodiment, when the compound represented by Formula (I-1) is used as the compound represented by Formula (I) in the production method of the present invention, in step (1), an antibody having a bioorthogonal functional group or bioorthogonal functional groups, represented by the above Formula (II-1) can be produced, and then in step (2), an antibody having a functional substance or functional substances, represented by the above Formula (III-1) can be produced.

Ab-E₁-E₂-E₃-B'-F (III-1)

wherein
Ab is an antibody,
E₁ is an electrophilic group coupled with a nucleophilic group in the antibody,
E₂ is a group represented by (a)-X-Y- [where X which binds to E₁ is C(R₁) (R₂) (where R₁ and R₂ are each independently a hydrogen atom or C₁₋₆ alkyl), N(R₃) (where R₃ is a hydrogen atom or C₁₋₆ alkyl), O, S, or Se, and Y which binds to E₃ is C(R₄) (R₅) (where R₄ and R₅ are each independently a hydrogen atom or C₁₋₆ alkyl), or (b) the following formula (i): 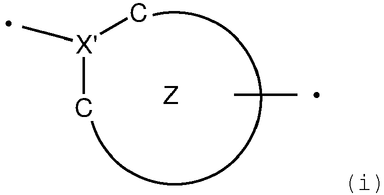
   (where the ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms. · is a bond),
   E₃ is a divalent group when E₂ is -X-Y-, and is a bond or a divalent group when E₂ is a group represented by Formula (i),
   B' is a divalent group comprising a portion formed by a reaction between the functional substance and the bioorthogonal functional group, and
   F is a functional substance.]

In a preferred specific embodiment, when the compound represented by Formula (1-2) is used as the compound represented by Formula (I-1) in the production method of the present invention, in step (1), an antibody having a bioorthogonal functional group or bioorthogonal functional groups represented by the above Formula (11-2) can be produced, and then in step (2), an antibody having a functional substance or functional substances, represented by the following Formula (III-2) can be produced.

Ab-E₁-X-Y-E₃-B'-F (III-2)

wherein
Ab, X, Y, B', and F are the same as those in the above Formula (III-1),
E₁ is a group selected from the group consisting of - C(=O)-, -SO₂-, and -CH₂-, and
E₃ is a divalent group.

In another preferred specific embodiment, when the compound represented by Formula (1-3) is used as the compound represented by Formula (I-1) in the production method of the present invention, in step (1), an antibody having a bioorthogonal functional group or bioorthogonal functional groups represented by the above Formula (11-3) can be produced, and then in step (2), an antibody having a functional substance or functional substances, represented by the following Formula (III-3) can be produced. 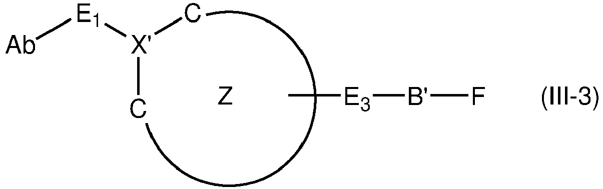 wherein
Ab, ring-constituting atom X', ring Z, B', and F are the same as those in Formula (III-1),
E₁ is a group selected from the group consisting of - C(=O)-, -SO₂-, and -CH₂-, and
E₃ is a bond or a divalent group.

When the compound represented by Formula (1-4) is used as the compound represented by Formula (1-3) in the production method of the present invention, preferably, in step (1), an antibody having a bioorthogonal functional group or bioorthogonal functional groups, represented by the following Formula (II-4) can be produced, and then in step (2), an antibody having a functional substance or functional substances, represented by the following Formula (III-4) can be produced. 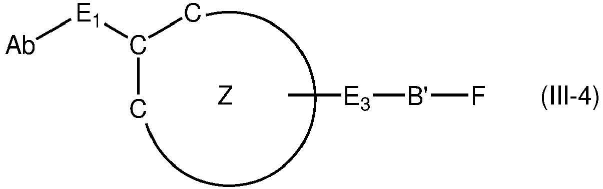 wherein
Ab, ring Z, B', and F are the same as those in Formula (III-1),
E₁ is a group selected from the group consisting of - C(=O)-, -SO₂-, and -CH₂-, and
E₃ is a bond or a divalent group.

Any symbols (e.g., E, E₁, E₂, E₃, and B) in Formula (III), (III-1), (III-2), (III-3) or (III-4) and the details of terms (e.g., antibody, electrophilic group, and bioorthogonal functional group) expressed in relation to the symbols (e.g., definitions, examples, and preferred examples) are common to those of the above Formula (I) or a formula having a subordinate concept thereof.

The antibody having a bioorthogonal functional group or bioorthogonal functional groups can react with the functional substance via the bioorthogonal functional group. Such a reaction can be conducted as appropriate under a condition incapable of causing denaturation or decomposition (e.g., cleavage of an amide bond) of proteins (a mild condition) as described above.

In the reaction system, a molar ratio (Z/Y) of the functional substance (Z) to the antibody (Y) having a bioorthogonal functional group or bioorthogonal functional groups is not limited to a particular ratio because it varies in accordance with the types of the bioorthogonal functional group, the functional substance, and the antibody, the number of sites in the antibody to be modified (e.g., DAR), and the like. However, the molar ratio (Z/Y) is e.g., 0.1 to 100, preferably 0.5 to 80, more preferably 1 to 70, even more preferably 2 to 50, and particularly preferably 3 to 30.

Determination of the formation of the antibody having a functional substance or functional substances, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of functional substances that the antibody having a functional substance or functional substances has can be performed by electrophoresis, chromatography, or mass spectrometry, for example, and preferably mass spectrometry. The antibody having a functional substance or functional substances can be purified as appropriate by any method such as chromatography (e.g., the pieces of chromatography described above and affinity chromatography).

### 4. Antibody regioselectively having bioorthogonal functional group or functional substance, or salt thereof

The present invention provides an antibody regioselectively having a bioorthogonal functional group or a functional substance, or a salt thereof.

The antibody regioselectively having a bioorthogonal functional group or bioorthogonal functional groups or a salt thereof is an antibody regioselectively having a bioorthogonal functional group or bioorthogonal functional groups represented by Formula (II-1). As the antibody regioselectively having a bioorthogonal functional group or bioorthogonal functional groups represented by the above Formula (II-1), the antibody regioselectively having a bioorthogonal functional group or bioorthogonal functional groups represented by the above Formula (11-2) or (11-3) is preferably provided. As the antibody regioselectively having a bioorthogonal functional group or bioorthogonal functional groups represented by the above Formula (II-3), the antibody regioselectively having a bioorthogonal functional group or bioorthogonal functional groups represented by the above Formula (11-4) is more preferably provided. Any symbols (e.g., E, E₁, E₂, E₃, and B) in Formula (II), (II-1), (II-2), (II-3) or (II-4) and the details of terms (e.g., antibody, electrophilic group, and bioorthogonal functional group) expressed in relation to the symbols (e.g., definitions, examples, and preferred examples) are common to those of the above Formula (I) or a formula having a subordinate concept thereof.

The antibody regioselectively having a functional substance or functional substances or a salt thereof is an antibody regioselectively having a functional substance or functional substances represented by the above Formula (III-1). As the antibody regioselectively having a functional substance or functional substances represented by the above Formula (III-1), the antibody regioselectively having a functional substance or functional substances represented by the above Formula (III-2) or (III-3) is preferably provided. As the antibody regioselectively having a functional substance or functional substances represented by the above Formula (III-3), the antibody regioselectively having a functional substance or functional substances represented by the above Formula (III-4) is more preferably provided. Any symbols (e.g., E, E₁, E₂, E₃, and B) in Formula (III), (III-1), (III-2), (III-3) or (III-4) and the details of terms (e.g., antibody, electrophilic group, and bioorthogonal functional group) expressed in relation to the symbols (e.g., definitions, examples, and preferred examples) are common to those of the above Formula (I) or a formula having a subordinate concept thereof.

The antibody regioselectively having a bioorthogonal functional group or bioorthogonal functional groups or a functional substance or functional substances is the same as the above-described antibody. Such an antibody is preferably a monoclonal antibody. Examples of the isotype of the monoclonal antibody include IgG (e.g., IgG1, IgG2, IgG3, and IgG4), IgM, IgA, IgD, IgE, and IgY. The monoclonal antibody is a full-length antibody or an antibody fragment (e.g., F(ab')₂, Fab', Fab, Fv, and a single-chain antibody); the full-length antibody is preferred. Such an antibody is particularly preferably a human antibody, a humanized antibody, or a chimeric antibody having human IgG (e.g., IgG1, IgG2, IgG3, and IgG4) in a constant region.

The antibody regioselectively having a bioorthogonal functional group or bioorthogonal functional groups or a functional substance or functional substances is preferably an antibody having a bioorthogonal functional group or bioorthogonal functional groups or a functional substance or functional substances only in a constant region of the antibody, and more preferably an antibody having a bioorthogonal functional group or bioorthogonal functional groups or a functional substance or functional substances only in an Fc region of the antibody.

When the antibody regioselectively having a bioorthogonal functional group or bioorthogonal functional groups or a functional substance or functional substances comprises one or more specific amino acid residues (e.g., a lysine residue, a tyrosine residue, a threonine residue, a serine residue, or a cysteine residue) in a target region consisting of 1 to 50 continuous amino acid residues [e.g., (a) a region consisting of amino acid residues at positions 246 to 248 in the human IgG Fc region, (b) a region consisting of amino acid residues at positions 288 to 290 in the human IgG Fc region, or (c) a region consisting of an amino acid residue at position 317 in the human IgG Fc region] and comprises five or more of the specific amino acid residues in a non-target region other than the target region, the antibody regioselectively having a bioorthogonal functional group or bioorthogonal functional groups or a functional substance or functional substances can have the bioorthogonal functional group or the functional substance with 30% or more regioselectivity in the one or more specific amino acid residues comprised in the target region. The regioselectivity may be e.g., preferably 40% or more, more preferably 50% or more, even more preferably 60% or more, and particularly preferably 70% or more, 80% or more, 90% or more, 95% or more, 96% or more, 97% or more, 98% or more, 99% or more, or 100%.

The antibody regioselectively having a bioorthogonal functional group or bioorthogonal functional groups or a functional substance or functional substances can have the bioorthogonal functional group (that is, a structural unit represented by E-B) or the functional substance (that is, a structural unit represented by E-B'-F) depending on the number of heavy chains. When the antibody having a bioorthogonal functional group or bioorthogonal functional groups or a functional substance or functional substances has a plurality of (e.g., 1 to 8, preferably 1 to 4, more preferably 2) antibody heavy chains, the antibody regioselectively having a bioorthogonal functional group or bioorthogonal functional groups or a functional substance or functional substances can regioselectively have a bioorthogonal functional group or a functional substance in the same target region of the plurality of antibody heavy chains. That is, in the antibody regioselectively having a bioorthogonal functional group or bioorthogonal functional groups or a functional substance or functional substances, the modification mode by the bioorthogonal functional group or the functional substance can be the same between a plurality of (e.g., two) heavy chains.

The antibody regioselectively having a bioorthogonal functional group or bioorthogonal functional groups or a functional substance or functional substances can have the same or different types of bioorthogonal functional groups (e.g., a structural unit represented by E-B) or the same or different types of functional substances (e.g., a structural unit represented by E-B'-F) in a plurality of (e.g., 2 to 5, preferably 2 to 4, more preferably 2 or 3) target regions of one antibody heavy chain. In this case, in the antibody regioselectively having a bioorthogonal functional group or bioorthogonal functional groups or a functional substance or functional substances, the modification mode by the bioorthogonal functional group or the functional substance can be the same between a plurality of (e.g., two) heavy chains.

### 5. Compound having affinity substance to antibody and functional substance, or salt thereof

The present invention provides a compound having an affinity substance to an antibody and a functional substance, represented by Formula (IV), or a salt thereof.

A-L-E-F (IV)

wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
F is a functional substance, and
the leaving group has ability to be cleaved and eliminated from E by a reaction between the nucleophilic group and the electrophilic group.

In the compound having an affinity substance to an antibody and a functional substance, or a salt thereof, the definitions, examples, and preferred examples of the affinity substance to an antibody (A), the divalent group comprising a leaving group (L), the divalent group comprising an electrophilic group (E), and the functional substance (F) are the same as those described above. Consequently, in the compound having an affinity substance to an antibody and a functional substance, or a salt thereof, A, L, and E can be specified in a similar manner to A, L, and E in the compound having an affinity substance to an antibody and a bioorthogonal functional group, or a salt thereof (for example, refer to Formula (I)). In the compound having an affinity substance to an antibody and a functional substance, or a salt thereof, F can be specified in a similar manner to F in the antibody having a functional substance or functional substances or a salt thereof (for example, refer to Formula (III)).

In a preferred embodiment, the compound represented by Formula (IV) may be a compound represented by the following Formula (IV-1):

A-L₁-L₂-E₁-E₂-E₃-F (IV-1)

wherein
A and F are the same as those in Formula (IV),
L₁ is a bond or a divalent group,
L₂ is a leaving group,
E₁ is an electrophilic group (i) coupled with a leaving group and (ii) having ability to react with a nucleophilic group in an antibody,
E₂ is a group represented by (a)-X-Y- [where X which binds to E₁ is C(R₁) (R₂) (where R₁ and R₂ are each independently a hydrogen atom or C₁₋₆ alkyl), N(R₃) (where R₃ is a hydrogen atom or C₁₋₆ alkyl), O, S, or Se, and Y which binds to E₃ is C(R₄) (R₅) (where R₄ and R₅ are each independently a hydrogen atom or C₁₋₆ alkyl), or (b) the following formula (i): 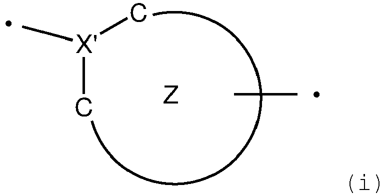
   (where the ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms. · is a bond),
   E₃ is a divalent group when E₂ is -X-Y-, and is a bond or a divalent group when E₂ is a group represented by Formula (i), and
   the leaving group has ability to be cleaved and eliminated from E₁ by a reaction between the nucleophilic group and the electrophilic group].

In Formula (IV-1), the definitions, examples, and preferred examples of the leaving group represented by L₂ are the same as those described for (a) to (c) in the above "1-3. Divalent group (L) comprising leaving group".

In a preferred specific embodiment, the compound represented by Formula (IV-1) may be a compound represented by the following Formula (IV-2):

A-L₁-L₂-E₁-X-Y-E₃-F (IV-2)

wherein
A, L₁, X, Y, and F are the same as those in Formula (IV-1),
L₂ is
   (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R) -, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-, - SO₂-, and -CH₂-, and
E₃ is a divalent group.

In another preferred specific embodiment, the compound represented by Formula (IV-1) may be a compound represented by the following Formula (IV-3): 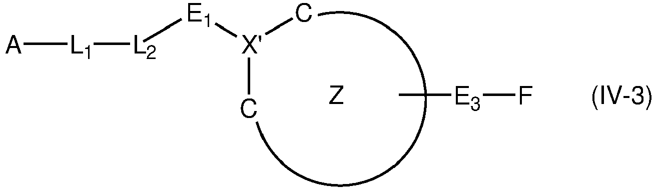 wherein
A, L₁, ring Z, ring-constituting atom X', and F are the same as those in the above Formula (IV-1),
L₂ is
   (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-,-SO₂-, and -CH₂-, and
E₃ is a bond or a divalent group.

The compound represented by Formula (IV-3) may be preferably a compound represented by the following formula (IV-4) : 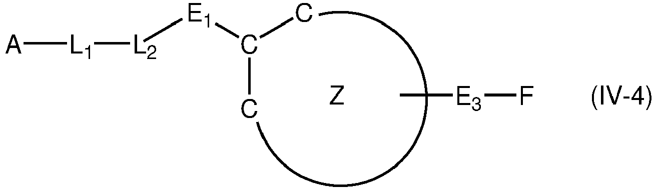 wherein
A, L₁, and F are the same as those in the above Formula (IV-1),
L₂ is
   (a) ring P-Q- [where ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl),
   (b) heteroarylene, or
   (c) -Q- [where Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- (where R is a hydrogen atom or C₁₋₆ alkyl)],
E₁ is selected from the group consisting of -C(=O)-, - SO₂-, and -CH₂-,
ring Z is a divalent cyclic group in which all of a ring-constituting atom which binds to E₁ and ring-constituting atoms on both sides thereof are carbon atoms, and
E₃ is a bond or a divalent group.

In the compounds represented by the above Formulae (IV-1) to (IV-4), the definitions, examples, and preferred examples of A, L₁, L₂, E₁, E₂, E₃, F, -X-Y-, C₁₋₆ alkyl in R₁ to R₅, and the group represented by Formula (i) (e.g., a divalent cyclic group or a divalent heterocyclic group) are the same as those described above. The definitions, examples, and preferred examples of (a) to (c) each of which is L₂, ring Z (e.g., a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms), ring P, and a group such as Q (e.g., C₁₋₆ alkyl in R) are also the same as those described above.

The compound having an affinity substance to an antibody and a functional substance, or a salt thereof can be prepared as appropriate by reacting a compound having an affinity substance to an antibody and a bioorthogonal functional group, or a salt thereof with a functional substance as described above via the bioorthogonal functional group. Such a reaction can be conducted in an appropriate reaction system such as an organic solvent system or an aqueous solution system at an appropriate temperature (e.g., about 15°C to 200°C). The reaction system may comprise an appropriate catalyst. The reaction time is e.g., 1 minute to 20 hours, preferably 10 minutes to 15 hours, more preferably 20 minutes to 10 hours, and even more preferably 30 minutes to 8 hours.

In the reaction system, a molar ratio (Y/X) of the compound having an affinity substance to an antibody and a bioorthogonal functional group, or a salt thereof to the functional substance (X) is not limited to a particular ratio because it varies in accordance with the types of the structural unit and the affinity substance, the number of sites in the affinity substance to be modified with the structural unit, and the like. However, the molar ratio (Y/X) is e.g., 0.01 to 100, preferably 0.05 to 20, and more preferably 0.1 to 10.

Determination of the formation of the compound having an affinity substance to an antibody and a functional substance, or a salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. The compound having an affinity substance to an antibody and a functional substance, or a salt thereof can be purified as appropriate by any method such as chromatography (e.g., the pieces of chromatography described above and affinity chromatography).

### 6. Method for producing antibody having functional substance using compound having affinity substance to antibody and functional substance, or salt thereof

The present invention provides a method for producing an antibody having a functional substance or functional substances or a salt thereof, the method comprising the following.

Reacting a compound having an affinity substance to an antibody and a functional substance, represented by the following Formula (IV):

A-L-E-F (IV)

wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
F is a functional substance, and
the leaving group has ability to be cleaved and eliminated from E by a reaction between the nucleophilic group and the electrophilic group, or a salt thereof with an antibody
to form an antibody having a functional substance or functional substances, represented by the following Formula (V) :

Ab-E-F (V)

wherein
Ab is an antibody, and
E and F are the same as those in the above Formula (IV), or a salt thereof.

The antibody having a functional substance or functional substances produced by the production method of the present invention is preferably an antibody regioselectively having a functional substance or functional substances. In this case, the antibody regioselectively having a functional substance or functional substances, represented by Formula (V) can be produced. The antibody regioselectively having a functional substance or functional substances is preferably an antibody having a functional substance or functional substances only in a constant region, and more preferably an antibody having a functional substance or functional substances only in an Fc region.

When the antibody (Ab) comprises one or more specific amino acid residues (e.g., a lysine residue, a tyrosine residue, a threonine residue, a serine residue, or a cysteine residue) in a target region consisting of 1 to 50 continuous amino acid residues [e.g., a region consisting of amino acid residues at positions 246 to 248 in the human IgG Fc region, (b) a region consisting of amino acid residues at positions 288 to 290 in the human IgG Fc region, or (c) a region consisting of an amino acid residue at position 317 in the human IgG Fc region] and comprises five or more of the specific amino acid residues in a non-target region other than the target region, a partial structure other than the antibody can bind to one or more specific amino acid residues comprised in the target region with 30% or more regioselectivity. The regioselectivity may be e.g., preferably 40% or more, more preferably 50% or more, even more preferably 60% or more, and particularly preferably 70% or more, 80% or more, 90% or more, 95% or more, 96% or more, 97% or more, 98% or more, 99% or more, or 100%.

The antibody having a functional substance or functional substances produced by the production method of the present invention can have a functional substance (that is, a structural unit represented by E-F) depending on the number of heavy chains. Consequently, by using an antibody having a plurality of (e.g., 1 to 8, preferably 1 to 4, more preferably 2) antibody heavy chains in the production method of the present invention, it is possible to produce an antibody regioselectively having a plurality of structural units represented by E-F (or a plurality of structural units having a subordinate concept thereof) in the same target region of the plurality of antibody heavy chains. For example, by using an antibody having two antibody heavy chains (e.g., IgG, IgD, IgE, F(ab')₂ antibody, Fc region protein, or Fc fusion protein) in the production method of the present invention, it is possible to produce an antibody regioselectively having two structural units represented by E-F in the same target region of the two antibody heavy chains. That is, in the antibody having a functional substance or functional substances, the modification mode by the functional substance can be the same between a plurality of (e.g., two) heavy chains.

The antibody having a functional substance or functional substances can have the same or different types of functional substances (e.g., a structural unit represented by E-F) in a plurality of (e.g., 2 to 5, preferably 2 to 4, more preferably 2 or 3) target regions of one antibody heavy chain. In this case, in the antibody having a functional substance or functional substances, the modification mode by the functional substance can be the same between a plurality of (e.g., two) heavy chains.

The production method of the present invention may comprise subjecting a formed antibody to a specific treatment to form an antibody having a functional substance or functional substances and further modified. Examples of such a specific treatment include an antibody fragmentation treatment (e.g., a treatment with a specific protease such as papain or pepsin).

When the compound represented by Formula (IV) or a salt thereof is used in the production method of the present invention, an antibody having a functional substance or functional substances, represented by the above Formula (V) can be produced.

In a preferred embodiment, when a compound represented by Formula (IV-1) is used as the compound represented by Formula (IV) in the production method of the present invention, an antibody having a functional substance or functional substances, represented by the following Formula (V-1) can be produced.

Ab-E₁-E₂-E₃-F (V-1)

wherein
Ab is an antibody, and
E₁, E₂, E₃, and F are the same as those in the above Formula (IV-1).

In a preferred specific embodiment, when a compound represented by Formula (IV-2) is used as the compound represented by Formula (IV-1) in the production method of the present invention, an antibody having a functional substance or functional substances, represented by the following Formula (V-2) can be produced.

Ab-E₁-X-Y-E₃-F (V-2)

wherein
Ab is an antibody, and
E₁, X, Y, E₃, and F are the same as those in the above Formula (IV-2).

In another preferred specific embodiment, when a compound represented by Formula (IV-3) is used as the compound represented by Formula (IV-1) in the production method of the present invention, an antibody having a functional substance or functional substances, represented by the following Formula (V-3) can be produced. 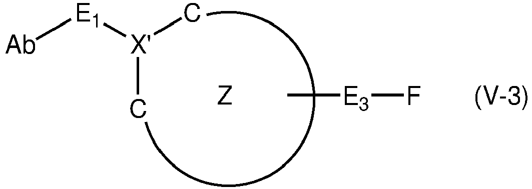 wherein
Ab is an antibody, and
E₁, ring Z, ring-constituting atoms X', E₃, and F are the same as those in the above Formula (IV-3).

When a compound represented by Formula (IV-4) is used as the compound represented by Formula (IV-3) in the production method of the present invention, an antibody having a functional substance or functional substances, represented by the following Formula (V-4) can be preferably produced. 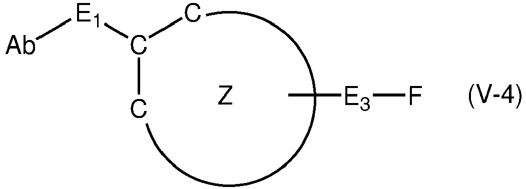 wherein
Ab is an antibody, and
E₁, ring Z, E₃, and F are the same as those in the above Formula (IV-4).

The compound having an affinity substance to an antibody and a functional substance, or a salt thereof has the electrophilic group in E or the electrophilic group in E₁, and therefore can react with the antibody. Such a reaction can be conducted as appropriate under a condition incapable of causing denaturation or decomposition (e.g., cleavage of an amide bond) of proteins (a mild condition). Such a reaction can be conducted in an appropriate reaction system such as a buffer at room temperature (e.g., about 15°C to 30°C), for example. The pH of the buffer is e.g., 5 to 9, preferably 5.5 to 8.5, and more preferably 6.0 to 8.0. The buffer may comprise an appropriate catalyst. The reaction time is e.g., 1 minute to 20 hours, preferably 10 minutes to 15 hours, more preferably 20 minutes to 10 hours, and even more preferably 30 minutes to 8 hours. For the details of such a reaction, refer to G. J. L. Bernardes et al., Chem. Rev., 115, 2174 (2015); G. J. L. Bernardes et al., Chem. Asian. J., 4,630 (2009); B. G. Davies et al., Nat. Commun., 5, 4740 (2014); A. Wagner et al., Bioconjugate. Chem., 25,825 (2014), for example.

In the reaction system, a molar ratio (Y/X) of the compound having an affinity substance to an antibody and a functional substance, or a salt thereof (Y) to the antibody (X) is not limited to a particular ratio because it varies in accordance with the types of the compound having an affinity substance to an antibody and a functional substance, or a salt thereof, and the antibody, the number of sites in the antibody to be modified by the compound having an affinity substance to an antibody and a bioorthogonal functional group, or a salt thereof (e.g., DAR), and the like. However, the molar ratio (Y/X) is e.g., 0.1 to 100, preferably 0.5 to 80, more preferably 1 to 70, even more preferably 2 to 50, and particularly preferably 3 to 30.

Determination of the formation of the antibody having a functional substance or functional substances, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of functional substances that the antibody having a functional substance or functional substances has can be performed by electrophoresis, chromatography, or mass spectrometry, for example, and preferably mass spectrometry. The antibody having a functional substance or functional substances can be purified as appropriate by any method such as chromatography (e.g., the pieces of chromatography described above and affinity chromatography).

### 7. Salt

In the present invention, examples of the salt include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 8. Uses

The compound of the present invention having an affinity substance to an antibody and a bioorthogonal functional group, or a salt thereof is useful for regioselective modification of an antibody by the bioorthogonal functional group, for example. Consequently, the present invention provides a reagent of regioselectively modifying an antibody by a bioorthogonal functional group, the reagent comprising a compound having an affinity substance to an antibody and a bioorthogonal functional group, or a salt thereof. In the reagent of regioselectively modifying an antibody by a bioorthogonal functional group, the details of the compound having an affinity substance to an antibody and a bioorthogonal functional group, or a salt thereof (e.g., definitions, examples, and preferred examples) are the same as those described above.

The compound of the present invention having an affinity substance to an antibody and a functional substance, or a salt thereof is useful for regioselective modification of an antibody by the functional substance, for example. Consequently, the present invention provides a reagent of regioselectively modifying an antibody by a functional substance, the reagent comprising a compound having an affinity substance to an antibody and a functional substance, or a salt thereof. In the reagent of regioselectively modifying an antibody by a functional substance, the details of the compound having an affinity substance to an antibody and a functional substance, or a salt thereof (e.g., definitions, examples, and preferred examples) are the same as those described above.

The details (e.g., definitions, examples, and preferred examples) of the regioselective modification of an antibody modified in the regioselective modifying reagent of the present invention are the same as those described above.

The regioselective modifying reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Tris-hydrochloric acid buffer, a carbonic acid-bicarbonic acid buffer, an aqueous boric acid solution, a glycine-sodium hydroxide buffer, and a citric acid buffer); buffers are preferred. The pH of solutions is e.g., 5.0 to 9.0 and preferably 5.5 to 8.5. The regioselective modifying reagent of the present invention can be provided in a liquid form or a powder form (e.g., freeze-dried powder).

The antibody regioselectively having a bioorthogonal functional group or bioorthogonal functional groups or a salt thereof is useful as an intermediate in preparation of an antibody regioselectively having a functional substance or functional substances or a salt thereof, for example.

The antibody regioselectively having a functional substance or functional substances or a salt thereof is useful as pharmaceuticals or reagents (e.g., diagnostic reagents and reagents for research), for example, and particularly as pharmaceuticals. It is reported that when the number of bonds and the bond positions of a drug of an antibody drug conjugate (ADC) are changed, pharmacokinetics, a releasing rate of the drug, and effects change. Given these circumstances, next-generation ADCs are required to control the number and positions of a drug to be conjugated. It is believed that when the number and positions are constant, the problems of expected efficacy, variations in conjugate medicines, and lot difference, or what is called regulation, will be solved. The antibody or salt thereof of the present invention regioselectively having a functional substance or functional substances can solve such a problem of regulation. Consequently, the antibody or salt thereof of the present invention regioselectively having a functional substance or functional substances may be provided in the form of a pharmaceutical composition. Such a pharmaceutical composition may comprise a pharmaceutically allowable carrier in addition to the antibody or salt thereof regioselectively having a functional substance or functional substances. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The antibody or salt thereof of the present invention regioselectively having a functional substance or functional substances may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set as appropriate.

### EXAMPLES

The present invention is explained by the following Examples in more detail, but the present invention is not limited to the following Examples.

### [Example 1: Synthesis of IgG1 Fc affinity substance]

### (1-1) Synthesis of affinity peptide to antibody

All the peptides illustrated below, which are affinity substances to an antibody, were prepared by similar methods. A compound having an N-terminal capped with an acetyl group (compounds 1, 2, and 32 to 53) and a compound having an N-terminal capped with 3-(triphenylmethylthio) propionic acid (compounds 3, 31, and 54) were prepared by solid phase peptide synthesis using a Rink amide resin by Fmoc method, and stirred for three hours in a solution of trifluoroacetic acid : water : triisopropylsilane : ethanedithiol = 94 : 2.5 : 1.0 : 2.5, thereby performing cutting out from the resin and deprotection. The resin was removed by filtration, diethyl ether was added for precipitation, and the diethyl ether was removed by decantation to obtain a peptide as crude crystals. This peptide was purified by preparative HPLC to obtain an affinity peptide as a product.

Each of compounds 35 to 53, which are peptides each having an S-S bond in a molecule thereof (excluding a peptide containing methionine), was synthesized by a method described below after a linear peptide as a precursor was obtained by the method described above. Tens mg of the obtained precursor was dissolved in 1 mL of DMSO, 100 µL of NH3/MeOH and 10 µL of H₂O₂ were added thereto, and the resulting mixture was stirred overnight. It was determined by LCMS that the reaction was completed, and then the product was purified by preparative HPLC to obtain an affinity peptide as a target product.

Each of compounds 33 and 34, which are peptides each having an S-S bond in a molecule thereof (containing methionine), was synthesized by a method described below after a linear peptide as a precursor was obtained by the method described above. Tens mg of the obtained precursor was dissolved in 20 mL of 0.1 M Tris-HCl buffer (pH 8.00), 5.0 eq of glutathione oxidation type was added thereto, and the resulting mixture was stirred overnight. It was determined by LCMS that the reaction was completed, and then the product was purified by preparative HPLC to obtain an affinity peptide as a target product. 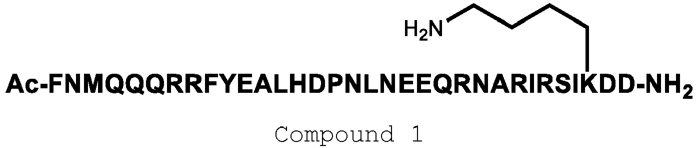 (Amino acid sequence of peptide portion: SEQ ID NO: 5)
MS (ESI)m/z:z=3 1392[M+3H]³⁺,z=4 1044[M+4H]⁴⁺ 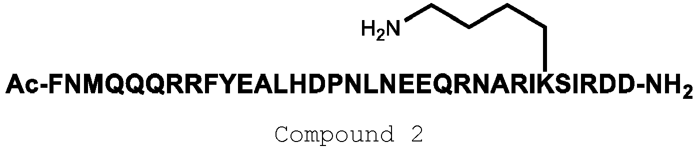 (Amino acid sequence of peptide portion: SEQ ID NO: 6)
MS (ESI)m/z:z=3 1392[M+3H]³,z=4 1044[M+4H]⁴⁺ 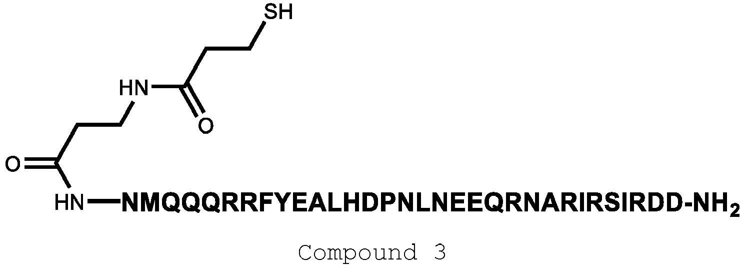 (Amino acid sequence of peptide portion: SEQ ID NO: 7)
MS (ESI)m/z:z=3 1478[M+3H]³⁺,z=4 1108[M+4H]⁴⁺ 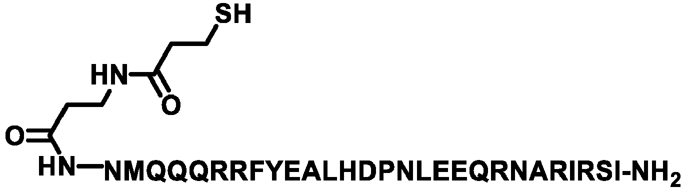 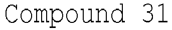 (Amino acid sequence of peptide portion: SEQ ID NO: 97)
MS (ESI)m/z:z=2 1892 [M+2H]²⁺, Z=3 1262 [M+3H]³⁺, Z=4 946 [M+4H]⁴⁺, z=5 757 [M+5H]⁵⁺ 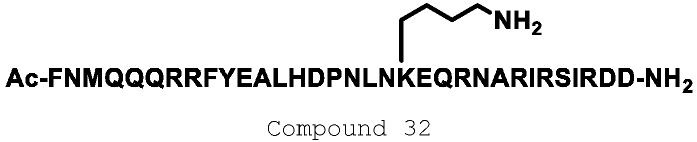 (Amino acid sequence of peptide portion: SEQ ID NO: 98)
MS (ESI)m/z:z=3 1401 [M+3H]³⁺, Z=4 1051 [M+4H]⁴⁺, z=5 841 [M+5H]⁵⁺ 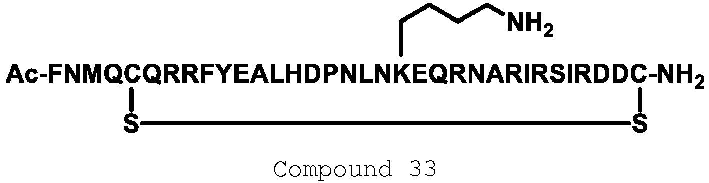 (Amino acid sequence of peptide portion: SEQ ID NO: 41)
MS (ESI)m/z:z=3 1426 [M+3H]³⁺, Z=4 1070 [M+4H]⁴⁺, z=5 859 [M+5H]⁵⁺ 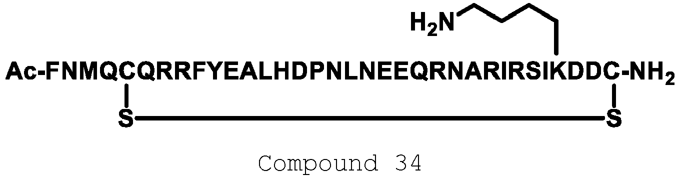 (Amino acid sequence of peptide portion: SEQ ID NO: 42)
MS (ESI)m/z:z=3 1417 [M+3H]³⁺, Z=4 1063 [M+4H]⁴⁺, z=5 851 [M+5H]⁵⁺ 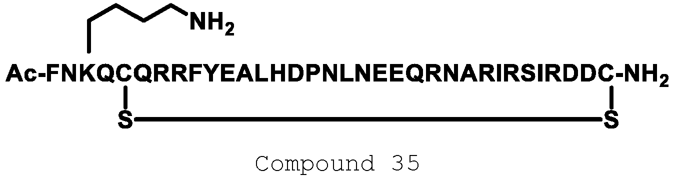 (Amino acid sequence of peptide portion: SEQ ID NO: 43)
MS (ESI)m/z:z=3 1425 [M+3H]³⁺, Z=4 1069 [M+4H]⁴⁺, z=5 855 [M+5H]⁵⁺ 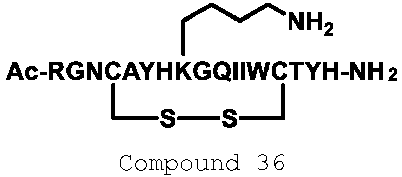 (Amino acid sequence of peptide portion: SEQ ID NO: 46)
MS (ESI)m/z:z=1 2090 [M+1H]⁺, Z=2 1045 [M+2H]²⁺, Z=3 697 [M+3H]³⁺ 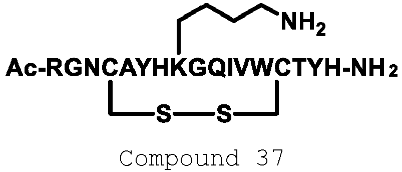 (Amino acid sequence of peptide portion: SEQ ID NO: 47)
MS (ESI)m/z:z=1 2074 [M+1H]⁺, Z=2 1037 [M+2H]²⁺, Z=3 692 [M+3H]³⁺ 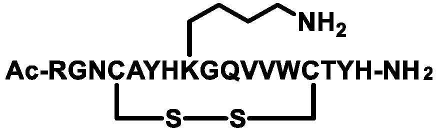 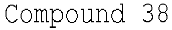 (Amino acid sequence of peptide portion: SEQ ID NO: 48)
MS (ESI)m/z:z=1 2061 [M+1H]⁺, Z=2 1031 [M+2H]²⁺, Z=3 687 [M+3H]³⁺ 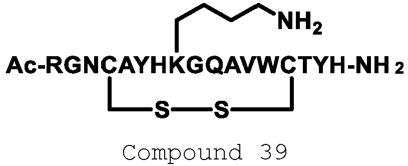 (Amino acid sequence of peptide portion: SEQ ID NO: 49)
MS (ESI)m/z:z=1 2032 [M+1H]⁺, Z=2 1016 [M+2H]²⁺, Z=3 678 [M+3H]³⁺ 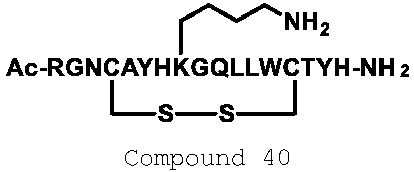 (Amino acid sequence of peptide portion: SEQ ID NO: 50)
MS (ESI)m/z:z=1 2089 [M+1H]⁺, Z=2 1044 [M+2H]²⁺, Z=3 696 [M+3H]³⁺ 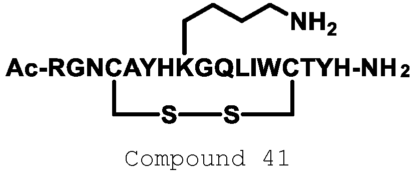 (Amino acid sequence of peptide portion: SEQ ID NO: 51)
MS (ESI)m/z:z=1 2088 [M+1H]⁺, Z=2 1044 [M+2H]²⁺, Z=3 696 [M+3H]³⁺ 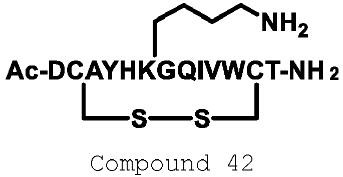 (Amino acid sequence of peptide portion: SEQ ID NO: 52)
MS (ESI)m/z:z=1 1561 [M+H]⁺, Z=2 781 [M+2H]²⁺ 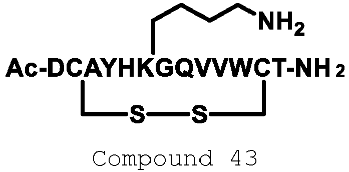 (Amino acid sequence of peptide portion: SEQ ID NO: 53)
MS (ESI)m/z:z=1 1548 [M+1H]⁺, Z=2 774 [M+2H]²⁺ 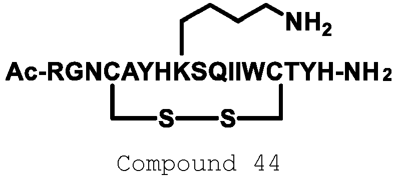 (Amino acid sequence of peptide portion: SEQ ID NO: 55)
MS (ESI)m/z:z=2 1059 [M+2H]²⁺, Z=3 706 [M+3H]³⁺ 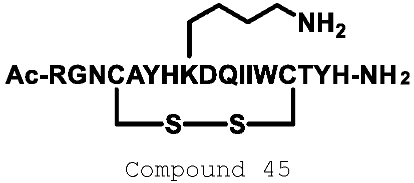 (Amino acid sequence of peptide portion: SEQ ID NO: 57)
MS (ESI)m/z:z=2 1074 [M+2H]²⁺, Z=3 716 [M+3H]³⁺ 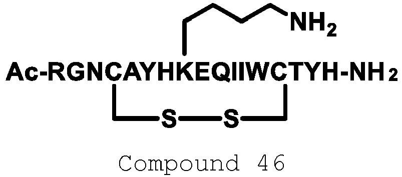 (Amino acid sequence of peptide portion: SEQ ID NO: 59)
MS (ESI)m/z:z=2 1081 [M+2H]²⁺, Z=3 721 [M+3H]³⁺, Z=4 541 [M+4H]⁴⁺ 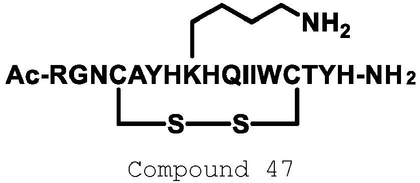 (Amino acid sequence of peptide portion: SEQ ID NO: 63)
MS (ESI)m/z:z=2 1085 [M+2H]²⁺, Z=3 723 [M+3H]³⁺, Z=4 543 [M+4H]⁴⁺ 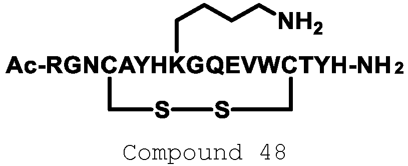 (Amino acid sequence of peptide portion: SEQ ID NO: 71)
MS (ESI)m/z:z=2 1045 [M+2H]²⁺, Z=3 697 [M+3H]³⁺ 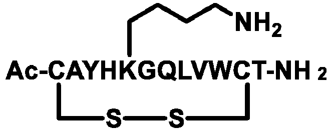 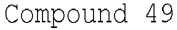 (Amino acid sequence of peptide portion: SEQ ID NO: 72)
MS (ESI)m/z:z=1 1345 [M+1H]⁺, Z=2 673 [M+2H]²⁺ 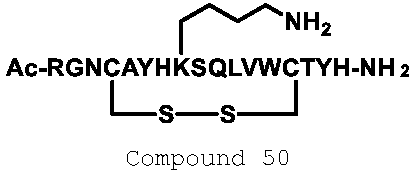 (Amino acid sequence of peptide portion: SEQ ID NO: 77)
MS (ESI)m/z:z=2 1052 [M+2H]²⁺, Z=3 702 [M+3H]³⁺ 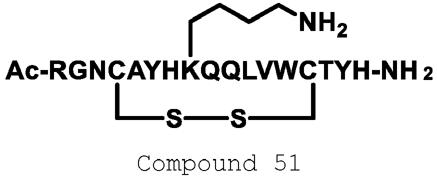 (Amino acid sequence of peptide portion: SEQ ID NO: 81)
MS (ESI)m/z:z=2 1073 [M+2H]²⁺, Z=3 715 [M+3H]³⁺ 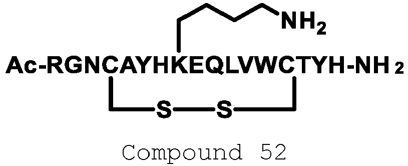 (Amino acid sequence of peptide portion: SEQ ID NO: 82)
MS (ESI)m/z:z=2 1073 [M+2H]²⁺, Z=3 716 [M+3H]³⁺ 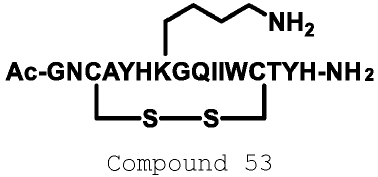 (Amino acid sequence of peptide portion: SEQ ID NO: 99)
MS (ESI)m/z:z=2 966 [M+2H]²⁺, Z=3 644 [M+3H]³⁺ 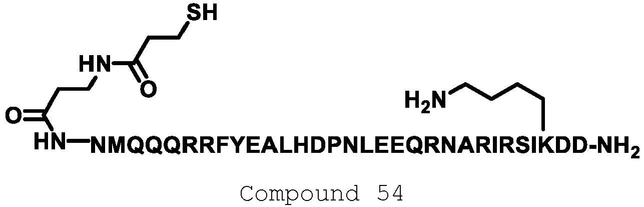 (Amino acid sequence of peptide portion: SEQ ID NO: 100)
MS (ESI)m/z:z=3 1381 [M+3H]³⁺, Z=4 1036 [M+4H]⁴⁺, z=5 829 [M+5H]⁵⁺

### (1-2) Synthesis of affinity peptide azide adduct to antibody

The peptide-azide adduct (compounds 4 and 5) illustrated below was synthesized as follows. Using an amino acid in which only a Lys residue to be functionalized later with a mtt group, a compound having an N-terminal capped with an acetyl group was prepared by solid phase peptide synthesis with a Rink amide resin by Fmoc method. The product was stirred in a solution of dichloromethane : trifluoroacetic acid : triisopropylsilane = 90 : 5 : 5 for one hour to deprotect only the mtt group. The resin was washed with DMF, then dissolved in triethylamine (50 molar equivalents), azidoacetyl acid NHS ester (10 molar equivalents), and DMF 4 mL), and added. The resulting mixture was stirred for 16 hours. The solution was removed, and then the product was stirred in a solution of trifluoroacetic acid : water : triisopropylsilane = 95 : 2.5 : 2.5 for one hour, thereby performing cutting out from the resin and deprotection. The resin was removed by filtration, diethyl ether was added for precipitation, and the diethyl ether was removed by decantation to obtain a peptide as crude crystals. This peptide was purified by preparative HPLC to obtain an affinity peptide azide adduct as a product. 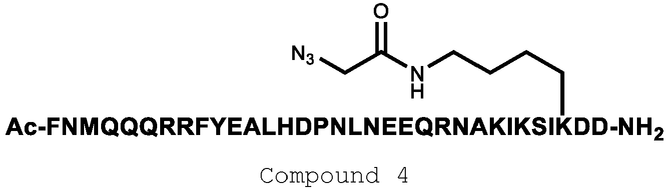 (Amino acid sequence of peptide portion: SEQ ID NO: 8)
MS(ESI)m/z:z=3 1401[M+3H]³⁺,z=4 1051[M+4H]⁴⁺ 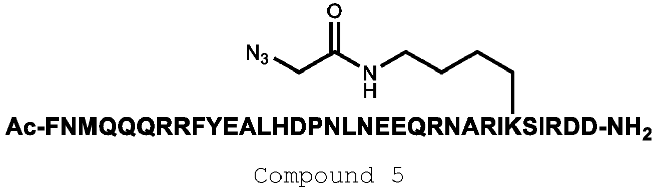 (Amino acid sequence of peptide portion: SEQ ID NO: 6)
MS(ESI)m/z:z=3 1420[M+3H]³⁺,z=4 1065[M+4H]⁴⁺

### [Example 2: Synthesis of antibody-modifying linker and coupling thereof with IgG1 Fc-affinity peptide azide adduct]

### (2-1) Synthesis of Imidazolylcarbonyl Compound

### (2-1-1) Synthesis of Imidazolylcarbonyl Compound (compound 6)

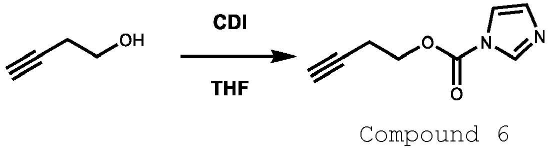

3-Butyn-1-ol (0.100 g, 1.32 mmol) and carbonyldiimidazole (263 mg, 1.62 mmol) were dissolved in a THF solvent and stirred at room temperature for one hour. The reaction solution was diluted with ethyl acetate and was washed with water and brine. Thereafter, sodium sulfate was added thereto, and the resulting mixture was allowed to stand for five minutes. Sodium sulfate was removed by filtration, and the resulting solution was concentrated under reduced pressure to obtain a crude product, which was then purified by silica gel column chromatography. The fraction containing the product was collected and concentrated under reduced pressure to obtain 1H-imidazole-1-carboxylic acid-3-butynyl ester (0.180 g, 1.10 mmol) corresponding to compound 6.
¹H NMR(400MHz,Chloroform-d) δ 2.08(t, J=2.7Hz, 1H), 2.73(td, J=6.6, 2.7Hz, 2H),4.54(t,J=6.6Hz,2 H), 7.11(s, 1H), 7.43(s, 1H), 8.18(s, 1H).
MS(ESI) m/z:165 [M+Na]⁺

### (2-1-2) Synthesis of Imidazolylcarbonyl Compound (compound 7)

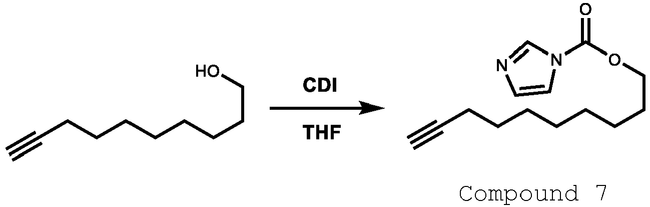

9-Decin-1-ol (0.100 g, 0.745 mmol) and carbonyldiimidazole (148 mg, 0.916 mmol) were dissolved in a THF solvent and stirred at room temperature for one hour. The reaction solution was diluted with ethyl acetate and was washed with water and brine. Thereafter, sodium sulfate was added thereto, and the resulting mixture was allowed to stand for five minutes. Sodium sulfate was removed by filtration, and the resulting solution was concentrated under reduced pressure to obtain a crude product, which was then purified by silica gel column chromatography. The fraction containing the product was collected and concentrated under reduced pressure to obtain 1H-imidazole-1-carboxylic acid-9-butynyl ester (0.152 g, 0.612 mmol) corresponding to compound 7.
¹H NMR(400MHz,Chloroform-d) δ 1.33-1.50(m, 10H), 1.82(m, 2H), 1.96(t, J=2.6Hz, 1H) 2.21(td, J=6.8, 2.6H z,2H), 4.43(t, J=6.8Hz, 2H)7.10(s, 1H), 7.45(brs, 1H), 8.16(s, 1H).
MS(ESI) m/z:271[M+Na]⁺

### (2-1-3) Synthesis of Imidazolylcarbonyl Compound (compound 9)

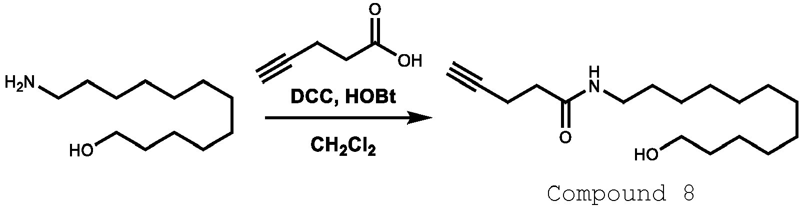

N,N'-dicyclohexylcarbodiimide (113 mg, 0.546 mmol), 1-hydroxybenzotriazole monohydrate (78.1 mg, 0.546 mmol), and 4-pentynoic acid (53.6 mg, 0.546 mmol) were dissolved in a dichloromethane solvent, and stirred at room temperature for one. Thereafter, 12-amino-dodecanol (0.100 g, 0.497 mmol) was added thereto, and the resulting mixture was stirred at room temperature for four hours. The reaction solution was diluted with ethyl acetate and was washed with water and brine. Thereafter, sodium sulfate was added thereto, and the resulting mixture was allowed to stand for five minutes. Sodium sulfate was removed by filtration, and the resulting solution was concentrated under reduced pressure to obtain 12-(pent-4-yn-1-oxo) aminododecan-1-ol (0.102 g, 0.363 mmol) corresponding to compound 8.
MS(ESI) m/z:281[M+H]⁺

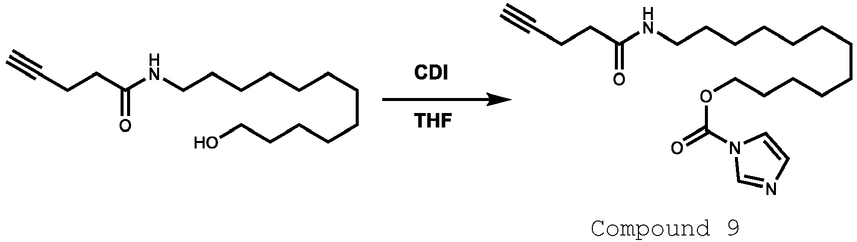

12-(Pent-4-ynamido) dodecan-1-ol (25.0 mg, 0.089 mmol) and carbonyldiimidazole (29.1 mg, 0.178 mmol) were dissolved in a THF solvent and stirred at room temperature for one hour. The reaction solution was diluted with ethyl acetate and was washed with water and brine. Thereafter, sodium sulfate was added thereto, and the resulting mixture was allowed to stand for five minutes. Sodium sulfate was removed by filtration, and the resulting solution was concentrated under reduced pressure to obtain a crude product, which was then purified by silica gel column chromatography. The fraction containing the product was collected and concentrated under reduced pressure to obtain 1H-imidazole-1-carboxylic acid-12-(pent-4-yn-1-oxo) aminododecanyl ester (12.0 mg, 0.032 mmol) corresponding to compound 9.
¹H NMR(400MHz,Chloroform-d) δ 1.23-1.50(m, 20H),1.81(t, J=2.6Hz, 1H),2.36(m,2H)2.53(td,J=6.8,2.6H z, 2H),3.23(t, J=6.8Hz, 2H)4.41(t, J=6.8Hz, 2H), 5.64(brs, 1H), 7.0 7(s, 1H), 7.43(brs, 1H), 8.13(s, 1H).
MS(ESI) m/z:398[M+Na]⁺

### (2-2) Synthesis of Imidazolylcarbonyl Compound and Coupling Thereof with Affinity Peptide Azide Compound

The affinity peptide azide compound (compound 4) synthesized in Example 1 was coupled with the imidazolylcarbonyl compounds (compounds 6, 7, and 9) synthesized in Example 2 by similar methods. The peptide azide compound was dissolved in 100 mM phosphate buffer (pH 7.0), and aminoguanidine hydrochloride (20 molar equivalents), sodium ascorbate (20 molar equivalents), and 100 mg/mL imidazolylcarbonyl compound (3 molar equivalents) dimethyl sulfoxide solution were added thereto. A separately prepared aqueous solution of copper sulfate monohydrate (4 molar equivalents) and tris(3-hydroxypropyltriazolylmethyl) amine (20 molar equivalents) was added to this reaction mixture, and the resulting mixture was stirred. The reaction was monitored by LC-MS, and disappearance of the raw materials was determined. Thereafter, the product was concentrated and diluted with water repeatedly four times by ultrafiltration (Amicon Ultra, 3K MWCO), and the resulting aqueous solution was freeze-dried to obtain a peptide-imidazolylcarbonyl compound (compounds 10, 11, and 12) illustrated below. 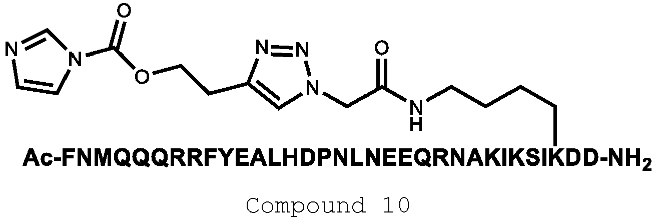 (Amino acid sequence of peptide portion: SEQ ID NO: 8)
MS (ESI)m/z:z=3 1455[M+3H]³⁺,z=4 1092[M+4H]⁴⁺ 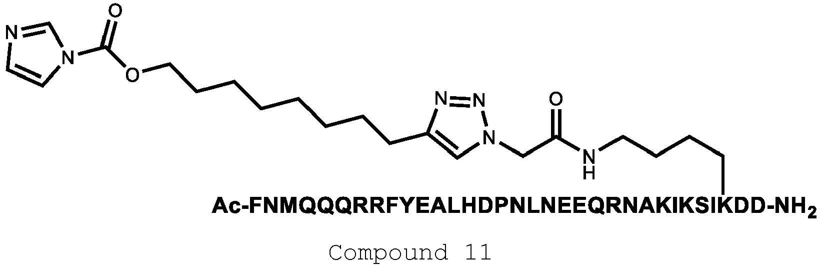 (Amino acid sequence of peptide portion: SEQ ID NO: 8)
MS (ESI)m/z:z=3 1483[M+3H]³⁺,z=4 1113[M+4H]⁴⁺ 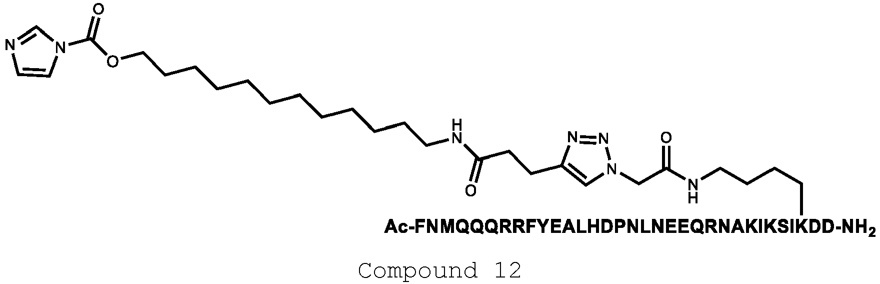 (Amino acid sequence of peptide portion: SEQ ID NO: 8)
MS (ESI)m/z:z=3 1525[M+3H]³⁺,z=4 1144[M+4H]⁴⁺

### [Reference Example 1: Verification of difference in reactivity due to difference in number of atoms from affinity peptide to antibody-modifying group (electrophilic group) by model experiment]

### (1-1) Synthesis of IgG Antibody Trastuzumab-Peptide Conjugate

20 µg of anti-HER2 IgG antibody trastuzumab (Chugai Pharmaceutical Co. Ltd.) was dissolved in 2.0 µL of 100 mM HEPES buffer (pH 7.2), 20 molar equivalents of the peptide-imidazolylcarbonyl compound (compounds 10, 11, and 12) synthesized in Example 2 (2-2) was added to the antibody, and the resulting mixture was stirred at 37°C for four hours. The reaction solution was subjected to water substitution by ultrafiltration (Amicon Ultra, 3K MWCO) to remove the peptide reagent, and the reaction was thereby stopped, thus obtaining an IgG antibody trastuzumab-peptide conjugate.

### (1-2) Analysis of IgG Antibody Trastuzumab-Peptide Conjugate by SDS-PAGE

The three types of IgG antibody trastuzumab-peptide conjugates obtained in (1-1) using three types of peptide-imidazolylcarbonyl compounds (compounds 10, 11, and 12) were subjected to SDS-PAGE (Mini-PROTEAN TGX gel, 4-20%, Bio-RAD; under reduction conditions; stained with Coomasie Brilliant Blue G-250 Stain). A result thereof is illustrated in FIG. 2. From this result, it was found that the modification reaction of the antibody progressed when compounds 10 and 11 were used, and did not progress when compound 12 was used.

### [Example 3: Synthesis of antibody affinity reagent loaded with maleimide]

### (3-1) Synthesis of protected thiol compound

### (3-1-1) Synthesis of Protected Thiol Compound (compound 14)

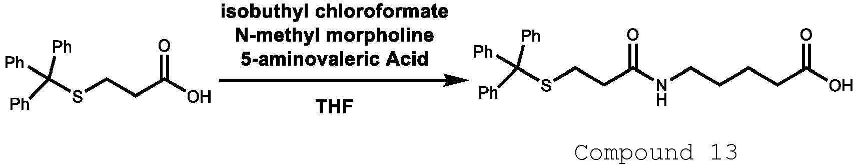

3-(Triphenylmethylthio) propionic acid (100 mg, 0.287 mmol) was dissolved in THF, isobutyl chlorocarbonate (42.7 µL, 0.316 mmol) and N-methylmorpholine (69.4 µL, 0.631 mmol) were added thereto at 0°C, and the resulting mixture was stirred for 30 minutes to prepare a corresponding mixed anhydride. 5-Aminovaleric acid (33.6 mg, 0.287 mmol) was dissolved in a 1 M aqueous sodium hydroxide solution at room temperature, and then the THF solution of the mixed anhydride described above was added dropwise thereto at room temperature. The resulting mixture was stirred for 16 hours at room temperature. Thereafter, the reaction solution was washed with water and ethyl acetate, and an aqueous phase was collected. A 6 M aqueous hydrochloric acid solution was added to the aqueous phase to adjust the pH within the system to 3.0, then separatory extraction with ethyl acetate was performed, then an organic phase was washed with brine, and then magnesium sulfate anhydride was added thereto, and allowed to stand for five minutes. Magnesium sulfate was removed by filtration, and the resulting solution was concentrated under reduced pressure to obtain a crude product, which was then purified by silica gel column chromatography. The fraction containing the product was collected and concentrated under reduced pressure to obtain 5-[3-(triphenylmethyl) sulfanyl-propyl-1-oxo] amino-hexanoic acid (110 mg, 0.246 mmol) corresponding to compound 13.
¹H NMR(400MHz,Chloroform-d) δ 1.66(m, 3H), 2.03(t, J=7.3Hz, 2H), 2.39(t, J=7.3Hz, 2H), 2.52(t, J=7 .3Hz, 2H), 3.22(q, J=6.6Hz, 2H),5.37(s, 1H),7.19-7.36(m, 9H), 7.40-7.49(m, 6H).
MS(ESI) m/z:470[M+Na]⁺

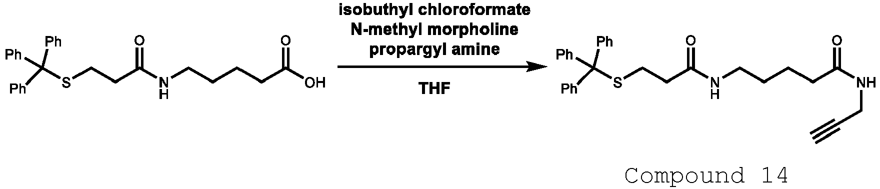

5-(3-Triphenylmethyl-propyl-1-oxo) amino-hexanoic acid (20.2 mg, 0.045 mmol) was dissolved in THF, isobutyl chlorocarbonate (6.70 µL, 0.050 mmol) and N-methylmorpholine (10.9 µL, 0.0991 mmol) were added thereto at 0°C, and the resulting mixture was stirred for 30 minutes to prepare a corresponding mixed anhydride. Propargylamine (16.6 µL, 0.226 mmol) was dissolved in a 1 M aqueous sodium hydroxide solution at room temperature, and then the THF solution of the mixed anhydride described above was added dropwise thereto at room temperature. The resulting mixture was stirred for 16 hours at room temperature. Thereafter, the reaction solution was washed with water and ethyl acetate, and an aqueous phase was collected. A 6 M aqueous hydrochloric acid solution was added to the aqueous phase to adjust the pH within the system to 3.0, then separatory extraction with ethyl acetate was performed, then an organic phase was washed with brine, and then magnesium sulfate anhydride was added thereto, and allowed to stand for five minutes. Magnesium sulfate was removed by filtration, and the resulting solution was concentrated under reduced pressure to obtain a crude product, which was then purified by silica gel column chromatography. The fraction containing the product was collected and concentrated under reduced pressure to obtain N-propynyl-5-(3-triphenylmethylsulfanyl-propyl-1-oxo) amino-hexanamide (20.0 mg, 0.041 mmol) corresponding to compound 14.
¹H NMR(400MHz,Chloroform-d) δ 1.52(dt, J=8.2, 6.5Hz, 2H), 1.59-1.72(m, 2H), 2.02-2.09(m,2H),2.17-2.28(m,2H)2.52(t,J=7.2Hz,2H),3.21(brs,2H),4.01(dd,J=5.3,2.6 Hz, 2H), 6.04(s, 1H), 7.19-7.35(m, 9H), 7.40-7.49(m, 6H).
MS(ESI) m/z:507[M+H]⁺

### (3-1-2) Synthesis of Protected Thiol Compound (compound 15)

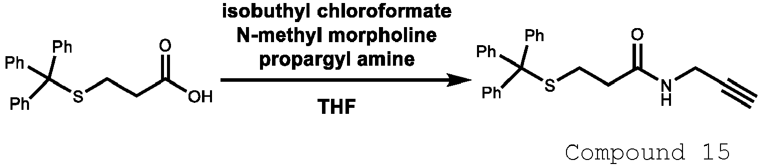

3-(Triphenylmethylthio) propionic acid (100 mg, 0.287 mmol) was dissolved in THF, isobutyl chlorocarbonate (42.7 µL, 0.316 mmol) and N-methylmorpholine (69.4 µL, 0.631 mmol) were added thereto at 0°C, and the resulting mixture was stirred for 30 minutes to prepare a corresponding mixed anhydride. Propargylamine (105 µL, 1.43 mmol) was dissolved in a 1 M aqueous sodium hydroxide solution at room temperature, and then the THF solution of the mixed anhydride described above was added dropwise thereto at room temperature. The resulting mixture was stirred for 16 hours at room temperature. Thereafter, the reaction solution was washed with water and ethyl acetate, and an aqueous phase was collected. A 6 M aqueous hydrochloric acid solution was added to the aqueous phase to adjust the pH within the system to 3.0, then separatory extraction with ethyl acetate was performed, then an organic phase was washed with brine, and then magnesium sulfate anhydride was added thereto, and allowed to stand for five minutes. Magnesium sulfate was removed by filtration, and the resulting solution was concentrated under reduced pressure to obtain N-propynyl-3-triphenylmethylsulfanyl-propanamide (110 mg, 0.286 mmol) corresponding to compound 15.
¹H NMR(400MHz,Chloroform-d) δ 2.01(t, J=7.1Hz, 2H), 2.24(t, J=2.8Hz, 1H), 2.53(t, J=7.6Hz, 2H), 3. 99(m, 2H), 5.40(brs, 1H), 7.19-7.35(m, 9H), 7.40-7.49(m, 6H).
MS(ESI) m/z:408[M+Na]⁺

### (3-2) Coupling of affinity peptide with protected thiol compound

### (3-2-1) Synthesis of Peptide-Protected Thiol Coupled Compound (compound 16)

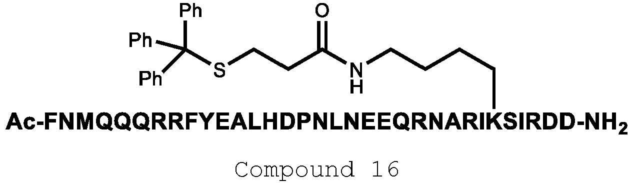

(Amino acid sequence of peptide portion: SEQ ID NO: 6)

Compound 5 synthesized in Example 1 was dissolved in N,N'-dimethylformamide. 3-(Triphenylmethylthio) propionic acid (5 molar equivalents), 1-ethyl-3-(3-dimethylaminopropyl) carbodiimide hydrochloride (15 molar equivalents), and 1-hydroxybenzotriazole (15 molar equivalents) were dissolved in N,N'-dimethylformamide and added to the system. The resulting mixture was stirred at room temperature for 12 hours and then purified by preparative HPLC. A fraction containing a target product was determined by ESI-MS, followed by freeze-drying, thus obtaining a peptide-protected thiol coupled compound (compound 16) as a product.
MS (ESI)m/z:z=3 1502[M+3H]³⁺, z=4 1125[M+4H]⁴⁺

### (3-2-2) Synthesis of Peptide-Protected Thiol Coupled Compound (compounds 17 and 18)

The affinity peptide azide compound (compound 5) synthesized in Example 1 was coupled with the protected thiol compounds (compounds 17 and 18) synthesized in (4-1-1) and (4-1-2) by similar methods. The peptide azide compound was dissolved in 100 mM phosphate buffer (pH 7.0), and aminoguanidine hydrochloride (20 molar equivalents), sodium ascorbate (20 molar equivalents), and 100 mg/mL protected thiol compound (3 molar equivalents) dimethyl sulfoxide solution were added thereto. A separately prepared aqueous solution of copper sulfate monohydrate (4 molar equivalents) and tris(3-hydroxypropyltriazolylmethyl) amine (20 molar equivalents) was added to this reaction mixture, and the resulting mixture was stirred. The reaction was monitored by LC-MS, and disappearance of the raw materials was determined. Thereafter, the product was concentrated and diluted with water repeatedly four times by ultrafiltration (Amicon Ultra-4, 3K MWCO), and the resulting aqueous solution was freeze-dried to obtain a peptide-protected thiol coupled compound (compounds 17 and 18) . 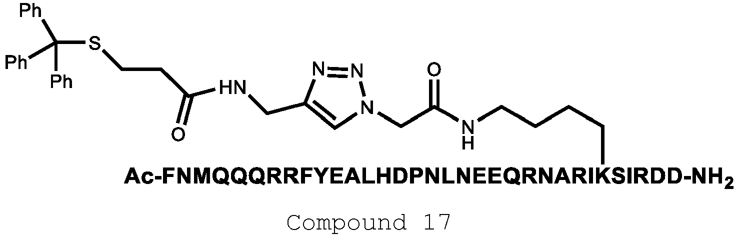 (Amino acid sequence of peptide portion: SEQ ID NO: 6)
MS(ESI) m/z:z=3 1548[M+3H]³⁺, z=4 1161[M+4H]⁴⁺ 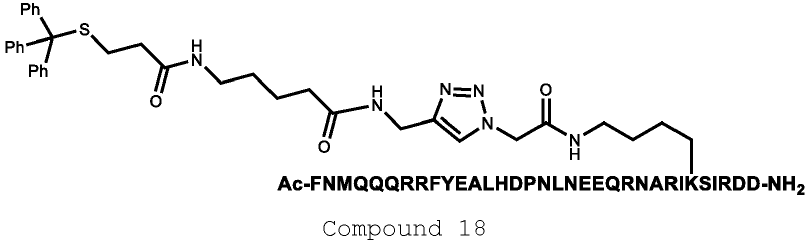 (Amino acid sequence of peptide portion: SEQ ID NO: 6)
MS(ESI) m/z:z=3 1581[M+3H]³⁺, z=4 1186[M+4H]⁴⁺

### (3-3) Synthesis of peptide-protected thiol coupled compound (compounds 19, 20, and 21)

The affinity peptide-protected thiol coupled compound (compounds 16, 17, and 18) synthesized in (3-2) was stirred with a trifluoroacetic acid : dichloromethane = 1 : 1 solution for one hour to remove a triphenylmethyl group. The completion of the reaction was determined by LC-MS, and then the product was purified by preparative HPLC. A fraction containing a target product was determined by ESI-MS, followed by freeze-drying, thus obtaining a thiol-peptide coupled compound (compounds 19, 20, and 21) as a product. 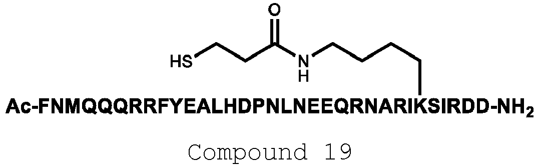 (Amino acid sequence of peptide portion: SEQ ID NO: 6)
MS(ESI) m/z:z=3 1454[M+3H]³⁺, z=4 1091[M+4H]⁴⁺ 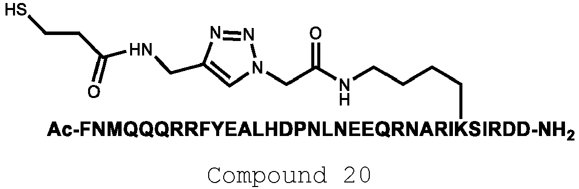 (Amino acid sequence of peptide portion: SEQ ID NO: 6)
MS(ESI) m/z:z=3 1468[M+3H]³⁺, z=4 1111[M+4H]⁴⁺ 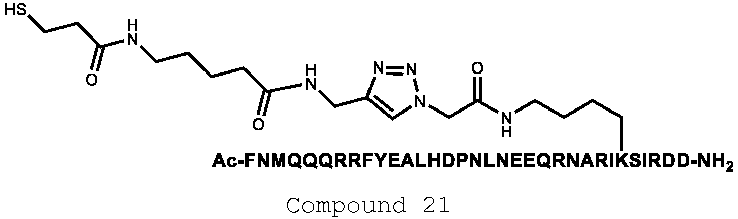 (Amino acid sequence of peptide portion: SEQ ID NO: 6)
m/z:z=3 1501[M+3H]³⁺, z=4 1126[M+4H]⁴⁺

### (3-4) Synthesis of antibody affinity peptide reagent loaded with maleimide (1) (compounds 22, 23, and 24)

The thiol-peptide coupled compound (compounds 19, 20, and 21) synthesized in (3-3) was dissolved in dimethyl sulfoxide, a dimethyl sulfoxide solution of N-hydroxymaleimide (20 molar equivalent) was added thereto, and the resulting mixture was stirred for one hour. Disappearance of the raw materials was determined by LC-MS. Thereafter, trans-4-[(2,5-dihydro-1H-pyrroli-1-yl) methyl)] cyclohexanecarboxylic acid (40 molar equivalents), 1-ethyl-3-(3-dimethylaminopropyl) carbodiimide hydrochloride (30 molar equivalents), and 1-hydroxybenzotriazole (30 molar equivalents) were added thereto, and the resulting mixture was stirred for four hours. The completion of the reaction was determined by LC-MS, and then the product was purified by preparative HPLC. A fraction containing a target product was determined by ESI-MS, followed by freeze-drying, thus obtaining a maleimide-affinity peptide reagent (compounds 22, 23, and 24) as a product. 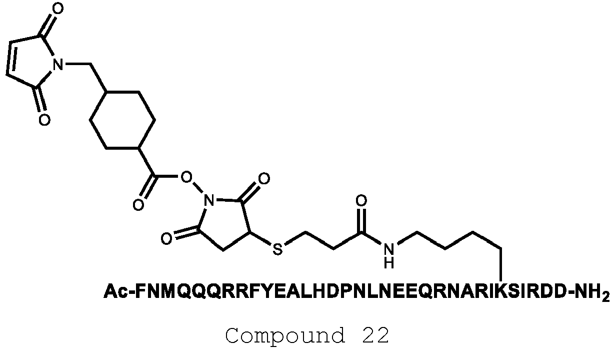 (Amino acid sequence of peptide portion: SEQ ID NO: 6)
MS(ESI) m/z:z=3 1532[M+3H]³⁺, z=4 1150[M+4H]⁴⁺ 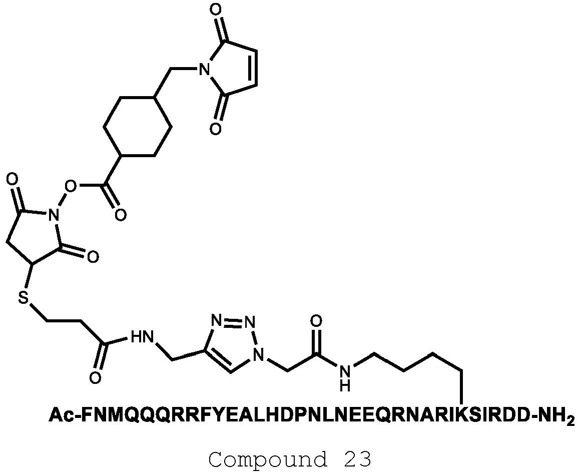 (Amino acid sequence of peptide portion: SEQ ID NO: 6)
MS(ESI) m/z:z=3 1578[M+3H]³⁺, z=4 1184[M+4H]⁴⁺ 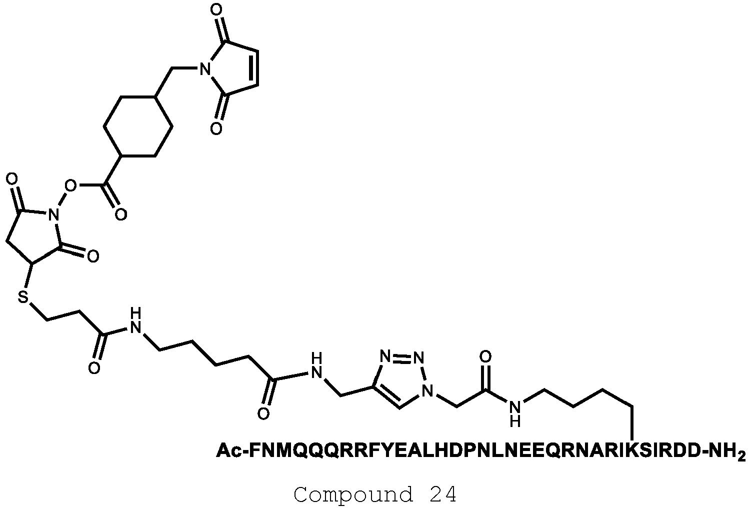 (Amino acid sequence of peptide portion: SEQ ID NO: 6)
MS(ESI) m/z:z=3 1611[M+3H]³⁺, z=4 1208[M+4H]⁴⁺

### (3-5) Synthesis of antibody affinity peptide reagent loaded with maleimide (2) (compounds 56 and 58)

### (3-5-1) Synthesis of N-hydroxysuccinimide-peptide coupled compound (compound 55)

Compound 3 synthesized in (1-1) was dissolved in dimethyl sulfoxide, a dimethyl sulfoxide solution of triethylamine (1 equivalent) and N-hydroxymaleimide (1.2 equivalents) was added thereto, and the resulting mixture was stirred at room temperature for one hour. The completion of the reaction was determined by LC-MS, and then the product was purified by preparative HPLC. A fraction containing a target product was determined by ESI-MS, followed by freeze-drying, thus obtaining an N-hydroxysuccinimide-peptide coupled compound (compound 55) as a product. 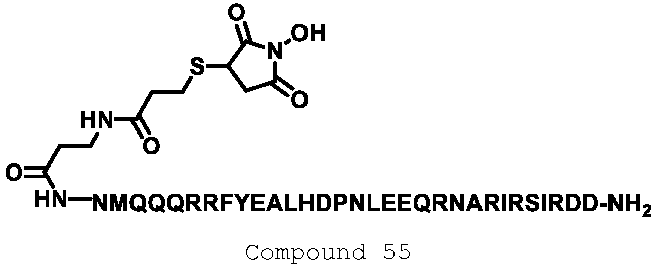 (Amino acid sequence of peptide portion: SEQ ID NO: 7)
MS (ESI)m/z:z=3 1429 [M+3H]³⁺, Z=4 1072 [M+4H]⁴⁺, z=5 857 [M+5H]⁵⁺, z=6 715 [M+6H]⁶⁺

### (3-5-2) Synthesis of antibody affinity peptide reagent loaded with maleimide (compound 56)

The N-hydroxysuccinimide-peptide coupled compound obtained in (3-5-1) and 4-(N-maleimidomethyl) cyclohexanecarboxylic acid N-succinimidyl (20 equivalents) were dissolved in dimethyl sulfoxide, and triethylamine (8 equivalents) was added thereto. The resulting mixture was stirred at room temperature for two hours, and then the progress of the reaction was determined by LC-MS. The product was purified by preparative HPLC, and a fraction containing a target product was determined by ESI-MS, followed by freeze-drying, thus obtaining an antibody affinity peptide reagent (compound 56) loaded with maleimide as the target product. 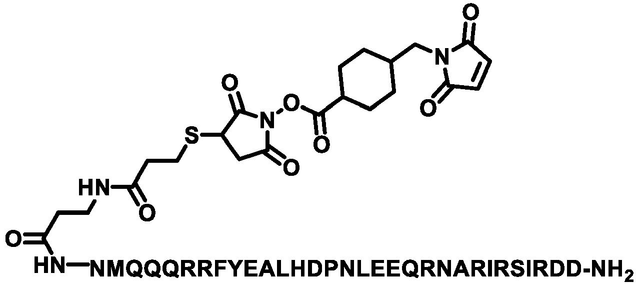 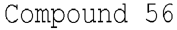 (Amino acid sequence of peptide portion: SEQ ID NO: 7)
MS (ESI)m/z:z=3 1502 [M+3H]³⁺, Z=4 1126 [M+4H]⁴⁺, z=5 901 [M+5H]⁵⁺, z=6 751 [M+6H]⁶⁺

### (3-5-3) Synthesis of N-hydroxysuccinimide-peptide coupled compound (compound 57)

The antibody affinity peptide (compound 36) synthesized in Example 1 was dissolved in dimethylsulfoxide, 2-iminothiolane hydrochloride (10 molar equivalents) and diisopropylethylamine (15 molar equivalents) were added thereto, and the resulting mixture was stirred for one hour. Disappearance of the raw materials was determined by LC-MS. Thereafter, a dimethyl sulfoxide solution of N-hydroxymaleimide (20 molar equivalent) was added thereto, and the resulting mixture was stirred for one hour. The progress of the reaction was determined by LC-MS, and then the product was purified by preparative HPLC. A fraction containing a target product was determined by ESI-MS, followed by freeze-drying, thus obtaining a hydroxymaleimide-affinity peptide (compound 57) as a product. 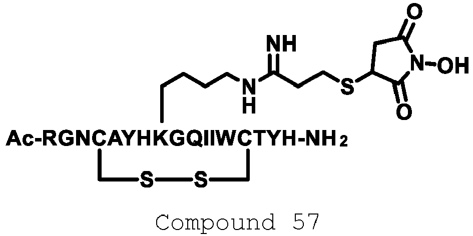 (Amino acid sequence of peptide portion: SEQ ID NO: 46)
MS (ESI)m/z:z=2 1152 [M+2H]²⁺, Z=3 769 [M+3H]³⁺

### (3-5-4) Synthesis of antibody affinity peptide reagent loaded with maleimide (compound 58)

The N-hydroxysuccinimide-peptide coupled compound (H1) obtained in (3-5-2) and 4-[(2,5-dioxo-2,5-dihydro-1H-pyrrol-1-yl) methyl] beizoic acid (20 equivalents) were dissolved in dimethyl sulfoxide, and triethylamine (8 equivalents) was added thereto. The resulting mixture was stirred at room temperature for two hours, and then the progress of the reaction was determined by LC-MS. The product was purified by preparative HPLC, and a fraction containing a target product was determined by ESI-MS, followed by freeze-drying, thus obtaining an antibody affinity peptide reagent (compound 58) loaded with maleimide as the target product. 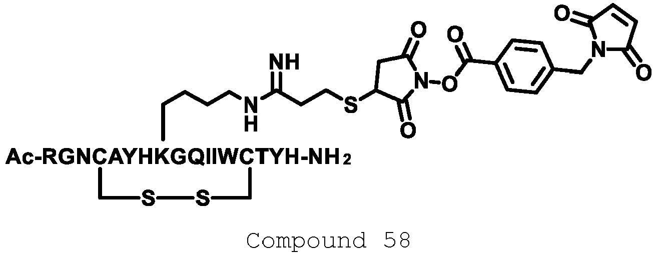 (Amino acid sequence of peptide portion: SEQ ID NO: 46)
MS (ESI)m/z:z=2 1259 [M+2H]²⁺, Z=3 840 [M+3H]³⁺

### [Example 4: Introduction of regioselective maleimide to anti-HER2 IgG antibody trastuzumab and analysis thereof]

### (4-1) Synthesis of peptide reagent having thiol

Compound 25 below was synthesized by solid phase peptide synthesis using 2-chlorotrityl resin by Fmoc method. After the solid phase synthesis, the product was stirred in a solution of trifluoroacetic acid : water : triisopropylsilane = 95 : 2.5 : 2.5 for one hour, thereby performing cutting out from the resin and deprotection. The resin was removed by filtration, diethyl ether was added for precipitation, and the diethyl ether was removed by decantation to obtain a peptide as crude crystals. This peptide was purified by preparative HPLC to obtain compound 25 below as a product. Compound 25 is not an affinity peptide but a model peptide compound instead of a medicine, which should be introduced via a maleimide group to an antibody regioselectively having maleimide, which is a bioorthogonal functional group. 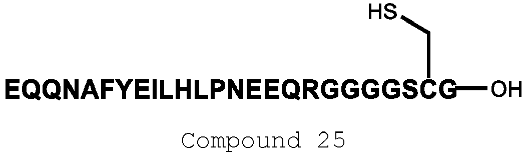 (Amino acid sequence of peptide portion: SEQ ID NO: 9)
MS (ESI) m/z:z=2 1482[M+2H]²⁺, z=3 988[M+3H]³⁺

### (4-2) Regiospecific modification of IgG antibody trastuzumab with maleimide-affinity peptide reagent

The maleimide-affinity peptide reagent (compound 22,23,24) synthesized in (4-4) of Example 4 was dissolved in N,N'-dimethylformamide to be 2.18 mM. 20.0 µg of anti-HER2 IgG antibody trastuzumab (Chugai Pharmaceutical Co., Ltd.) was dissolved in 14.8 µL of 50 mM HEPES buffer (pH 7.2), the peptide reagent (10 to 40 molar equivalents) was added to the antibody, and the resulting mixture was stirred at 37°C for four hours. The reaction solution was subjected to solvent substitution to a 20 mM acetate buffer (pH 4.5) by ultrafiltration (Amicon Ultra, 10K MWCO). By repeating the same operation three times, the affinity peptide reagent was removed to obtain maleimide-modified IgG antibody trastuzumab.

### (4-3) Conjugation of maleimide-modified IgG antibody trastuzumab with peptide reagent having thiol

The maleimide-modified IgG antibody trastuzumab synthesized in (5-2) of Example 5 was subjected to solvent substitution to a 50 mM HEPES buffer (pH 7.2) by ultrafiltration (Amicon Ultra, 10K MWCO) to return the pH in the system to neutrality. An aqueous solution of compound 25 adjusted to 10 mg/mL (80 molar equivalents to the antibody) was added thereto, and the resulting mixture was stirred for 16 hours. The reaction solution was subjected to water substitution by ultrafiltration (Amicon Ultra, 10K MWCO) to remove the peptide reagent, and the reaction was thereby stopped, thus obtaining an IgG antibody trastuzumab-peptide conjugate.

### (4-4) Analysis of IgG antibody trastuzumab-peptide conjugate by SDS-PAGE

The IgG antibody trastuzumab-peptide conjugate obtained in (4-3) was subjected to SDS-PAGE (Mini-PROTEAN TGX gel, 4 to 20%, Bio-RAD; under reduction conditions; stained with Coomasie Brilliant Blue G-250 Stain). A result thereof is illustrated in FIG. 3. From this result, it was found that the modification reaction of the antibody progressed when compounds 22 and 23 were used, and did not progress when compound 24 was used in the maleimide regioselective introduction reaction (5-2).

### (4-5) Determination of heavy chain selectivity of maleimide-modified IgG antibody trastuzumab under reduction conditions by ESI-TOFMS analysis

### (4-5-1) Synthesis of maleimide-modified IgG antibody trastuzumab (1)

The affinity peptide reagent loaded with maleimide (compound 56) synthesized in (3-5-2) of Example 3 was dissolved in N,N'-dimethylformamide to be 2.0 mM. 250 µg of anti-HER2 IgG antibody trastuzumab (Chugai Pharmaceutical Co., Ltd.) was dissolved in 230 µL of 50 mM HEPES buffer (pH 7.2), 20 molar equivalents of 2.0 mM peptide reagent was added to the antibody, and the resulting mixture was stirred at 37°C for 16 hours. The resulting solution was concentrated by ultrafiltration (Amicon Ultra, 10k MWCO) to obtain a maleimide-modified IgG antibody trastuzumab.

### (4-5-2) Determination of heavy chain selectivity of maleimide-modified trastuzumab under reduction conditions by ESI-TOFMS analysis (1)

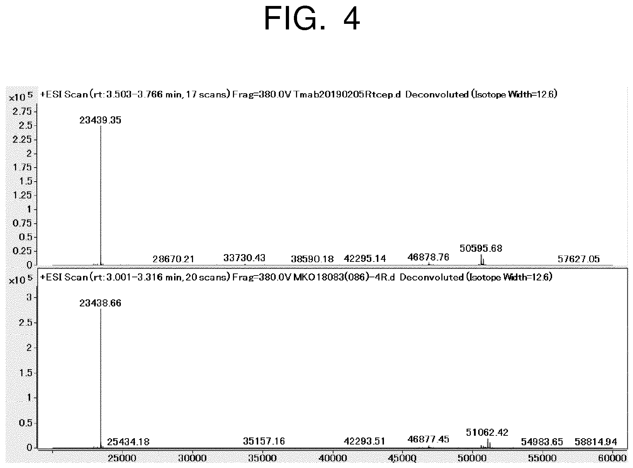

To the maleimide-modified trastuzumab synthesized in (4-5-1), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50596 and 50757, and a light chain peak was observed at 23439. For a reaction product, peaks were observed at 51062 and 51224 with maleimide reacted with tris(2-carboxyethyl) phosphine introduced to the heavy chain, and a light chain peak was observed at 23439, the same as that of the raw material. FIG. 4 illustrates a result of ESI-TOFMS analysis.

### (4-5-3) Synthesis of maleimide-modified IgG antibody trastuzumab (2)

Using the affinity peptide reagent loaded with maleimide (compound 58) synthesized in (3-5-4) of Example 3 in a similar manner to (4-5-1), azide-modified IgG antibody trastuzumab was obtained.

### (4-5-4) Determination of heavy chain selectivity of maleimide-modified trastuzumab under reduction conditions by ESI-TOFMS analysis (2)

A treatment was performed in a similar manner to (4-5-2), and the mass was measured by ESI-TOFMS; for the raw material trastuzumab used as a control, heavy chain peaks were observed at 50602 and 50764, and a light chain peak was observed at 23443. For a reaction product, peaks were observed at 50818 and 50979 with maleimide reacted with N-acetylcysteine introduced to the heavy chain, and a light chain peak was observed at 23443, the same as that of the raw material.

### [Example 5: Synthesis of antibody-modifying reagent loaded with azide]

### (5-1) Synthesis of antibody-modifying reagent (Comparative Example compound) having hetero atom at β-position of antibody-modifying group (electrophilic group)

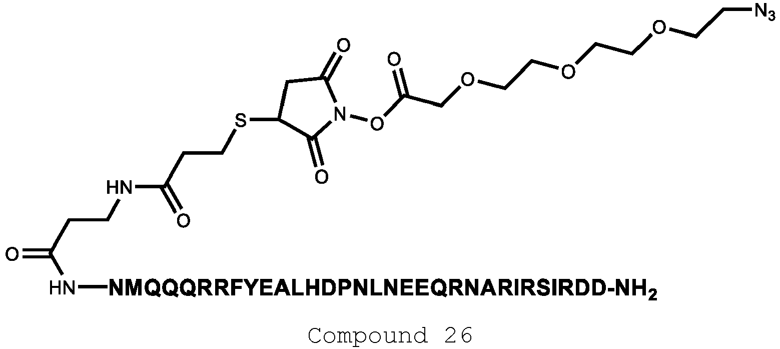

(Amino acid sequence of peptide portion: SEQ ID NO: 7)

The antibody affinity peptide (compound 2) synthesized in Example 1 was dissolved in dimethyl sulfoxide, a dimethyl sulfoxide solution of N-hydroxymaleimide (20 molar equivalents) was added thereto, and the resulting mixture was stirred for one hour. Disappearance of the raw materials was determined by LC-MS. Thereafter, 11-azido-3,6,9-trioxaundecanoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-PEG-affinity peptide reagent (compound 26) as a product.
MS (ESI)m/z:z=3 1501[M+3H]³⁺, z=4 1125[M+4H]⁴⁺

### (5-2) Synthesis of antibody-modifying reagent having carbon atom or hetero atom at β-position of antibody-modifying group (electrophilic group)

### (5-2-1) Synthesis of antibody-modifying reagent (compounds 27, 59, and 60) by coupling of azide reagent with affinity peptide

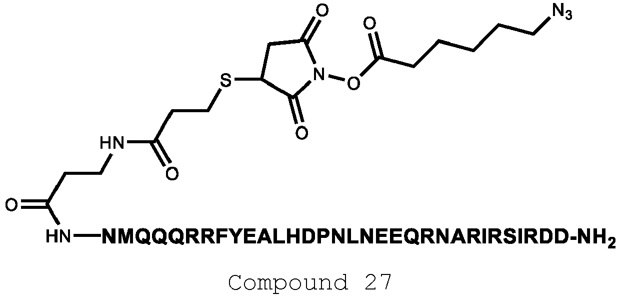

(Amino acid sequence of peptide portion: SEQ ID NO: 7)

The antibody affinity peptide (compound 3) synthesized in Example 1 was dissolved in dimethyl sulfoxide, a dimethyl sulfoxide solution of N-hydroxymaleimide (20 molar equivalents) was added thereto, and the resulting mixture was stirred for one hour. Disappearance of the raw materials was determined by LC-MS. Thereafter, 6-azido-hexanoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-alkyl-affinity peptide reagent (compound 27) as a product.
MS (ESI)m/z:z=3 1475[M+3H]³⁺, z=4 1107[M+4H]⁴⁺

Using the N-hydroxysuccinimide-peptide coupled compound (compound 57) synthesized in Example 3, an azide-alkyl-affinity peptide reagent (compound 59) was obtained by a similar method. 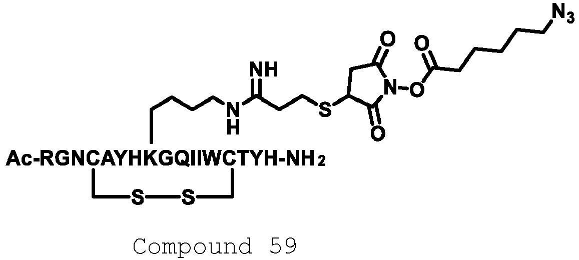 (Amino acid sequence of peptide portion: SEQ ID NO: 46)
MS (ESI)m/z:z=2 1222 [M+2H]²⁺, Z=3 815 [M+3H]³⁺

6-Azido-hexanoic acid was replaced with azidoacetic acid, and synthesis was performed in a similar manner to obtain an azide-alkyl-affinity peptide reagent (compound 60). Note that compound 60 is a Comparative Example compound having a hetero atom at a β-position of the antibody-modifying group (electrophilic group). 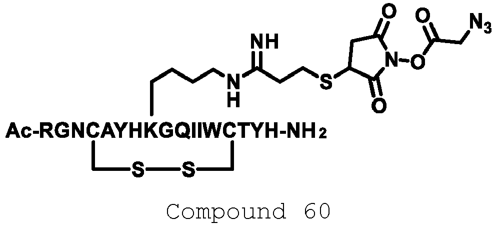 (Amino acid sequence of peptide portion: SEQ ID NO: 46)
MS (ESI)m/z:z=2 1193 [M+2H]²⁺, Z=3 796 [M+3H]³⁺

### (5-2-2) Synthesis of antibody-modifying reagent (compounds 28 and 61) by coupling of azide reagent with affinity peptide

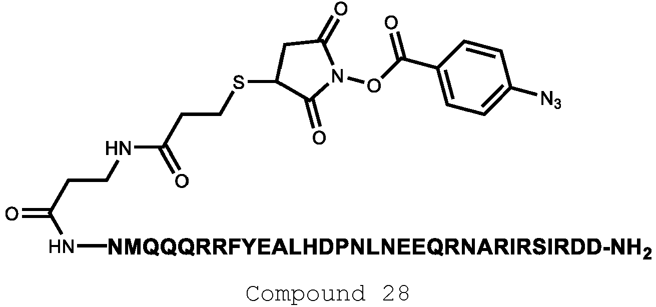

(Amino acid sequence of peptide portion: SEQ ID NO: 7)

The antibody affinity peptide (compound 3) synthesized in Example 1 was dissolved in dimethyl sulfoxide, a dimethyl sulfoxide solution of N-hydroxymaleimide (20 molar equivalents) was added thereto, and the resulting mixture was stirred for one hour. Disappearance of the raw materials was determined by LC-MS. Thereafter, 4-azidobenzoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-phenyl-affinity peptide reagent (compound 28) as a product.
MS (ESI)m/z:z=3 1478[M+3H]³⁺, z=4 1108[M+4H]⁴⁺

Using the antibody affinity peptide (compound 31) synthesized in Example 1, an azide-phenyl-affinity peptide reagent (compound 61) was obtained by a similar method. 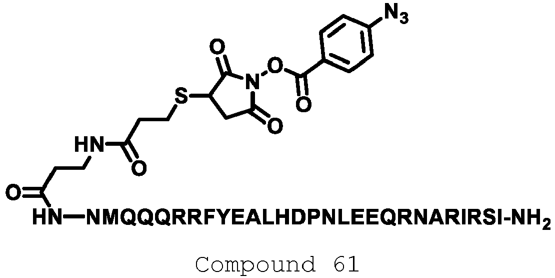 (Amino acid sequence of peptide portion: SEQ ID NO: 97)
MS (ESI)m/z:z=3 1348 [M+3H]³⁺, Z=4 1011 [M+4H]⁴⁺

### (5-2-3) Synthesis of antibody-modifying reagent (compounds 29 and 62 to 83) by coupling of azide reagent with affinity peptide

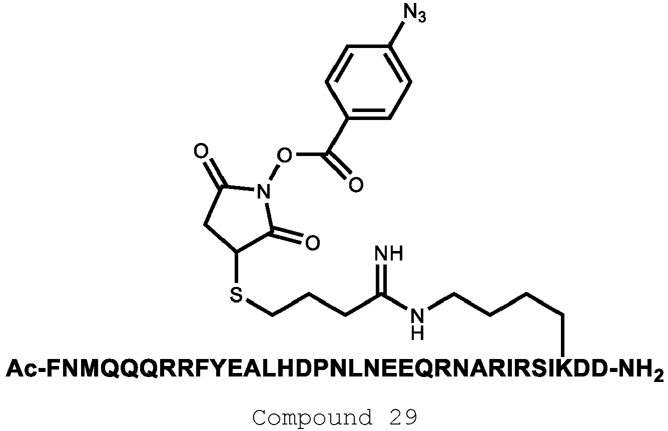

(Amino acid sequence of peptide portion: SEQ ID NO: 5)

The antibody affinity peptide (compound 3) synthesized in Example 1 was dissolved in dimethylsulfoxide, 2-iminothiolane hydrochloride (10 molar equivalents) and diisopropylethylamine (15 molar equivalents) were added thereto, and the resulting mixture was stirred for one hour. Disappearance of the raw materials was determined by LC-MS. Thereafter, a dimethyl sulfoxide solution of N-hydroxymaleimide (20 molar equivalent) was added thereto, and the resulting mixture was stirred for one hour. The progress of the reaction was determined by LC-MS. Thereafter, 4-azidobenzoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-phenyl-affinity peptide reagent (compound 29) as a product.
MS (ESI)m/z:z=3 1512[M+3H]³⁺, z=4 1134[M+4H]⁴⁺

Using the antibody affinity peptides (compounds 32 to 53) synthesized in Example 1, antibody-modifying reagents by coupling of azide reagents illustrated below with the affinity peptides (compounds 62 to 83) were synthesized by a similar technique.

An antibody-modifying reagent synthesized using the antibody affinity peptide (compound 32) synthesized in Example 1 is as follows. 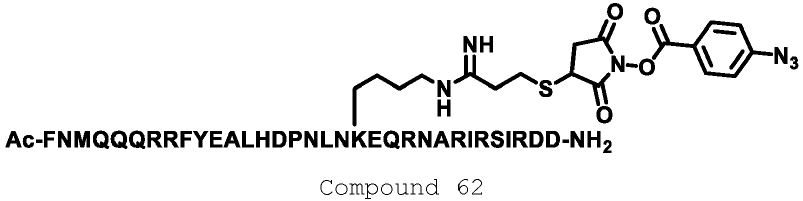 (Amino acid sequence of peptide portion: SEQ ID NO: 98)
MS (ESI)m/z:z=3 1521[M+3H]³⁺, z=4 1141[M+4H]⁴⁺

An antibody-modifying reagent synthesized using the antibody affinity peptide (compound 33) synthesized in Example 1 is as follows. 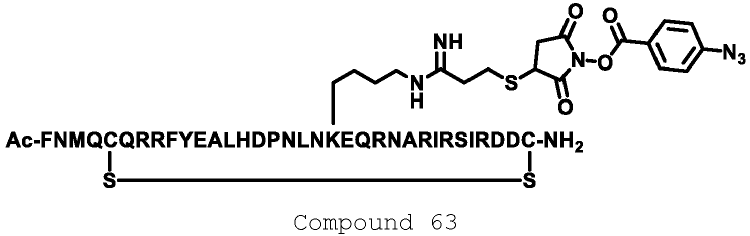 (Amino acid sequence of peptide portion: SEQ ID NO: 41)
MS (ESI)m/z:z=3 1546 [M+3H]³⁺, Z=4 1159 [M+4H]⁴⁺

An antibody-modifying reagent synthesized using the antibody affinity peptide (compound 34) synthesized in Example 1 is as follows. 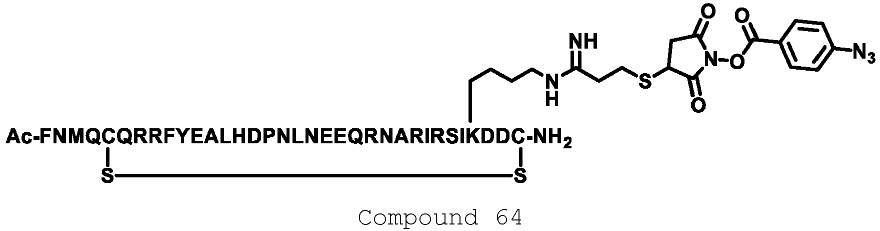 (Amino acid sequence of peptide portion: SEQ ID NO: 42)
MS (ESI)m/z:z=3 1537 [M+3H]³⁺, Z=4 1153 [M+4H]⁴⁺

An antibody-modifying reagent synthesized using the antibody affinity peptide (compound 35) synthesized in Example 1 is as follows. 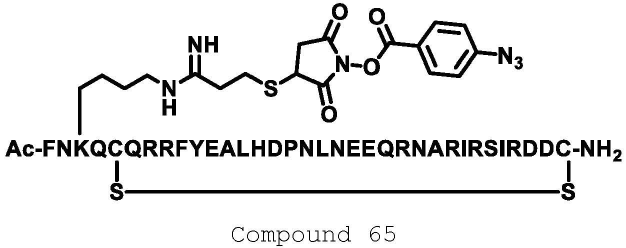 (Amino acid sequence of peptide portion: SEQ ID NO: 43)
MS (ESI)m/z:z=3 1545 [M+3H]³⁺, Z=4 1159 [M+4H]⁴⁺

An antibody-modifying reagent synthesized using the antibody affinity peptide (compound 36) synthesized in Example 1 is as follows. 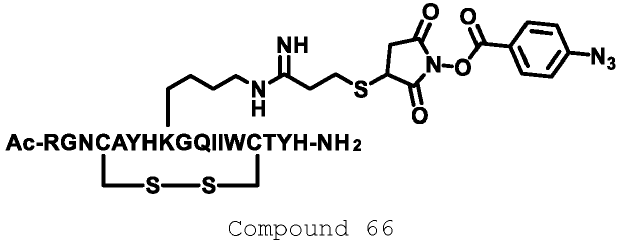 (Amino acid sequence of peptide portion: SEQ ID NO: 46)
MS (ESI)m/z:z=2 1225[M+H]⁺, z=3 817[M+H]⁺

An antibody-modifying reagent synthesized using the antibody affinity peptide (compound 37) synthesized in Example 1 is as follows. 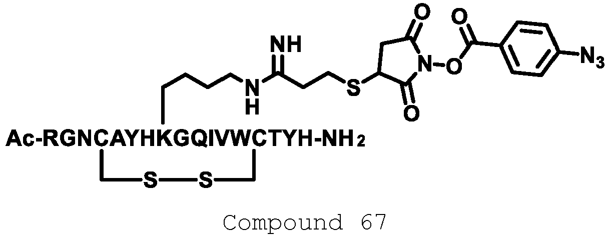 (Amino acid sequence of peptide portion: SEQ ID NO: 47)
MS (ESI)m/z:z=2 1217 [M+2H]²⁺, z=3 812 [M+3H]³⁺

An antibody-modifying reagent synthesized using the antibody affinity peptide (compound 38) synthesized in Example 1 is as follows. 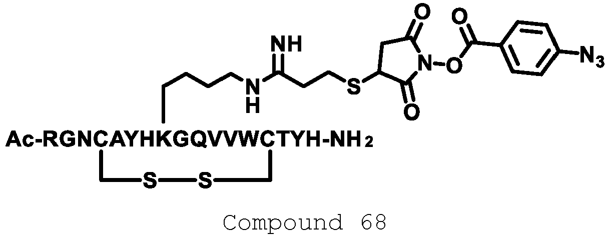 (Amino acid sequence of peptide portion: SEQ ID NO: 48)
MS (ESI)m/z:z=2 1211 [M+2H]²⁺, z=3 807 [M+3H]³⁺

An antibody-modifying reagent synthesized using the antibody affinity peptide (compound 39) synthesized in Example 1 is as follows. 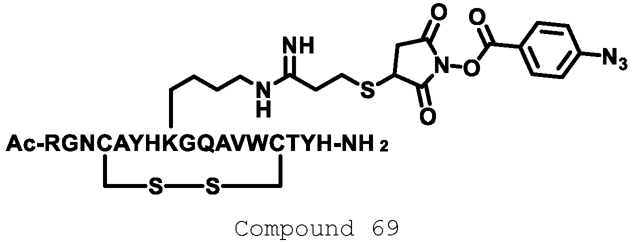 (Amino acid sequence of peptide portion: SEQ ID NO: 49)
MS (ESI)m/z:z=2 1196 [M+2H]²⁺, z=3 798 [M+3H]³⁺

An antibody-modifying reagent synthesized using the antibody affinity peptide (compound 40) synthesized in Example 1 is as follows. 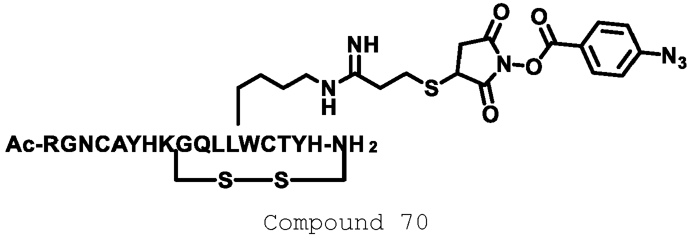 (Amino acid sequence of peptide portion: SEQ ID NO: 50)
MS (ESI)m/z:z=2 1224 [M+2H]²⁺, z=3 817 [M+3H]³⁺

An antibody-modifying reagent synthesized using the antibody affinity peptide (compound 41) synthesized in Example 1 is as follows. 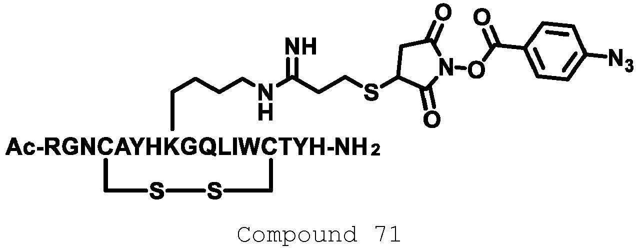 (Amino acid sequence of peptide portion: SEQ ID NO: 51)
MS (ESI)m/z:z=2 1224 [M+2H]²⁺, z=3 817 [M+3H]³⁺

An antibody-modifying reagent synthesized using the antibody affinity peptide (compound 42) synthesized in Example 1 is as follows. 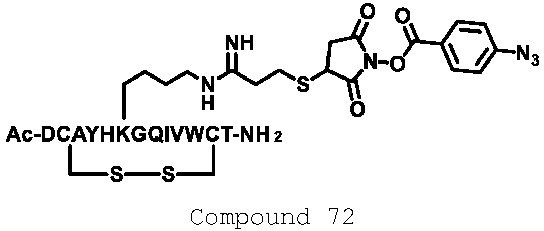 (Amino acid sequence of peptide portion: SEQ ID NO: 52)
MS (ESI)m/z:z=2 961 [M+2H]²⁺

An antibody-modifying reagent synthesized using the antibody affinity peptide (compound 43) synthesized in Example 1 is as follows. 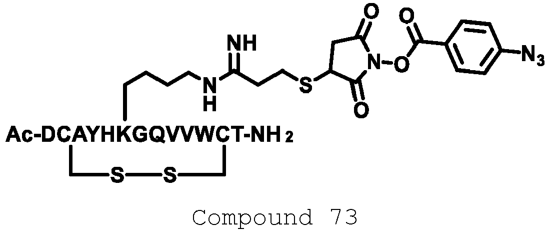 (Amino acid sequence of peptide portion: SEQ ID NO: 53)
MS (ESI)m/z:z=1 1909[M+H]⁺, z=3 955[M+H]⁺

An antibody-modifying reagent synthesized using the antibody affinity peptide (compound 44) synthesized in Example 1 is as follows. 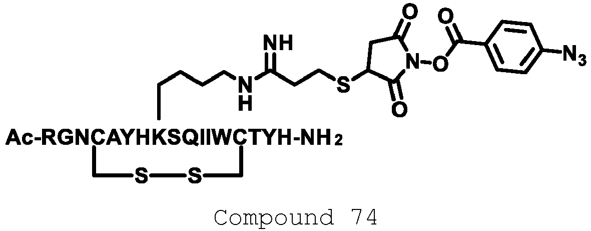 (Amino acid sequence of peptide portion: SEQ ID NO: 55)
MS (ESI)m/z:z=2 1239 [M+2H]²⁺, z=3 827 [M+3H]³⁺

An antibody-modifying reagent synthesized using the antibody affinity peptide (compound 45) synthesized in Example 1 is as follows. 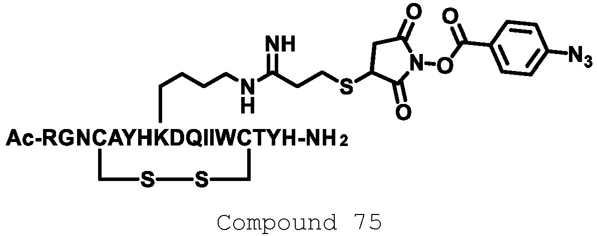 (Amino acid sequence of peptide portion: SEQ ID NO: 57)
MS (ESI)m/z:z=2 1253 [M+2H]²⁺, z=3 836 [M+3H]³⁺

An antibody-modifying reagent synthesized using the antibody affinity peptide (compound 46) synthesized in Example 1 is as follows. 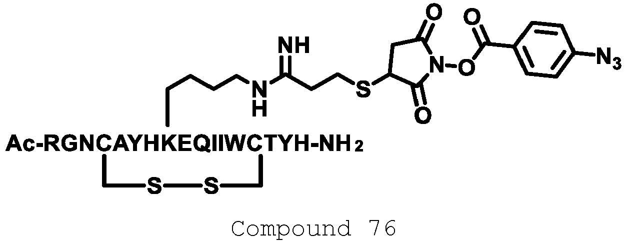 (Amino acid sequence of peptide portion: SEQ ID NO: 59)
MS (ESI)m/z:z=2 1260 [M+2H]²⁺, z=3 841 [M+3H]³⁺

An antibody-modifying reagent synthesized using the antibody affinity peptide (compound 47) synthesized in Example 1 is as follows. 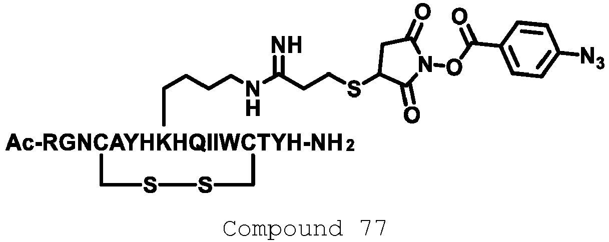 (Amino acid sequence of peptide portion: SEQ ID NO: 63)
MS (ESI)m/z:z=2 1265[M+H]⁺, z=3 844 [M+H]^{÷}

An antibody-modifying reagent synthesized using the antibody affinity peptide (compound 48) synthesized in Example 1 is as follows. 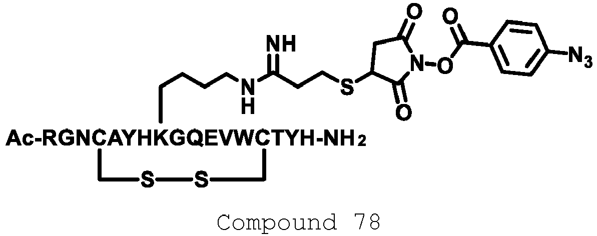 (Amino acid sequence of peptide portion: SEQ ID NO: 71)
MS (ESI)m/z:z=2 1225 [M+2H]²⁺, z=3 817 [M+3H]³⁺

An antibody-modifying reagent synthesized using the antibody affinity peptide (compound 49) synthesized in Example 1 is as follows. 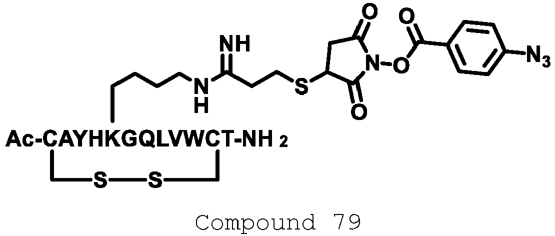 (Amino acid sequence of peptide portion: SEQ ID NO: 72)
MS (ESI)m/z:z=2 853 [M+2H]²⁺

An antibody-modifying reagent synthesized using the antibody affinity peptide (compound 50) synthesized in Example 1 is as follows. 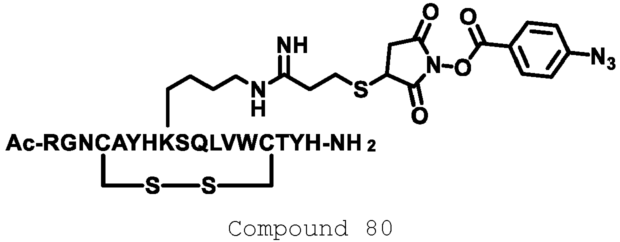 (Amino acid sequence of peptide portion: SEQ ID NO: 77)
MS (ESI)m/z:z=2 1232 [M+2H]²⁺, z=3 822 [M+3H]³⁺

An antibody-modifying reagent synthesized using the antibody affinity peptide (compound 51) synthesized in Example 1 is as follows. 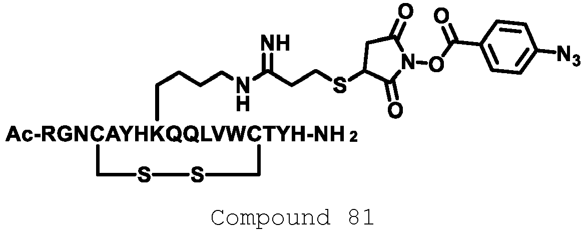 (Amino acid sequence of peptide portion: SEQ ID NO: 81)
MS (ESI)m/z:z=2 1253 [M+2H]²⁺, z=3 836 [M+3H]³⁺

An antibody-modifying reagent synthesized using the antibody affinity peptide (compound 52) synthesized in Example 1 is as follows. 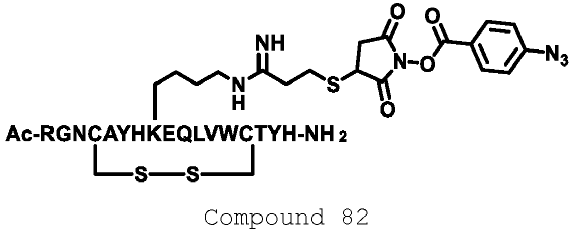 (Amino acid sequence of peptide portion: SEQ ID NO: 82)
MS (ESI)m/z:z=2 1254 [M+2H]²⁺, z=3 836 [M+3H]³⁺

An antibody-modifying reagent synthesized using the antibody affinity peptide (compound 53) synthesized in Example 1 is as follows. 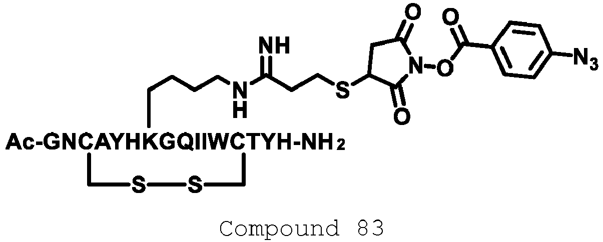 (Amino acid sequence of peptide portion: SEQ ID NO: 99)
MS (ESI)m/z:z=2 1147 [M+2H]²⁺, z=3 764 [M+3H]³⁺

### [Example 6: Regiospecific modification of IgG1 Fc with IgG1 Fc affinity peptide reagent and analysis by ESI-TOFMS]

### (6-1) Antibody modification reaction with antibody-modifying reagent having carbon atom at β-position of antibody-modifying group (electrophilic group)

### (6-1-1) Antibody Modification Reaction Using Affinity Peptide Reagent (compound 27, 59) (azide-modified antibody 1, 2)

The affinity peptide reagent loaded with azide (compound 27) synthesized in Example 5 was dissolved in N,N'-dimethylformamide to be 0.22 mM. 200 µg of anti-HER2 IgG antibody trastuzumab (Chugai Pharmaceutical Co., Ltd.) was dissolved in 1200 µL of 50 mM HEPES buffer (pH 7.2), 20 molar equivalents of 0.22 mM peptide reagent was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The reaction mixture was purified with Protein A (Aspire Protein A Tips, Thermo), and the eluate was substituted with 9.57 mM PBS buffer (pH 7.0) and concentrated by ultrafiltration (Amicon Ultra, 10K MWCO) to obtain azide-modified IgG antibody trastuzumab (azide-modified antibody 1).

An antibody modification reaction was performed using the affinity peptide reagent loaded with azide (compound 59) by a similar technique to obtain azide-modified IgG antibody trastuzumab (azide-modified antibody 2) .

### (6-1-2) Antibody Modification Reaction Using Affinity Peptide Reagent (compounds 28 and 61) (azide-modified antibody 3, 4)

The affinity peptide reagent loaded with azide (compound 28) synthesized in Example 5 was dissolved in N,N'-dimethylformamide to be 0.22 mM. 200 µg of anti-HER2 IgG antibody trastuzumab (Chugai Pharmaceutical Co., Ltd.) was dissolved in 1200 µL of 50 mM HEPES buffer (pH 7.2), 20 molar equivalents of 0.22 mM peptide reagent was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The reaction mixture was purified with Protein A (Aspire Protein A Tips, Thermo), and the eluate was substituted with 9.57 mM PBS buffer (pH 7.0) and concentrated by ultrafiltration (Amicon Ultra, 10K MWCO) to obtain azide-modified IgG antibody trastuzumab (azide-modified antibody 3) .

An antibody modification reaction was performed using the affinity peptide reagent loaded with azide (compound 61) by a similar technique to obtain azide-modified IgG antibody trastuzumab (azide-modified antibody 4) .

### (6-1-3) Antibody Modification Reaction Using Affinity Peptide Reagent (compounds 29 and 62 to 83) (azide-modified antibodies 5 to 27)

The affinity peptide reagent loaded with azide (compound 29) synthesized in Example 5 was dissolved in N,N'-dimethylformamide to be 0.22 mM. 200 µg of anti-HER2 IgG antibody trastuzumab (Chugai Pharmaceutical Co., Ltd.) was dissolved in 1200 µL of 50 mM HEPES buffer (pH 7.2), 20 molar equivalents of 0.22 mM peptide reagent was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The reaction mixture was purified with Protein A (Aspire Protein A Tips, Thermo), and the eluate was substituted with 9.57 mM PBS buffer (pH 7.0) and concentrated by ultrafiltration (Amicon Ultra, 10K MWCO) to obtain azide-modified IgG antibody trastuzumab (azide-modified antibody 5) .

An antibody modification reaction was performed using the affinity peptide reagent loaded with azide (compound 62) by a similar technique to obtain azide-modified IgG antibody trastuzumab (azide-modified antibody 6) .

An antibody modification reaction was performed using the affinity peptide reagent loaded with azide (compound 63) by a similar technique to obtain azide-modified IgG antibody trastuzumab (azide-modified antibody 7) .

An antibody modification reaction was performed using the affinity peptide reagent loaded with azide (compound 64) by a similar technique to obtain azide-modified IgG antibody trastuzumab (azide-modified antibody 8) .

An antibody modification reaction was performed using the affinity peptide reagent loaded with azide (compound 65) by a similar technique to obtain azide-modified IgG antibody trastuzumab (azide-modified antibody 9) .

An antibody modification reaction was performed using the affinity peptide reagent loaded with azide (compound 66) by a similar technique to obtain azide-modified IgG antibody trastuzumab (azide-modified antibody 10) .

An antibody modification reaction was performed using the affinity peptide reagent loaded with azide (compound 67) by a similar technique to obtain azide-modified IgG antibody trastuzumab (azide-modified antibody 11) .

An antibody modification reaction was performed using the affinity peptide reagent loaded with azide (compound 68) by a similar technique to obtain azide-modified IgG antibody trastuzumab (azide-modified antibody 12) .

An antibody modification reaction was performed using the affinity peptide reagent loaded with azide (compound 69) by a similar technique to obtain azide-modified IgG antibody trastuzumab (azide-modified antibody 13) .

An antibody modification reaction was performed using the affinity peptide reagent loaded with azide (compound 70) by a similar technique to obtain azide-modified IgG antibody trastuzumab (azide-modified antibody 14) .

An antibody modification reaction was performed using the affinity peptide reagent loaded with azide (compound 71) by a similar technique to obtain azide-modified IgG antibody trastuzumab (azide-modified antibody 15) .

An antibody modification reaction was performed using the affinity peptide reagent loaded with azide (compound 72) by a similar technique to obtain azide-modified IgG antibody trastuzumab (azide-modified antibody 16) .

An antibody modification reaction was performed using the affinity peptide reagent loaded with azide (compound 73) by a similar technique to obtain azide-modified IgG antibody trastuzumab (azide-modified antibody 17) .

An antibody modification reaction was performed using the affinity peptide reagent loaded with azide (compound 74) by a similar technique to obtain azide-modified IgG antibody trastuzumab (azide-modified antibody 18) .

An antibody modification reaction was performed using the affinity peptide reagent loaded with azide (compound 75) by a similar technique to obtain azide-modified IgG antibody trastuzumab (azide-modified antibody 19) .

An antibody modification reaction was performed using the affinity peptide reagent loaded with azide (compound 76) by a similar technique to obtain azide-modified IgG antibody trastuzumab (azide-modified antibody 20) .

An antibody modification reaction was performed using the affinity peptide reagent loaded with azide (compound 77) by a similar technique to obtain azide-modified IgG antibody trastuzumab (azide-modified antibody 21) .

An antibody modification reaction was performed using the affinity peptide reagent loaded with azide (compound 78) by a similar technique to obtain azide-modified IgG antibody trastuzumab (azide-modified antibody 22) .

An antibody modification reaction was performed using the affinity peptide reagent loaded with azide (compound 79) by a similar technique to obtain azide-modified IgG antibody trastuzumab (azide-modified antibody 23) .

An antibody modification reaction was performed using the affinity peptide reagent loaded with azide (compound 80) by a similar technique to obtain azide-modified IgG antibody trastuzumab (azide-modified antibody 24) .

An antibody modification reaction was performed using the affinity peptide reagent loaded with azide (compound F81) by a similar technique to obtain azide-modified IgG antibody trastuzumab (azide-modified antibody 25) .

An antibody modification reaction was performed using the affinity peptide reagent loaded with azide (compound 82) by a similar technique to obtain azide-modified IgG antibody trastuzumab (azide-modified antibody 26) .

An antibody modification reaction was performed using the affinity peptide reagent loaded with azide (compound 83) by a similar technique to obtain azide-modified IgG antibody trastuzumab (azide-modified antibody 27) .

### (6-1-4) Antibody Modification Reaction Other Than Trastumab Using Affinity Peptide Reagent (compound 66) (azide-modified antibodies 28 to 31)

The affinity peptide reagent loaded with azide (compound 66) synthesized in Example 5 was dissolved in N,N'-dimethylformamide to be 2.0 mM. 200 µg of anti-TNF-α IgG antibody adalimumab (Eisai Co., Ltd.) was dissolved in 1200 µL of 50 mM HEPES buffer (pH 7.2), 20 molar equivalents of 2.0 mM peptide reagent was added to the antibody, and the resulting mixture was stirred at 25°C for 16 hours. 50 µL of a 10 mg/mL aqueous Lys solution was added to the reaction solution, and the resulting mixture was stirred for 30 minutes to stop the reaction. The reaction mixture was purified with Protein A (Aspire Protein A Tips, Thermo), and the eluate was substituted with 9.57 mM PBS buffer (pH 7.0) and concentrated by ultrafiltration (Amicon Ultra, 10K MWCO) to obtain azide-modified IgG antibody adalimumab (azide-modified antibody 28) .

An antibody modification reaction was performed using an anti-RANKL IgG antibody denosumab (Daiichi Sankyo Co., Ltd.) by a similar technique to the above to obtain azide-modified IgG antibody denosumabumab (azide-modified antibody 29).

An antibody modification reaction was performed using an anti-IL-4/13 IgG antibody dupilumab (Sanofi) by a similar technique to the above to obtain azide-modified IgG antibody dupilumab (azide-modified antibody 30).

An antibody modification reaction was performed using an anti-CD20 IgG antibody rituximab (Roche) by a similar technique to the above to obtain azide-modified IgG antibody rituximab (azide-modified antibody 31).

### (6-2) Determination of heavy chain selectivity of azide-modified IgG antibody trastuzumab under reduction conditions by ESI-TOFMS analysis

### (6-2-1) Determination of heavy chain selectivity of azide-modified trastuzumab (azide-modified antibodies 1 and 2) under reduction conditions by ESI-TOFMS analysis when compounds 27 and 59 are used in modification reaction

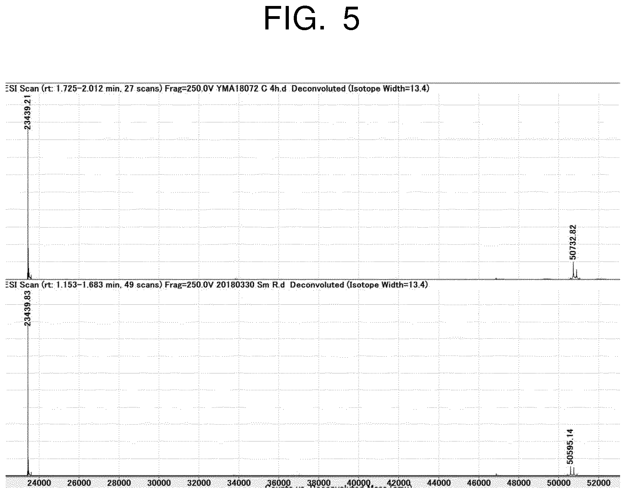

To the azide-modified trastuzumab (azide-modified antibody 1) synthesized in (6-1-1) of Example 6, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50595 and 50757, and a light chain peak was observed at 23440. For a reaction product, peaks were observed at 50733 and 50895 with alkyl azide introduced to the heavy chain, and a light chain peak was observed at 23440, the same as that of the raw material. FIG. 5 illustrates a result of ESI-TOFMS analysis.

ESI-TOF MS analysis was also performed on the azide-modified antibody 2 by a similar technique. For the raw material trastuzumab used as a control, heavy chain peaks were observed at 50602 and 50764, and a light chain peak was observed at 23443. For a reaction product, peaks were observed at 50742 and 50904 with alkyl azide introduced to the heavy chain, and a light chain peak was observed at 23443, the same as that of the raw material.

### (6-2-2) Determination of heavy chain selectivity of azide-modified trastuzumab (azide-modified antibodies 3 and 4) under reduction conditions by ESI-TOFMS analysis when compounds 28 and 61 are used in modification reaction

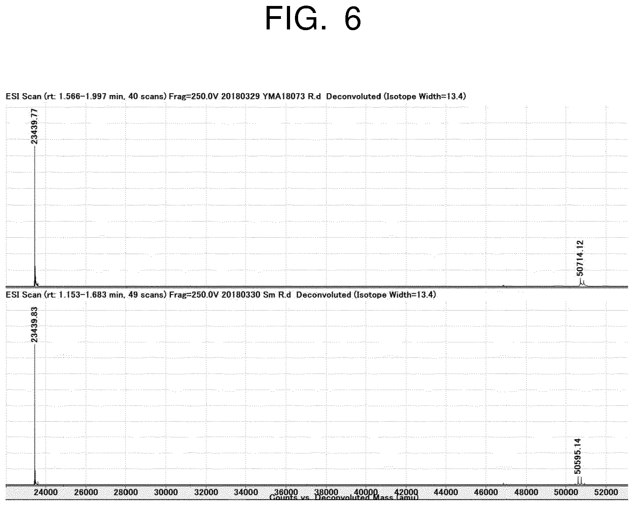

To the azide-modified trastuzumab (azide-modified antibody 3) synthesized in (6-1-2) of Example 6, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50595 and 50757, and a light chain peak was observed at 23440. For a reaction product, peaks were observed at 50714 and 50876 with aminebenzoic acid introduced to the heavy chain, and a light chain peak was observed at 23440, the same as that of the raw material. Note that the azidobenzoic acid-introduced compound was reduced to an aminebenzoic acid-introduced compound by a reduction reaction of 7 mM tris(2-carboxyethyl) phosphine salt, which was a pretreatment for the measurement. FIG. 6 illustrates a result of ESI-TOFMS analysis.

ESI-TOF MS analysis was also performed on the azide-modified antibody 4 by a similar technique. For the raw material trastuzumab used as a control, heavy chain peaks were observed at 50597 and 50758, and a light chain peak was observed at 23441. For a reaction product, peaks were observed at 50716 and 50877 with aminebenzoic acid introduced to the heavy chain, and a light chain peak was observed at 23441, the same as that of the raw material.

### (6-2-3) Determination of heavy chain selectivity of azide-modified trastuzumab (azide-modified antibodies 5 to 27) under reduction conditions by ESI-TOFMS analysis when compounds 29 and 62 to 83 are used in modification reaction

To the azide-modified trastuzumab (azide-modified antibody 5) synthesized in (6-1-3) of Example 6, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50595 and 50757, and a light chain peak was observed at 23440. For a reaction product, peaks were observed at 50714 and 50876 with aminebenzoic acid introduced to the heavy chain, and a light chain peak was observed at 23440, the same as that of the raw material. Note that the azidobenzoic acid-introduced compound was reduced to an aminebenzoic acid-introduced compound by the pretreatment for the measurement.

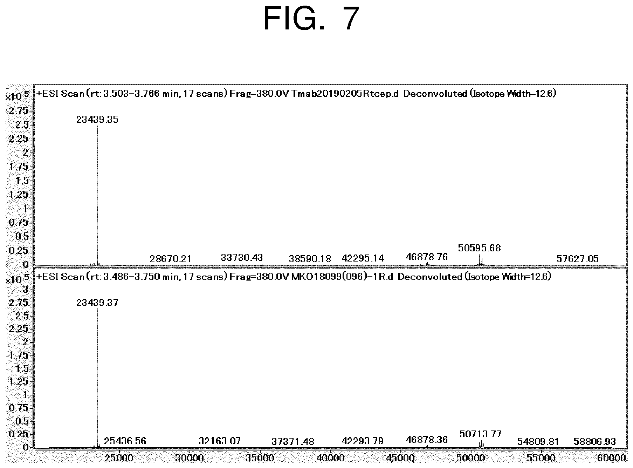

ESI-TOF MS analysis was also performed on the azide-modified antibody 6 by a similar technique. For the raw material trastuzumab used as a control, heavy chain peaks were observed at 50594 and 5076, and a light chain peak was observed at 23439. For a reaction product, peaks were observed at 50714 and 50875 with aminebenzoic acid introduced to the heavy chain, and a light chain peak was observed at 23439, the same as that of the raw material. FIG. 7 illustrates a result of ESI-TOFMS analysis.

ESI-TOF MS analysis was also performed on the azide-modified antibody 7 by a similar technique. For the raw material trastuzumab used as a control, heavy chain peaks were observed at 50594 and 5076, and a light chain peak was observed at 23439. For a reaction product, peaks were observed at 50713 and 50875, with aminebenzoic acid introduced to the heavy chain, and a light chain peak was observed at 23439, the same as that of the raw material.

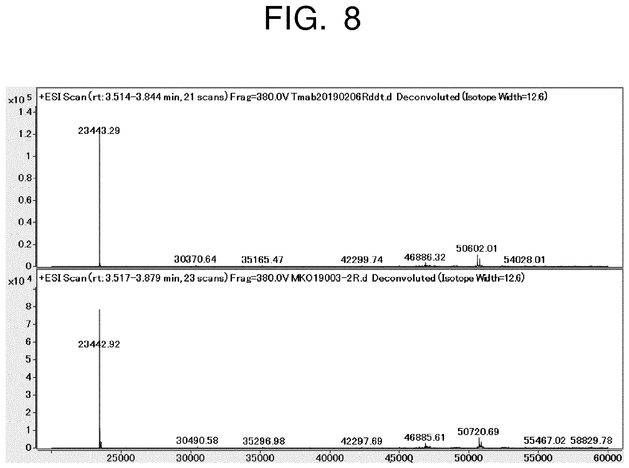

ESI-TOF MS analysis was also performed on the azide-modified antibody 8 by a similar technique. For the raw material trastuzumab used as a control, heavy chain peaks were observed at 50602 and 50764, and a light chain peak was observed at 23443. For a reaction product, peaks were observed at 50721 and 50883 with aminebenzoic acid introduced to the heavy chain, and a light chain peak was observed at 23442, the same as that of the raw material. FIG. 8 illustrates a result of ESI-TOFMS analysis.

ESI-TOF MS analysis was also performed on the azide-modified antibody 9 by a similar technique. For the raw material trastuzumab used as a control, heavy chain peaks were observed at 50595 and 50757, and a light chain peak was observed at 23440. For a reaction product, peaks were observed at 50714 and 50875 with aminebenzoic acid introduced to the heavy chain, and a light chain peak was observed at 23440, the same as that of the raw material.

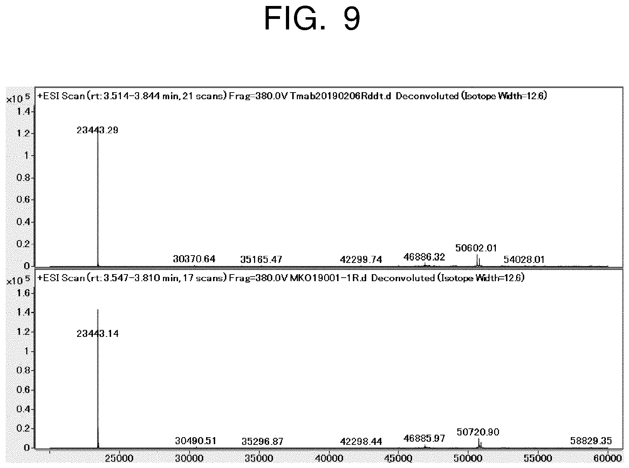

ESI-TOF MS analysis was also performed on the azide-modified antibody 10 by a similar technique. For the raw material trastuzumab used as a control, heavy chain peaks were observed at 50602 and 50764, and a light chain peak was observed at 23443. For a reaction product, peaks were observed at 50721 and 50883 with aminebenzoic acid introduced to the heavy chain, and a light chain peak was observed at 23443, the same as that of the raw material. FIG. 9 illustrates a result of ESI-TOFMS analysis.

ESI-TOF MS analysis was also performed on the azide-modified antibody 11 by a similar technique. For the raw material trastuzumab used as a control, heavy chain peaks were observed at 50595 and 50757, and a light chain peak was observed at 23440. For a reaction product, peaks were observed at 50713 and 50874 with aminebenzoic acid introduced to the heavy chain, and a light chain peak was observed at 23440, the same as that of the raw material.

ESI-TOF MS analysis was also performed on the azide-modified antibody 12 by a similar technique. For the raw material trastuzumab used as a control, heavy chain peaks were observed at 50595 and 50757, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 13 by a similar technique. For the raw material trastuzumab used as a control, heavy chain peaks were observed at 50594 and 50756, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 14 by a similar technique. For the raw material trastuzumab used as a control, heavy chain peaks were observed at 50594 and 50756, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 15 by a similar technique. For the raw material trastuzumab used as a control, heavy chain peaks were observed at 50594 and 50756, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 16 by a similar technique. For the raw material trastuzumab used as a control, heavy chain peaks were observed at 50594 and 50756, and a light chain peak was observed at 23440. For a reaction product, peaks were observed at 50714 and 50875 with aminebenzoic acid introduced to the heavy chain, and a light chain peak was observed at 23440, the same as that of the raw material.

ESI-TOF MS analysis was also performed on the azide-modified antibody 17 by a similar technique. For the raw material trastuzumab used as a control, heavy chain peaks were observed at 50595 and 50757, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 18 by a similar technique. For the raw material trastuzumab used as a control, heavy chain peaks were observed at 50594 and 50756, and a light chain peak was observed at 23439. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 19 by a similar technique. For the raw material trastuzumab used as a control, heavy chain peaks were observed at 50595 and 50757, and a light chain peak was observed at 23440. For a reaction product, peaks were observed at 50714 and 50875 with aminebenzoic acid introduced to the heavy chain, and a light chain peak was observed at 23440, the same as that of the raw material.

ESI-TOF MS analysis was also performed on the azide-modified antibody 20 by a similar technique. For the raw material trastuzumab used as a control, heavy chain peaks were observed at 50595 and 50757, and a light chain peak was observed at 23440. For a reaction product, peaks were observed at 50714 and 50875 with aminebenzoic acid introduced to the heavy chain, and a light chain peak was observed at 23440, the same as that of the raw material.

ESI-TOF MS analysis was also performed on the azide-modified antibody 21 by a similar technique. For the raw material trastuzumab used as a control, heavy chain peaks were observed at 50595 and 50757, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 22 by a similar technique. For the raw material trastuzumab used as a control, heavy chain peaks were observed at 50595 and 50757, and a light chain peak was observed at 23440. For a reaction product, peaks were observed at 50714 and 50875 with aminebenzoic acid introduced to the heavy chain, and a light chain peak was observed at 23440, the same as that of the raw material.

ESI-TOF MS analysis was also performed on the azide-modified antibody 23 by a similar technique. For the raw material trastuzumab used as a control, heavy chain peaks were observed at 50597 and 50759, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 24 by a similar technique. For the raw material trastuzumab used as a control, heavy chain peaks were observed at 50597 and 50759, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 25 by a similar technique. For the raw material trastuzumab used as a control, heavy chain peaks were observed at 50597 and 50759, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 26 by a similar technique. For the raw material trastuzumab used as a control, heavy chain peaks were observed at 50597 and 50759, and a light chain peak was observed at 23440. For a reaction product, peaks were observed at 50714 and 50876 with aminebenzoic acid introduced to the heavy chain, and a light chain peak was observed at 23440, the same as that of the raw material.

ESI-TOF MS analysis was also performed on the azide-modified antibody 27 by a similar technique. For the raw material trastuzumab used as a control, heavy chain peaks were observed at 50602 and 50764, and a light chain peak was observed at 23443. For a reaction product, peaks were observed at 50721 and 50883 with aminebenzoic acid introduced to the heavy chain, and a light chain peak was observed at 23440, the same as that of the raw material.

### (6-2-3) Determination of heavy chain selectivity of azide-modified antibody (azide modified antibodies 28 to 31) under reduction conditions by ESI-TOFMS analysis when compound 5 is used in modification reaction

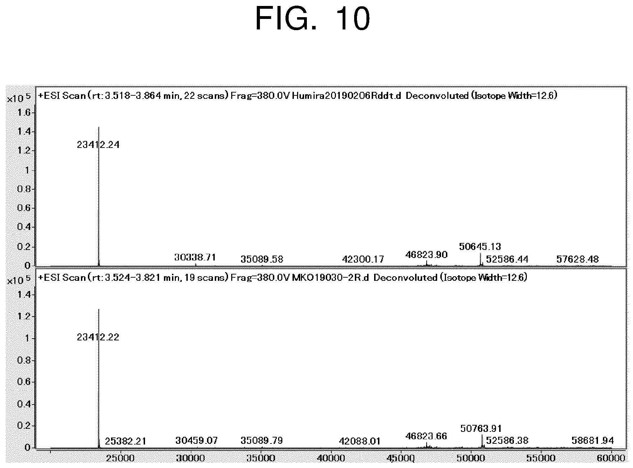

To the azide-modified adalimumab (azide-modified antibody 28) synthesized in (6-1-4) of Example 6, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material adalimumab used as a control, heavy chain peaks were observed at 50645 and 50764, and a light chain peak was observed at 23412. For a reaction product, peaks were observed at 50763 and 50926 with aminebenzoic acid introduced to the heavy chain, and a light chain peak was observed at 23412, the same as that of the raw material. Note that the azidobenzoic acid-introduced compound was reduced to an aminebenzoic acid-introduced compound by a reduction reaction of 7 mM tris(2-carboxyethyl) phosphine salt, which was a pretreatment for the measurement (hereinafter the same). FIG. 10 illustrates a result of ESI-TOFMS analysis.

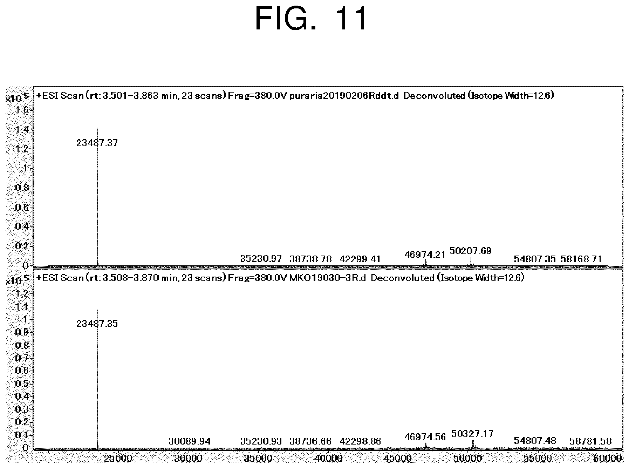

ESI-TOF MS analysis was also performed on the azide-modified antibody 29 (denosumab-azide-modified antibody) by a similar technique. For the raw material denosumab used as a control, heavy chain peaks were observed at 50208 and 50371, and a light chain peak was observed at 23487. For a reaction product, peaks were observed at 50327 and 50490 with aminebenzoic acid introduced to the heavy chain, and a light chain peak was observed at 23487, the same as that of the raw material. FIG. 11 illustrates a result of ESI-TOFMS analysis.

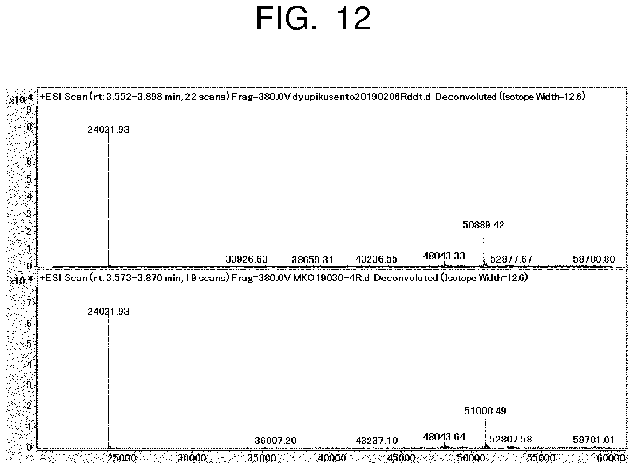

ESI-TOF MS analysis was also performed on the azide-modified antibody 30 (dupilumab-azide-modified antibody) by a similar technique. For the raw material dupilumab used as a control, a heavy chain peak was observed at 50889, and a light chain peak was observed at 24022. For a reaction product, a peak was observed at 51009 with aminebenzoic acid was introduced to the heavy chain, and a light chain peak was observed at 24022, the same as that of the raw material. FIG. 12 illustrates a result of ESI-TOFMS analysis.

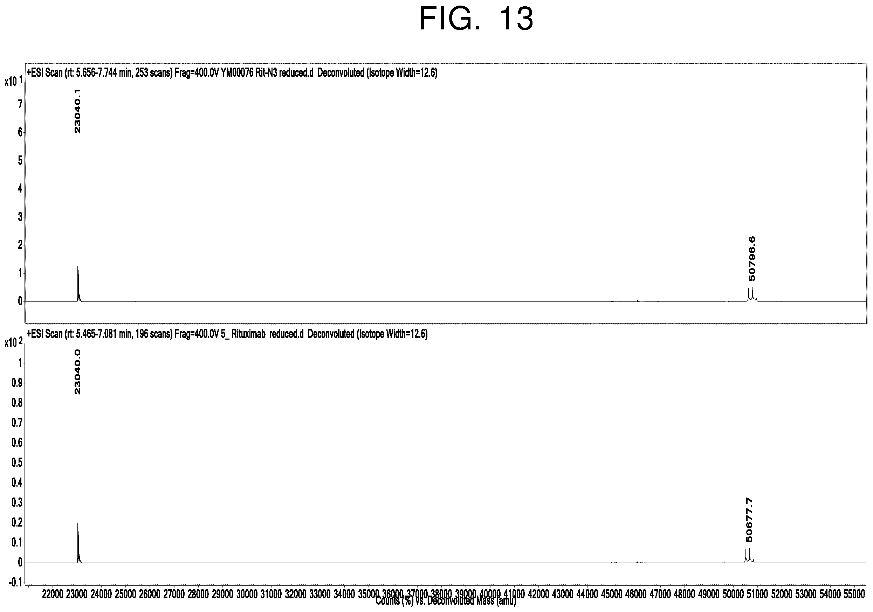

ESI-TOF MS analysis was also performed on the azide-modified antibody 31 (rituximab-azide-modified antibody) by a similar technique. For the raw material rituximab used as a control, heavy chain peaks were observed at 50515 and 50678, and a light chain peak was observed at 23040. For a reaction product, peaks were observed at 50635 and 50797 with aminebenzoic acid introduced to the heavy chain, and a light chain peak was observed at 23040, the same as that of the raw material (FIG. 13).

### [Comparative Example 1: Modification reaction of IgG1 Fc with peptide reagent having hetero atom at β-position and analysis by ESI-TOFMS]

The peptide reagent having a heteroatom at a β-position (compound 26) synthesized in Example 5 was dissolved in N,N'-dimethylformamide to be 0.22 mM. 200 µg of anti-HER2 IgG antibody trastuzumab (Chugai Pharmaceutical Co., Ltd.) was dissolved in 1200 µL of 50 mM HEPES buffer (pH 7.2), 120 µL of 0.22 mM peptide reagent (20 molar equivalents to the antibody) was added, and the resulting mixture was stirred at 37°C for 16 hours. 50 µL of a 10 mg/mL aqueous Lys solution was added to the reaction solution, and the resulting mixture was stirred for 30 minutes to stop the reaction. This solution was subjected to solvent substitution to a PBS solution by ultrafiltration (Amicon Ultra, 10K MWCO). Thereafter, 2.0 µL of 7 mM tris(2-carboxyethyl) phosphine hydrochloride solution (100 equivalents to the antibody) was added thereto, and the resulting mixture was allowed to stand at room temperature for 20 minutes. The mass was measured by ESI-TOFMS; for the raw material trastuzumab used as a control, heavy chain peaks were observed at 50595 and 50757, and a light chain peak was observed at 23440. Similarly, for a reaction product, heavy chain peaks were observed at 50595 and 50757, and a light chain peak was observed at 23440. It was determined that an antibody modification reaction with compound 26 having a hetero atom at a β-position of an antibody-modifying group (electrophilic group) had not progressed.

Similarly, an antibody modification reaction was performed using a peptide reagent (compound 60) having a hetero base paper at a β-position. The mass was measured by ESI-TOFMS; for the raw material trastuzumab used as a control, heavy chain peaks were observed at 50602 and 50764, and a light chain peak was observed at 23443. Similarly, for a reaction product, heavy chain peaks were observed at 50602 and 50764, and a light chain peak was observed at 23443. It was determined that an antibody modification reaction with compound 60 having a hetero atom at a β-position had not progressed.

From this result, it was found that the modification reaction of the antibody progressed when compounds 27, 28, and 29 were used, and did not progress when compound 26 was used in the azide regioselective introduction reaction (6-2).

### [Example 7: Synthesis of antibody-modifying reagent loaded with thiol protector]

### (7-1) Synthesis of antibody-modifying reagent (compound 84) by coupling of protected thiol reagent with affinity peptide

The hydroxymaleimide-affinity peptide (compound 57) synthesized in (3-5-3) was dissolved in a dimethylsulfoxide solvent. 3-(Acetylthio) propionic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an acetylthiol-affinity peptide reagent (compound 84) as a product. 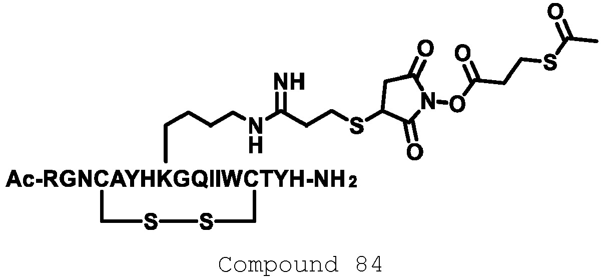 (Amino acid sequence of peptide portion: SEQ ID NO: 46)
MS (ESI)m/z:z=2 1217 [M+2H]²⁺, z=3 812 [M+3H]³⁺

### (7-2) Synthesis of antibody-modifying reagent (compound 85) by coupling of protected thiol reagent with affinity peptide

The hydroxymaleimide-affinity peptide (compound 57) synthesized in (3-5-3) was dissolved in a dimethylsulfoxide solvent. (S)-3-(benzoylthio)-2-methylpropionic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining a benzoylthiol-affinity peptide reagent (compound 85) as a product. 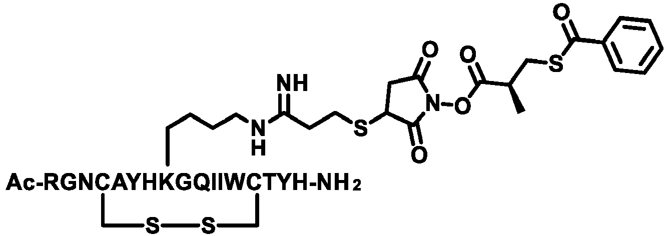 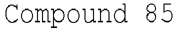 (Amino acid sequence of peptide portion: SEQ ID NO: 46)
MS (ESI)m/z:z=2 1255 [M+2H]²⁺, z=3 837 [M+3H]³⁺

### [Example 8: Regiospecific modification of IgG1 Fc with IgG1 Fc affinity peptide reagent loaded with thiol protector and analysis by ESI-TOFMS]

### (8-1) Antibody modification reaction with IgG1 Fc affinity peptide reagent loaded with thiol protector

### (8-1-1) Antibody Modification Reaction Using Affinity Peptide Reagent (compound 84)

The affinity peptide reagent loaded with a protected thiol (compound 84) synthesized in Example 7 was dissolved in N,N'-dimethylformamide to be 2.0 mM. 200 µg of anti-HER2 IgG antibody trastuzumab (Chugai Pharmaceutical Co., Ltd.) was dissolved in 200 µL of 50 mM HEPES buffer (pH 7.2), 20 molar equivalents of 2.0 mM peptide reagent was added to the antibody, and the resulting mixture was stirred at 25°C for three hours. The reaction solution was concentrated by ultrafiltration (Amicon Ultra, 10k MWCO) to obtain protected thiol-modified IgG antibody trastuzumab.

### (8-1-2) Antibody Modification Reaction Using Affinity Peptide Reagent (compound 85)

The affinity peptide reagent loaded with a protected thiol (compound 85) synthesized in Example 7 was dissolved in N,N'-dimethylformamide to be 2.0 mM. 200 µg of anti-HER2 IgG antibody trastuzumab (Chugai Pharmaceutical Co., Ltd.) was dissolved in 200 µL of 50 mM HEPES buffer (pH 7.2), 20 molar equivalents of 2.0 mM peptide reagent was added to the antibody, and the resulting mixture was stirred at 25°C for three hours. The reaction solution was concentrated by ultrafiltration (Amicon Ultra, 10k MWCO) to obtain protected thiol-modified IgG antibody trastuzumab.

### (8-2) Determination of heavy chain selectivity of protected thiol-modified IgG antibody trastuzumab under reduction conditions by ESI-TOFMS analysis

### (8-2-1) Determination of heavy chain selectivity of azide-modified trastuzumab under reduction conditions by ESI-TOFMS analysis when affinity peptide reagent (compound 84) is used in modification reaction

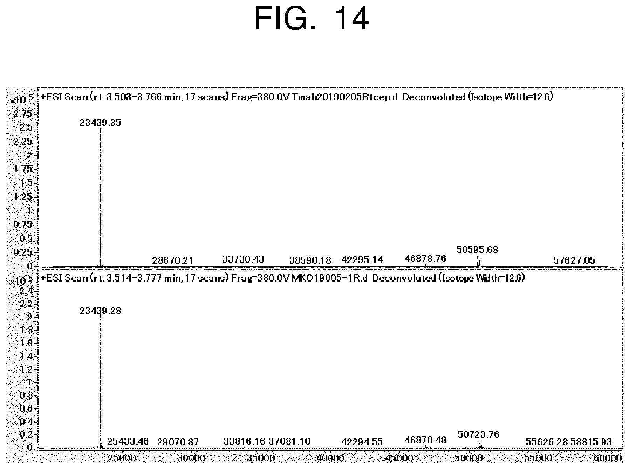

To the protected thiol-modified trastuzumab synthesized in (8-1-1), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50594 and 50756, and a light chain peak was observed at 23439. For a reaction product, peaks were observed at 50723 and 50885 with an acetyl protected thiol group introduced to the heavy chain, and a light chain peak was observed at 23439, the same as that of the raw material. FIG. 14 illustrates a result of ESI-TOFMS analysis.

### (8-2-2) Determination of heavy chain selectivity of trastuzumab-protected thiol compound under reduction conditions by ESI-TOFMS analysis when affinity peptide reagent (compound 3) is used in modification reaction

To the protected thiol-modified trastuzumab synthesized in (8-1-2), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50594 and 50756, and a light chain peak was observed at 23439. For a reaction product, peaks were observed at 50800 and 50962 with a benzoyl protected thiol group introduced to the heavy chain, and a light chain peak was observed at 23439, the same as that of the raw material.

### (8-3) Determination of formation of thiol-modified IgG antibody trastuzumab

### (8-3-1) Deprotection of acetyl protected thiol-modified IgG antibody trastuzumab

The acetyl protected thiol-modified IgG antibody antibody trastuzumab obtained in (8-2-1) was substituted with a 0.5 M aqueous hydroxylamine solution of 20 mM ethylenediamine (pH 6 preparation), and the resulting solution was allowed to stand at room temperature for one hour. The reaction solution was concentrated by ultrafiltration (Amicon Ultra, 10k MWCO) to obtain thiol-modified IgG antibody trastuzumab.

### (8-3-2) Determination of thiol-modified IgG antibody trastuzumab by ESI-TOFMS analysis

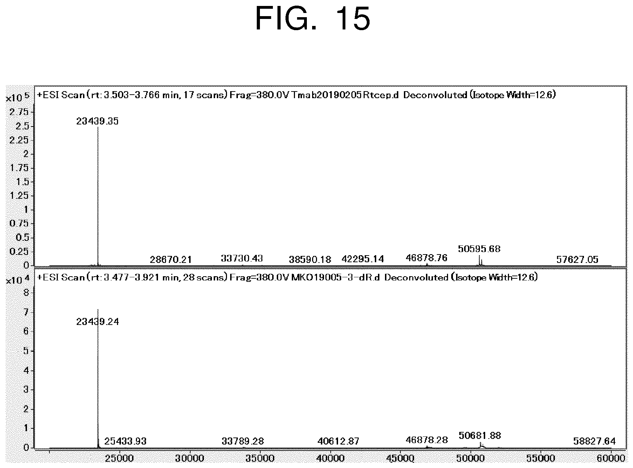

To the thiol-modified trastuzumab deprotected in (8-3-1), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for a reaction product, peaks were observed at 50681 and 50844 with a thiol deprotected with an acetyl group in the heavy chain, and it was determined that the acetyl group was removed to form a thiol. FIG. 15 illustrates a result of ESI-TOFMS analysis.

### (8-3-3) Deprotection of benzoyl protected thiol-modified IgG antibody trastuzumab

The benzoyl protected thiol-modified IgG antibody antibody trastuzumab obtained in (8-2-2) was substituted with a 0.5 M aqueous hydroxylamine solution of 20 mM ethylenediamine (pH 6 preparation), and the resulting solution was allowed to stand at room temperature for one hour. The reaction solution was concentrated by ultrafiltration (Amicon Ultra, 10k MWCO) to obtain each thiol-modified IgG antibody trastuzumab.

### (8-3-4) Determination of thiol-modified IgG antibody trastuzumab by ESI-TOFMS analysis

To the thiol-modified trastuzumab deprotected in (8-3-3), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for a reaction product, peaks were observed at 50696 and 50858 with a thiol deprotected with a benzoyl group in the heavy chain, and it was determined that the benzoyl group was removed to form a thiol.

### [Example 9: Regioselective modification of IgG1 Fc in multiple different target regions with IgG1 Fc affinity peptide reagent and analysis by ESI-TOFMS]

### (9-1) Regioselective modification in multiple different target regions using two types of affinity peptide reagents stepwise and analysis by ESI-TOFMS

### (9-1-1) Regiospecific antibody modification reaction at a plurality of points using two types of affinity peptide reagents (compounds 66 and 64) stepwise

The affinity peptide reagent loaded with azide (compound 66) synthesized in Example 5 was dissolved in N,N'-dimethylformamide to be 2.04 mM. 300 µg of anti-HER2 IgG antibody trastuzumab (Chugai Pharmaceutical Co., Ltd.) was dissolved in 300 µL of 50 mM sodium acetate buffer (pH 4.7), 20 molar equivalents of 2.04 mM peptide reagent was added to the antibody, and the resulting mixture was stirred at 25°C for three hours. A peptide reagent residue was removed by ultrafiltration (Amicon Ultra, 10k MWCO), and the obtained concentrate was diluted with 200 µL of 50 mM HEPES buffer (pH 7.2). 20 molar equivalents of 2.0 mM N,N'-dimethylformamide solution of the affinity peptide reagent loaded with azide (compound 64) synthesized in Example 5 was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The resulting solution was concentrated by ultrafiltration (Amicon Ultra, 10k MWCO) to obtain azide-modified IgG antibody trastuzumab.

### (9-1-2) Determination of heavy chain selectivity of azide-modified IgG antibody trastuzumab under reduction conditions by ESI-TOFMS analysis

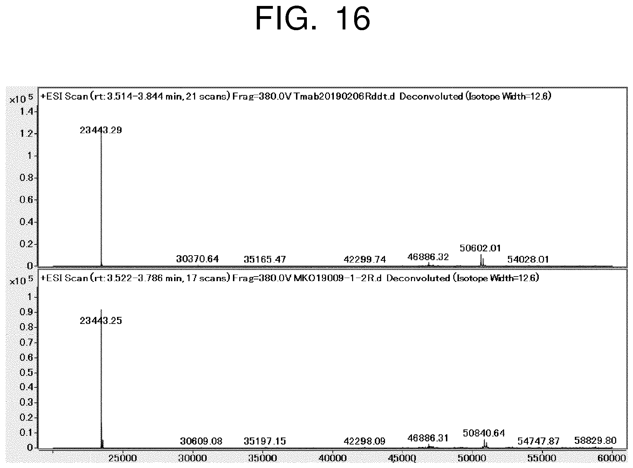

To the azide-modified trastuzumab synthesized in (9-1-1) of Example 9, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50602 and 50764, and a light chain peak was observed at 23443. For a reaction product, peaks were observed at 50841 and 51002 with two molecules of aminebenzoic acid were introduced to the heavy chain. A light chain peak was observed at 23443, the same as that of the raw material. Note that the azidobenzoic acid-introduced compound was reduced to an aminebenzoic acid-introduced compound by the pretreatment for the measurement. FIG. 16 illustrates a result of ESI-TOFMS analysis.

### (9-1-3) Regioselective modification reaction in multiple different target regions using two types of affinity peptide reagents (compounds 64 and 66) stepwise

The affinity peptide reagent loaded with azide (compound 64) synthesized in Example 5 was dissolved in N,N'-dimethylformamide to be 2.0 mM. 300 µg of anti-HER2 IgG antibody trastuzumab (Chugai Pharmaceutical Co., Ltd.) was dissolved in 300 µL of 50 mM HEPES buffer (pH 7.2), 20 molar equivalents of 2.0 mM peptide reagent was added to the antibody, and the resulting mixture was stirred at 37°C for 16 hours. A peptide reagent residue was removed by ultrafiltration (Amicon Ultra, 10k MWCO), and the obtained concentrate was diluted with 200 µL of 50 mM sodium acetate buffer (pH 4.7). 20 molar equivalents of 2.04 mM N,N'-dimethylformamide solution of the affinity peptide reagent loaded with azide (compound 66) synthesized in Example 5 was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The resulting solution was concentrated by ultrafiltration (Amicon Ultra, 10k MWCO) to obtain azide-modified IgG antibody trastuzumab.

### (9-1-4) Determination of heavy chain selectivity of azide-modified IgG antibody trastuzumab under reduction conditions by ESI-TOFMS analysis

The mass was measured by ESI-TOFMS by a similar method to (9-1-2) of Example 9. For the raw material trastuzumab used as a control, heavy chain peaks were observed at 50602 and 50764, and a light chain peak was observed at 23443. For a reaction product, peaks were observed at 50840 and 51002 with two molecules of aminebenzoic acid were introduced to the heavy chain. A light chain peak was observed at 23443, the same as that of the raw material. Note that the azidobenzoic acid-introduced compound was reduced to an aminebenzoic acid-introduced compound by the pretreatment for the measurement.

### (9-1-5) Regiospecific antibody modification reaction at a plurality of points using two types of affinity peptide reagents (compounds 84 and 64) stepwise

The affinity peptide reagent loaded with a thiol protector (compound 84) synthesized in Example 7 was dissolved in N,N'-dimethylformamide to be 2.04 mM. 300 µg of anti-HER2 IgG antibody trastuzumab (Chugai Pharmaceutical Co., Ltd.) was dissolved in 300 µL of 50 mM sodium acetate buffer (pH 4.7), 20 molar equivalents of 2.04 mM peptide reagent was added to the antibody, and the resulting mixture was stirred at 25°C for three hours. A peptide reagent residue was removed by ultrafiltration (Amicon Ultra, 10k MWCO), and the obtained concentrate was diluted with 200 µL of 50 mM HEPES buffer (pH 7.2). 20 molar equivalents of 2.0 mM N,N'-dimethylformamide solution of the affinity peptide reagent loaded with azide (compound 64) synthesized in Example 5 was added to the antibody, and the resulting mixture was stirred at 37°C for 16 hours. 50 µL of a 10 mg/mL aqueous Lys solution was added to the reaction solution, and the resulting mixture was stirred for 30 minutes to stop the reaction. The resulting solution was concentrated by ultrafiltration (Amicon Ultra, 10k MWCO) to obtain azide-modified IgG antibody trastuzumab.

### (9-1-6) Determination of heavy chain selectivity of azide-thiol-modified IgG antibody trastuzumab under reduction conditions by ESI-TOFMS analysis

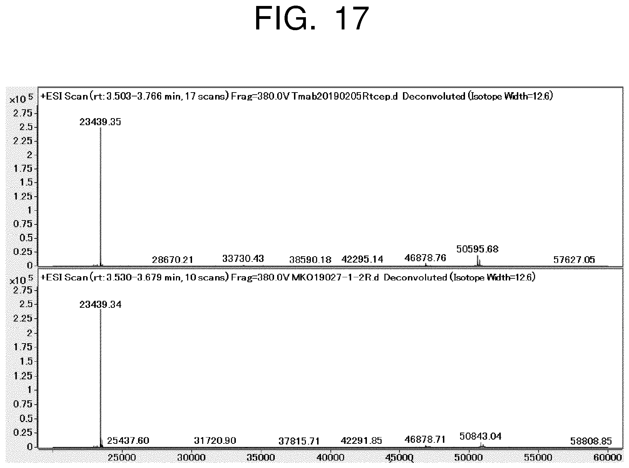

The mass of the azide-modified IgG antibody trastuzumab synthesized in (9-1-5) was measured by ESI-TOFMS by a similar method to (9-1-2) of Example 9. For the raw material trastuzumab used as a control, heavy chain peaks were observed at 50597 and 50757, and a light chain peak was observed at 23439. For a reaction product, peaks were observed at 50843 and 51005 with aminebenzoic acid and an acetyl protected thiol group introduced to the heavy chain. A light chain peak was observed at 23443, the same as that of the raw material. Note that the azidobenzoic acid-introduced compound was reduced to an aminebenzoic acid-introduced compound by the pretreatment for the measurement. FIG. 17 illustrates a result of ESI-TOFMS analysis.

### (9-2) Regioselective modification in multiple different target regions using affinity peptide having a plurality of derivatization sites and analysis by ESI-TOFMS

### (9-2-1) Synthesis of affinity peptide (compound 86) having a plurality of derivatization sites

The antibody affinity peptide (compound 54) synthesized in Example 1 was dissolved in dimethylsulfoxide, 2-iminothiolane hydrochloride (10 molar equivalents) and diisopropylethylamine (15 molar equivalents) were added thereto, and the resulting mixture was stirred for one hour. Disappearance of the raw materials was determined by LC-MS. Thereafter, a dimethyl sulfoxide solution of N-hydroxymaleimide (20 molar equivalent) was added thereto, and the resulting mixture was stirred for one hour. The progress of the reaction was determined by LC-MS. Thereafter, 4-azidobenzoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining a di(azide-phenyl)-affinity peptide reagent (compound 86) as a product. 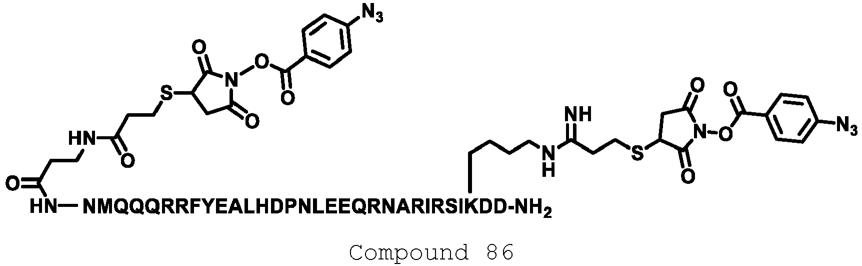 (Amino acid sequence of peptide portion: SEQ ID NO: 100)
MS (ESI)m/z:z=3 1587 [M+3H]³⁺, 3 1191 [M+4H]⁴⁺

### (9-2-2) Regiospecific antibody modification reaction at a plurality of points using affinity peptide having a plurality of derivatization sites

The di(azide-phenyl)-affinity peptide reagent (compound 86) synthesized in (9-2-1) of Example 9 was dissolved in N,N'-dimethylformamide to be 2.0 mM. 250 µg of anti-HER2 IgG antibody trastuzumab (Chugai Pharmaceutical Co., Ltd.) was dissolved in 250 µL of 50 mM HEPES buffer (pH 7.2), 20 molar equivalents of 2.0 mM peptide reagent was added to the antibody, and the resulting mixture was stirred at 25°C for three hours. The reaction solution was concentrated by ultrafiltration (Amicon Ultra, 10k MWCO) to obtain azide-modified IgG antibody trastuzumab.

### (9-2-3) Determination of heavy chain selectivity of azide-modified IgG antibody trastuzumab under reduction conditions by ESI-TOFMS analysis

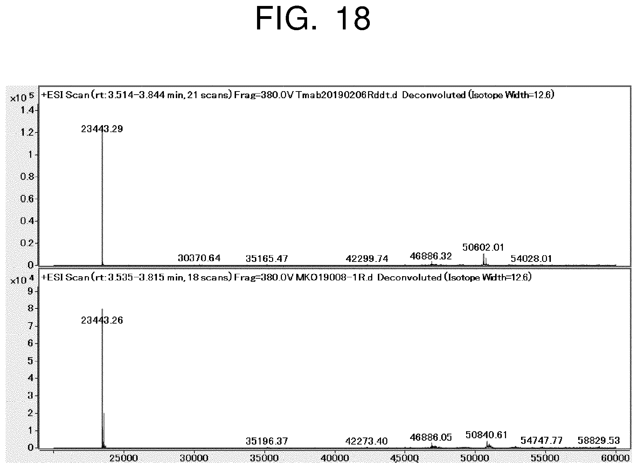

To the azide-modified trastuzumab synthesized in (9-2-2) of Example 9, 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50602 and 50764, and a light chain peak was observed at 23443. For a reaction product, peaks were observed at 50841 and 51003 with two molecules of aminebenzoic acid were introduced to the heavy chain. A light chain peak was observed at 23443, the same as that of the raw material. Note that the azidobenzoic acid-introduced compound was reduced to an aminebenzoic acid-introduced compound by the pretreatment for the measurement. FIG. 18 illustrates a result of ESI-TOFMS analysis.

### [Example 10: Conjugation of regiospecific azide-introduced trastuzumab with arbitrary compound and ESI-TOFMS analysis of product]

### (10-1) Conjugation of regiospecific azide-introduced trastuzumab with Cy3-cycloalkyne

### (10-1-1) Conjugation of regiospecific azide-introduced trastuzumab with Cy3-DBCO and ESI-TOFMS analysis

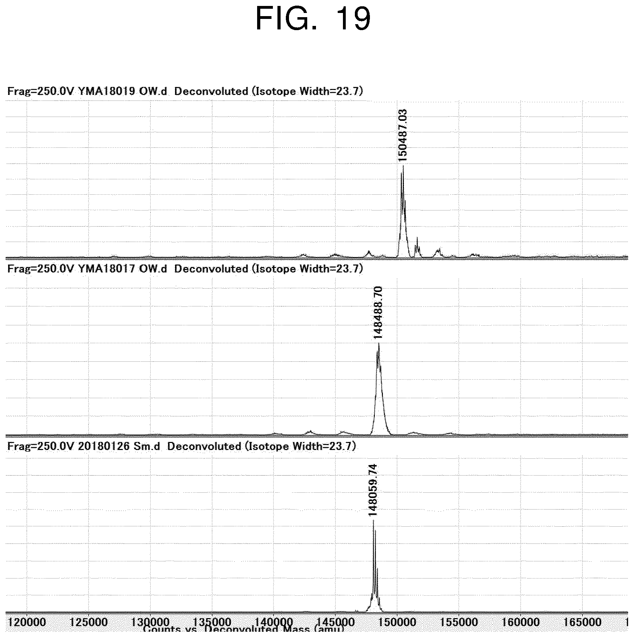

The azidobenzoic acid-introduced trastuzumab synthesized in (6-1-2) of Example 6 was dissolved in 20 mM PBS buffer to be 0.3 mM, then 20 µL of dibenzocyclooctyne-Cy3 manufactured by Sigma Aldrich (0.94 mM, dissolved in DMF) was added thereto, and the resulting mixture was stirred at room temperature for 12 hours. The reaction solution was subjected to water substitution by ultrafiltration (Amicon Ultra, 10K MWCO) to stop the reaction. The mass was measured by ESI-TOFMS; for the trastuzumab, a peak was observed at 148060, for the azide-introduced trastuzumab synthesized in (6-1-2), a peak was observed at 148489, and for a reaction product, a peak was observed at 150487 with two molecules of Cy3 introduced (FIG. 19).

### (10-1-2) ESI-TOFMS Analysis under Reduction Conditions

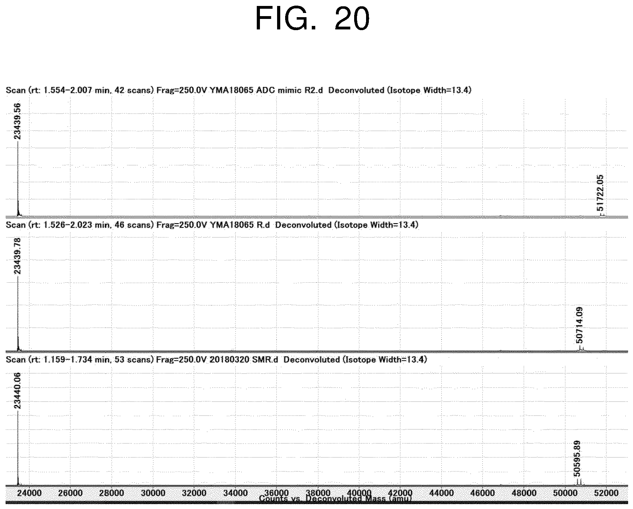

To the trastuzumab-Cy3 conjugate PBS solution synthesized in (10-1-1), 2 µL of a 7 mM tris(2-carboxyethyl) phosphine hydrochloride solution (100 equivalents to the trastuzumab-Cy3 conjugate) was added, and the resulting mixture was stirred at room temperature for 20 minutes. The mass was measured by ESI-TOFMS; for the raw material azide-introduced trastuzumab synthesized in (6-1-1) of Example 6, heavy chain peaks were observed at 50714 and 50876, and a light chain peak was observed at 23440, whereas for the trastuzumab-Cy3 conjugate, peaks were observed at 51722 and 51885 with Cy3 introduced to the heavy chain, and a light chain peak was observed at 23440, the same as that of the raw material (FIG. 20). Note that the azidobenzoic acid-introduced compound was reduced to an aminebenzoic acid-introduced compound by a reduction reaction of 7 mM tris(2-carboxyethyl) phosphine salt, which was a pretreatment for the measurement.

### (10-2) Conjugation of regiospecific azide-introduced trastuzumab with peptide-cycloalkyne

### (10-2-1) Synthesis of peptide-cycloalkyne

A peptide portion of compound 30 below was synthesized by solid phase peptide synthesis using 2-chlorotrityl resin by Fmoc method. After the solid phase synthesis, 1-hydroxybenzotriazole (2 molar equivalents), 1-ethyl-3-(3-dimethylaminopropyl) carbodiimide hydrochloride (2 molar equivalents), triethylamine (2 molar equivalents), and 5-(5,6-dihydro-11,12-didehydrodibenzo[b,f][b,f]azocin-5(6H)-yl)-oxopentanoic acid (2.2 molar equivalents) were added at any time, and the resulting mixture was stirred for 19 hours. The product was stirred in a solution of trifluoroacetic acid : water : triisopropylsilane = 95 : 2.5 : 2.5 for one hour, thereby performing cutting out from the resin and deprotection, and triethylamine was added to the filtrate after cutting for neutralization. The resin was removed by filtration, diethyl ether was added for precipitation, and the diethyl ether was removed by decantation to obtain a peptide as crude crystals. This peptide was purified by preparative HPLC to obtain compound 30 below as a product. 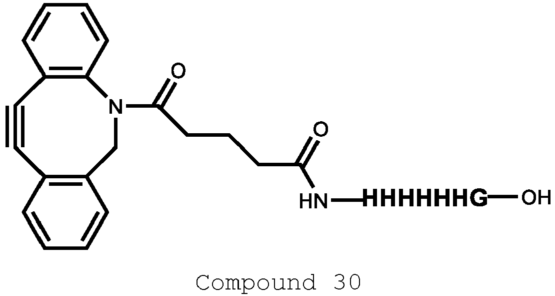 (Amino acid sequence of peptide portion: SEQ ID NO: 50)
MS (ESI) m/z:1200[M+H]⁺

### (10-2-2) Conjugation of regiospecific azide-introduced trastuzumab with peptide-cycloalkyne and ESI-TOFMS analysis

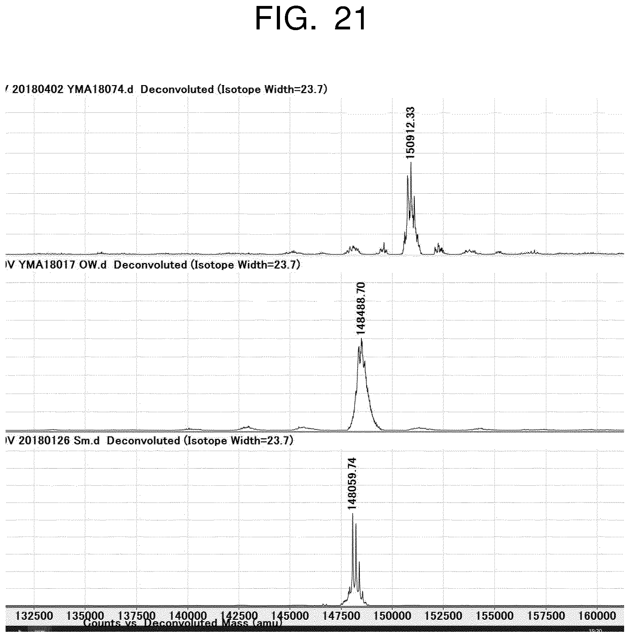

The azidobenzoic acid-introduced trastuzumab synthesized in (6-1-2) of Example 6 was dissolved in 20 mM PBS buffer to be 0.3 mM. Thereafter, 20 µL of a 0.94 mM aqueous solution of the peptide-cycloalkyne (compound 29) synthesized in (7-2-1) of Example 7 was added thereto, and the resulting mixture was stirred at room temperature for 12 hours. The reaction solution was subjected to water substitution by ultrafiltration (Amicon Ultra, 10K MWCO) to stop the reaction. The mass was measured by ESI-TOFMS; for the trastuzumab, a peak was observed at 148060, for the azide-introduced trastuzumab synthesized in (6-1-2), a peak was observed at 148489, and for a reaction product, a peak was observed at 150912 with two peptides introduced (FIG. 21) .

### (10-2-3) ESI-TOFMS analysis under reduction conditions

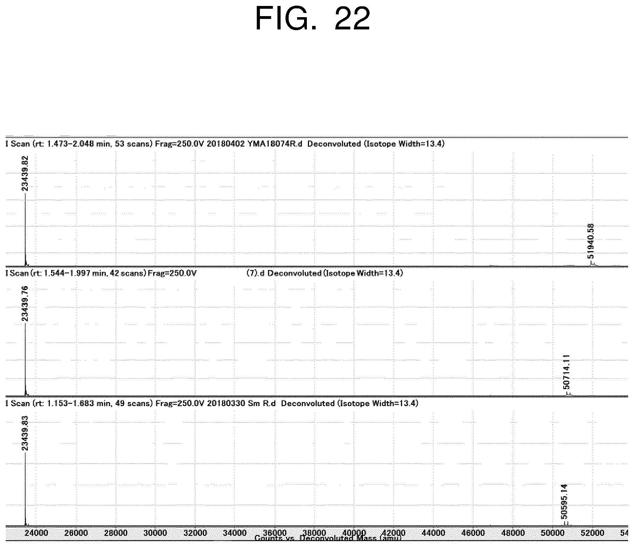

To the trastuzumab-peptide conjugate PBS solution synthesized in (10-2-2), 2 µL of a 7 mM tris(2-carboxyethyl) phosphine hydrochloride solution (100 equivalents to the trastuzumab-peptide conjugate) was added, and the resulting mixture was stirred at room temperature for 20 minutes. The mass was measured by ESI-TOFMS; for the raw material azide-introduced trastuzumab synthesized in (6-1-1) of Example 6, heavy chain peaks were observed at 50714 and 50876, and a light chain peak was observed at 23440, whereas for the trastuzumab-peptide conjugate, peaks were observed at 51941 and 52102 with peptide introduced to the heavy chain, and a light chain peak was observed at 23440, the same as that of the raw material (FIG. 22). Note that the azidobenzoic acid-introduced compound was reduced to an aminebenzoic acid-introduced compound by a reduction reaction of 7 mM tris(2-carboxyethyl) phosphine salt, which was a pretreatment for the measurement.

### (10-3) Conjugation of regiospecific thiol-introduced trastuzumab with maleimide compound and ESI-TOFMS analysis

### (10-3-1) Conjugation of regiospecific thiol-introduced trastuzumab with maleimide compound and ESI-TOFMS analysis

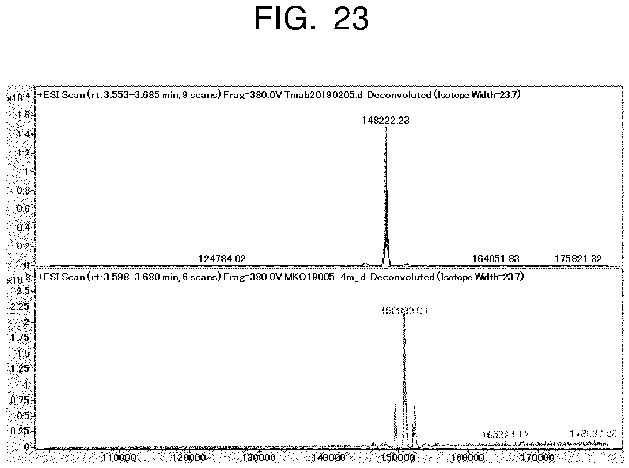

The thiol-introduced trastuzumab synthesized in (8-3-1) of Example 8 was dissolved in 20 mM PBS buffer to be 0.3 mM, then 5 molar equivalents of m-dPEG (registered trademark)₂₄-MAL was added thereto, and the resulting mixture was stirred at room temperature for 12 hours. The reaction solution was processed by ultrafiltration (Amicon Ultra, 10k MWCO). The mass was measured by ESI-TOFMS; for the trastuzumab, a peak was observed at 14822, for the thiol-introduced trastuzumab synthesized in (8-3-3), a peak was observed at 148243, and for a reaction product, a peak was observed at 150880 with two molecules of m-dPEG (registered trademark) ₂₄-MAL introduced (FIG. 23).

### (10-3-2) ESI-TOFMS analysis under reduction conditions

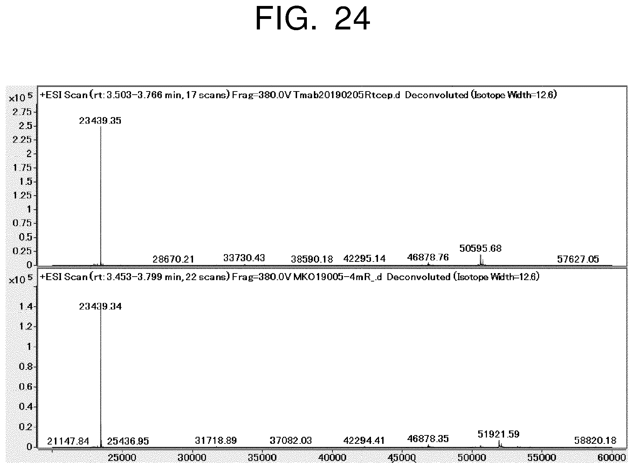

To the trastuzumab-peg conjugate PBS solution synthesized in (10-3-1), 2 µL of a 7 mM tris(2-carboxyethyl) phosphine hydrochloride solution (100 equivalents to the trastuzumab-peg conjugate) was added, and the resulting mixture was stirred at room temperature for 20 minutes. The mass was measured by ESI-TOFMS; for the raw material thiol-introduced trastuzumab synthesized in (8-3-3) of Example 8, heavy chain peaks were observed at 50682 and 50844, and a light chain peak was observed at 23449, whereas for a reaction product, peaks were observed at 51922 and 52083 with m-dPEG (registered trademark) ₂₄-MAL introduced to the heavy chain, and a light chain peak was observed at 23449, the same as that of the raw material (FIG. 24).

### [Example 11: Determination of regiospecific modification of bioorthogonal functional group-introduced trastuzumab]

### (11-1) Glycolysis of Bioorthogonal Functional Group-Introduced Trastuzumab

### (11-1-1) Glycolysis of Azidobenzoic Acid-Introduced Trastuzumab (1)

Using PNGase F (NEW ENGLAND BioLabs, catalog number P0704), glycolysis and post-treatment were performed on the azidobenzoic acid-introduced trastuzumab (azide-modified antibody 3) synthesized in (6-1-2) of Example 6 according to manufacturer's protocol and the technique of patent (WO 2017/191817.

### (11-1-2) Glycolysis of Azidobenzoic acid-Introduced Trastuzumab (2)

Using PNGase F (NEW ENGLAND BioLabs, catalog number P0704), glycolysis and post-treatment were performed on the azidobenzoic acid-introduced trastuzumab (azide-modified antibody 5) synthesized in (6-1-3) of Example 6 according to manufacturer's protocol and the technique of patent (WO 2017/191817).

### (11-1-3) Glycolysis of Maleimide-Introduced Trastuzumab (3) (K246/248)

Using PNGase F (NEW ENGLAND BioLabs, catalog number P0704), glycolysis and post-treatment were performed on the maleimide-introduced trastuzumab synthesized in (4-5-1) of Example 4 according to manufacturer's protocol and the technique of patent (WO 2017/19181757) in the presence of 3-mercaptopropionic acid (20 molar equivalents).

### (11-1-4) Glycolysis of Alkyl Azide-Introduced Trastuzumab (4) (K246/248)

Using PNGase F (NEW ENGLAND BioLabs, catalog number P0704), glycolysis and post-treatment were performed on the alkyl azide-introduced trastuzumab (azide-modified antibody 2) synthesized in (6-1-1) of Example 6 according to manufacturer's protocol and the technique of patent (WO 2017/19181757).

### (11-1-5) Glycolysis of Azidobenzoic Acid-Introduced Trastuzumab (5) (K317)

Using PNGase F (NEW ENGLAND BioLabs, catalog number P0704), glycolysis and post-treatment were performed on the azidobenzoic acid-introduced trastuzumab (azide-modified antibody 6) synthesized in (6-1-3) of Example 6 according to manufacturer's protocol and the technique of patent (WO 2017/19181757).

### (11-1-6) Glycolysis of Azidobenzoic Acid-Introduced Trastuzumab (6) (K288/290)

Using PNGase F (NEW ENGLAND BioLabs, catalog number P0704), glycolysis and post-treatment were performed on the azidobenzoic acid-introduced trastuzumab (azide-modified antibody 8) synthesized in (6-1-3) of Example 6 according to manufacturer's protocol and the technique of patent (WO 2017/19181757).

### (11-1-7) Glycolysis of Azidobenzoic Acid-Introduced Trastuzumab (7) (K246/248)

Using PNGase F (NEW ENGLAND BioLabs, catalog number P0704), glycolysis and post-treatment were performed on the azidobenzoic acid-introduced trastuzumab (azide-modified antibody 10) synthesized in (6-1-3) of Example 6 according to manufacturer's protocol and the technique of patent (WO 2017/19181757).

### (11-1-8) Glycolysis of Acetyl Protected Thiol-Introduced trastuzumab (8) (K246/248)

Using PNGase F (NEW ENGLAND BioLabs, catalog number P0704), glycolysis and post-treatment were performed on the acetyl protected thiol-introduced trastuzumab synthesized in (8-1-3) of Example 8 according to manufacturer's protocol and the technique of patent (WO 2017/19181757).

### (11-1-9) Glycolysis of Azidobenzoic Acid-Multiple Target Region Introduced Trastuzumab (9)

Using PNGase F (NEW ENGLAND BioLabs, catalog number P0704), glycolysis and post-treatment were performed on the azidobenzoic acid-multiple target region introduced trastuzumab synthesized in (9-1-3) of Example 9 according to manufacturer's protocol and the technique of patent (WO 2017/19181757).

### (11-1-10) Glycolysis of Azide-Thiol-Multiple Target Region Introduced Trastuzumab (10)

Using PNGase F (NEW ENGLAND BioLabs, catalog number P0704), glycolysis and post-treatment were performed on the azide-thiol-multiple target region introduced trastuzumab synthesized in (9-1-4) of Example 9 according to manufacturer's protocol and the technique of patent (WO 2017/19181757).

### (11-1-11) Glycolysis of Azidobenzoic Acid-Multiple Target Region Introduced Denosumab (11)

Using PNGase F (NEW ENGLAND BioLabs, catalog number P0704), glycolysis and post-treatment were performed on the azidobenzoic acid-introduced denosumab (azide-modified antibody 29) synthesized in (6-1-4) of Example 6 according to manufacturer's protocol and the technique of patent (WO 2017/19181757).

### (11-1-12) Glycolysis of Azidobenzoic Acid-Multiple Target Region Introduced Dupilumab (12)

Using PNGase F (NEW ENGLAND BioLabs, catalog number P0704), glycolysis and post-treatment were performed on the azidobenzoic acid-introduced dupilumab (azide-modified antibody 30) synthesized in (6-1-4) of Example 6 according to manufacturer's protocol and the technique of patent (WO 2017/19181757).

### (11-2) Trypsin Treatment of Azidobenzoic Acid-Introduced Trastuzumab

### (11-2-1) Trypsin Treatment of Glycosylated Azidobenzoic Acid-Introduced Trastuzumab (1)

To a 1.5 mL low-adsorption micro test tube, 3.4 µL of a sample solution of the glycosylated azidobenzoic acid-introduced trastuzumab obtained in (11-1-1) and 6.6 µL of 50 mM ammonium hydrogencarbonate buffer were added to be 10 µL in total. A 50 mM ammonium hydrogencarbonate buffer and 10 µL of a 20 mM aqueous dithiothreitol solution dissolved in 20% trifluoroethanol were added thereto. The resulting mixture was heated at 65°C for one hour, then 10 µL of a 50 mM aqueous iodoacetamide solution was added thereto, and the resulting mixture was reacted in a dark place at room temperature for 30 minutes while being shaken at 300 rpm. After the reaction, 40 µL of a 50 mM ammonium hydrogencarbonate buffer was added thereto, the resulting mixture was stirred, 10 µL of a 20 ng/µL aqueous trypsin solution was added thereto, and the resulting mixture was subjected to enzyme digestion at 37°C for 18 hours. After the digestion, 2 µL of a 20% aqueous trifluoroacetic acid solution was added thereto to stop the reaction. Thereafter, the solution was diluted 10 times with a 50 mM ammonium hydrogen carbonate buffer or 0.5% trifluoroacetate. The diluted solution was used for LC-MS/MS measurement.

### (11-2-2) Trypsin Treatment of Glycosylated Azidobenzoic Acid-Introduced Trastuzumab (2)

To a 1.5 mL low-adsorption micro test tube, 10 µL of a sample solution of the glycosylated azidobenzoic acid-introduced trastuzumab obtained in (11-1-2), a 50 mM ammonium hydrogencarbonate buffer, and 10 µL of a 20 mM aqueous dithiothreitol solution dissolved in 20% trifluoroethanol were added. The resulting mixture was heated at 65°C for one hour, then 10 µL of a 50 mM aqueous iodoacetamide solution was added thereto, and the resulting mixture was reacted in a dark place at room temperature for 30 minutes while being shaken at 300 rpm. After the reaction, 40 µL of a 50 mM ammonium hydrogencarbonate buffer was added thereto, the resulting mixture was stirred, 10 µL of a 20 ng/µL aqueous trypsin solution was added thereto, and the resulting mixture was subjected to enzyme digestion at 37°C for 18 hours. After the digestion, 2 µL of a 20% aqueous trifluoroacetic acid solution was added thereto to stop the reaction. Thereafter, the solution was diluted 10 times with a 50 mM ammonium hydrogen carbonate buffer or 0.5% trifluoroacetate. The diluted solution was used for LC-MS/MS measurement.

### (11-2-3) Trypsin Treatment of Glycosylated Maleimide-introduced Trastuzumab (3)

To a 1.5 mL low-adsorption micro test tube, 10 µL of a sample solution of the glycosylated maleimide-introduced trastuzumab obtained in (11-1-3) was added, and a similar treatment to 11-2-1 was performed to obtain a sample for LC-MS/MS measurement.

### (11-2-4) Trypsin Treatment of Glycosylated Alkyl Azide-Introduced Trastuzumab (4)

To a 1.5 mL low-adsorption micro test tube, 10 µL of a sample solution of the glycosylated alkyl azide-introduced trastuzumab obtained in (11-1-4) was added, and a similar treatment to 11-2-1 was performed to obtain a sample for LC-MS/MS measurement.

### (11-2-5) Trypsin Treatment of Glycosylated Azidobenzoic Acid-Introduced Trastuzumab (5)

To a 1.5 mL low-adsorption micro test tube, 10 µL of a sample solution of the glycosylated azidobenzoic acid-introduced trastuzumab obtained in (11-1-5) was added, and a similar treatment to 11-2-1 was performed to obtain a sample for LC-MS/MS measurement.

### (11-2-6) Trypsin Treatment of Glycosylated Azidobenzoic Acid-Introduced Trastuzumab (6)

To a 1.5 mL low-adsorption micro test tube, 10 µL of a sample solution of the glycosylated azidobenzoic acid-introduced trastuzumab obtained in (11-1-6) was added, and a similar treatment to 11-2-1 was performed to obtain a sample for LC-MS/MS measurement.

### (11-2-7) Trypsin Treatment of Glycosylated Azidobenzoic Acid-Introduced Trastuzumab (7)

To a 1.5 mL low-adsorption micro test tube, 10 µL of a sample solution of the glycosylated azidobenzoic acid-introduced trastuzumab obtained in (11-1-7) was added, and a similar treatment to 11-2-1 was performed to obtain a sample for LC-MS/MS measurement.

### (11-2-8) Trypsin Treatment of Glycosylated Acetylthiol-Introduced Trastuzumab (8)

To a 1.5 mL low-adsorption micro test tube, 10 µL of a sample solution of the glycosylated acetylthiol-introduced trastuzumab obtained in (11-1-8) was added, and a similar treatment to 11-2-1 was performed to obtain a sample for LC-MS/MS measurement.

### (11-2-9) Trypsin Treatment of Glycosylated Azidobenzoic Acid-Introduced Trastuzumab (9)

To a 1.5 mL low-adsorption micro test tube, 10 µL of a sample solution of the glycosylated azidobenzoic acid-introduced trastuzumab obtained in (11-1-9) was added, and a similar treatment to 11-2-1 was performed to obtain a sample for LC-MS/MS measurement.

### (11-2-10) Trypsin Treatment of Glycosylated Acetylthiol and Azidobenzoic Acid-Introduced Trastuzumab (10)

To a 1.5 mL low-adsorption micro test tube, 10 µL of a sample solution of the glycosylated acetylthiol and azidobenzoic acid-introduced trastuzumab obtained in (11-1-8) was added, and a similar treatment to 11-2-1 was performed to obtain a sample for LC-MS/MS measurement.

### (11-2-11) Trypsin Treatment of Glycosylated Azidobenzoic Acid-Introduced Denosumab (11)

To a 1.5 mL low-adsorption micro test tube, 10 µL of a sample solution of the glycosylated azidobenzoic acid-introduced denosumab obtained in (11-1-11) was added, and a similar treatment to 11-2-1 was performed to obtain a sample for LC-MS/MS measurement.

### (11-2-12) Trypsin Treatment of Glycosylated Azidobenzoic Acid-Introduced Dupilumab (12)

To a 1.5 mL low-adsorption micro test tube, 10 µL of a sample solution of the glycosylated azidobenzoic acid-introduced dupilumab obtained in (11-1-12) was added, and a similar treatment to 11-2-1 was performed to obtain a sample for LC-MS/MS measurement.

### (11-3) LC-MS/MS Measurement Conditions for Trastuzumab

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 0.3-1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Acquisition
Activation Type:Collision Induced Dissociation(CID)

Data acquisition was performed using Xcalibur 3.0 (Thermo Fisher Scientific) and Thermo Orbitrap Fusion Tune Application 2.0 (Thermo Fisher Scientific) as accompanying software.

### (11-4) Analysis Conditions for Modified Site of Trastuzumab, Denosumab, and Dupilumab

Modified site analysis of an LC-MS/MS measurement result was performed using BioPharma Finder 3.0 (Thermo Fisher Scientific).

The analysis with BioPharma Finder was performed by setting S/N threshold to 1 and setting MS Noise Level to 0.01% of a peak top intensity. A digestive enzyme was set to Trypsin, and e Specificity was set to High. Furthermore, Mass Tolerance at the time of peak detection was set to 4 ppm, and Mass Accuracy at the time of peptide identification was set to 5 ppm. For Static Modification, Carbamidomethyl (+57.021 Da) was set as modification of a cysteine residue with iodoacetamide. For Dynamic Modifications, oxidation of methionine (+15.995 Da) and a modified compound to a lysine residue (maleimide introduced compound (+219.090 Da), mercaptopropionic acid-added maleimide-introduced compound (+325.098 Da), alkyl azide-introduced compound (+139.075Da), alkylamine-introduced compound (+113.084Da), azidobenzoic acid-introduced compound (+145.028Da), aminebenzoic acid-introduced compound (+119.037Da), acetylthiol-introduced compound (+130.009Da), and carbamidomethylated acetylthiol-introduced compound (+145.020 Da)) were set. In addition, a filter was set such that only those with a Confidence Score of 80 or higher and with observed MS/MS were obtained.

Note that during the digestion, reductive alkylation with dithiothreitol and iodoacetamide is performed, and therefore the alkyl azide introduced compound is partially reduced to an alkyl amine-introduced compound, the azidobenzoic acid-introduced compound is reduced to an aminebenzoic acid-introduced compound, and the acetylthiol-introduced compound is carbamidomethylated.

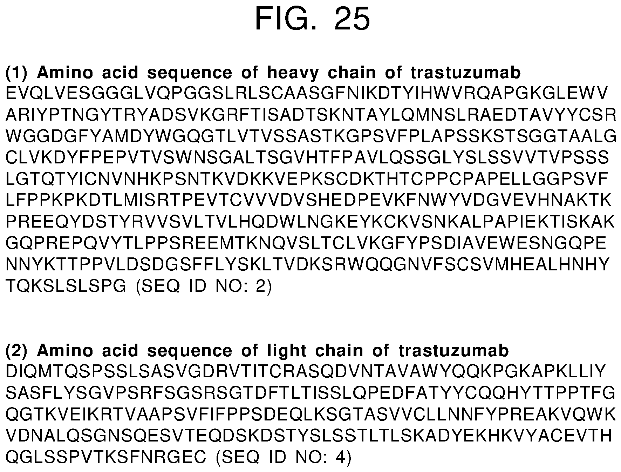

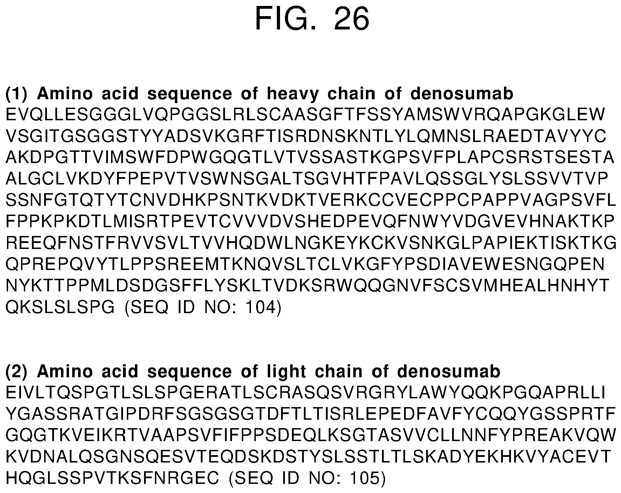

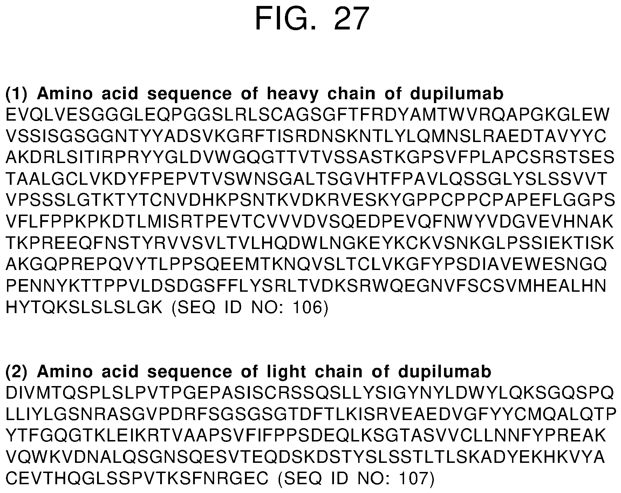

As data on an amino acid sequence to be searched for a modified site, (1) and (2) illustrated in FIG. 25 were used for trastuzumab, (1) and (2) illustrated in FIG. 26 were used for denosumab, and (1) and (2) illustrated in FIG. 27 were used for dupilumab.

### (11-5) Analysis Result of Modified Site of Digest by LC-MS/MS

### (11-5-1) Analysis Result of Modified Site of Trypsin Digest by LC-MS/MS (1)

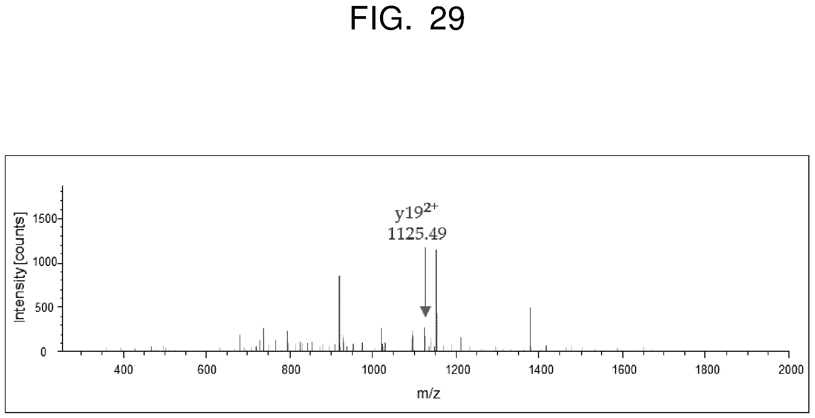

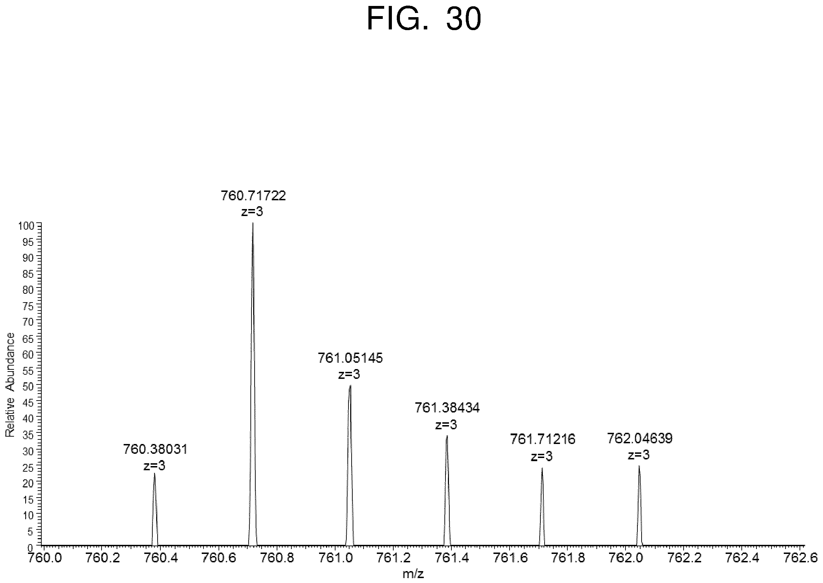

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of azide-modified trastuzumab (aminebenzoic acid-introduced compound (+119.037Da)) obtained in (11-2-1) (measured value: m/z 945.73328; theoretical value: 945.73350; and tetravalent) was observed (FIG. 29); and from a CID spectrum, a product ion of m/z 1125.49 (theoretical value: 1125.11) corresponding to divalent y19 indicating modification of a lysine residue at position 246 or 248 in EU numbering of the heavy chain was determined (FIG. 30).

### (11-5-2) Analysis Result of Modified Site of Trypsin Digest by LC-MS/MS (2)

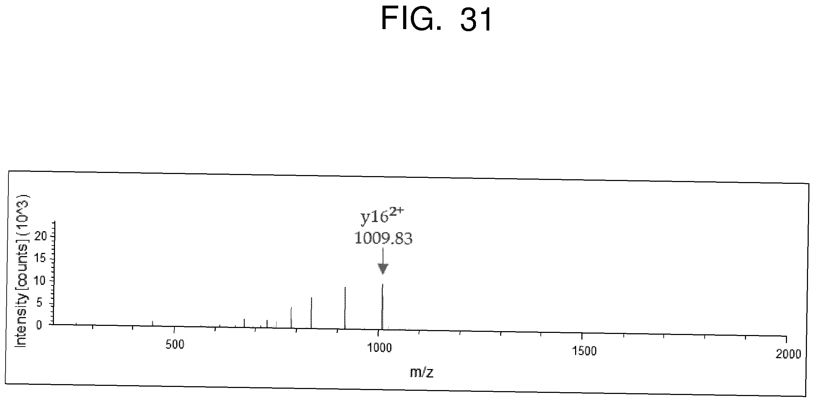

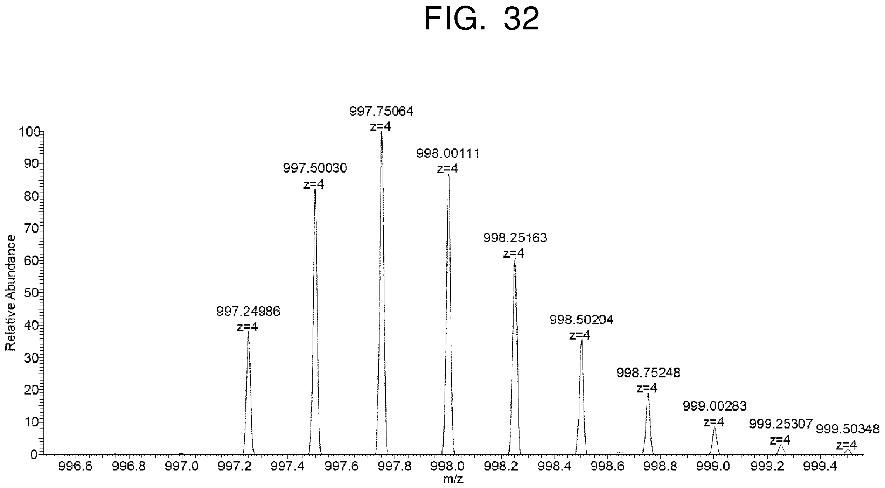

After analysis using LC-MS/MS, an MS spectrum of the peptide fragment of FNWYVDGVEVHNAKTKPR (SEQ ID NO: 12), which is a peptide consisting of 18 amino acid residues comprising a modified site to a lysin residue by trypsin digestion of azide-modified trastuzumab (aminebenzoic acid-introduced compound (+119.037Da)) obtained in (11-2-2) (measured value: m/z 760.38287; theoretical value: 760.38390; and trivalent) was observed (FIG. 31), and from a CID spectrum, a product ion of m/z 1009.83 (theoretical value: 1009.52) corresponding to divalent y16 indicating modification of a lysine residue at position 288 or 290 in EU numbering of the heavy chain was determined (FIG. 32).

### (11-5-3) Analysis Result of Modified Site of Trypsin Digest by LC-MS/MS (3)

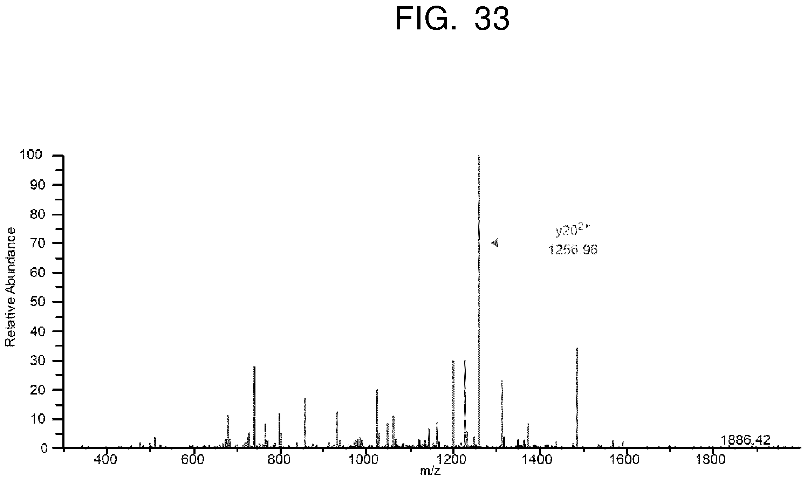

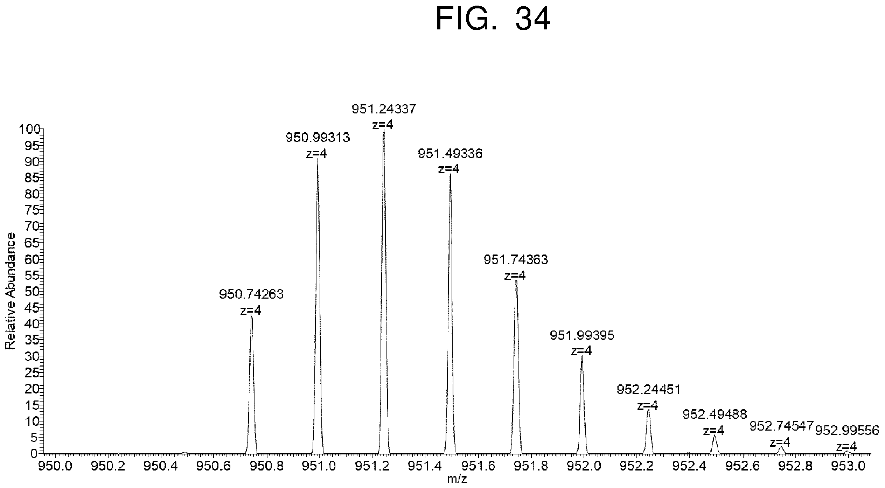

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of maleimide MPA-modified trastuzumab (mercaptopropionic acid-added maleimide-introduced compound (+325.098 Da)) obtained in (11-2-3) (measured value: m/z 997.24986; theoretical value: 997.24908; and tetravalent) was observed (FIG. 33); and from a CID spectrum, a product ion of m/z 1256.96 (theoretical value: 1256.65) corresponding to divalent y20 indicating modification of a lysine residue at position 246 or 248 in EU numbering of the heavy chain was determined (FIG. 34).

From this result, it was found that in (11-2-3) above, conjugation progressed regioselectively at Lys246 and Lys248 in EU numbering on the heavy chain of the antibody.

### (11-5-4) Analysis Result of Modified Site of Trypsin Digest by LC-MS/MS (4)

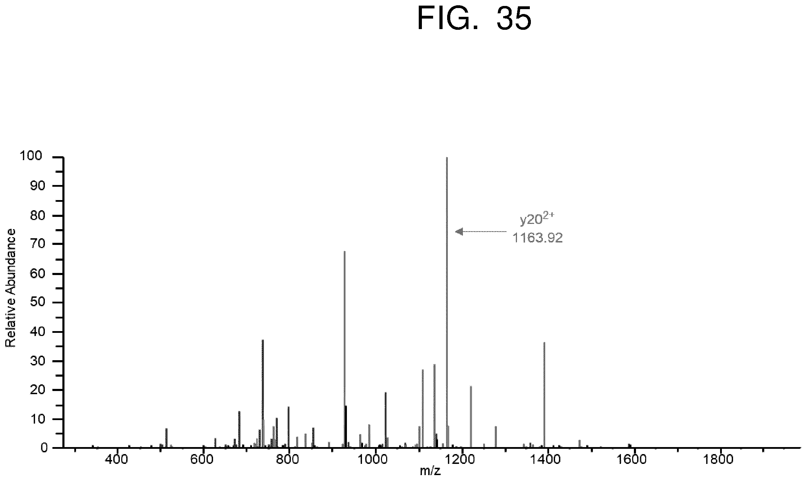

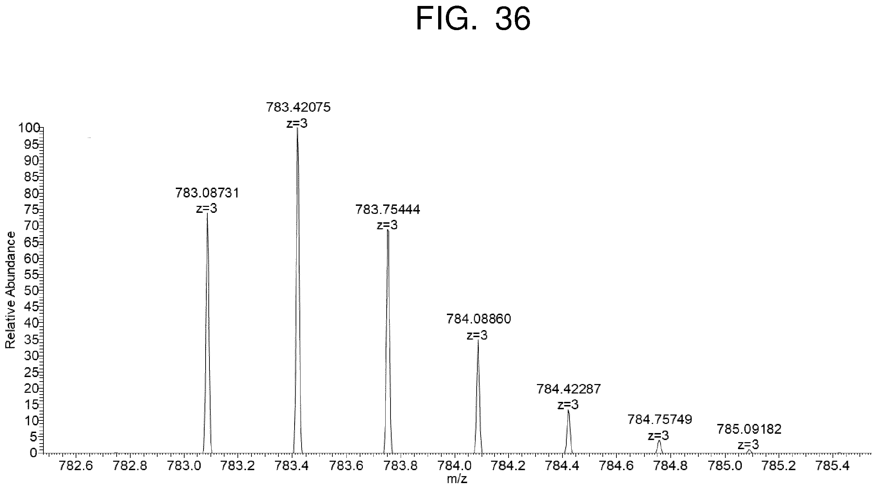

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of alkyl azide-modified trastuzumab (alkyl azide-introduced compound (+139.075Da)) obtained in (11-2-4) (measured value: m/z 950.74263; theoretical value: 950.74313; and tetravalent) was observed (FIG. 35); and from a CID spectrum, a product ion of m/z 1163.92 (theoretical value: 1163.64) corresponding to divalent y20 indicating modification of a lysine residue at position 246 or 248 in EU numbering of the heavy chain was determined (FIG. 36).

From this result, it was found that in (11-2-4) above, conjugation progressed regioselectively at Lys246 and Lys248 in EU numbering on the heavy chain of the antibody.

### (11-5-5) Analysis Result of Modified Site of Trypsin Digest by LC-MS/MS (5)

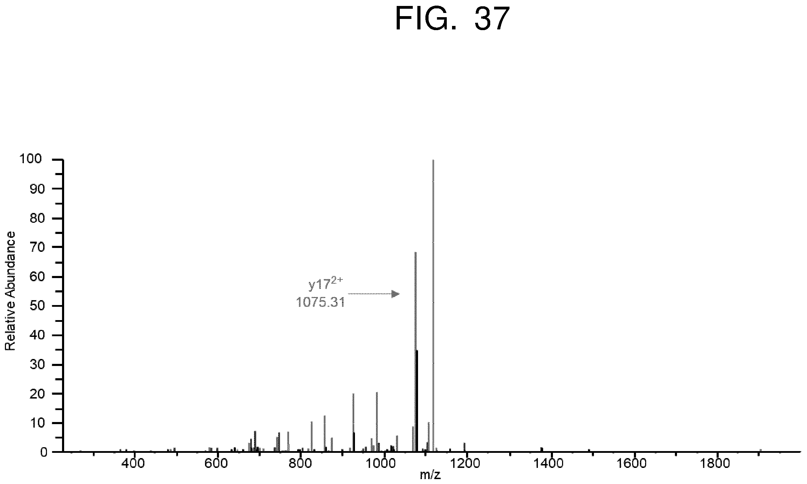

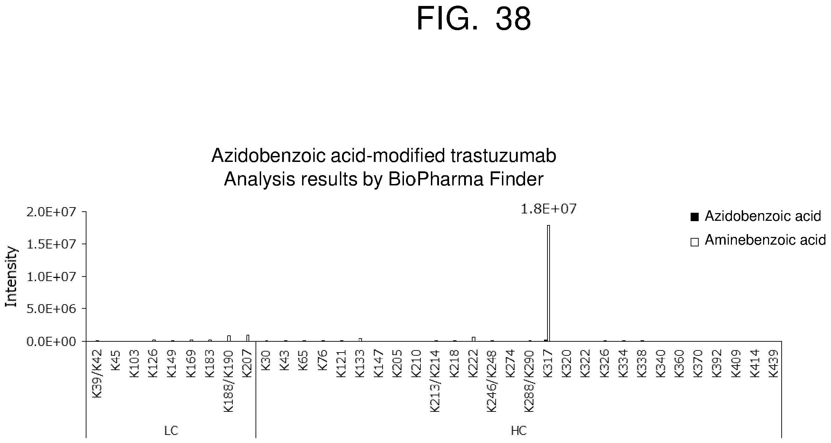

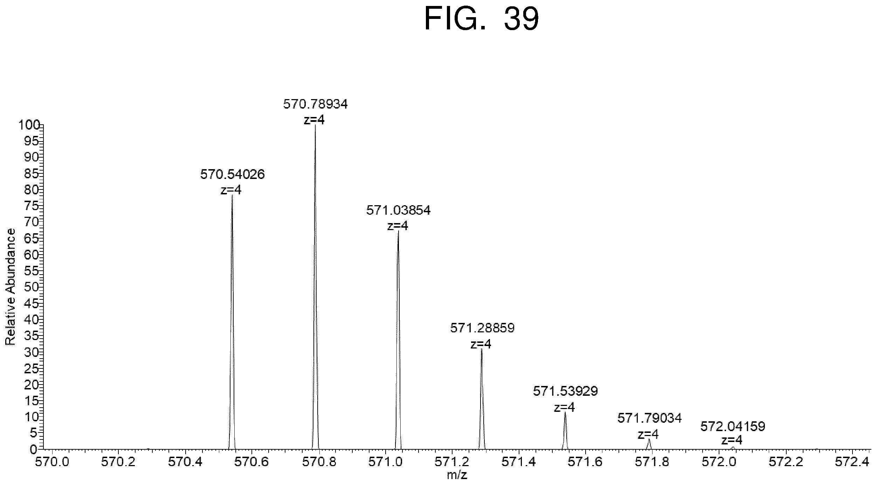

After analysis using LC-MS/MS, an MS spectrum of the peptide fragment of VVSVLTVLHQDWLNGKEYK (SEQ ID NO: 101), which is a peptide consisting of 19 amino acid residues comprising a modified site to a lysin residue by trypsin digestion of azidobenzoic acid-modified trastuzumab (aminebenzoic acid-introduced compound (+119.037Da)) obtained in (11-2-5) (measured value: m/z 783.08731; theoretical value: 783.08690; and trivalent) was observed (FIG. 37), and from a CID spectrum, a product ion of m/z 1075.31 (theoretical value: 1075.06) corresponding to divalent y17 indicating modification of a lysine residue at position 317 in EU numbering of the heavy chain was determined (FIG. 38). In addition, analysis with BioPharma Finder indicated that modification to a lysine residue at position 317 occurred highly selectively (FIG. 39).

From this result, it was found that in (11-2-5) above, conjugation progressed regioselectively at Lys317 in EU numbering on the heavy chain of the antibody.

### (11-5-6) Analysis Result of Modified Site of Trypsin Digest by LC-MS/MS (6)

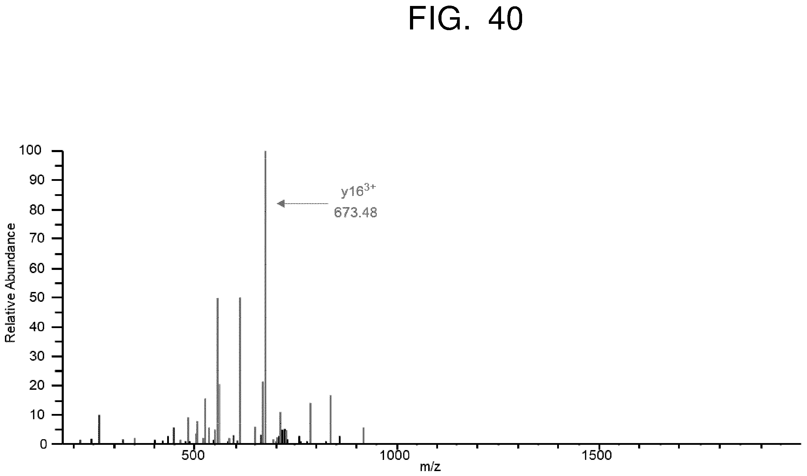

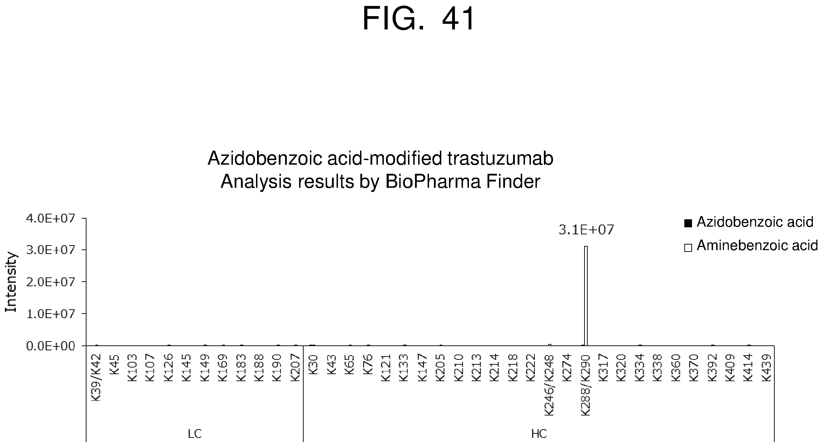

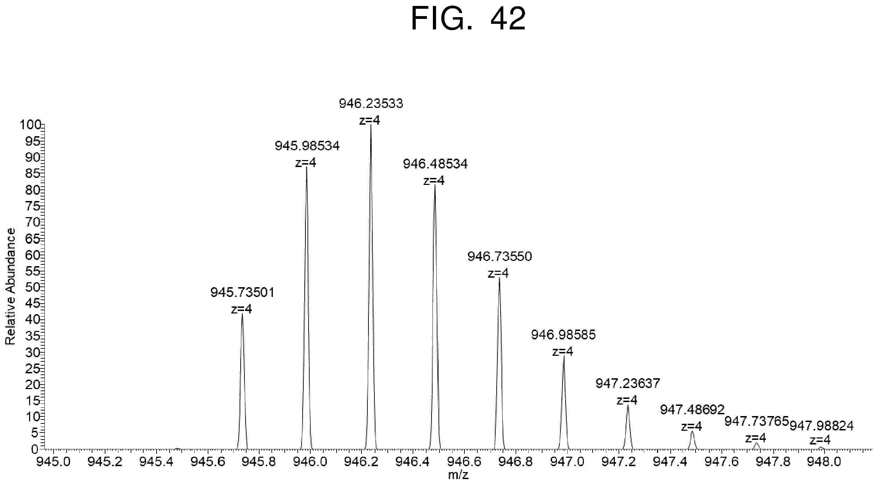

After analysis using LC-MS/MS, an MS spectrum of the peptide fragment of FNWYVDGVEVHNAKTKPR (SEQ ID NO: 12), which is a peptide consisting of 18 amino acid residues comprising a modified site to a lysin residue by trypsin digestion of azidobenzoic acid-modified trastuzumab (aminebenzoic acid-introduced compound (+119.037Da)) obtained in (11-2-6) (measured value: m/z 570.54026; theoretical value: 570.53991; and tetravalent) was observed (FIG. 40), and from a CID spectrum, a product ion of m/z 673.48 (theoretical value: 673.35) corresponding to trivalent y16 indicating modification of a lysine residue at position 288 or 290 in EU numbering of the heavy chain was determined (FIG. 41). In addition, analysis with BioPharma Finder indicated that modification to a lysine residue at position 288 or 290 occurred highly selectively (FIG. 42).

From this result, it was found that in (11-2-6) above, conjugation progressed regioselectively at Lys288 and Lys290 in EU numbering on the heavy chain of the antibody.

### (11-5-7) Analysis Result of Modified Site of Trypsin Digest by LC-MS/MS (7)

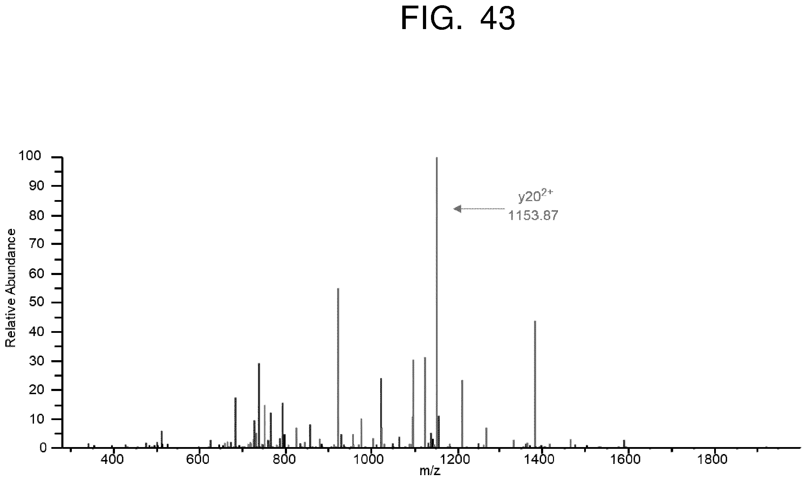

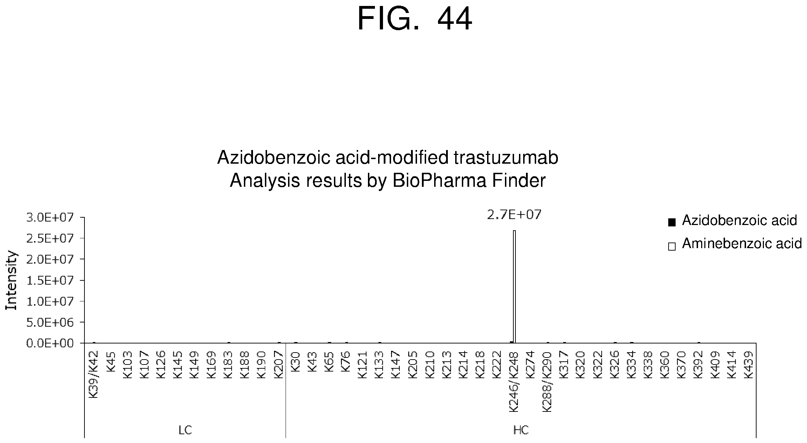

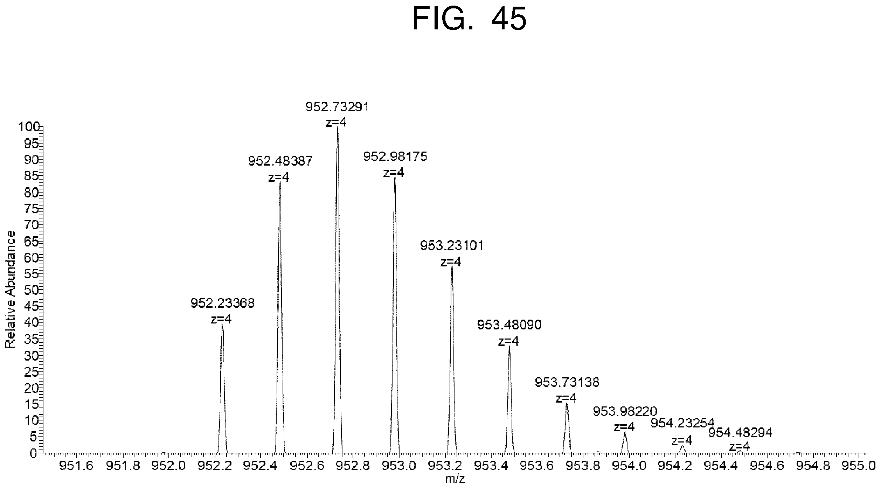

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of azidobenzoic acid-modified trastuzumab (aminebenzoic acid-introduced compound (+119.037Da)) obtained in (11-2-7) (measured value: m/z 945.73501; theoretical value: 945.73376; and tetravalent) was observed (FIG. 43); and from a CID spectrum, a product ion of m/z 1153.87 (theoretical value: 1153.62) corresponding to divalent y20 indicating modification of a lysine residue at position 246 or 248 in EU numbering of the heavy chain was determined (FIG. 44). In addition, analysis with BioPharma Finder indicated that modification to a lysine residue at position 246 or 248 occurred highly selectively (FIG. 45).

From this result, it was found that in (11-2-7) above, conjugation progressed regioselectively at Lys246 and Lys248 in EU numbering on the heavy chain of the antibody.

### (11-5-8) Analysis Result of Modified Site of Trypsin Digest by LC-MS/MS (8)

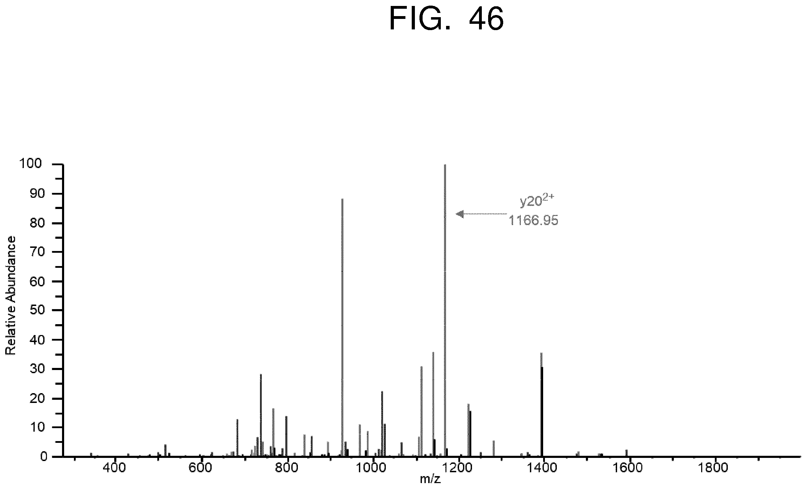

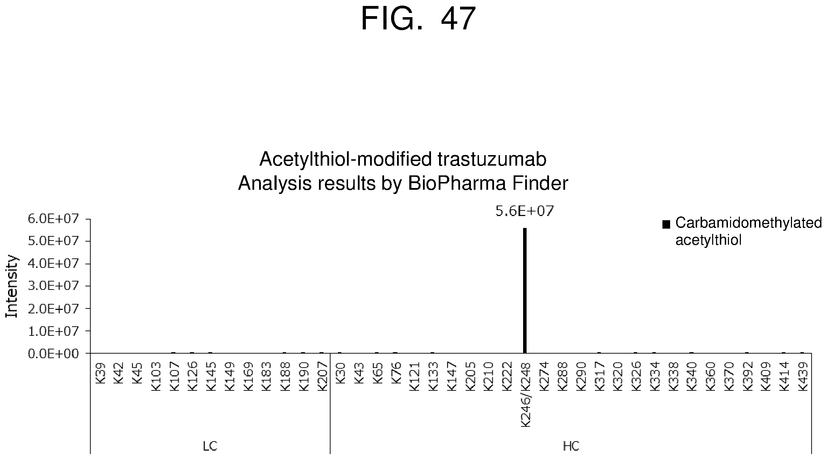

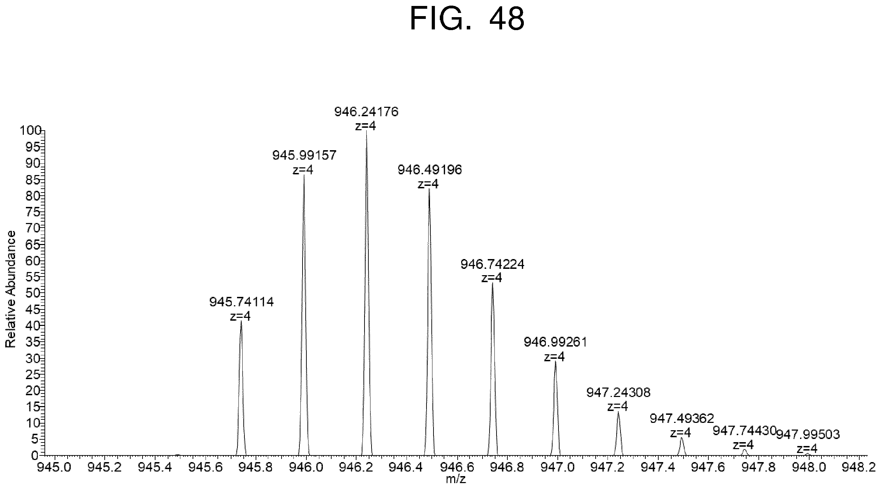

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of acetylthiol-modified trastuzumab (carbamidomethylated acetylthiol-introduced compound (+145.020Da)) obtained in (11-2-8) (measured value: m/z 952.23368; theoretical value: 952.22942; and tetravalent) was observed (FIG. 46); and from a CID spectrum, a product ion of m/z 1166.95 (theoretical value: 1166.61) corresponding to divalent y20 indicating modification of a lysine residue at position 246 or 248 in EU numbering of the heavy chain was determined (FIG. 47). In addition, analysis with BioPharma Finder indicated that modification to a lysine residue at position 246 or 248 occurred highly selectively (FIG. 48).

From this result, it was found that in (11-2-8) above, conjugation progressed regioselectively at Lys246 and Lys248 in EU numbering on the heavy chain of the antibody.

### (11-5-9) Analysis Result of Modified Site of Trypsin Digest by LC-MS/MS (9)

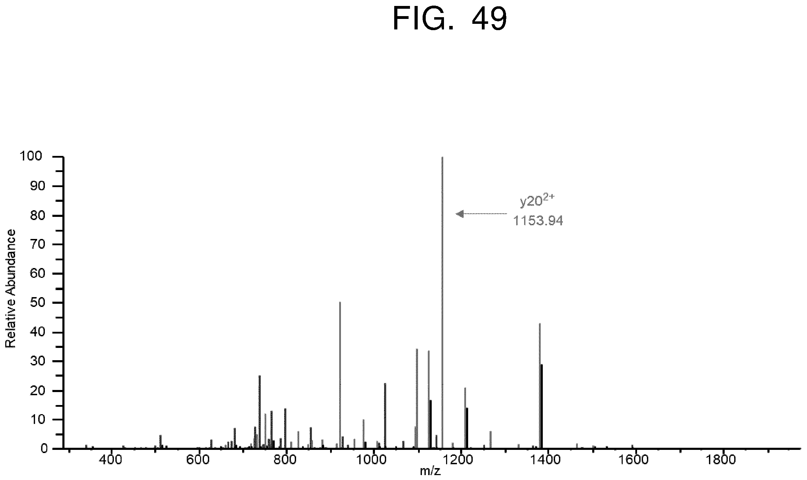

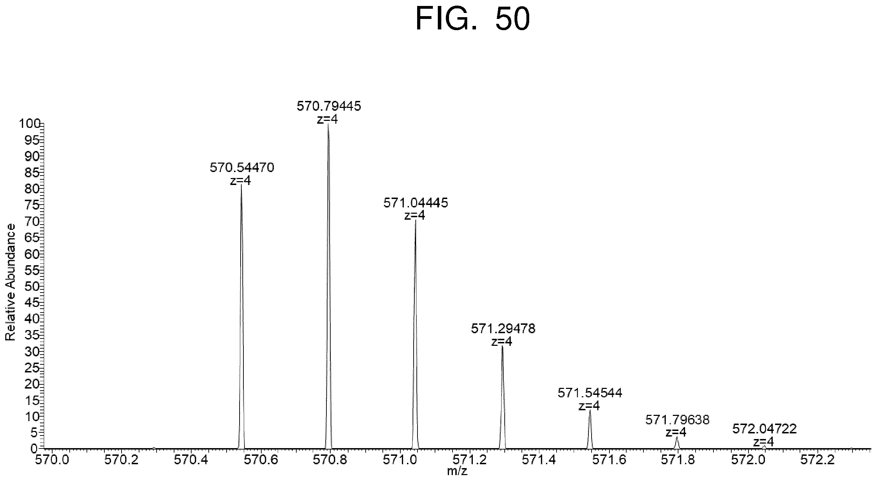

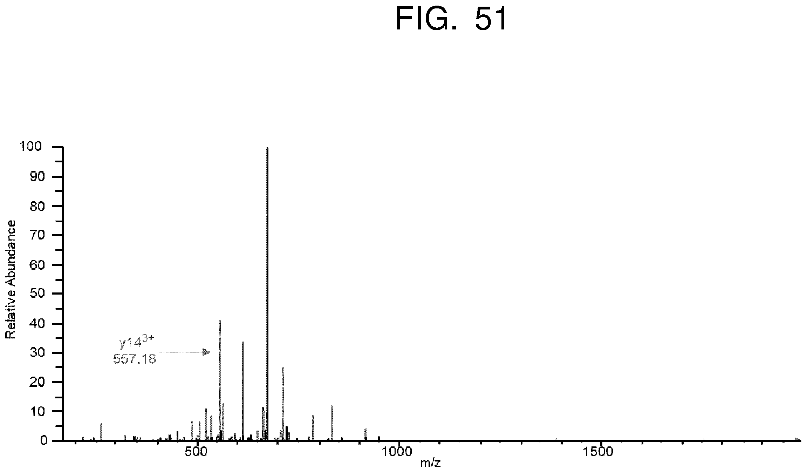

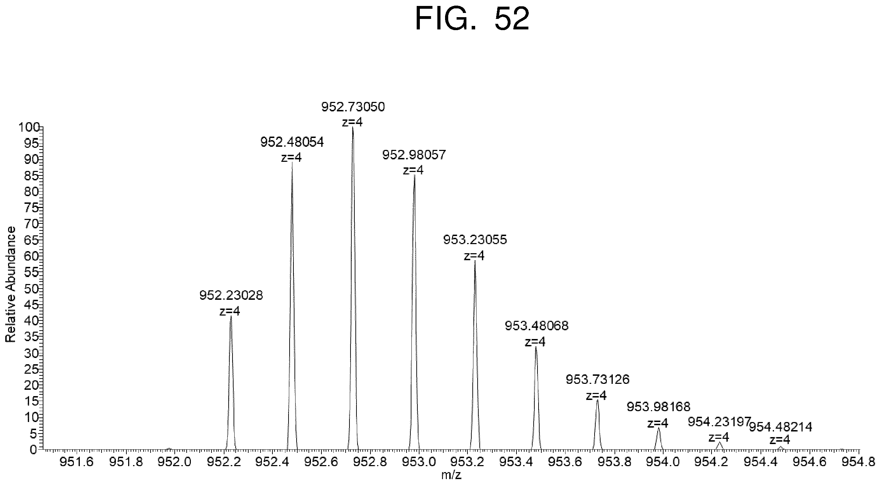

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of azidobenzoic acid-modified trastuzumab (aminebenzoic acid-introduced compound (+119.037Da)) obtained in (11-2-9) (measured value: m/z 945.74114; theoretical value: 945.73376; and tetravalent) was observed (FIG. 49); and from a CID spectrum, a product ion of m/z 1153.94 (theoretical value: 1153.62) corresponding to divalent y20 indicating modification of a lysine residue at position 246 or 248 in EU numbering of the heavy chain was determined (FIG. 50). An MS spectrum of the peptide fragment of FNWYVDGVEVHNAKTKPR (SEQ ID NO: 12), which is a peptide consisting of 18 amino acid residues comprising a modified site to a lysin residue (aminebenzoic acid-introduced compound (+119.037Da)) (measured value: m/z 570.54470; theoretical value: 570.53991; and tetravalent) was observed (FIG. 51), and from a CID spectrum, a product ion of m/z 557.18 (theoretical value: 556.97) corresponding to trivalent y14 indicating modification of a lysine residue at position 288 or 290 in EU numbering of the heavy chain was determined (FIG. 52).

From this result, it was found that in (11-2-9) above, conjugation progressed regioselectively at Lys246 or Lys248 and Lys288 or Lys290 in EU numbering on the heavy chain of the antibody.

### (11-5-10) Analysis Result of Modified Site of Trypsin Digest by LC-MS/MS (10)

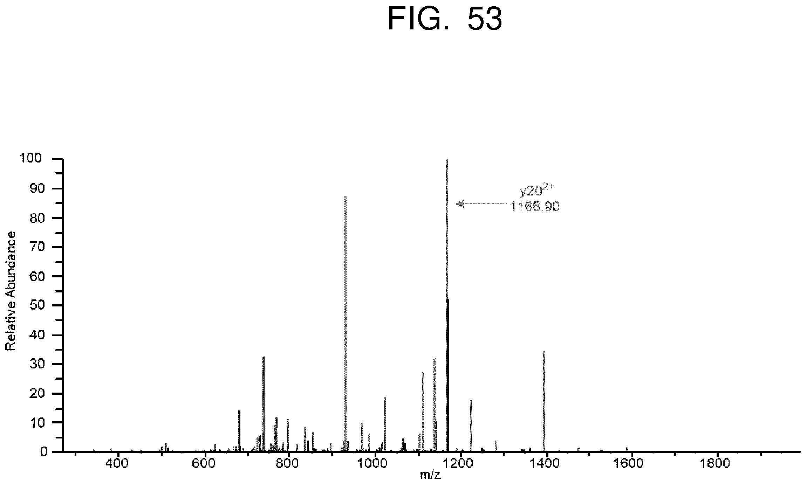

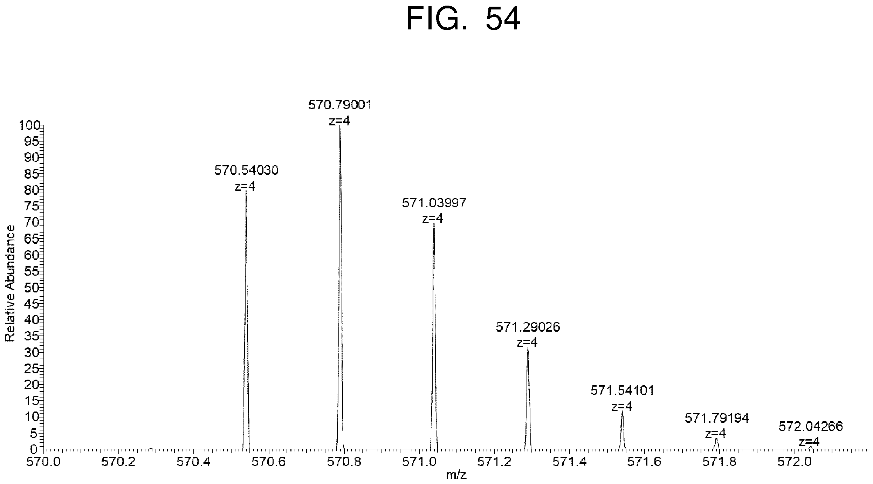

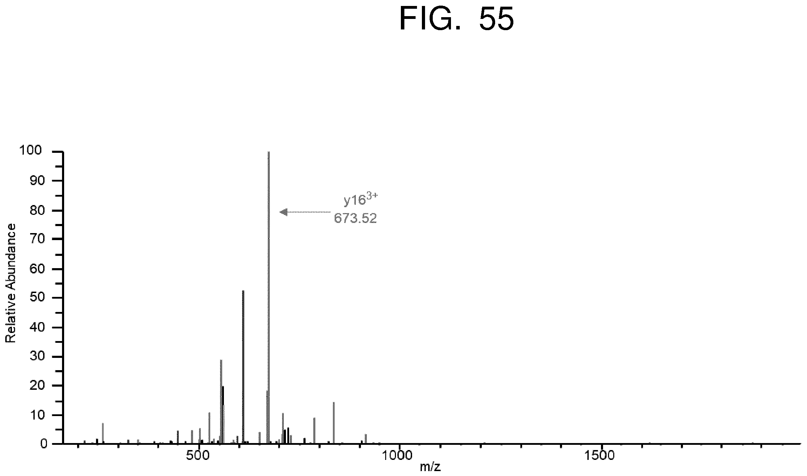

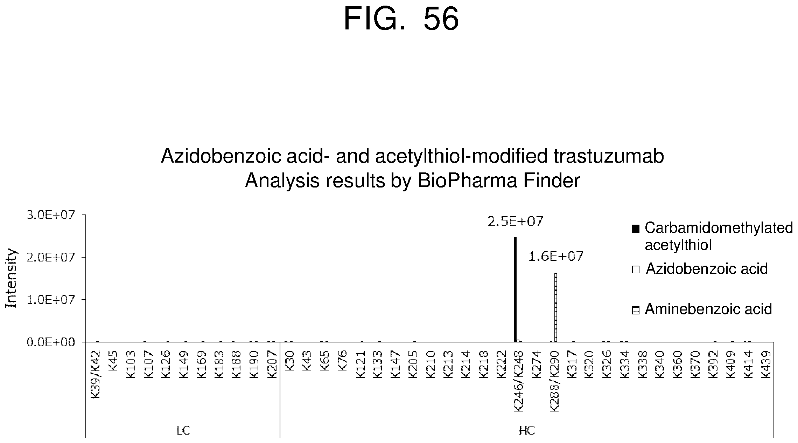

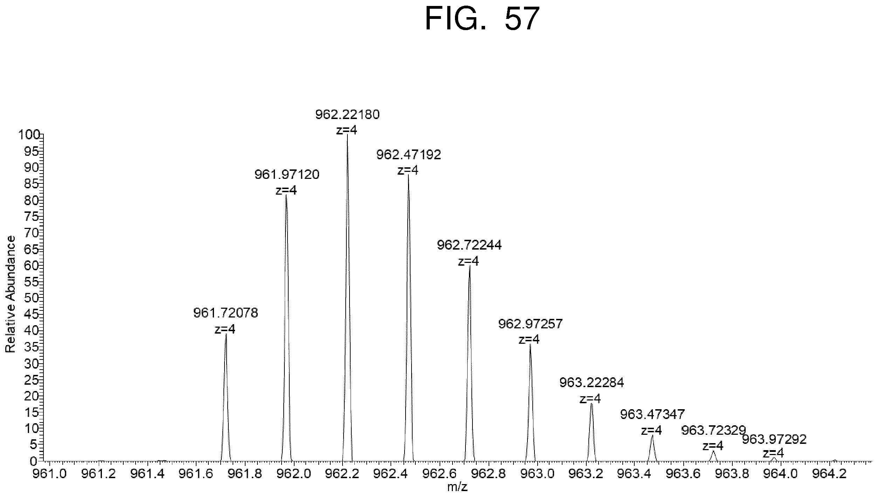

After analysis using LC-MS/MS, an MS spectrum of the peptide fragment of THTCPPCPAPELLGGPSVFLFPPKPKDTLMISR (SEQ ID NO: 11), which is a peptide consisting of 33 amino acid residues comprising a modified site to a lysine residue by trypsin digestion of acetylthiol and azidobenzoic acid-modified trastuzumab (carbamidomethylated acetylthiol-introduced compound (+119.037Da)) obtained in (11-2-10) (measured value: m/z 952.23028; theoretical value: 952.22942; and tetravalent) was observed (FIG. 53); and from a CID spectrum, a product ion of m/z 1166.90 (theoretical value: 1166.61) corresponding to divalent y20 indicating modification of a lysine residue at position 246 or 248 in EU numbering of the heavy chain was determined (FIG. 54). An MS spectrum of the peptide fragment of FNWYVDGVEVHNAKTKPR (SEQ ID NO: 12), which is a peptide consisting of 18 amino acid residues comprising a modified site to a lysin residue (an aminebenzoic acid-introduced compound (+119.037Da)) (measured value: m/z 570.54030; theoretical value: 570.53991; and tetravalent) was observed (FIG. 55), and from a CID spectrum, a product ion of m/z 673.52 (theoretical value: 673.35) corresponding to trivalent y16 indicating modification of a lysine residue at position 288 or 290 in EU numbering of the heavy chain was determined (FIG. 56). Furthermore, analysis with BioPharma Finder indicated that carbamidomethylated acetylthiol modification to a lysine residue at position 246 or 248, and aminebenzoic acid modification to a lysine residue at position 288 or 290 occurred highly selectively (FIG. 57).

From this result, it was found that in (11-2-10) above, conjugation progressed regioselectively at Lys246 or Lys248 and Lys288 or Lys290 in EU numbering on the heavy chain of the antibody.

### (11-5-11) Analysis Result of Modified Site of Trypsin Digest by LC-MS/MS (11)

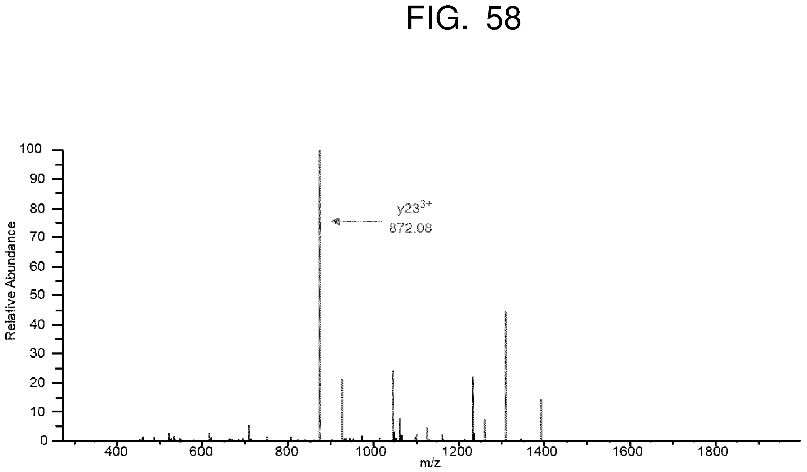

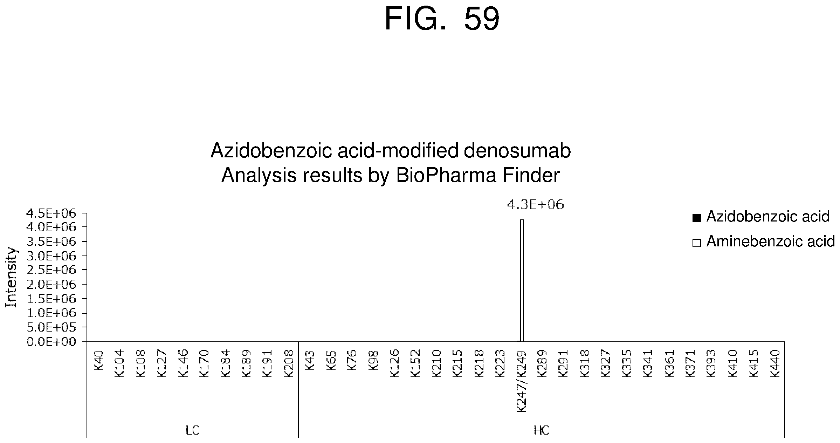

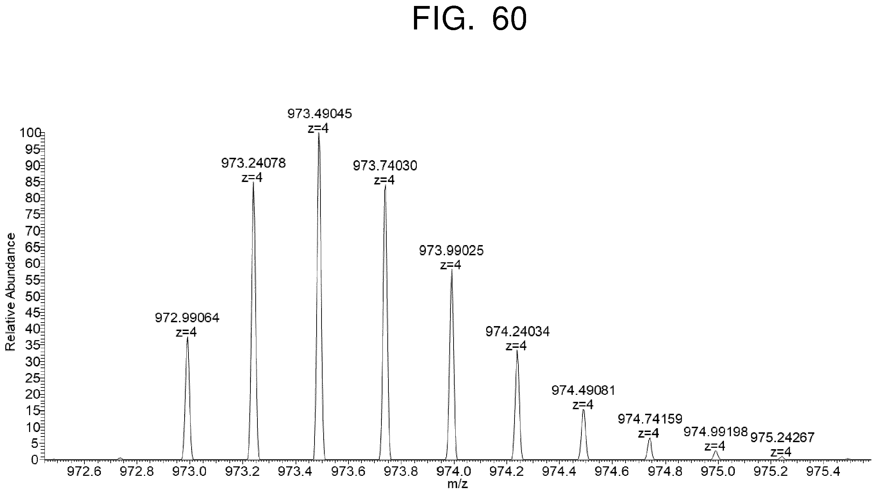

After analysis using LC-MS/MS, an MS spectrum of the peptide fragment of CCVECPPCPAPPVAGPSVFLFPPKPKDTLMISR (SEQ ID NO: 102), which is a peptide consisting of 33 amino acid residues comprising a modified site to a lysine residue by trypsin digestion of azidobenzoic acid-modified denosumab (aminebenzoic acid-introduced compound (+119.037Da)) obtained in (11-2-11) (measured value: m/z 961.72078; theoretical value: 961.71980; and tetravalent) was observed (FIG. 58); and from a CID spectrum, a product ion of m/z 872.08 (theoretical value: 871.81) corresponding to trivalent y23 indicating modification of a lysine residue at position 247 or 249 in the number in the sequence of the heavy chain (that is, the N-terminal amino acid is the first, hereinafter the same) was determined (FIG. 59). In addition, analysis with BioPharma Finder indicated that modification to a lysine residue at position 247 or 249 occurred highly selectively (FIG. 60).

From this result, it was found that in (11-2-11) above, conjugation progressed regioselectively at Lys247 or Lys249 in the number in the sequence on the heavy chain of the antibody.

### (11-5-12) Analysis Result of Modified Site of Trypsin Digest by LC-MS/MS (12)

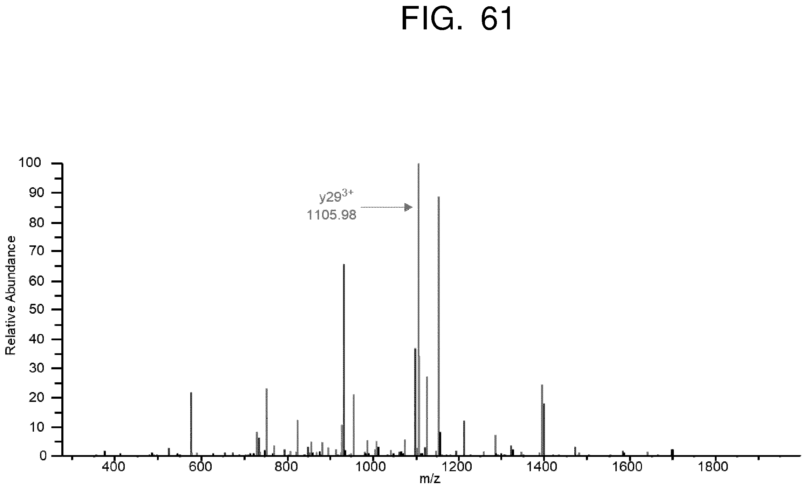

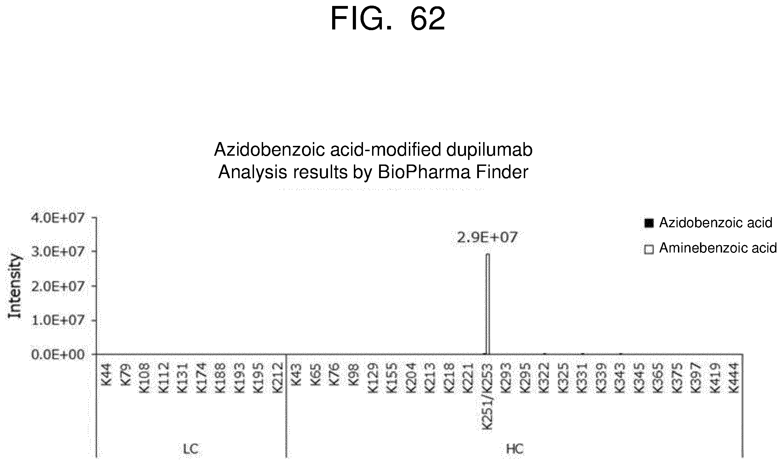

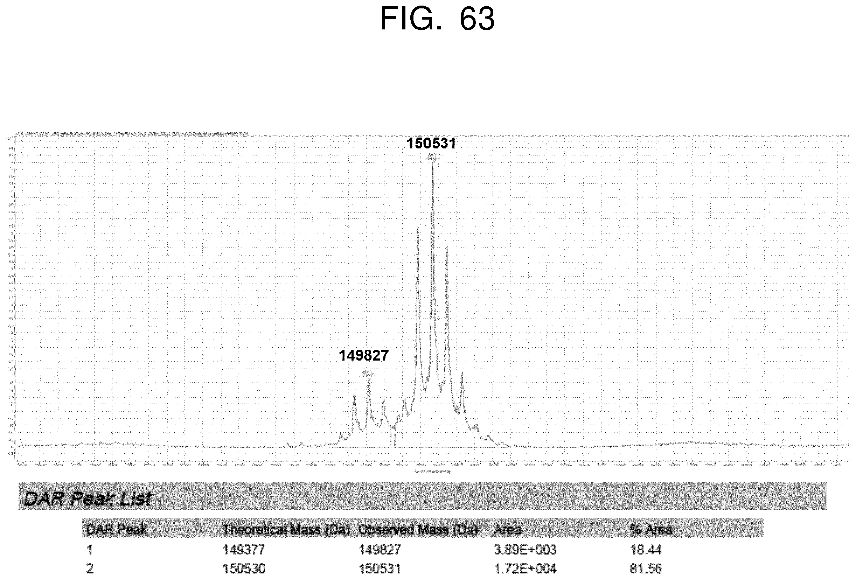

After analysis using LC-MS/MS, an MS spectrum of the peptide fragment of YGPPCPPCPAPEFLGGPSVFLFPPKPKDTLMISR (SEQ ID NO: 103), which is a peptide consisting of 34 amino acid residues comprising a modified site to a lysine residue by trypsin digestion of azidobenzoic acid-modified dupilumab (aminebenzoic acid-introduced compound (+119.037Da)) obtained in (11-2-12) (measured value: m/z 972.99064; theoretical value: 972.98886; and tetravalent) was observed (FIG. 61); and from a CID spectrum, a product ion of m/z 1105.98 (theoretical value: 1105.58) corresponding to trivalent y29 indicating modification of a lysine residue at position 251 or 253 in the number in the sequence on the heavy chain was determined (FIG. 62). In addition, analysis with BioPharma Finder indicated that modification to a lysine residue at position 251 or 253 occurred highly selectively (FIG. 63).

From this result, it was found that in (11-2-12) above, conjugation progressed regioselectively at Lys251 or Lys253 in the number in the sequence on the heavy chain of the antibody.

### [Example 12: Synthesis of four types of antibody drug conjugates (ADC) of trastuzumab-DMl, trastuzumab-MMAE, rituximab-DM1, and rituximab-MMAE]

### (12-1) Conjugation of regiospecific azide-introduced trastuzumab with DBCO-DM1

### (12-1-1) Synthesis of regioselective trastuzumab-DM1 conjugate

0.9 mg of the azidobenzoic acid-introduced trastuzumab (azido antibody 3) synthesized in (6-1-2) of Example 6 was dissolved in 473 µL of 100 mM PBS, 10 mM EDTA buffer. Thereafter, 51 µL of N,N'-dimethylformamide and DBCO-DM1 (manufactured by Abzena, compound 87) were dissolved in N,N'-dimethylacetamide to be 4 mM, and 2 µL of the resulting solution was added and stirred at 25°C for 16 hours. After the reaction, through extraction with a NAP-5 column, DM1 was removed.

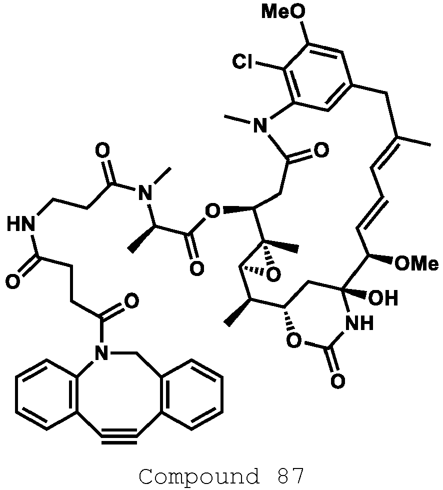

### (12-1-2) Calculation of average DAR by ESI-TOFMS

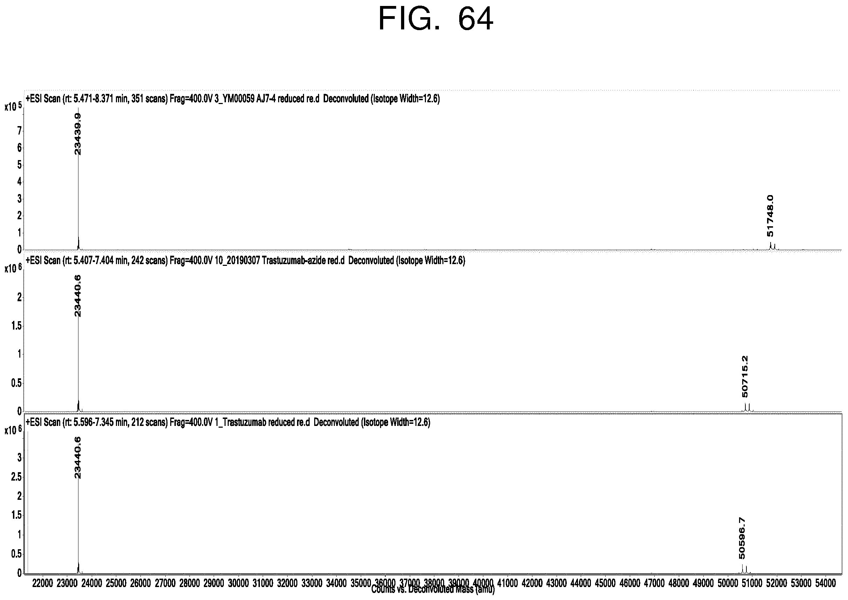

For ESI-TOFMS, Agilent 6550 coupled to Agilent 1290 UPLC with DAD detector, Autosampler cooling and Thermostatted column compartment manufactured by Agilent Technologies, Inc. was used. For software, MassHunter was used to calculate DAR. For a product, peaks were observed at 150531 with two molecules of DM1 introduced and at 149827 with one molecule of DM1 introduced, and an average DAR was calculated to be 1.8 (FIG. 64).

### (12-1-3) ESI-TOFMS analysis under reduction conditions

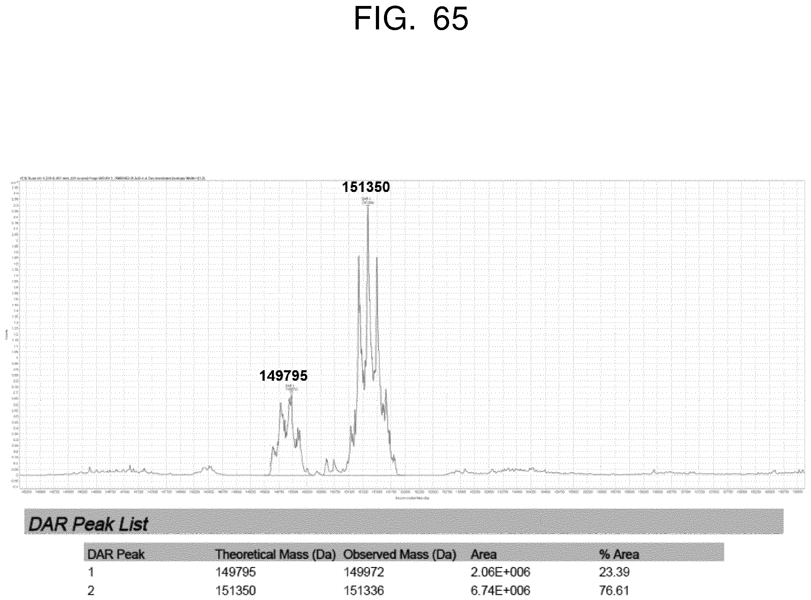

To the trastuzumab-DM1 conjugate PBS solution synthesized in (12-1-1), 2 µL of a 7 mM tris(2-carboxyethyl) phosphine hydrochloride solution (100 equivalents to the trastuzumab-DM1 conjugate) was added, and the resulting mixture was stirred at room temperature for 20 minutes. The mass was measured by ESI-TOFMS; for the raw material azide-introduced trastuzumab synthesized in (6-1-1) of Example 6, heavy chain peaks were observed at 50715 and 50877, and a light chain peak was observed at 23440, whereas for the trastuzumab-DM1 conjugate, peaks were observed at 51748 and 519097 with DM1 introduced to the heavy chain, and a light chain peak was observed at 23440, the same as that of the raw material (FIG. 65). Note that the azidobenzoic acid-introduced compound was reduced to an aminebenzoic acid-introduced compound by a reduction reaction of 7 mM tris(2-carboxyethyl) phosphine salt, which was a pretreatment for the measurement.

### (12-2) Conjugation of regiospecific azide-introduced trastuzumab with DBCO-MMAE

### (12-2-1) Synthesis of regioselective trastuzumab-MMAE conjugate

0.5 mg of the azidobenzoic acid-introduced trastuzumab (azido antibody 3) synthesized in (6-1-2) of Example 6 was reacted with DBCO-MMAE (manufactured by Abzena, compound 88) by a similar technique to (12-1-1) of Example 12. After the reaction, through extraction with a NAP-5 column, DBCO-MMAE was removed.

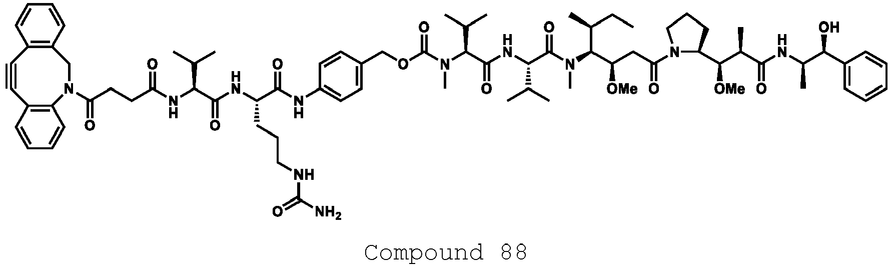

### (12-2-2) Calculation of average DAR by ESI-TOFMS

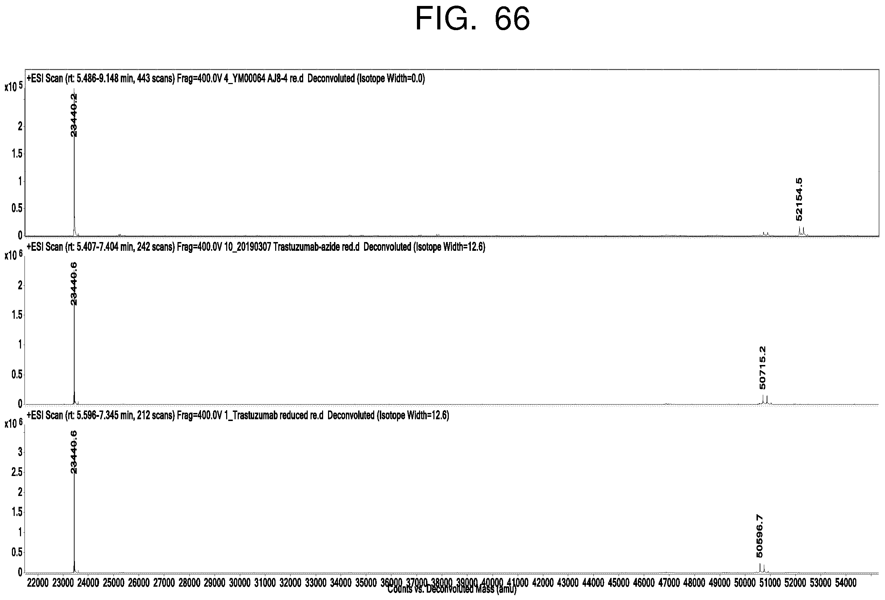

DAR was calculated by a similar technique to (12-1-2) of Example 12. For a product, peaks were observed at 151336 with two molecules of MMAE introduced and at 149972 with one molecule of MMAE introduced, and an average DAR was calculated to be 1.8 (FIG. 66).

### (12-2-3) ESI-TOFMS analysis under reduction conditions

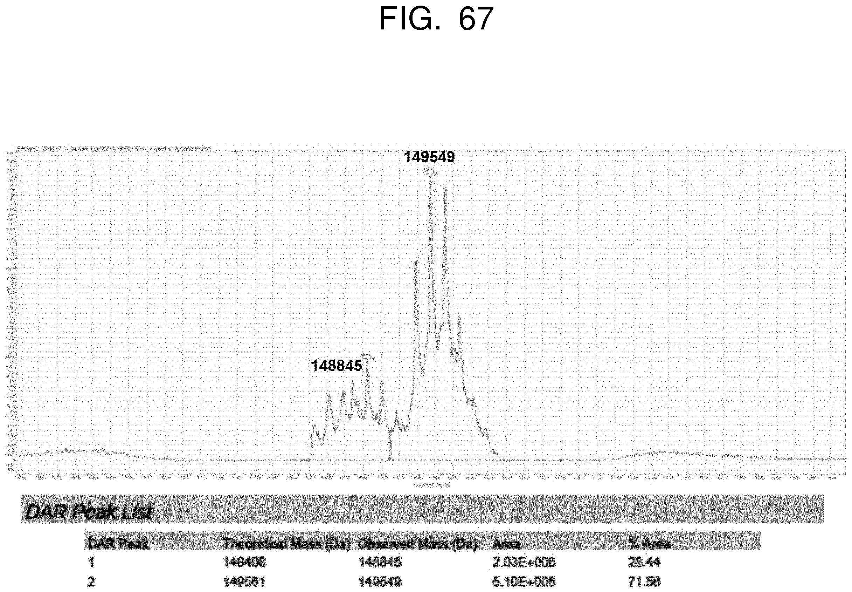

To the trastuzumab-MMAE conjugate PBS solution synthesized in (12-2-1), 2 µL of a 7 mM tris(2-carboxyethyl) phosphine hydrochloride solution (100 equivalents to the trastuzumab-DM1 conjugate) was added, and the resulting mixture was stirred at room temperature for 20 minutes. The mass was measured by ESI-TOFMS; for the raw material azide-introduced trastuzumab synthesized in (6-1-1) of Example 6, heavy chain peaks were observed at 50715 and 50877, and a light chain peak was observed at 23440, whereas for the trastuzumab-MMAE conjugate, peaks were observed at 52154 and 52316 with MMAE introduced to the heavy chain, and a light chain peak was observed at 23440, the same as that of the raw material (FIG. 67). Note that the azidobenzoic acid-introduced compound was reduced to the aminebenzoic acid-introduced compound by the reduction reaction of 7 mM tris(2-carboxyethyl) phosphine salt, which was a pretreatment for the measurement.

### (12-3) Conjugation of regiospecific azide-introduced rituximab with DBCO-DM1

### (12-3-1) Synthesis of regioselective rituximab-DM1 conjugate

The azidobenzoic acid-introduced rituximab (azide-modified antibody 101) synthesized in (6-1-4) of Example 6 was reacted with DBCO-DM1 (manufactured by Abzena, compound 87) by a similar technique to (12-1-1) of Example 12. After the reaction, through extraction with a NAP-5 column, DBCO-DM1 was removed.

### (12-3-2) Calculation of average DAR by ESI-TOFMS

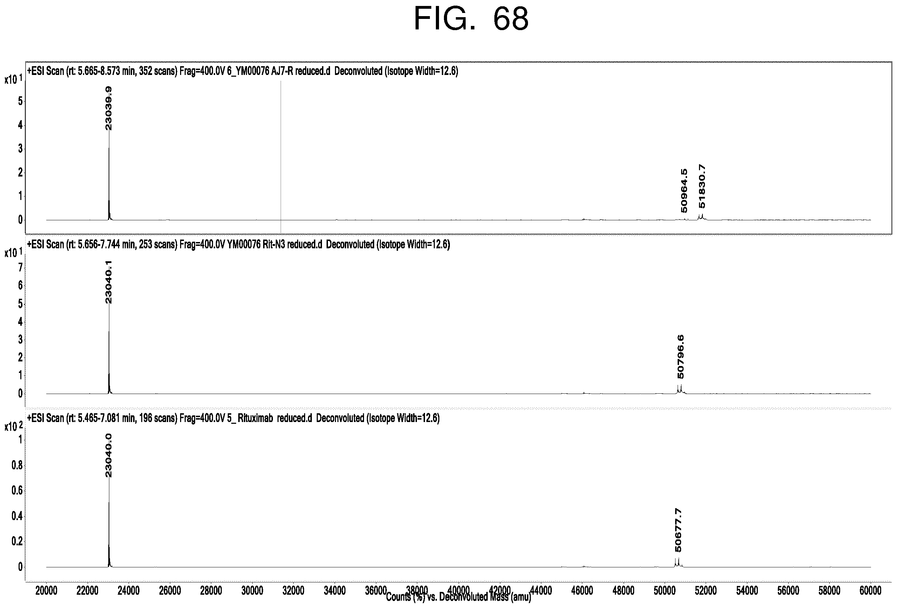

DAR was calculated by a similar technique to (12-1-2) of Example 12. For a product, peaks were observed at 149549 with two molecules of DM1 introduced and at 148845 with one molecule of DM1 introduced, and an average DAR was calculated to be 1.8 (FIG. 68).

### (12-3-3) ESI-TOFMS analysis under reduction conditions

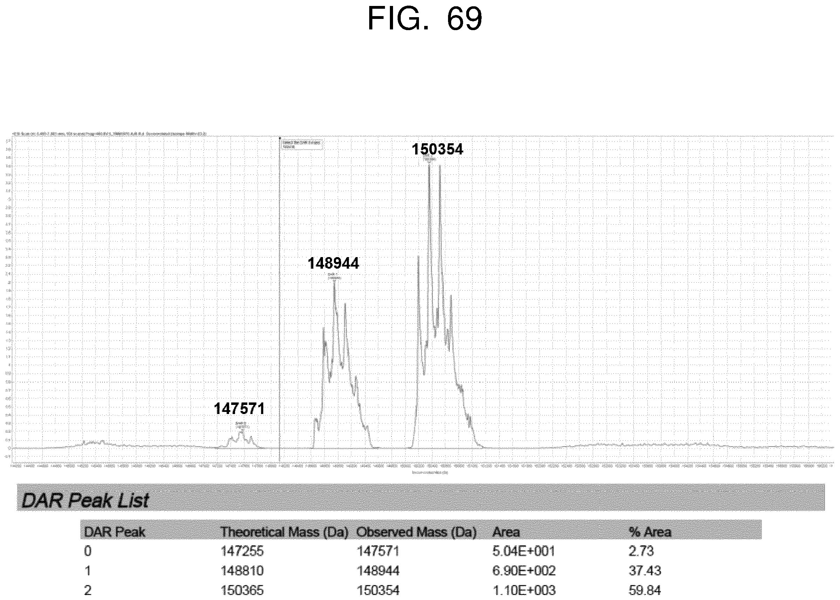

To the rituximab-DM1 conjugate PBS solution synthesized in (12-3-1), 2 µL of a 7 mM tris(2-carboxyethyl) phosphine hydrochloride solution (100 equivalents to the trastuzumab-DM1 conjugate) was added, and the resulting mixture was stirred at room temperature for 20 minutes. The mass was measured by ESI-TOFMS; for the raw material azide-introduced rituximab synthesized in (6-1-4) of Example 6, heavy chain peaks were observed at 50635 and 50797, and a light chain peak was observed at 23040, whereas for the rituximab-DM1 conjugate, peaks were observed at 51668 and 51831 with DM1 introduced to the heavy chain, and a light chain peak was observed at 23040, the same as that of the raw material (FIG. 69). Note that the azidobenzoic acid-introduced compound was reduced to the aminebenzoic acid-introduced compound by the reduction reaction of 7 mM tris(2-carboxyethyl) phosphine salt, which was a pretreatment for the measurement.

### (12-4) Conjugation of regiospecific azide-introduced rituximab with DBCO-MMAE

### (12-4-1) Synthesis of regioselective rituximab-MMAE conjugate

The azidobenzoic acid-introduced rituximab (azide-modified antibody 101) synthesized in (6-1-4) of Example 6 was reacted with DBCO-MMAE (manufactured by Abzena, compound 88) by a similar technique to (12-1-1) of Example 12. After the reaction, through extraction with a NAP-5 column, DBCO-MMAE was removed.

### (12-4-2) Calculation of average DAR by ESI-TOFMS

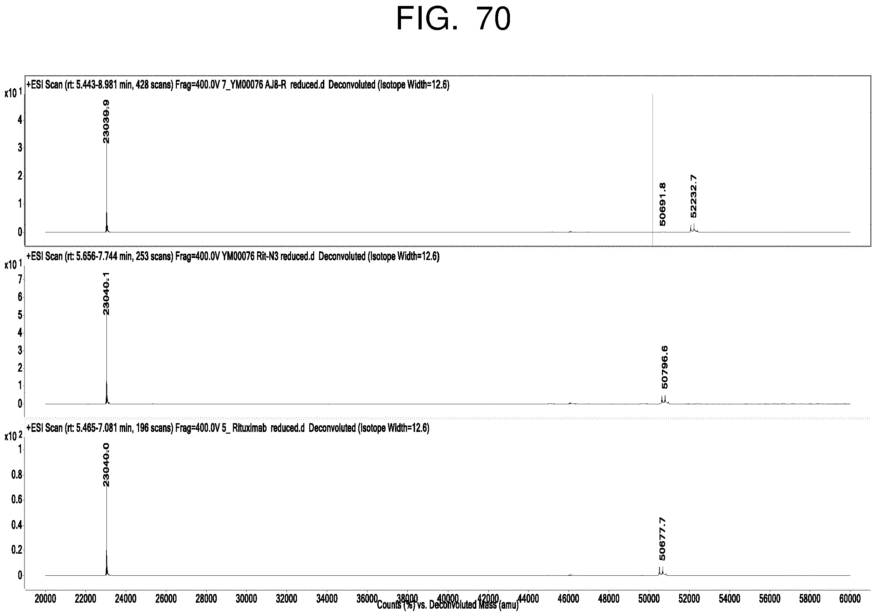

DAR was calculated by a similar technique to (12-2) of Example 12. For a product, peaks were observed at 150354 with two molecules of MMAE introduced and at 148994 with one molecule of MMAE introduced, and an average DAR was calculated to be 1.6 (FIG. 70).

### (12-4-3) ESI-TOFMS analysis under reduction conditions

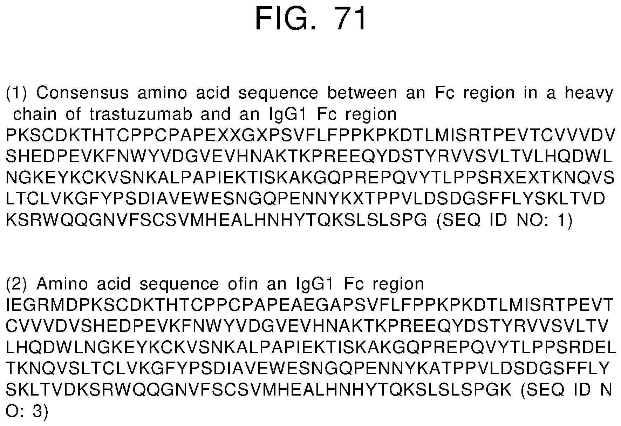

To the rituximab-MMAE conjugate PBS solution synthesized in (12-4-1), 2 µL of a 7 mM tris(2-carboxyethyl) phosphine hydrochloride solution (100 equivalents to the trastuzumab-MMAE conjugate) was added, and the resulting mixture was stirred at room temperature for 20 minutes. The mass was measured by ESI-TOFMS; for the raw material azide-introduced rituximab synthesized in (6-1-4) of Example 6, heavy chain peaks were observed at 50635 and 50797, and a light chain peak was observed at 23040, whereas for the rituximab-MMAE conjugate, peaks were observed at 52071 and 52233 with MMAE introduced to the heavy chain, and a light chain peak was observed at 23040, the same as that of the raw material (FIG. 71). Note that the azidobenzoic acid-introduced compound was reduced to an aminebenzoic acid-introduced compound by a reduction reaction of 7 mM tris(2-carboxyethyl) phosphine salt, which was a pretreatment for the measurement.

### [Example 13: Introduction of regiospecific payload model to IgG1 Fc with IgG1 Fc affinity peptide reagent loaded with payload model and analysis by ESI-TOFMS]

### (13-1) Synthesis of IgG1 Fc affinity peptide reagent loaded with payload model

### (13-1-1) Synthesis of IgG1 Fc Affinity Peptide Reagent Loaded with Payload Model (compound 89)

The antibody affinity peptide (compound 36) synthesized in Example 1 was dissolved in dimethylsulfoxide, 2-iminothiolane hydrochloride (10 molar equivalents) and diisopropylethylamine (15 molar equivalents) were added thereto, and the resulting mixture was stirred for one hour. Disappearance of the raw materials was determined by LC-MS. Thereafter, a dimethyl sulfoxide solution of N-hydroxymaleimide (20 molar equivalent) was added thereto, and the resulting mixture was stirred for one hour. The progress of the reaction was determined by LC-MS. Thereafter, Fmoc-N-amide-dPEG12 acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an Fmoc-N-amido-dPEG12-affinity peptide reagent (compound 89) as a product. 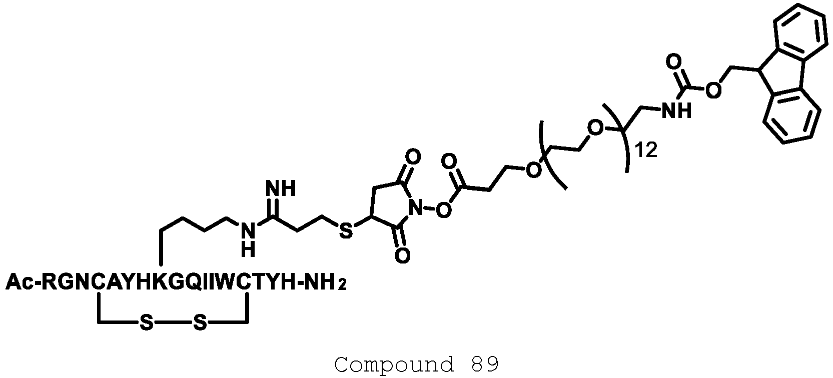 (Amino acid sequence of peptide portion: SEQ ID NO: 46)
MS (ESI)m/z:z=2 1563 [M+2H]²⁺, 3 1043 [M+3H]³⁺

### (13-1-2) Synthesis of IgG1 Fc Affinity Peptide Reagent Loaded with Payload Model (compound 90)

A biotin-affinity peptide reagent (compound 90) was obtained in a similar manner to Example (13-1-1). 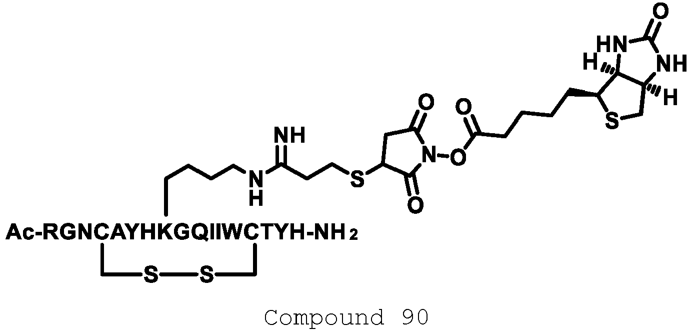 (Amino acid sequence of peptide portion: SEQ ID NO: 46)
MS (ESI)m/z:z=2 1563 [M+2H]²⁺, z=3 1043 [M+3H]³⁺

### (13-2) Introduction of regiospecific payload model to IgG1 Fc with IgG1 Fc affinity peptide reagent loaded with payload model and ESI-TOFMS analysis

### (13-2-1) Introduction of regiospecific payload model to IgG1 Fc with IgG1 Fc affinity peptide reagent loaded with payload model dPEG12 and ESI-TOFMS analysis

The affinity peptide reagent loaded with a payload model (compound 89) synthesized in (13-1-1) was dissolved in N,N'-dimethylformamide to be 2.0 mM. 200 µg of anti-HER2 IgG antibody trastuzumab (Chugai Pharmaceutical Co., Ltd.) was dissolved in 200 µL of 50 mM HEPES buffer (pH 7.2), 20 molar equivalents of 2.0 mM peptide reagent was added to the antibody, and the resulting mixture was stirred at 25°C for three hours. The reaction solution was concentrated by ultrafiltration (Amicon Ultra, 10k MWCO) to obtain a payload model dPEG12-introduced IgG antibody.

The mass was measured by ESI-TOFMS; for the trastuzumab, a peak was observed at 148061, whereas for a reaction product, a peak was observed at 149867 with two molecules of Fmoc-dPEG12 introduced.

### (13-2-2) Introduction of regiospecific payload model to IgG1 Fc with IgG1 Fc affinity peptide reagent loaded with payload model dPEG12 and ESI-TOFMS analysis

Using the affinity peptide reagent loaded with a payload model (compound 90) synthesized in (13-1-2), similar examination to (13-2-1) was performed to obtain a payload model biotin-introduced IgG antibody.

### (13-3) Analysis of introduction of regiospecific payload model to IgG1 Fc with IgG1 Fc affinity peptide reagent loaded with payload model by ESI-TOFMS under reduction conditions

### (13-3-1) Analysis of introduction of regiospecific payload model to IgG1 Fc with IgG1 Fc affinity peptide reagent loaded with payload model dPEG12 by ESI-TOFMS under reduction conditions

To the trastuzumab-payload model synthesized in (13-2-1), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50602 and 50764, and a light chain peak was observed at 23443. For a reaction product, peaks were observed at 51425 and 51584 with Fmoc-dPEG12 introduced to the heavy chain, and a light chain peak was observed at 23443, the same as that of the raw material.

### (13-3-2) Analysis of introduction of regiospecific payload model to IgG1 Fc with IgG1 Fc affinity peptide reagent loaded with payload model biotin by ESI-TOFMS under reduction conditions

To the trastuzumab-payload model synthesized in (13-2-2), 2.0 µL of a 7 mM tris(2-carboxyethyl) phosphine hydrochloride solution (100 equivalents to the antibody) was added, and the resulting mixture was allowed to stand at room temperature for 20 minutes. The mass was measured by ESI-TOFMS; for the raw material trastuzumab used as a control, heavy chain peaks were observed at 50596 and 50757, and a light chain peak was observed at 23439. For a reaction product, peaks were observed at 50820 and 50982 with biotin introduced to the heavy chain, and a light chain peak was observed at 23439, the same as that of the raw material.

### [Example 14: Introduction of regiospecific payload to IgG1 Fc with IgG1 Fc affinity peptide reagent loaded with payload and analysis by ESI-TOFMS]

### (14-1) Synthesis of IgG1 Fc affinity peptide reagent loaded with MMAE (compound 91)

The Fc affinity peptide reagent (compound 66) synthesized in Example 5 was dissolved in N,N'-dimethylformamide to be 0.22 mM. DBCO-MMAE (manufactured by Abzena, compound 91) was added thereto, and the resulting mixture was stirred at 25°C for 16 hours. Disappearance of the raw materials was determined by LC-MS, and an Fc affinity peptide reagent loaded with MMAE as a product was obtained. 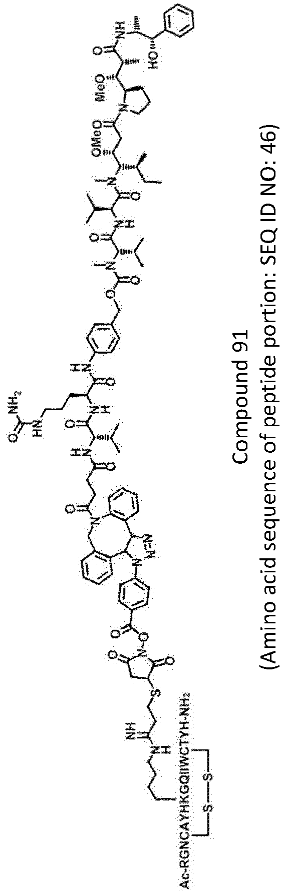 MS (ESI)m/z:z=2 1930[M+2H]²⁺, z=3 1287[M+3H]3+

### (14-2) Synthesis of IgG1 Fc regioselective trastuzumab-MMAE conjugate with IgG1 Fc affinity peptide reagent loaded with MMAE and ESI-TOFMS analysis

200 µg of anti-HER2 IgG antibody trastuzumab (Chugai Pharmaceutical Co., Ltd.) was reacted with the Fc affinity peptide reagent loaded with MMAE (compound 91) synthesized in (14-1) by a similar technique to (13-2) of Example 13. The reaction solution was concentrated by ultrafiltration (Amicon Ultra, 10k MWCO) to obtain a regioselective trastuzumab-MMAE conjugate.

As a result, the mass was measured by ESI-TOFMS, and peaks were observed at 151336 with two molecules of MMAE introduced and at 149972 with one molecule of MMAE introduced.

### [Example 15: Regiospecific modification of IgG1 Fc with IgG1 Fc affinity peptide reagent having linker derived from glutamic acid residue and analysis by ESI-TOFMS]

### (15-1) Synthesis of affinity peptide to antibody

Compound 92 was synthesized and obtained by the method described in (1-1). 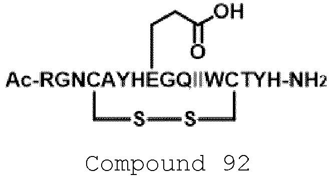 (Amino acid sequence of peptide portion: SEQ ID NO: 108)
MS (ESI)m/z:z=2 1046.4 [M+2H]²⁺, Z=3 698.3 [M+3H]³⁺

### (15-2) Synthesis of antibody-modifying reagent by coupling of azide reagent with affinity peptide

The antibody affinity peptide synthesized in (15-1) was dissolved in dimethyl sulfoxide, 1-ethyl-3-(3-dimethylaminopropyl) carbodiimide hydrochloride (40 molar equivalents) and 1-hydroxybenzotriazole (40 molar equivalents) were added thereto, and the resulting mixture was stirred. A 2-aminoethanol solution (40 molar equivalent) and triethylamine (1 molar equivalent) were added thereto, and the resulting mixture was stirred at room temperature for one hour. LC-MS measurement was performed, and separation and purification were performed by reverse phase HPLC. Each fraction was determined by MS, and a fraction containing a target product was freeze-dried. The obtained compound was dissolved in dimethylsulfoxide, triethylamine (1 molar equivalent) and N-hydroxymaleimide (1.2 molar equivalents) were added thereto, and the resulting mixture was stirred at room temperature for one hour. LC-MS measurement was performed to determine progress of the reaction. 4-Azidobenzoic acid (40 molar equivalents), 1-ethyl-3-(3-dimethylaminopropyl) carbodiimide hydrochloride (32 molar equivalents), and 1-hydroxybenzotriazole (32 molar equivalents) were added thereto, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-phenyl-affinity peptide reagent (compound 93) as a product. 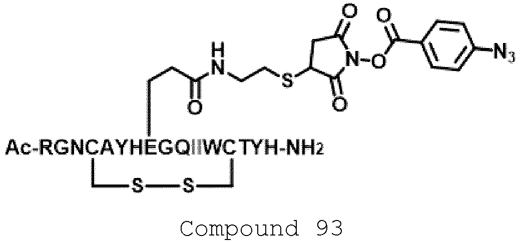 (Amino acid sequence of peptide portion: SEQ ID NO: 108)
MS (ESI)m/z:z=2 1204.3 [M+2H]²⁺, Z=3 803.3 [M+3H]³⁺

### (15-3) Antibody modification reaction using affinity peptide reagent (compound 93)

Using the affinity peptide loaded with azide (compound 93) synthesized in (15-2), a reaction was caused by the method described in (6-1-3) to obtain an IgG antibody trastuzumab-azide-modified antibody.

### (15-4) Determination of heavy chain selectivity of IgG antibody trastuzumab-azide-modified antibody under reduction conditions by ESI-TOFMS analysis

The azide-modified trastuzumab obtained in (15-3) was subjected to reduction and ESI-TOFMS analysis by the method described in (6-2-3) of Example 6. For the raw material trastuzumab used as a control, heavy chain peaks were observed at 50602 and 50764, and a light chain peak was observed at 23443. For a reaction product, peaks were observed at 50725 and 50885 with aminebenzoic acid introduced to the heavy chain, and a light chain peak was observed at 23446.

### [Example 16: Regiospecific modification of IgG1 Fc with IgG1 Fc affinity peptide reagent having thioglycolate as leaving group and analysis by ESI-TOFMS]

### (16-1) Synthesis of linker having thioglycolate as leaving group (compound 95)

Dithiodiglycolic acid was dissolved in dichloromethane, glycine t-butyl ester (2.2 molar equivalents), N,N-diisopropylamine (4.8 molar equivalents), and 1-ethyl-3-(3-dimethylaminopropyl) carbodiimide hydrochloride (2.4 molar equivalents) were added thereto, and the resulting mixture was stirred for one hour. The reaction solution was separated and washed with water, and then the obtained organic layer was concentrated under reduced pressure. The obtained residue was dissolved in N,N-dimethylformamide/water (1 : 1), tris(2-carboxyethyl) phosphine hydrochloride (1.3 molar equivalents) was added thereto, and the resulting mixture was stirred at room temperature for one hour. The solution was extracted with ethyl acetate, and then concentrated under reduced pressure and subjected to silica gel column treatment with ethyl acetate/hexane to obtain compound 94. 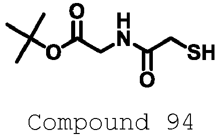 MS (ESI) [2M+H]⁺ =411, [M-H]⁻ =204

Compound 94 was dissolved in dichloromethane, 4-azidobenzoic acid (1 molar equivalent), 1H-benzotriazol-1-yloxytripyrrolidinophosphonium hexafluorophosphate (1.2 molar equivalents), and N,N-diisopropylethylamine (1.5 molar equivalents) were added thereto, and the resulting mixture was stirred at room temperature for 30 minutes. The reaction solution was purified by a silica gel column. Thereafter, the obtained white crystals were dissolved in dichloromethane/trifluoroacetic acid (1 : 1), and the resulting solution was stirred at room temperature for one hour. The reaction solution was freeze-dried to obtain a linker having a thioglycolate as a leaving group (compound 95) . 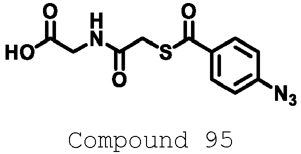 MS (ESI) m/z:z=1 295 [M+H]¹⁺

### (16-2) Synthesis of antibody-modifying reagent by coupling of azide reagent with affinity peptide

To the antibody affinity peptide (compound 36) synthesized in (1-1), the linker (compound 95) synthesized in (16-1) (40 molar equivalents), 1-ethyl-3-(3-dimethylaminopropyl) carbodiimide hydrochloride (32 molar equivalents), and 1-hydroxybenzotriazole (32 molar equivalents) were added, and the resulting mixture was stirred for two hours. The completion of the reaction was determined by LC-MS, and then the product was purified by preparative HPLC. A fraction containing a target product was determined by ESI-MS, followed by freeze-drying, thus obtaining an azide-phenyl-affinity peptide reagent (compound 96) as a product. 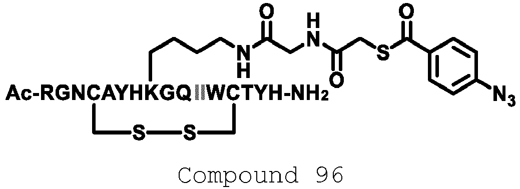 (Amino acid sequence of peptide portion: SEQ ID NO: 46)
MS (ESI)m/z:z=2 1183.3 [M+2H]²⁺, Z=3 789.2 [M+3H]³⁺

### (16-3) Antibody modification reaction using affinity peptide reagent (compound 96)

Using the affinity peptide loaded with azide (compound 96) synthesized in (16-2), a reaction was caused by the method described in (6-1-3) to obtain an IgG antibody trastuzumab-azide-modified antibody.

### (16-4) Determination of heavy chain selectivity of IgG antibody trastuzumab-azide-modified antibody under reduction conditions by ESI-TOFMS analysis

The azide-modified trastuzumab obtained in (16-3) was subjected to reduction and ESI-TOFMS analysis by the method described in (6-2-3) of Example 6. For the raw material trastuzumab used as a control, heavy chain peaks were observed at 50602 and 50764, and a light chain peak was observed at 23443. For a reaction product, peaks were observed at 50720 and 50882 with aminebenzoic acid introduced to the heavy chain, and a light chain peak was observed at 23443, the same as that of the raw material.

### (Summary)

To summarize the above results, the outlines of the amino acid sequence of the affinity peptide used in Examples and the antibody having a bioorthogonal functional group or bioorthogonal functional groups capable of being produced using a compound produced in Examples are as follows.

**Table 1. Summary of affinity peptides**

| Amino acid sequence | SEQ ID NO |
|---|---|
| FNMQQQRRFYEALHDPNLNEEQRNARIRSIKDD | 5 |
| FNMQQQRRFYEALHDPNLNEEQRNARIKSIRDD | 6 |
| β-Ala-NMQQQRRFYEALHDPNLNEEQRNARIRSIRDD | 7 |
| FNMQQQRRFYEALHDPNLNEEQRNAKIKSIKDD | 8 |
| FNMQCQRRFYEALHDPNLNKEQRNARIRSIRDDC | 41 |
| FNMQCQRRFYEALHDPNLNEEQRNARIRSIKDDC | 42 |
| FNKQCQRRFYEALHDPNLNEEQRNARIRSIRDDC | 43 |
| β-Ala-NMQQQRRFYEALHDPNLEEQRNARIRSI | 97 |
| FNMQQQRRFYEALHDPNLNKEQRNARIRSIRDD | 98 |
| β-Ala-NMQQQRRFYEALHDPNLEEQRNARIRSIKDD | 100 |
| | |
| RGNCAYHKGQIIWCTYH | 46 |
| RGNCAYHKGQIVWCTYH | 47 |
| RGNCAYHKGQVVWCTYH | 48 |
| RGNCAYHKGQAVWCTYH | 49 |
| RGNCAYHKGQLLWCTYH | 50 |
| RGNCAYHKGQLIWCTYH | 51 |
| DCAYHKGQIVWCT | 52 |
| DCAYHKGQVVWCT | 53 |
| RGNCAYHKSQIIWCTYH | 55 |
| RGNCAYHKDQIIWCTYH | 57 |
| RGNCAYHKEQIIWCTYH | 59 |
| RGNCAYHKHQIIWCTYH | 63 |
| RGNCAYHKGQEVWCTYH | 71 |
| CAYHKGQLVWC | 72 |
| RGNCAYHKSQLVWCTYH | 77 |
| RGNCAYHKQQLVWCTYH | 81 |
| RGNCAYHKEQLVWCTYH | 82 |
| GNCAYHKGQIIWCTYH | 99 |
| RGNCAYHEGQIIWCTYH | 108 |

For the antibody, it was indicated that a specific amino acid residue including the following amino acid residues can be modified regioselectively:

### (1) Lysine residue at position 246 and/or 248

For example, refer to Example 11 (11-5-1), (11-5-3), (11-5-4), (11-5-7), (11-5-8), (11-5-9), and (11-5-10) regarding modification of a lysine residue at position 246 and/or 248 of trastuzumab, Example 11 (11-5-11) regarding modification of a lysine residue at position 247 and/or 249 of denosumab (corresponding to a lysine residue at position 246 and/or 248 of human IgG in EU numbering), and Example 11 (11-5-12) regarding modification of a lysine residue at position 251 and/or 253 of dupilumab (corresponding to a lysine residue at position 246 and/or 248 of human IgG in EU numbering);

### (2) Lysine residue at position 288 and/or 290

For example, refer to Example 11 (11-5-2), (11-5-6), (11-5-9), and (11-5-10);

### (3) Lysine residue at position 317

For example, refer to Example 11 (11-5-5).

Furthermore, the compound having an affinity substance to an antibody and a bioorthogonal functional group, represented by the following Formula (I), is described with reference to part of Examples as Tables 2 and 3 below:

A-L-E-B (I)

wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group.

**Table 2. Relationship between compound having affinity substance to antibody, and bioorthogonal functional group, and antibody having bioorthogonal functional groups which can be produced by using the same (1)**

| Example | Specific structure of compound | Antibody having bioorthogonal functional groups which can be produced by using the compound* |
|---|---|---|
| | □ **Affiritysubstance(A)** | |
| | 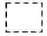 **Leaving group (L2)** | |
| | ○ **Electrophile group (E1)** | |
| | 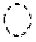 **Bloorthogonal functional group (B)** | |
| 3-4 | 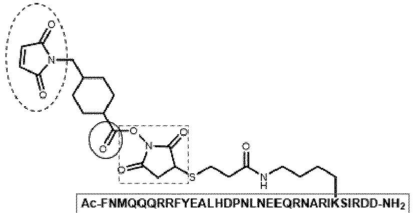 | 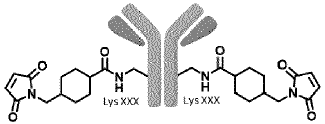 |
| | The above-amino acid sequence: SEQ ID NO:6 | |
| 3-4 | 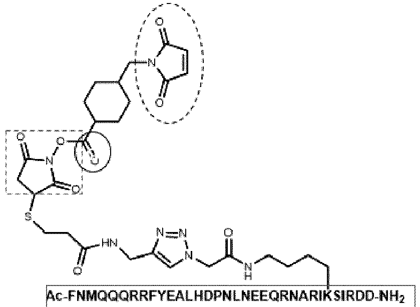 | |
| | The above-amino acid sequence: SEQ ID NO:6 | |
| 3-5-2 | 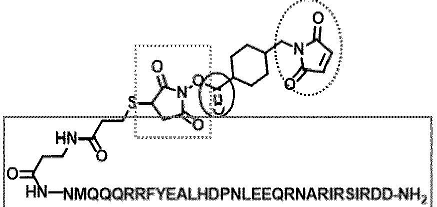 | |
| | The above-amino acid sequence: SEQ ID NO:7 | |

| | | |
|---|---|---|
| * An NH-alkyl portion extending from an antibody is derived from side chain of lysine residue in the antibody (hereafter the same applies). | | |

**Table 3. Relationship between compound having affinity substance to antibody, and bioorthogonal functional group, and antibody having bioorthogonal functional groups which can be produced by using the same (2) (excerpt)**

| Example | Specific structure of compound | Antibody having bioorthogonal functional groups which can be produced by using the compound* |
|---|---|---|
| | □ **Affiritysubstance(A)** | |
| | 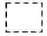 **Leaving group (L2)** | |
| | ○ **Electrophile group (E1)** | |
| | 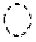 **Bloorthogonal functional group (B)** | |
| 3-5-4 | 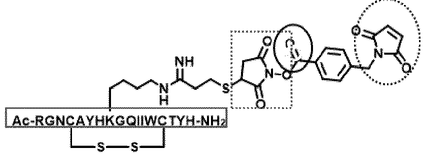 | 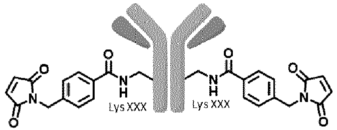 |
| | The above-amino acid sequence: SEQ ID NO:46 | |
| 5-2-1 | 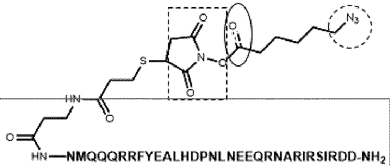 | 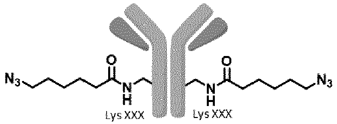 |
| | The above-amino acid sequence: SEQ ID NO:7 | |
| | 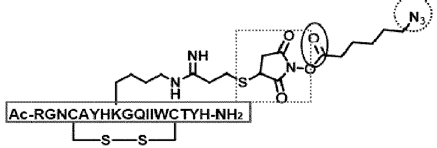 | |
| | The above-amino acid sequence: SEQ ID NO:46 | |
| 5-2-2 | For example, the following compound: | |
| | 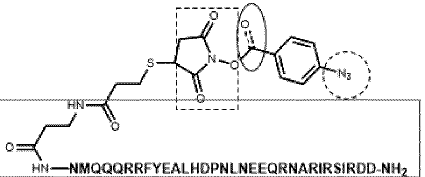 | 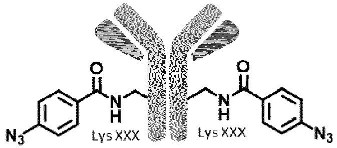 |
| | The above-amino acid sequence: SEQ ID NO:7 | |

**Table 4. Relationship between compound having affinity substance to antibody, and bioorthogonal functional group, and antibody having bioorthogonal functional groups which can be produced by using the same (3) (excerpt)**

| Example | Specific structure of compound | Antibody having bioorthogonal functional groups which can be produced by using the compound* |
|---|---|---|
| | □ **Affiritysubstance(A)** | |
| | 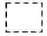 **Leaving group (L2)** | |
| | ○ **Electrophile group (E1)** | |
| | 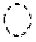 **Bloorthogonal functional group (B)** | |
| 5-2-3 | For example, the following compound: | 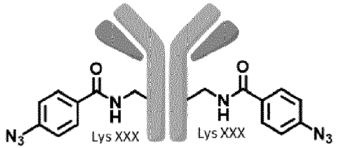 |
| | 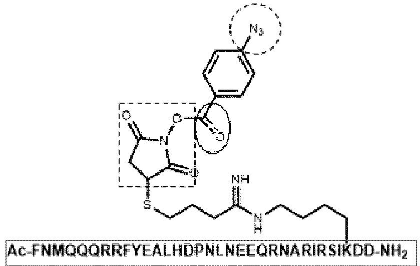 | |
| | The above-amino acid sequence: SEQ ID NO:5 | |
| | 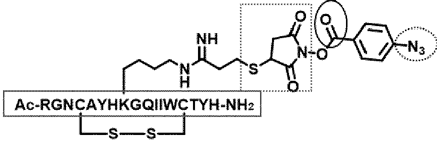 | |
| | The above-amino acid sequence: SEQ ID NO:46 | |
| 15-1 | 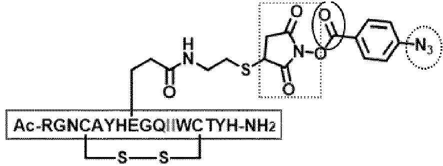 | |
| | The above-amino acid sequence: SEQ ID NO:108 | |
| 16-2 | 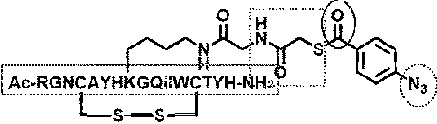 | |
| | The above-amino acid sequence: SEQ ID NO:46 | |

**Table 5. Relationship between compound having affinity substance to antibody, and bioorthogonal functional group, and antibody having bioorthogonal functional groups which can be produced by using the same (4) (excerpt)**

| Example | Specific structure of compound | Antibody having bioorthogonal functional groups which can be produced by using the compound* |
|---|---|---|
| | □ **Affiritysubstance(A)** | |
| | 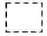 **Leaving group (L2)** | |
| | ○ **Electrophile group (E1)** | |
| | 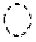 **Bloorthogonal functional group (B)** | |
| 7-1 | 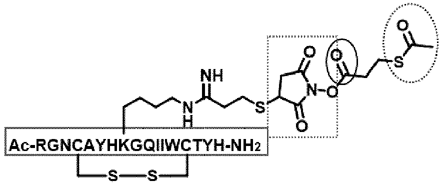 | 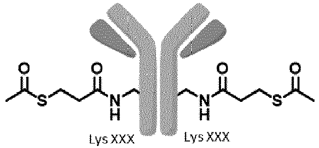 |
| | The above-amino acid sequence: SEQ ID NO:46 | |
| 9-1-1 | 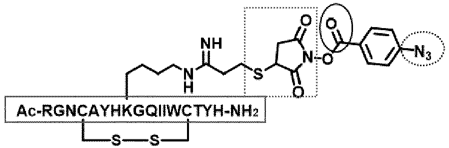 | 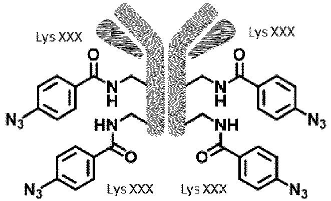 |
| | The above-amino acid sequence: SEQ ID NO:46 | |
| | 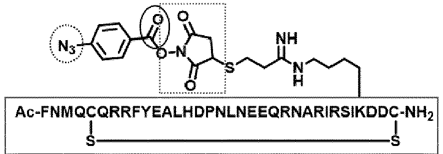 | |
| | The above-amino acid sequence: SEQ ID NO:42 | |

**Table 6. Relationship between compound having affinity substance to antibody, and bioorthogonal functional group, and antibody having bioorthogonal functional groups which can be produced by using the same (5) (excerpt)**

| Example | Specific structure of compound | Antibody having bioorthogonal functional groups which can be produced by using the compound* |
|---|---|---|
| | □ **Affiritysubstance(A)** | |
| | 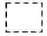 **Leaving group (L2)** | |
| | ○ **Electrophile group (E1)** | |
| | 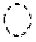 **Bloorthogonal functional group (B)** | |
| 9-1-5 | 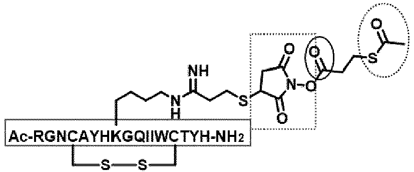 | 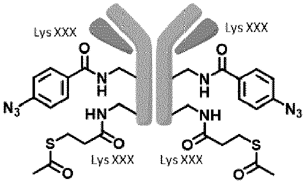 |
| | The above-amino acid sequence: SEQ ID NO:46 | |
| | 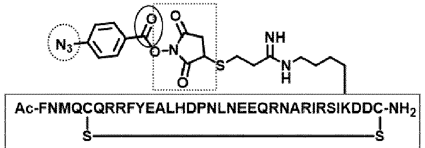 | |
| | The above-amino acid sequence: SEQ ID NO:42 | |
| 9-2-1 | 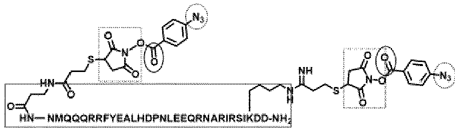 | 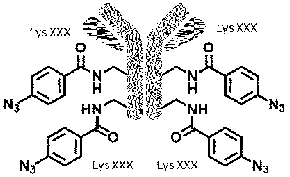 |
| | The above-amino acid sequence: SEQ ID NO:100 | |

It has been demonstrated from the foregoing that in a compound having an affinity substance to an antibody, a cleavable portion, and a reactive group, the antibody can be modified regioselectively by adjusting factors such as the type of the affinity substance, the length of a linker between the affinity substance and the reactive group, and the position in the affinity substance to which the linker is introduced as appropriate.

### Industrial Applicability

For example, the present invention is useful for production of a regioselectively modified antibody.

### [Sequence Listing]

SEQUENCE LISTING

<110>  Ajinomoto Co., Inc.

<120>  Compounds and salts thereof comprising an affinity substance to
       an antibody, and a bioorthogonal group

<130>  PAMA-31638

<150>  JP2018-113962
<151>  2018-06-14

<160>  108

<170>  PatentIn version 3.5

<210>  1
<211>  230
<212>  PRT
<213>  Artificial Sequence

<220>
<223>  Fc region of human IgG1


<220>
<221>  misc_feature
<222>  (18)..(19)
<223>  Xaa can be any naturally occurring amino acid

<220>
<221>  misc_feature
<222>  (21)..(21)
<223>  Xaa can be any naturally occurring amino acid

<220>
<221>  misc_feature
<222>  (140)..(140)
<223>  Xaa can be any naturally occurring amino acid

<220>
<221>  misc_feature
<222>  (142)..(142)
<223>  Xaa can be any naturally occurring amino acid

<220>
<221>  misc_feature
<222>  (177)..(177)
<223>  Xaa can be any naturally occurring amino acid

<400>  1

Pro Lys Ser Cys Asp Lys Thr His Thr Cys Pro Pro Cys Pro Ala Pro
1               5                   10                  15


Glu Xaa Xaa Gly Xaa Pro Ser Val Phe Leu Phe Pro Pro Lys Pro Lys
            20                  25                  30


Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asp Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Xaa Glu Xaa Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Xaa Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly
225                 230

<210>  2
<211>  449
<212>  PRT
<213>  Artificial Sequence

<220>
<223>  Heavy chain of Trastuzumab (humanized IgG1 monoclonal antibody)

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asp Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210>  3
<211>  237
<212>  PRT
<213>  Artificial Sequence

<220>
<223>  Fc region of human IgG1 antibody

<400>  3

Ile Glu Gly Arg Met Asp Pro Lys Ser Cys Asp Lys Thr His Thr Cys
1               5                   10                  15

Pro Pro Cys Pro Ala Pro Glu Ala Glu Gly Ala Pro Ser Val Phe Leu
            20                  25                  30

Phe Pro Pro Lys Pro Lys Asp Thr Leu Met Ile Ser Arg Thr Pro Glu
        35                  40                  45

Val Thr Cys Val Val Val Asp Val Ser His Glu Asp Pro Glu Val Lys
    50                  55                  60

Phe Asn Trp Tyr Val Asp Gly Val Glu Val His Asn Ala Lys Thr Lys
65                  70                  75                  80

Pro Arg Glu Glu Gln Tyr Asp Ser Thr Tyr Arg Val Val Ser Val Leu
                85                  90                  95

Thr Val Leu His Gln Asp Trp Leu Asn Gly Lys Glu Tyr Lys Cys Lys
            100                 105                 110

Val Ser Asn Lys Ala Leu Pro Ala Pro Ile Glu Lys Thr Ile Ser Lys
        115                 120                 125

Ala Lys Gly Gln Pro Arg Glu Pro Gln Val Tyr Thr Leu Pro Pro Ser
    130                 135                 140

Arg Asp Glu Leu Thr Lys Asn Gln Val Ser Leu Thr Cys Leu Val Lys
145                 150                 155                 160

Gly Phe Tyr Pro Ser Asp Ile Ala Val Glu Trp Glu Ser Asn Gly Gln
                165                 170                 175

Pro Glu Asn Asn Tyr Lys Ala Thr Pro Pro Val Leu Asp Ser Asp Gly
            180                 185                 190

Ser Phe Phe Leu Tyr Ser Lys Leu Thr Val Asp Lys Ser Arg Trp Gln
        195                 200                 205

Gln Gly Asn Val Phe Ser Cys Ser Val Met His Glu Ala Leu His Asn
    210                 215                 220

His Tyr Thr Gln Lys Ser Leu Ser Leu Ser Pro Gly Lys
225                 230                 235

<210> 4
<211> 214
<212> PRT
<213> Artificial Sequence

<220>
<223> Light chain of Trastuzumab (humanized IgG1 monoclonal antibody)

<400>  4

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  33
<212>  PRT
<213>  Artificial Sequence

<220>
<223>  Peptide having an affinity to Fc region of human IgG.  The amino
      acid residue at N-terminus may be acetylated, and the amino acid
      residue at C-terminus may be aminated.

<400>  5

Phe Asn Met Gln Gln Gln Arg Arg Phe Tyr Glu Ala Leu His Asp Pro
1               5                   10                  15

Asn Leu Asn Glu Glu Gln Arg Asn Ala Arg Ile Arg Ser Ile Lys Asp
            20                  25                  30

Asp

<210>  6
<211>  33
<212>  PRT
<213>  Artificial Sequence

<220>
<223>  Peptide having an affinity to Fc region of human IgG.  The amino
      acid residue at N-terminus may be acetylated, and the amino acid
      residue at C-terminus may be aminated.

<400>  6

Phe Asn Met Gln Gln Gln Arg Arg Phe Tyr Glu Ala Leu His Asp Pro
1               5                   10                  15

Asn Leu Asn Glu Glu Gln Arg Asn Ala Arg Ile Lys Ser Ile Arg Asp
            20                  25                  30

Asp

<210>  7
<211>  33
<212>  PRT
<213>  Artificial Sequence

<220>
<223>  Peptide having an affinity to Fc region of human IgG.  The amino
      acid residue at N-terminus may be acetylated, and the amino acid
      residue at C-terminus may be aminated.

<220>
<221>  MISC_FEATURE
<222>  (1)..(1)
<223>  beta-alanine

<400>  7

Xaa Asn Met Gln Gln Gln Arg Arg Phe Tyr Glu Ala Leu His Asp Pro

1               5                   10                  15

Asn Leu Asn Glu Glu Gln Arg Asn Ala Arg Ile Arg Ser Ile Arg Asp
            20                  25                  30

Asp

<210>  8
<211>  33
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  8

Phe Asn Met Gln Gln Gln Arg Arg Phe Tyr Glu Ala Leu His Asp Pro
1               5                   10                  15

Asn Leu Asn Glu Glu Gln Arg Asn Ala Lys Ile Lys Ser Ile Lys Asp
            20                  25                  30

Asp

<210>  9
<211>  25
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  9

Glu Gln Gln Asn Ala Phe Tyr Glu Ile Leu His Leu Pro Asn Glu Glu
1               5                   10                  15

Gln Arg Gly Gly Gly Gly Ser Cys Gly
            20                  25

<210>  10
<211>  7
<212>  PRT
<213>  Artificial Sequence

<220>
<223>  Model for peptide drug

<400>  10

His His His His His His Gly
1               5

<210>  11
<211>  33
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  11

Phe Asn Met Gln Gln Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp

<210>  12
<211>  34
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  12

Phe Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  13
<211>  33
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<220>
<221>  MISC_FEATURE
<222>  (1)..(1)
<223>  beta-alanine

<400>  13

Xaa Asn Met Gln Gln Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp

<210>  14
<211>  34
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<220>
<221>  MISC_FEATURE
<222>  (1)..(1)
<223>  beta-alanine

<400>  14

Xaa Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  15
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  15

Xaa Xaa Xaa Cys Xaa Xaa Xaa Xaa Xaa Xaa Ile Ile Trp Cys Xaa Xaa
1               5                   10                  15

Xaa

<210>  16
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,

leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  16

Xaa Xaa Xaa Cys Xaa Xaa Xaa Xaa Xaa Xaa Ile Val Trp Cys Xaa Xaa
1               5                   10                  15


Xaa




<210>  17
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
      glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
      acide, phenylalanine, tyrosine, tryptophan, histidine, threonine,
      leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  17

Xaa Xaa Xaa Cys Xaa Xaa Xaa Xaa Xaa Xaa Val Val Trp Cys Xaa Xaa
1               5                   10                  15


Xaa




<210>  18
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues

may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE

<222>  (17)..(17)
<223>  absent, or histidine

<400>  18

Xaa Xaa Xaa Cys Xaa Xaa Xaa Xaa Xaa Xaa Ala Val Trp Cys Xaa Xaa
1               5                   10                  15

Xaa

<210>  19
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE

<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  19

Xaa Xaa Xaa Cys Xaa Xaa Xaa Xaa Xaa Xaa Leu Leu Trp Cys Xaa Xaa
1               5                   10                  15


Xaa




<210>  20
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>

<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  20

Xaa Xaa Xaa Cys Xaa Xaa Xaa Xaa Xaa Xaa Leu Ile Trp Cys Xaa Xaa
1               5                   10                  15

Xaa

<210>  21
<211>  17
<212>  PRT
<213>  Artificial Sequence

<220>

<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  21

Xaa Xaa Xaa Cys Xaa Xaa Xaa Xaa Xaa Xaa Leu Val Phe Cys Xaa Xaa
1               5                   10                  15

Xaa

<210>  22
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
      glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  22

Xaa Xaa Xaa Cys Xaa Xaa Xaa Xaa Xaa Xaa Gln Val Trp Cys Xaa Xaa
1               5                   10                  15


Xaa




<210>  23
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)

<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  23

Xaa Xaa Xaa Cys Xaa Xaa Xaa Xaa Xaa Xaa Glu Val Trp Cys Xaa Xaa
1               5                   10                  15


Xaa




<210>  24
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>

<221>  MISC_FEATURE
<222>  (16)..(16)
<223>  absent, or tyrosine

<220>
<221>  MISC_FEATURE
<222>  (17)..(17)
<223>  absent, or histidine

<400>  24

Xaa Xaa Xaa Cys Xaa Xaa Xaa Xaa Xaa Xaa Leu Val Trp Cys Xaa Xaa
1               5                   10                  15

Xaa

<210>  25
<211>  12
<212>  PRT
<213>  Artificial Sequence

<220>
<223>  Peptide having an affinity to CH2 region of human IgG

<400>  25

Glu Pro Ile His Arg Ser Thr Leu Thr Ala Leu Leu
1               5                   10

<210>  26
<211>  10
<212>  PRT
<213>  Artificial Sequence

<220>
<223>  Peptide having an affinity to Fc region of human IgG

<400>  26

Phe Ala Arg Leu Val Ser Ser Ile Arg Tyr
1               5                   10

<210>  27
<211>  10
<212>  PRT
<213>  Artificial Sequence

<220>
<223>  Peptide having an affinity to Fc region of human IgG

<400>  27

Phe Gly Arg Leu Val Ser Ser Ile Arg Tyr
1               5                   10

<210>  28
<211>  10

<212>  PRT
<213>  Artificial Sequence

<220>
<223>  Peptide having an affinity to Fc region of human IgG

<400>  28

Thr Trp Lys Thr Ser Arg Ile Ser Ile Phe
1               5                   10


<210>  29
<211>  4
<212>  PRT
<213>  Artificial Sequence

<220>
<223>  Peptide having an affinity to specific region of human IgG

<400>  29

Gln Ser Tyr Pro
1


<210>  30
<211>  6
<212>  PRT
<213>  Artificial Sequence

<220>
<223>  Peptide having an affinity to Fc region of human IgG

<400>  30

His Trp Arg Gly Trp Val
1               5


<210>  31
<211>  6
<212>  PRT
<213>  Artificial Sequence

<220>
<223>  Peptide having an affinity to Fc region of human IgG

<400>  31

His Tyr Phe Lys Phe Asp
1               5


<210>  32
<211>  6
<212>  PRT
<213>  Artificial Sequence

<220>
<223>  Peptide having an affinity to Fc region of human IgG

<400>  32

His Phe Arg Arg His Leu
1               5

<210>  33
<211>  4
<212>  PRT
<213>  Artificial Sequence

<220>
<223>  Peptide having an affinity to Fc region of human IgG

<400>  33

Asp Ala Ala Gly
1

<210>  34
<211>  8
<212>  PRT
<213>  Artificial Sequence

<220>
<223>  Peptide having an affinity to Fc region of human IgG

<400>  34

Asn Ala Arg Lys Phe Tyr Lys Gly
1               5

<210>  35
<211>  8
<212>  PRT
<213>  Artificial Sequence

<220>
<223>  Peptide having an affinity to Fc region of human IgG

<400>  35

Asn Lys Phe Arg Gly Lys Tyr Lys
1               5

<210>  36
<211>  34
<212>  PRT
<213>  Artificial Sequence

<220>
<223>  Phenylalanine residue at N-terminus may be acetylated and two
       cysteine residues may be linked by disulfide bridge

<400>  36

Phe Asn Met Gln Cys Gln Arg Arg Phe Tyr Glu Ala Leu His Asp Pro
1               5                   10                  15

Asn Leu Asn Glu Glu Gln Arg Asn Ala Lys Ile Lys Ser Ile Arg Asp

                20                  25                  30

Asp Cys

<210>  37
<211>  34
<212>  PRT
<213>  Artificial Sequence

<220>
<223>  Lysine residue at N-terminus may be acetylated and two cysteine
       residues may be linked by disulfide bridge

<400>  37

Lys Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  38
<211>  34
<212>  PRT
<213>  Artificial Sequence

<220>
<223>  Phenylalanine residue at N-terminus may be acetylated and two
       cysteine residues may be linked by disulfide bridge

<400>  38

Phe Asn Met Gln Cys Gln Lys Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  39
<211>  34
<212>  PRT
<213>  Artificial Sequence

<220>
<223>  Phenylalanine residue at N-terminus may be acetylated and two
       cysteine residues may be linked by disulfide bridge

<400>  39

Phe Asn Met Gln Cys Gln Arg Arg Phe Tyr Glu Ala Lys His Asp Pro
1               5                   10                  15

Asn Leu Asn Glu Glu Gln Arg Asn Ala Arg Ile Arg Ser Ile Arg Asp
            20                  25                  30

Asp Cys

<210>  40
<211>  34
<212>  PRT
<213>  Artificial Sequence

<220>
<223>  Phenylalanine residue at N-terminus may be acetylated and two
       cysteine residues may be linked by disulfide bridge

<400>  40

Phe Asn Met Gln Cys Gln Arg Arg Phe Tyr Glu Ala Leu His Asp Pro
1               5                   10                  15

Asn Leu Asn Glu Glu Gln Arg Lys Ala Arg Ile Arg Ser Ile Arg Asp
            20                  25                  30

Asp Cys

<210>  41
<211>  34
<212>  PRT
<213>  Artificial Sequence

<220>
<223>  Phenylalanine residue at N-terminus may be acetylated and two
       cysteine residues may be linked by disulfide bridge

<400>  41

Phe Asn Met Gln Cys Gln Arg Arg Phe Tyr Glu Ala Leu His Asp Pro
1               5                   10                  15

Asn Leu Asn Lys Glu Gln Arg Asn Ala Arg Ile Arg Ser Ile Arg Asp
            20                  25                  30

Asp Cys

<210>  42
<211>  34
<212>  PRT
<213>  Artificial Sequence

<220>
<223>  Phenylalanine residue at N-terminus may be acetylated and two
       cysteine residues may be linked by disulfide bridge

<400>  42

Phe Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Lys Asp
            20                  25                  30

Asp Cys

<210>  43
<211>  34
<212>  PRT
<213>  Artificial Sequence

<220>
<223>  Phenylalanine residue at N-terminus may be acetylated and two
       cysteine residues may be linked by disulfide bridge

<400>  43

Phe Asn Lys Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  44
<211>  34
<212>  PRT
<213>  Artificial Sequence

<220>
<223>  Phenylalanine residue at N-terminus may be acetylated and two
       cysteine residues may be linked by disulfide bridge

<400>  44

Phe Asn Met Gln Cys Lys Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  45
<211>  34
<212>  PRT
<213>  Artificial Sequence

<220>
<223>  Phenylalanine residue at N-terminus may be acetylated and two
       cysteine residues may be linked by disulfide bridge

<400>  45

Phe Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Lys
            20                  25                  30

Asp Cys

<210>  46
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  46

Arg Gly Asn Cys Ala Tyr His Lys Gly Gln Ile Ile Trp Cys Thr Tyr
1               5                   10                  15

His

<210>  47
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  47

Arg Gly Asn Cys Ala Tyr His Lys Gly Gln Ile Val Trp Cys Thr Tyr
1               5                   10                  15

His

<210>  48
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  48

Arg Gly Asn Cys Ala Tyr His Lys Gly Gln Val Val Trp Cys Thr Tyr
1               5                   10                  15

His

<210>  49
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  49

Arg Gly Asn Cys Ala Tyr His Lys Gly Gln Ala Val Trp Cys Thr Tyr
1               5                   10                  15

His

<210>  50
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  50

Arg Gly Asn Cys Ala Tyr His Lys Gly Gln Leu Leu Trp Cys Thr Tyr
1               5                   10                  15

His

<210>  51
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  51

Arg Gly Asn Cys Ala Tyr His Lys Gly Gln Leu Ile Trp Cys Thr Tyr
1               5                   10                  15

His

<210>  52
<211>  13
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  52

Asp Cys Ala Tyr His Lys Gly Gln Ile Val Trp Cys Thr
1               5                   10

<210>  53
<211>  13
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  53

Asp Cys Ala Tyr His Lys Gly Gln Val Val Trp Cys Thr
1               5                   10

<210>  54
<211>  13
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  54

Asp Cys Ala Tyr His Lys Gly Gln Ala Val Trp Cys Thr
1               5                   10


<210>  55
<211>  17
<212>  PRT
<213>  Artificial Sequence

<220>
<223>  eptide having an affinity to Fc region of human IgG.  The amino
      acid residue at N-terminus may be acetylated, the amino acid
      residue at C-terminus may be aminated and two cysteine residues
      may be linked by disulfide bridge.

<400>  55

Arg Gly Asn Cys Ala Tyr His Lys Ser Gln Ile Ile Trp Cys Thr Tyr
1               5                   10                  15


His




<210>  56
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  56

Arg Gly Asn Cys Ala Tyr His Lys Asn Gln Ile Ile Trp Cys Thr Tyr
1               5                   10                  15


His




<210>  57
<211>  17
<212>  PRT
<213>  Artificial Sequence

<220>
<223>  Peptide having an affinity to Fc region of human IgG.  The amino
      acid residue at N-terminus may be acetylated, the amino acid

residue at C-terminus may be aminated and two cysteine residues
      may be linked by disulfide bridge.

<400>  57

Arg Gly Asn Cys Ala Tyr His Lys Asp Gln Ile Ile Trp Cys Thr Tyr
1               5                   10                  15

His

<210>  58
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  58

Arg Gly Asn Cys Ala Tyr His Lys Gln Gln Ile Ile Trp Cys Thr Tyr
1               5                   10                  15

His

<210>  59
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  59

Arg Gly Asn Cys Ala Tyr His Lys Glu Gln Ile Ile Trp Cys Thr Tyr
1               5                   10                  15

His

<210>  60
<211>  17
<212>  PRT
<213>  Artificial Sequence

<220>
<223>  Peptide having an affinity to Fc region of human IgG.  The amino
       acid residue at N-terminus may be acetylated, the amino acid

residue at C-terminus may be aminated and two cysteine residues
      may be linked by disulfide bridge.

<400>  60

Arg Gly Asn Cys Ala Tyr His Lys Phe Gln Ile Ile Trp Cys Thr Tyr
1               5                   10                  15

His

<210>  61
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  61

Arg Gly Asn Cys Ala Tyr His Lys Tyr Gln Ile Ile Trp Cys Thr Tyr
1               5                   10                  15

His

<210>  62
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  62

Arg Gly Asn Cys Ala Tyr His Lys Trp Gln Ile Ile Trp Cys Thr Tyr
1               5                   10                  15

His

<210>  63
<211>  17
<212>  PRT
<213>  Artificial Sequence

<220>
<223>  Peptide having an affinity to Fc region of human IgG.  The amino
       acid residue at N-terminus may be acetylated, the amino acid

residue at C-terminus may be aminated and two cysteine residues
may be linked by disulfide bridge.

<400> 63

Arg Gly Asn Cys Ala Tyr His Lys His Gln Ile Ile Trp Cys Thr Tyr
1               5                   10                  15

His

<210> 64
<211> 17
<212> PRT
<213> Artificial Sequence

<220>
<223> Peptide having an affinity to Fc region of human IgG. The amino
acid residue at N-terminus may be acetylated, the amino acid
residue at C-terminus may be aminated and two cysteine residues
may be linked by disulfide bridge.

<400> 64

Arg Gly Asn Cys Ala Tyr His Lys Thr Gln Ile Ile Trp Cys Thr Tyr
1               5                   10                  15

His

<210> 65
<211> 17
<212> PRT
<213> Artificial Sequence

<220>
<223> Peptide having an affinity to Fc region of human IgG. The amino
acid residue at N-terminus may be acetylated, the amino acid
residue at C-terminus may be aminated and two cysteine residues
may be linked by disulfide bridge.

<400> 65

Arg Gly Asn Cys Ala Tyr His Lys Leu Gln Ile Ile Trp Cys Thr Tyr
1               5                   10                  15

His

<210> 66
<211> 11
<212> PRT
<213> Artificial Sequence

<220>
<223> Peptide having an affinity to Fc region of human IgG. The amino
acid residue at N-terminus may be acetylated, the amino acid

residue at C-terminus may be aminated and two cysteine residues
      may be linked by disulfide bridge.

<400>  66

Cys Ala Tyr His Lys Leu Gln Ile Val Trp Cys
1               5                   10

<210>  67
<211>  11
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  67

Cys Ala Tyr His Lys Leu Gln Leu Ile Trp Cys
1               5                   10

<210>  68
<211>  11
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  68

Cys Ala Tyr His Lys Ser Gln Ile Val Trp Cys
1               5                   10

<210>  69
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  69

Arg Gly Asn Cys Ala Tyr His Lys Gly Gln Leu Val Phe Cys Thr Tyr
1               5                   10                  15

His

<210>  70
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  70

Arg Gly Asn Cys Ala Tyr His Lys Gly Gln Gln Val Trp Cys Thr Tyr
1               5                   10                  15

His

<210>  71
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  71

Arg Gly Asn Cys Ala Tyr His Lys Gly Gln Glu Val Trp Cys Thr Tyr
1               5                   10                  15

His

<210>  72
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  72

Cys Ala Tyr His Lys Gly Gln Leu Val Trp Cys
1               5                   10

<210>  73
<211>  17
<212>  PRT

<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  73

Arg Gly Asn Cys Ala Tyr His Lys Ala Gln Leu Val Trp Cys Thr Tyr
1               5                   10                  15

His

<210>  74
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  74

Arg Gly Asn Cys Ala Tyr His Lys Val Gln Leu Val Trp Cys Thr Tyr
1               5                   10                  15

His

<210>  75
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  75

Arg Gly Asn Cys Ala Tyr His Lys Leu Gln Leu Val Trp Cys Thr Tyr
1               5                   10                  15

His

<210>  76
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  76

Arg Gly Asn Cys Ala Tyr His Lys Ile Gln Leu Val Trp Cys Thr Tyr
1               5                   10                  15

His

<210>  77
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  77

Arg Gly Asn Cys Ala Tyr His Lys Ser Gln Leu Val Trp Cys Thr Tyr
1               5                   10                  15

His

<210>  78
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  78

Arg Gly Asn Cys Ala Tyr His Lys Thr Gln Leu Val Trp Cys Thr Tyr
1               5                   10                  15

His

<210>  79
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  79

Arg Gly Asn Cys Ala Tyr His Lys Asn Gln Leu Val Trp Cys Thr Tyr
1               5                   10                  15

His

<210>  80
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  80

Arg Gly Asn Cys Ala Tyr His Lys Asp Gln Leu Val Trp Cys Thr Tyr
1               5                   10                  15

His

<210>  81
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  81

Arg Gly Asn Cys Ala Tyr His Lys Gln Gln Leu Val Trp Cys Thr Tyr
1               5                   10                  15

His

<210>  82
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  82

Arg Gly Asn Cys Ala Tyr His Lys Glu Gln Leu Val Trp Cys Thr Tyr
1               5                   10                  15

His

<210>  83
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  83

Arg Gly Asn Cys Ala Tyr His Lys Phe Gln Leu Val Trp Cys Thr Tyr
1               5                   10                  15

His

<210>  84
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  84

Arg Gly Asn Cys Ala Tyr His Lys Arg Gln Leu Val Trp Cys Thr Tyr
1               5                   10                  15

His

<210>  85
<211>  17
<212>  PRT

<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  85

Arg Gly Asn Cys Ala Tyr His Lys His Gln Leu Val Trp Cys Thr Tyr
1               5                   10                  15

His

<210>  86
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  86

Arg Gly Asn Cys Ala Tyr His Lys Trp Gln Leu Val Trp Cys Thr Tyr
1               5                   10                  15

His

<210>  87
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  87

Arg Gly Asn Cys Ala Tyr His Lys Tyr Gln Leu Val Trp Cys Thr Tyr
1               5                   10                  15

His

<210>  88
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  88

Arg Gly Asn Cys Ala Tyr Phe Lys Gly Gln Leu Val Trp Cys Thr Tyr
1               5                   10                  15

His

<210>  89
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  89

Arg Gly Asn Cys Ala Tyr Tyr Lys Gly Gln Leu Val Trp Cys Thr Tyr
1               5                   10                  15

His

<210>  90
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  90

Arg Gly Asn Cys Ala Tyr Trp Lys Gly Gln Leu Val Trp Cys Thr Tyr
1               5                   10                  15

His

<210>  91
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  91

Arg Gly Asn Cys Ala Tyr Arg Lys Gly Gln Leu Val Trp Cys Thr Tyr
1               5                   10                  15

His

<210>  92
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  92

Arg Gly Asn Cys Ala Tyr Gly Lys Gly Gln Leu Val Trp Cys Thr Tyr
1               5                   10                  15

His

<210>  93
<211>  12
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  93

Asp Cys Ala Tyr His Lys Gly Gln Leu Val Trp Cys
1               5                   10

<210>  94
<211>  12
<212>  PRT
<213>  Artificial Sequence

<220>
<223>  Peptide having an affinity to Fc region of human IgG.  The amino

acid residue at N-terminus may be acetylated, the amino acid
      residue at C-terminus may be aminated and two cysteine residues
      may be linked by disulfide bridge.

<400>  94

Asn Cys Ala Tyr His Lys Gly Gln Leu Val Trp Cys
1               5                   10


<210>  95
<211>  12
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  95

Cys Ala Tyr His Lys Gly Gln Leu Val Trp Cys Thr
1               5                   10


<210>  96
<211>  11
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  96

Cys Ala Tyr His Lys Ser Gln Leu Val Trp Cys
1               5                   10


<210>  97
<211>  28
<212>  PRT
<213>  Artificial Sequence

<220>
<223>  Peptide having an affinity to Fc region of human IgG.  The amino
      acid residue at N-terminus may be acetylated, and the amino acid
      residue at C-terminus may be aminated.

<400>  97

Asn Met Gln Gln Gln Arg Arg Phe Tyr Glu Ala Leu His Asp Pro Asn
1               5                   10                  15


Leu Glu Glu Gln Arg Asn Ala Arg Ile Arg Ser Ile
            20                  25

<210>  98
<211>  33
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  98

Phe Asn Met Gln Gln Gln Arg Arg Phe Tyr Glu Ala Leu His Asp Pro
1               5                   10                  15

Asn Leu Asn Lys Glu Gln Arg Asn Ala Arg Ile Arg Ser Ile Arg Asp
            20                  25                  30

Asp

<210>  99
<211>  16
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  99

Gly Asn Cys Ala Tyr His Lys Gly Gln Ile Ile Trp Cys Thr Tyr His
1               5                   10                  15

<210>  100
<211>  31
<212>  PRT
<213>  Artificial Sequence

<220>
<223>  Peptide having an affinity to Fc region of human IgG.  The amino
       acid residue at N-terminus may be acetylated, and the amino acid
       residue at C-terminus may be aminated.

<400>  100

Asn Met Gln Gln Gln Arg Arg Phe Tyr Glu Ala Leu His Asp Pro Asn
1               5                   10                  15

Leu Glu Glu Gln Arg Asn Ala Arg Ile Arg Ser Ile Lys Asp Asp
            20                  25                  30

<210>  101

<211>  19
<212>  PRT
<213>  Artificial Sequence

<220>
<223>  Peptice fragment containing lysin residue at position 317 in
       human IgG

<400>  101

Val Val Ser Val Leu Thr Val Leu His Gln Asp Trp Leu Asn Gly Lys
1               5                   10                  15

Glu Tyr Lys

<210>  102
<211>  33
<212>  PRT
<213>  Artificial Sequence

<220>
<223>  Peptice fragment containing lysin residue at position 247or 249
       in denosumab

<400>  102

Cys Cys Val Glu Cys Pro Pro Cys Pro Ala Pro Pro Val Ala Gly Pro
1               5                   10                  15

Ser Val Phe Leu Phe Pro Pro Lys Pro Lys Asp Thr Leu Met Ile Ser
            20                  25                  30

Arg

<210>  103
<211>  34
<212>  PRT
<213>  Artificial Sequence

<220>
<223>  Peptice fragment containing lysin residue at position 251or 253
       in dupixent

<400>  103

Tyr Gly Pro Pro Cys Pro Pro Cys Pro Ala Pro Glu Phe Leu Gly Gly
1               5                   10                  15

Pro Ser Val Phe Leu Phe Pro Pro Lys Pro Lys Asp Thr Leu Met Ile
            20                  25                  30

Ser Arg

<210>  104
<211>  447
<212>  PRT
<213>  Artificial Sequence

<220>
<223>  Amino acid sequence of heavy chain of denosumab

<400>  104

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Gly Ile Thr Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Pro Gly Thr Thr Val Ile Met Ser Trp Phe Asp Pro Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Asn Phe Gly Thr Gln Thr Tyr Thr Cys Asn Val Asp
        195                 200                 205

His Lys Pro Ser Asn Thr Lys Val Asp Lys Thr Val Glu Arg Lys Cys
    210                 215                 220

Cys Val Glu Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Phe Arg Val Val
    290                 295                 300

Ser Val Leu Thr Val Val His Gln Asp Trp Leu Asn Gly Lys Glu Tyr
305                 310                 315                 320

Lys Cys Lys Val Ser Asn Lys Gly Leu Pro Ala Pro Ile Glu Lys Thr
                325                 330                 335

Ile Ser Lys Thr Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Met Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445

<210>  105
<211>  215
<212>  PRT

<213>  Artificial Sequence

<220>
<223>  Amino acid sequence of light chain of denosumab

<400>  105

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Arg Gly Arg
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Phe Tyr Cys Gln Gln Tyr Gly Ser Ser Pro
                85                  90                  95

Arg Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 106
<211> 452
<212> PRT
<213> Artificial Sequence

<220>
<223> Amino acid sequence of heavy chain of dupixent

<400> 106

Glu Val Gln Leu Val Glu Ser Gly Gly Gly Leu Glu Gln Pro Gly Gly
1               5                   10                  15

Ser Leu Arg Leu Ser Cys Ala Gly Ser Gly Phe Thr Phe Arg Asp Tyr
            20                  25                  30

Ala Met Thr Trp Val Arg Gln Ala Pro Gly Lys Gly Leu Glu Trp Val
        35                  40                  45

Ser Ser Ile Ser Gly Ser Gly Gly Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Leu Ser Ile Thr Ile Arg Pro Arg Tyr Tyr Gly Leu
            100                 105                 110

Asp Val Trp Gly Gln Gly Thr Thr Val Thr Val Ser Ser Ala Ser Thr
        115                 120                 125

Lys Gly Pro Ser Val Phe Pro Leu Ala Pro Cys Ser Arg Ser Thr Ser
    130                 135                 140

Glu Ser Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Lys Thr Tyr Thr Cys
        195                 200                 205

Asn Val Asp His Lys Pro Ser Asn Thr Lys Val Asp Lys Arg Val Glu

210                 215                 220

Ser Lys Tyr Gly Pro Pro Cys Pro Pro Cys Pro Ala Pro Glu Phe Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

Gln Glu Asp Pro Glu Val Gln Phe Asn Trp Tyr Val Asp Gly Val Glu
        275                 280                 285

Val His Asn Ala Lys Thr Lys Pro Arg Glu Glu Gln Phe Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gly Leu Pro Ser Ser
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Gln Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Arg Leu Thr
                405                 410                 415

Val Asp Lys Ser Arg Trp Gln Glu Gly Asn Val Phe Ser Cys Ser Val
            420                 425                 430

Met His Glu Ala Leu His Asn His Tyr Thr Gln Lys Ser Leu Ser Leu
        435                 440                 445

Ser Leu Gly Lys
    450

<210>  107
<211>  219
<212>  PRT
<213>  Artificial Sequence

<220>
<223>  Amino acid sequence of light chain of dupixent

<400>  107

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu Tyr Ser
            20                  25                  30

Ile Gly Tyr Asn Tyr Leu Asp Trp Tyr Leu Gln Lys Ser Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Phe Tyr Tyr Cys Met Gln Ala
                85                  90                  95

Leu Gln Thr Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys

210                 215

<210>  108
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  108

Arg Gly Asn Cys Ala Tyr His Glu Gly Gln Ile Ile Trp Cys Thr Tyr
1               5                   10                  15

His

## Claims

1. A compound having an affinity substance to an antibody and a bioorthogonal functional group, represented by the following Formula (I):
A-L-E-B (I)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group, or
a salt thereof.

2. The compound or salt thereof according to claim 1, wherein the affinity substance is a peptide.

3. The compound or salt thereof according to claim 2, wherein the peptide is a peptide having ability to bind to a constant region of a monoclonal antibody.

4. The compound or salt thereof according to claim 2 or 3, wherein the peptide is a peptide having ability to bind to an Fc region of a monoclonal antibody.

5. The compound or salt thereof according to claim 4, wherein the peptide is a peptide having ability to bind to an Fc region of IgG.

6. The compound or salt thereof according to any one of claims 2 to 5, wherein the peptide has 10 to 40 amino acid residues.

7. The compound or salt thereof according to any one of claims 2 to 6, wherein the peptide comprises
(a) (a-1-1) the amino acid sequence of FNMQQQRRFYEALHDPNLNEEQRNARIRSIRDD (SEQ ID NO: 11) or
(a-1-2) the amino acid sequence of FNMQCQRRFYEALHDPNLNEEQRNARIRSIRDDC (SEQ ID NO: 12),
wherein any one to three amino acid residues in the sequence, which may be the same or different, are each substituted with one amino acid residue selected from the group consisting of a lysine residue, an aspartic acid residue, and a glutamic acid residue, or
(a-2-1) the amino acid sequence of β-Ala-NMQQQRRFYEALHDPNLNEEQRNARIRSIRDD (SEQ ID NO: 13) or
(a-2-2) the amino acid sequence of β-Ala-NMQCQRRFYEALHDPNLNEEQRNARIRSIRDDC (SEQ ID NO: 14),
wherein any one to three amino acid residues in the sequence, which may be the same or different, are each substituted with one amino acid residue selected from the group consisting of a lysine residue, an aspartic acid residue, and a glutamic acid residue, and
(b) an amino acid sequence having 85% or more identity to each of the amino acid sequences of SEQ ID NOs: 11 to 14.

8. The compound or salt thereof according to any one of claims 2 to 6, wherein
the peptide comprises any of amino acid sequences of
Formula 1-1: (X₀₋₃)ₐ-C-Xaa1-Xaa2-Xaa3-Xaa4-Xaa5-Xaa6-I-I-W-C-(X₀₋₃)_{b} (SEQ ID NO: 15),
Formula 1-2: (X₀₋₃)ₐ-C-Xaa1-Xaa2-Xaa3-Xaa4-Xaa5-Xaa6-I-V-W- C-(X₀₋₃)_{b} (SEQ ID NO: 16),
Formula 1-3: (X₀₋₃)ₐ-C-Xaa1-Xaa2-Xaa3-Xaa4-Xaa5-Xaa6-V-V-W-C-(X₀₋₃)_{b} (SEQ ID NO: 17),
Formula 1-4: (X₀₋₃)ₐ-C-Xaa1-Xaa2-Xaa3-Xaa4-Xaa5-Xaa6-A-V-W-C-(X₀₋₃)_{b} (SEQ ID NO: 18),
Formula 1-5: (X₀₋₃)ₐ-C-Xaa1-Xaa2-Xaa3-Xaa4-Xaa5-Xaa6-L-L-W-C-(X₀₋₃)_{b} (SEQ ID NO: 19),
Formula 1-6: (X₀₋₃)ₐ-C-Xaa1-Xaa2-Xaa3-Xaa4-Xaa5-Xaa6-L-I-W-C-(X₀₋₃)_{b} (SEQ ID NO: 20),
Formula 1-7: (X₀₋₃)ₐ-C-Xaa1-Xaa2-Xaa3-Xaa4-Xaa5-Xaa6-L-V-F-C-(X₀₋₃)_{b} (SEQ ID NO: 21),
Formula 1-8 (X₀₋₃)ₐ-C-Xaa1-Xaa2-Xaa3-Xaa4-Xaa5-Xaa6-Q-V-W-C-(X₀₋₃)_{b} (SEQ ID NO: 22),
Formula 1-9: (X₀₋₃)ₐ-C-Xaa1-Xaa2-Xaa3-Xaa4-Xaa5-Xaa6-E-V-W-C-(X₀₋₃)_{b} (SEQ ID NO: 23)
wherein
(X₀₋₃)ₐ is absence, or an arginine residue-glycine residue-asparagine residue, an aspartic acid residue, a glycine residue-asparagine residue, or an asparagine residue,
(X₀₋₃)_{b} is absence, or a threonine residue-tyrosine residue-histidine residue, or a threonine residue,
Xaa1 is an alanine residue,
Xaa2 is a tyrosine residue, a tryptophan residue, or a histidine residue,
Xaa3 is a histidine residue, a phenylalanine residue, a tyrosine residue, a tryptophan residue, an arginine residue, or a glycine residue,
Xaa4 is a lysine residue, an aspartic acid residue, or a glutamic acid residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue,
Xaa6 is a glutamine residue, a glutamic acid residue, an asparagine residue, or an aspartic acid residue, and Formula 2-1: (X₀₋₃')ₐ-C-(Xaa1')-(Xaa2')-(Xaa3')-(Xaa4')-(Xaa5')-(Xaa6')-L-V-W-C-(X₀₋₃')_{b} (SEQ ID NO: 24)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively.

9. The compound or salt thereof according to any one of claims 1 to 8, wherein the leaving group is (1) a group selected from the group consisting of -O-, -S-, -Se-, -SO₂-O-,- SO₂-N(R)-, -SO₂-, -C≡C-CH₂-O-, -N(OR)-, -N(R)-, and -ON(R)- where R is a hydrogen atom or C₁₋₆ alkyl, or (2) heteroarylene.

10. The compound or salt thereof according to any one of claims 1 to 9, wherein the nucleophilic group is selected from the group consisting of NH₂ in a side chain of a lysine residue, OH in a side chain of a tyrosine residue, OH in a side chain of a serine residue, OH in a side chain of a threonine residue, and SH in a side chain of a cysteine residue.

11. The compound or salt thereof according to any one of claims 1 to 10, wherein the electrophilic group is selected from the group consisting of -C(=O)-, -SO₂-, and -CH₂-.

12. The compound or salt thereof according to any one of claims 1 to 11, wherein the bioorthogonal functional group is selected from the group consisting of an azide residue, an aldehyde residue, a thiol residue, an alkyne residue, an alkene residue, a halogen residue, a tetrazine residue, a nitron residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue.

13. The compound or salt thereof according to any one of claims 1 to 12, wherein the bioorthogonal functional group is selected from the group consisting of an azide residue, a thiol residue, an alkyne residue, a maleimide residue, and a disulfide residue.

14. The compound or salt thereof according to any one of claims 1 to 13, wherein the compound represented by the above Formula (I) is a compound represented by Formula (I-1) below:
A-L₁-L₂-E₁-E₂-E₃-B (I-1)
wherein
A and B are the same as those in the above Formula (I),
L₁ is a bond or a divalent group,
L₂ is a leaving group,
E₁ is an electrophilic group (i) coupled with the leaving group and (ii) having ability to react with a nucleophilic group in the antibody,
E₂ is a group represented by (a) -X-Y-
wherein
X which binds to E₁ is C(R₁) (R₂) where R₁ and R₂ are each independently a hydrogen atom or C₁₋₆ alkyl, N(R₃) where R₃ is a hydrogen atom or C₁₋₆ alkyl, O, S, or Se, and
Y which binds to E₃ is C(R₄) (R₅) where R₄ and R₅ are each independently a hydrogen atom or C₁₋₆ alkyl, or
E₂ is a group represented by (b) the following formula (i) : 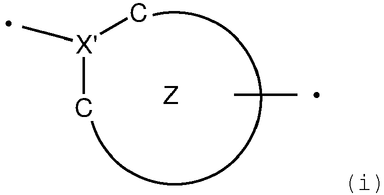
where ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and • is a bond,
when E₂ is -X-Y-, E₃ is a divalent group, and when E₂ is a group represented by Formula (i), E₃ is a bond or a divalent group, and
the leaving group has ability to be cleaved and eliminated from E₁ by a reaction between the nucleophilic group and the electrophilic group.

15. The compound or salt thereof according to claim 14, wherein the L₂ is
(a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N (R) -, -SO₂-, -C≡C-CH₂-O-, -N(OR)-, -N(R)-, and -O-N(R)- where R is a hydrogen atom or C₁₋₆ alkyl,
(b) heteroarylene, or
(c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- where R is a hydrogen atom or C₁₋₆ alkyl.

16. The compound or salt thereof according to claim 14 or 15, wherein the L₂ is a group selected from the group consisting of the following structural formulae: 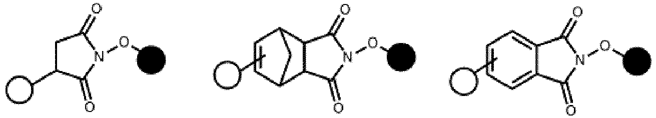 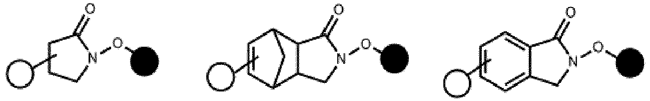 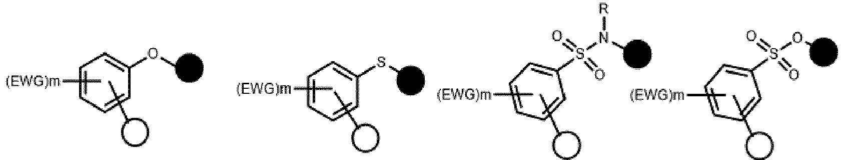 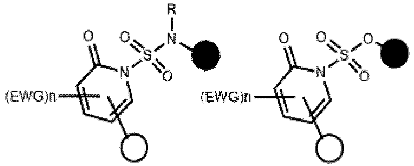 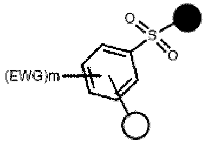 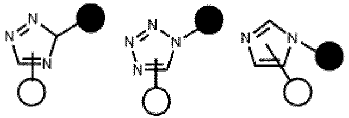 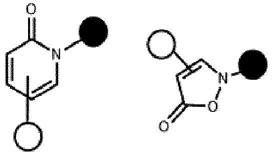 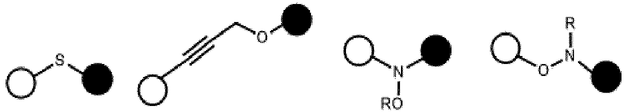
where EWG is an electron-withdrawing group,
m is an integer of 0 to 4,
n is an integer of 0 to 3,
R is a hydrogen atom or C₁₋₆ alkyl,
a symbol of "white circle" is a bond to L₁, and a symbol of "black circle" is a bond to E₁.

17. The compound or salt thereof according to any one of claims 1 to 16, wherein a main chain of L or L₁-L₂ coupling A with E consists of 20 or less atoms.

18. The compound or salt thereof according to any one of claims 14 to 17, wherein the compound represented by the above Formula (I-1) is a compound represented by the following Formula (1-2):
A-L₁-L₂-E₁-X-Y-E₃-B (1-2)
wherein
A, L₁, X, Y, and B are the same as those in the above Formula (I-1),
L₂ is
(a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- where R is a hydrogen atom or C₁₋₆ alkyl,
(b) heteroarylene, or
(c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- where R is a hydrogen atom or C₁₋₆ alkyl,
E₁ is selected from the group consisting of -C(=O)-, - SO₂-, and -CH₂-, and
E₃ is a divalent group.

19. The compound or salt thereof according to any one of claims 14 to 17, wherein the compound represented by the above Formula (I-1) is a compound represented by the following Formula (1-3): 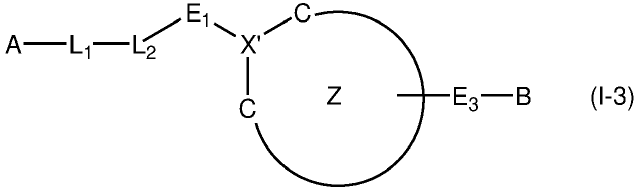 wherein
A, L₁, ring Z, and B are the same as those in the above Formula (I-1),
L₂ is
(a) ring P-Q- wherein ring P is selected from the group consisting of an arylene optionally substituted with an electron-withdrawing group, a heteroarylene optionally substituted with an electron-withdrawing group, optionally condensed 2,5-diketopyrrolidine, optionally condensed 2,6-diketopiperidine, optionally condensed 2-ketopyrrolidine, optionally condensed 2-ketopiperidine, and 2-pyridone, and Q is selected from the group consisting of -O-, -S-, -Se-, -SO₂-O-, -SO₂-N(R)-, -SO₂-, -C≡C-CH₂-O-, -N(OR)-, -N(R)-, and -O-N(R)- where R is a hydrogen atom or C₁₋₆ alkyl,
(b) heteroarylene, or
(c) -Q- wherein Q is selected from the group consisting of -O-, -S-, -Se-, -SO₂-O-, -SO₂-N(R)-, -SO₂-, - C≡C-CH₂-O-, -N(OR)-, -N(R)-, and -O-N(R)- (where R is a hydrogen atom or C₁₋₆ alkyl,
E₁ is selected from the group consisting of -C(=O)-, - SO₂-, and -CH₂-, and
E₃ is a bond or a divalent group.

20. A reagent of regioselectively modifying an antibody by a bioorthogonal functional group, the reagent comprising
a compound having an affinity substance to an antibody and a bioorthogonal functional group, represented by the following Formula (I):
A-L-E-B (I)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group, or
a salt thereof.

21. A method for producing an antibody having a bioorthogonal functional group or bioorthogonal functional groups or a salt thereof, the method comprising:
reacting a compound having an affinity substance to an antibody and a bioorthogonal functional group, represented by the following Formula (I):
A-L-E-B (I)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group, or a salt thereof with an antibody
to form an antibody having a bioorthogonal functional group or bioorthogonal functional groups, represented by the following Formula (II):
Ab-E-B (II)
wherein
E and B are the same as those in the above Formula (I), and
Ab is an antibody, or
a salt thereof.

22. A method for producing an antibody having a functional substance or functional substances or a salt thereof, the method comprising:
(1) reacting a compound having an affinity substance to an antibody and a bioorthogonal functional group, represented by the following Formula (I):
A-L-E-B (I)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
B is a bioorthogonal functional group, and
the leaving group has ability to be cleaved and eliminated from E by a reaction between the nucleophilic group and the electrophilic group, or a salt thereof with an antibody
to form an antibody having a bioorthogonal functional group or bioorthogonal functional groups, represented by the following Formula (II):
Ab-E-B (II)
wherein
E and B are the same as those in the above Formula (I), and
Ab is an antibody, or
a salt thereof; and
(2) reacting the antibody having a bioorthogonal functional group or bioorthogonal functional groups, represented by the above Formula (II), or a salt thereof with a functional substance via the bioorthogonal functional group
to form the antibody having a functional substance or functional substances, represented by the following Formula (III) :
Ab-E-B'-F (III)
wherein Ab is the same as that in Formula (II),
E is the same as that in Formula (I),
B' is a divalent group comprising a portion formed by a reaction between the functional substance and the bioorthogonal functional group, and
F is a functional substance, or a salt thereof.

23. An antibody regioselectively having a bioorthogonal functional group or bioorthogonal functional groups, represented by the following Formula (II-1):
Ab-E₁-E₂-E₃-B (II-1)
wherein
Ab is an antibody,
E₁ is an electrophilic group coupled with a nucleophilic group in the antibody,
E₂ is a group represented by (a) -X-Y-
wherein X which binds to E₁ is C(R₁) (R₂) where R₁ and R₂ are each independently a hydrogen atom or C₁₋₆ alkyl, N(R₃) where R₃ is a hydrogen atom or C₁₋₆ alkyl, O, S, or Se, and
Y which binds to E₃ is C(R₄) (R₅) where R₄ and R₅ are each independently a hydrogen atom or C₁₋₆ alkyl, or
E₂ is a group represented by (b) the following formula (i) : 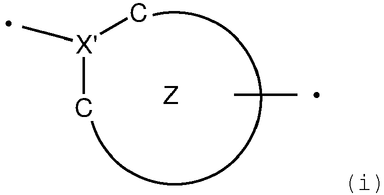
wherein ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and • is a bond,
when E₂ is -X-Y-, E₃ is a divalent group, and when E₂ is a group represented by Formula (i), E₃ is a bond or a divalent group, and
B is a bioorthogonal functional group, or
a salt thereof.

24. The antibody or salt thereof according to claim 23, wherein the antibody is an antibody having a bioorthogonal functional group or bioorthogonal functional groups only in a constant region of a monoclonal antibody.

25. The antibody or salt thereof according to claim 23 or 24, wherein the antibody is an antibody having a bioorthogonal functional group or bioorthogonal functional groups only in an Fc region of a monoclonal antibody.

26. The antibody or salt thereof according to any one of claims 23 to 25, wherein the antibody is a human IgG regioselectively having a bioorthogonal functional group or bioorthogonal functional groups in a region consisting of amino acid residues at positions 246 to 248 or 288 to 290 in a human IgG Fc region.

27. The antibody or salt thereof according to any one of claims 23 to 26, wherein the antibody represented by the above Formula (II-1) is an antibody regioselectively having a bioorthogonal functional group or bioorthogonal functional groups, represented by the following formula (II-2) :
Ab-E₁-X-Y-E₃-B (11-2)
wherein
Ab, X, Y, and B are the same as those in the above Formula (II-1),
E₁ is selected from the group consisting of -C(=O)-, - SO₂-, and -CH₂-, and
E₃ is a divalent group.

28. The antibody or salt thereof according to any one of claims 23 to 26, wherein the antibody represented by the above Formula (II-1) is an antibody regioselectively having a bioorthogonal functional group or bioorthogonal functional groups, represented by the following formula (II-3) : 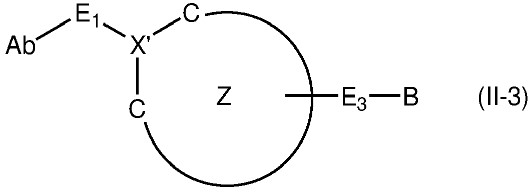 wherein
Ab, ring-constituting atom X', ring Z, and B are the same as those in the above Formula (II-1),
E₁ is selected from the group consisting of -C(=O)-, - SO₂-, and -CH₂-, and
E₃ is a bond or a divalent group.

29. An antibody regioselectively having a functional substance or functional substances, represented by the following Formula (III-1):
Ab-E₁-E₂-E₃-B'-F (III-1)
wherein
Ab is an antibody,
E₁ is an electrophilic group coupled with a nucleophilic group in the antibody,
E₂ is a group represented by (a) -X-Y-
wherein X which binds to E₁ is C(R₁) (R₂) where R₁ and R₂ are each independently a hydrogen atom or C₁₋₆ alkyl, N(R₃) where R₃ is a hydrogen atom or C₁₋₆ alkyl, O, S, or Se, and Y which binds to E₃ is C(R₄) (R₅) where R₄ and R₅ are each independently a hydrogen atom or C₁₋₆ alkyl, or E₂ is a group represented by (b) the following formula (i): 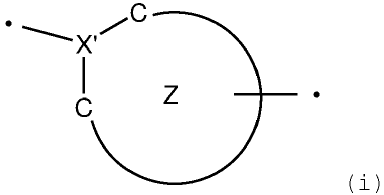
wherein ring Z is a divalent cyclic group in which all of a ring-constituting atom X' which binds to E₁ and ring-constituting atoms on both sides thereof are carbon atoms, or a divalent heterocyclic group in which the ring-constituting atom X' which binds to E₁ is a nitrogen atom, and ring-constituting atoms on both sides of the nitrogen atom are carbon atoms, and • is a bond,
E₃ is a divalent group when E₂ is -X-Y-, and is a bond or a divalent group when E₂ is a group represented by Formula (i),
B' is a divalent group comprising a portion formed by a reaction between the functional substance and the bioorthogonal functional group, and
F is a functional substance, or
a salt thereof.

30. The antibody or salt thereof according to claim 29, wherein the antibody is an antibody having a bioorthogonal functional group or bioorthogonal functional groups only in a constant region of a monoclonal antibody.

31. The antibody or salt thereof according to claim 29 or 30, wherein the antibody is an antibody having a bioorthogonal functional group or bioorthogonal functional groups only in an Fc region of a monoclonal antibody.

32. The antibody or salt thereof according to any one of claims 29 to 31, wherein the antibody is a human IgG regioselectively having a functional substance or functional substances in a region consisting of amino acid residues at positions 246 to 248 or 288 to 290 in a human IgG Fc region.

33. The antibody or salt thereof according to any one of claims 29 to 32, wherein the antibody represented by the above Formula (III-1) is an antibody regioselectively having a functional substance or functional substances, represented by the following formula (111-2):
Ab-E₁-X-Y-E₃-B'-F (III-2)
wherein
Ab, X, Y, B', and F are the same as those in the above Formula (III-1),
E₁ is selected from the group consisting of -C(=O)-, - SO₂-, and -CH₂-, and
E₃ is a divalent group.

34. The antibody or salt thereof according to any one of claims 29 to 32, wherein the antibody represented by the above Formula (III-1) is an antibody regioselectively having a functional substance or functional substances, represented by the following formula (111-3): 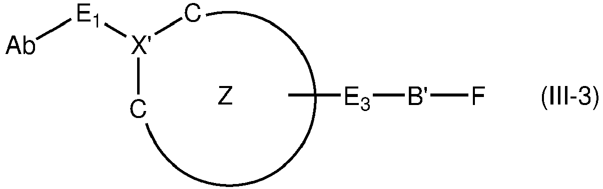 wherein
Ab, ring-constituting atom X', ring Z, B', and F are the same as those in the above Formula (III-1),
E₁ is selected from the group consisting of -C(=O)-, - SO₂-, and -CH₂-, and
E₃ is a bond or a divalent group.

35. A compound having an affinity substance to an antibody and a functional substance, represented by the following Formula (IV):
A-L-E-F (IV)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
F is a functional substance, and
the leaving group has ability to be cleaved and eliminated from E by a reaction between the nucleophilic group and the electrophilic group, or
a salt thereof.

36. A reagent of regioselectively modifying an antibody by a functional substance, represented by the following Formula (IV):
A-L-E-F (IV)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
F is a functional substance, and
the leaving group has ability to be cleaved and eliminated from E by a reaction between the nucleophilic group and the electrophilic group, or
a salt thereof.

37. A method for producing an antibody having a functional substance or functional substances or a salt thereof, the method comprising
reacting a compound having an affinity substance to an antibody and a functional substance, represented by the following Formula (IV):
A-L-E-F (IV)
wherein
A is an affinity substance to an antibody,
L is a divalent group comprising a leaving group,
E is a divalent group comprising an electrophilic group (i) coupled with the leaving group and (ii) having ability to react with a nucleophilic group in the antibody,
F is a functional substance, and
the leaving group has ability to be cleaved and eliminated from E by a reaction between the nucleophilic group and the electrophilic group, or a salt thereof with an antibody
to form the antibody having a functional substance or functional substances, represented by the following Formula (III) :
Ab-E-F (III)
wherein
Ab is an antibody, and
E and F are the same as those in the above Formula (IV), or a salt thereof.
